# EUROPÄISCHE PATENTANMELDUNG

(11) **EP 2 343 303 A1**
(43) Veröffentlichungstag der Anmeldung: **13.07.2011**
(21) Anmeldenummer: 11158787.9
(22) Anmeldetag: 05.10.2005
(51) Int. Cl.: C07D 513/04, C07D 277/84, A61K 31/428, A61K 31/429, A61P 35/00

(54) **PI3-Kinase Inhibitoren**

(30) Priorität: 07.10.2004 DE 102004048877; 09.02.2005 DE 102005005813
(62) Teilanmeldung aus: 05805652.4
(71) Anmelder: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: Breitfelder, Steffen, 88448 Attenweiler (DE); Maier, Udo, 89250 Senden (DE); Brandl, Trixi, 4123 Allschwil (CH); Hoenke, Christoph, 55218 Ingelheim (DE); Grauert, Matthias, 88400 Biberach (DE); Pautsch, Alexander, 89075 Ulm (DE); Hoffmann, Matthias, 88441 Mittelbiberach (DE); Kalkbrenner, Frank, 88448 Attenweiler (DE); Joergensen, Anne, 2450 København SV (DK); Schaenzle, Gerhard, 88400 Biberach (DE); Peters, Stefan, 88400 Biberach (DE); Buettner, Frank, 88448, Attenweiler (DE); Bauer, Eckhart, 88400 Biberach (DE)
(74) Vertreter: Hammann, Heinz

(57) **Zusammenfassung**

Es werden neue Verbindungen der Formel 1 bereitgestellt, die aufgrund ihrer pharmazeutischen Wirksamkeit als PI3-Kinase Modulator zur Anwendung auf therapeutischem Gebiet zur Behandlung von entzündlichen oder allergischen Erkrankungen gelangen können.

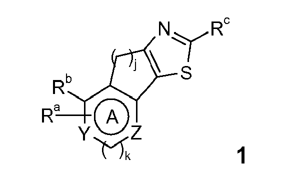

Beispielhaft seien hier entzündliche und allergische Atemwegserkrankungen, entzündliche Erkrankungen des Magen-Darm-Traktes und des Bewegungsapparates, entzündliche und allergische Hauterkrankungen, entzündliche Augenerkrankungen, Erkrankungen der Nasenschleimhaut, entzündliche oder allergische Krankheitszustände, bei denen Autoimmun-Reaktionen beteiligt sind oder Nierenentzündungen genannt.

## Beschreibung

### TECHNISCHES GEBIET

Phosphatidylinositol-3-Kinasen (PI3-Kinasen) sind ein Subfamilie der Lipidkinasen, die die Übertragung eines Phosphatrestes auf die 3'-Position des Inositolrings von Phosphoinositiden katalysieren (Vanhaesebroeck and Waterfield, Exp Cell Res. 1999 Nov 25;253(1):239-54).

Sie spielen eine wichtige Rolle bei zahlreichen zellulären Vorgängen wie z.B. Zellwachstums- und Differenzierungsvorgängen, der Steuerung von cytoskelettalen Veränderungen und der Regulation intrazellulärer Transportvorgänge (Vanhaesebroeck et al., Annu Rev Biochem. 2001; 70:535-602). Aufgrund ihrer *in vitro*-Spezifität für bestimmte Phosphoinositid-Substrate können die PI3-Kinasen in verschiedene Klassen eingeteilt werden. Die Mitglieder der Rezeptor-regulierten Klasse I sind heterodimere Enzyme, die aus einer 110-120 kDa schweren katalytischen (p110) und einer 50-101 kDa schweren nicht-katalytischen Untereinheit (p50, p55, p85, p101) aufgebaut sind. Die am höchsten konservierte Region bei allen PI3-Kinasen ist die C-terminal gelegene Kinase-Domäne. Sie weist Strukturmerkmale auf, die ebenfalls in den meisten bekannten Proteinkinasen zu finden sind. Dazu gehören z.B. auch hochkonservierte Aminosäuren, die für die Koordination des ATP-Moleküls verantwortlich sind (Walker et al., Nature. 1999 Nov 18; 402(6759):313-20).

Drei der vier Mitglieder der Klasse I PI3-Kinasen assoziieren konstitutiv mit einer 50-85 kDa schweren Adaptor-Untereinheit, von denen p85 der Prototyp ist. Die Interaktion erfolgt dabei über die sog. p85-Bindungsdomäne, die auf den katalytischen Untereinheiten der PI3-Kinase α, β und δ zu finden ist. Die drei Formen werden aufgrund dieses strukturellen Merkmals zu Klasse IA zusammengefasst. Die katalytische Untereinheit der PI3-Kinase γ, p110γ, assoziiert stattdessen mit einem 101 kDa schweren regulatorischen Protein, das als p101 bezeichnet wird. Sie bildet - bis jetzt als einziges Mitglied - die Klasse IB der PI3-Kinasen.Diese strukturelle Einteilung in Klasse IA und IB zeigt auch Parallelen in den funktionellen Eigenschaften der entsprechenden PI3-Kinase-Isoformen (Vanhaesebroeck and Waterfield, Exp Cell Res. 1999 Nov 25;253(1):239-54)

So werden die PI3-Kinase α, β und δ hauptsächlich durch Rezeptor-Tyrosin-Kinasen (RTKs) oder lösliche Tyrosinkinasen aktiviert. Die p85-Untereinheit dient hierbei als Adaptor, da sie mit ihren SH2-Domänen die phosphorylierten Tyrosinreste spezifischer Aminosäuresequenzen (YxxM) erkennen und binden kann. Die PI3Kγ hingegen wird hauptsächlich durch Gβγ-Untereinheiten aktiviert, die aus heterotrimeren G-Proteinen nach Aktivierung von heptahelikalen Rezeptoren freigesetzt werden. Durch diese unterschiedliche Kopplung an Zelloberflächen-Rezeptoren in Kombination mit einer mehr oder weniger restriktiven Expression ergeben sich zwangsläufig sehr unterschiedliche Aufgaben und Funktionen der 4 Klasse I PI3-Kinasen im intakten Organismus (Wymann et al., Trends Pharmacol Sci. 2003 Jul;24(7):366-76).

Viele unabhängige Befunde sprechen für eine Beteiligung von Klasse IA PI3-Kinasen an unkontrollierten Zellwachstums- und Differenzierungsvorgängen. So war die erste nachgewiesene PI3-Kinase Aktivität mit der transformierenden Aktivität von viralen Onkogenen assoziiert, wie z.B. das middle T Antigen von Polyomaviren, Src Tyrosinkinasen oder aktivierten Wachstumsfaktoren (Workman, Biochem Soc Trans. 2004 Apr; 32(Pt 2):393-6). Bei vielen Tumoren, wie z.B. Brustkrebs, Ovar- oder auch Pankreaskarzinom findet man eine Überaktivität von Akt/PKB, welches direkt durch die Lipidprodukte der Klasse I PI3-Kinasen aktiviert wird und so die Signale in die Zelle weiterleitet. Darüber hinaus wurde erst kürzlich gefunden, dass das PIK3CA-Gen, das für p110α codiert, in verschiedenen Tumorarten, wie Kolon-, Mamma- oder Lungenkarzinomen eine hohe Mutationshäufigkeit aufweist, von denen einige repräsentative als aktivierende Mutationen charakterisiert werden konnten (Samuels et al., Science. 2004 Apr 23;304(5670):554).

Das jüngste Mitglied der Klasse IA PI3-Kinasen, die PI3Kδ, ist restriktiver exprimiert als die PI3Kα und β. In sog. "knock-in"-Mäusen, bei denen die katalytische Untereinheit der PI3Kδ, p110δ, durch eine inaktive Mutante ersetzt wurde, konnte gezeigt werden, das dieses PI3K-Isoenzym eine spezifische Rolle in der Signalübertragung von B- und T-Lymphozyten nach Antigen-Rezeptor-Stimulation spielt (Okkenhaug et al., Science. 2002 Aug; 297(5583):1031-4). Dies sind Mechanismen, die vor allem bei Autoimmunerkrankungen, wie z.B. Morbus Crohn oder rheumatoide Arthritis eine Rolle spielen.

Die PI3Kγ wird fast ausschließlich durch Gᵢ-koppelnde heptahelikale Rezeptoren aktiviert. So konnte in Neutrophilen von Mäusen, die keine PI3Kγ exprimieren, keine PI3, 4, 5-P₃-Bildung beobachtet werden, wenn diese mit IL-8, fMLP, LTB₄ oder C5a stimuliert wurden (Hirsch et al., Science. 2000 Feb 11; 287(5455):1049-53). Dies zeigt, dass zumindest in diesem Zelltypus die PI3Kγ die einzige PI3-Kinase-Isoform ist, die an diese heptahelikalen Rezeptoren koppelt. Darüber hinaus zeigten isolierte Neutrophile und Makrophagen aus den PI3Kγ-defizienten Mäusen eine stark verminderte chemotaktische Aktivität bzw. Sauerstoffradikalproduktion gegenüber einer ganzen Reihe von Chemokinen und Chemoattraktoren. Ebenfalls vermindert war die IgE-vermittelte Aktivierung von Mastzellen, die aus p110γ-defizienten Mäusen isoliert worden waren. Als verantwortlicher Mechanismus wird ein positiver Rückkopplungsmechnismus diskutiert, bei dem die PI3Kγ durch Gᵢ-koppelnde Adenosin A₃-Rezeptoren aktiviert wird (Laffargue et al., Immunity. 2002 Mar;16(3):441-51).

Trotz dieser verminderten Fähigkeit auf Entzündungsmediatoren zu reagieren, sind die p110γ-defizienten Mäuse normal lebens- und fortpflanzungsfähig und haben die gleiche Lebenserwartung wie identisch gehaltene wildtypische Vergleichstiere. Daraus lässt sich schließen, dass die Klasse IB PI3Kγ eine zentrale Rolle bei der Aktivierung von verschiedene Entzündungszellen einnimmt, und daher Isoform-spezifische Inhibitoren eine attraktive Möglichkeit zur anti-inflammatorischen Therapie mit vergleichsweise geringen Nebenwirkungen darstellen (Ward and Finan, Curr Opin Pharmacol. 2003 Aug;3(4):426-34)
Neben ihrer Funktion in Leukozyten scheint die PI3Kγ trotz ihrer geringen Expression in Kardiomyozyten auch eine Rolle im kardiovaskulären System zu spielen. So zeigten p110γ-defiziente Mäuse eine Erhöhung der Herzmuskelkontraktilität, die vermutlich über eine Überproduktion von cAMP erklärt werden kann (Crackower et al., Cell. 2002 Sep 20; 110(6):737-49). Erst kürzlich konnte gezeigt werden, dass die PI3Kγ auch bei der Entstehung der kardialen Hypertrophie beteiligt ist. So zeigten p110γ-defiziente Mäuse in einem Isoproterenol-induzierten Herzinsuffizienz-Modell im Vergleich zu den wildtypischen Tieren eine deutlich verminderte Hypertrophie und Fibrose (Oudit et al., Circulation. 2003 Oct 28; 108(17):2147-52).

Aufgabe der vorliegenden Erfindung war es neue Verbindungen bereitzustellen, die aufgrund ihrer pharmazeutischen Wirksamkeit als PI3-Kinase Modulator zur Anwendung auf therapeutischem Gebiet zur Behandlung von entzündlichen oder allergischen Erkrankungen gelangen können. Beispielhaft seien hier entzündliche und allergische Atemwegserkrankungen, entzündliche Erkrankungen des Magen-Darm Traktes, rheumatoide Arthritis, entzündliche und allergische Hauterkrankungen, entzündliche Augenerkrankungen, Erkrankungen der Nasenschleimhaut, entzündliche oder allergische Krankheitszustände, bei denen Autoimmun-Reaktionen beteiligt sind oder Nierenentzündungen genannt.

### STAND DER TECHNIK

PI3-Kinase Inhibitoren zur Behandlung entzündlicher Krankheiten sind in der Literatur bekannt. So offenbart die WO 03/072557 5-Phenylthiazolderivate, WO 04/029055 zeigt annelierte Azolpyrimidine und WO 04/007491 Azolidinone-vinyl verknüpfte Benzolderivate. Weiterhin werden durch die beiden Schriften WO 04/052373 und WO 04/056820 Benzoxazin- in Benzoxazin-3-onderivate offenbart.

### DETAILLIERTE BESCHREIBUNG DER ERFINDUNG

Überraschenderweise wurde gefunden, dass die vorstehend genannten Aufgaben durch Verbindungen der Formel **1** gelöst werden. Dementsprechend betrifft die vorliegende Erfindung Verbindungen der Formel **1**, 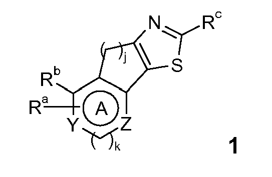 mit der Maßgabe, dass die mit A gekennzeichnete, kreisförmige Linie ein aromatisches System bedeutet; worin
- **Y**: Kohlenstoff, Stickstoffatom, Schwefel; bevorzugt Kohlenstoff, Stickstoff;
- **Z**: Kohlenstoff, Stickstoffatom, Schwefel; bevorzugt Kohlenstoff, Stickstoff;
- **j**: 1, 2 oder 3;
- **k**: 0 oder 1;
- **R^{a}**: H, COR⁸, NR⁹R¹⁰, NO₂, OR⁸, SR¹¹, SOR¹¹, SO₂R¹¹, NHCO-C₁₋₆-Alkyl-NH₂, oder ein Rest ausgewählt aus der Gruppe bestehend aus C₁₋₆-Alkyl, C₂₋₆-Alkenyl, C₃₋₈-Cycloalkyl, C₃₋₈-Cycloalkenyl, C₁₋₆-Haloalkyl, Aryl, C₇₋₁₁-Aralkyl, Spiro, Het, Heteroaryl und CH₂-O-Aryl, der gegebenenfalls substituiert sein kann;
- R⁸: C₁₋₆-Alkyl, C₃₋₆-Cycloalkyl, NH₂, Hetaryl oder Aryl, gegebenenfalls substituiert mit einem oder mehreren Halogen oder C₁-₄-Alkyl;
- R⁹: H, COOR¹², CONR¹² oder C₁₋₆-Alkyl, gegebenenfalls substituiert mit einem oder mehreren COOH, N(C₁₋₆-Alkyl)₂ oder Het, gegebenenfalls substituiert mit einem oder mehreren C₁₋₆-Alkyl; oder R⁹ bedeutet Het, gegebenenfalls substituiert mit einem oder mehreren C₁₋₄-Alkyl;
- R¹⁰: H, C₁₋₆-Alkyl, CO-C₁₋₆-Alkyl oder C₂₋₆-Alkinyl;
- R¹¹: C₁₋₆-Alkyl, gegebenenfalls substituiert mit einem oder mehreren N(C₁₋₄-Alkyl)₂;
- **R^{b}**: R⁴, OR⁴, -CH₂OR⁴, COR⁴, COOR⁴, CONR⁴R⁵, NR⁴R⁵, NR⁵COR⁴, NR⁵COOR⁴, NR⁵CONR⁴R⁵, NR⁵SOR⁴ oder NR⁵SO₂R⁴;
- R⁴, R⁵: H, C₁₋₆-Alkyl, C₁₋₆-Haloalkyl, C₁₋₆-Alkylen-OH, C₂₋₆-Alkenyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl;
oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
- **R^{c}**: NHR⁶ oder ein Rest ausgewählt aus der Gruppe bestehend aus 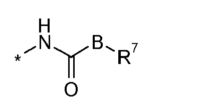 worin
B Bindung, C₁₋₆-Alkyl, C₂₋₆-Alkenyl oder C₂₋₆-Alkinyl;
R⁶ H oder ein Rest ausgewählt aus der Gruppe C₁₋₄-Alkyl, C₂₋₄-Alkenyl, C₂₋₄-Alkinyl, C₃₋₆-Cycloalkyl, C₃-₆-Clycloalkenyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
R⁷ H, C₁₋₄-Alkyl, C₂₋₄-Alkenyl, C₂₋₄-Alkinyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₆-Alkyl, (CH₂)₂₋₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}** = NHCONH-Et bedeutet.

Bevorzugt sind die oben genannten Verbindungen der Formel 1; worin
- **R^{a}**: ein Rest ausgewählt aus der Gruppe bestehend aus Aryl, C₇₋₁₁-Aralkyl und Heteroaryl, der gegebenenfalls substituiert durch einen oder mehrere Reste ausgewählt aus der Gruppe bestehend aus R¹, R² und R³ sein kann;
- **R¹** und R²: unabhängig voneinander C₁₋₆-Alkyl, C₂₋₆-Alkenyl C₂₋₆-Alkinyl, C₃-₆-Cycloalkyl, C₃₋₆-Cycloalkenyl, C₁₋₆-Haloalkyl, C₁₋₆-Alkylen-COOH, C₁₋₆-Alkoxy, Halogen, OH, CN, COR^{1.1}, O-C₁₋₄-Haloalkyl, NO₂ oder SR^{1.1}, SOR^{1.1}, SO₂R^{1.1}, Het oder Hetaryl,
R^{1.1} OH, C₁₋₆-Alkyl, C₂₋₆-Alkenyl C₂₋₆-Alkinyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 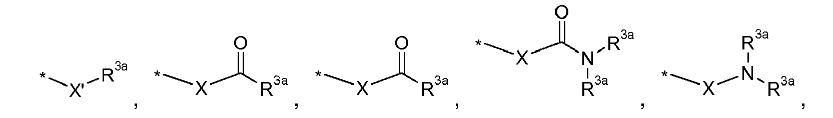 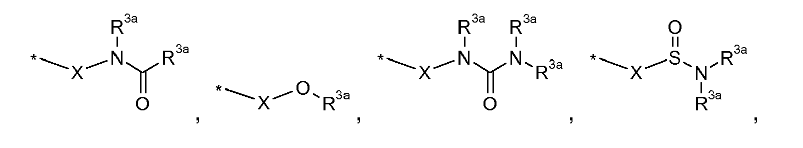 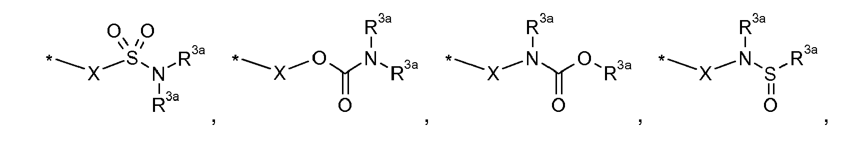 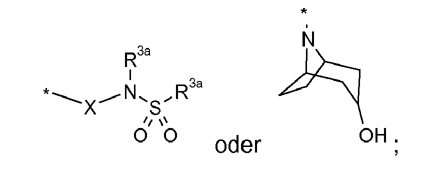 worin
X Bindung oder C₁₋₄-Alkylen;
X' C₁₋₄-Alkylen, C₂₋₄-Alkenylen oder C₁₋₄-Alkinylen
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} C₁₋₆-Alkyl, C₂₋₆-Alkenyl, OH, C₁₋₄-Alkylen-OH, C₁₋₄-Alkylen-NR^{3.1.1.1}R^{3.1.1.2}, COR^{3.1.1.1}, COOR^{3.1.1.1}, CONR^{3.1.1.1}R^{3.1.1.2}, NR^{3.1.1.1}R^{3.1.1.2}, Het, Hetaryl, NHCOR^{3.1.1.1}
R^{3.1.1.1} ein Rest ausgewählt aus der Gruppe bestehend aus H, C₁₋₄-Alkyl, Aryl und C₇₋₁₁-Aralkyl; gegebenenfalls substituiert mit einem Rest ausgewählt aus der Gruppe Halogen, OH und CN;
R^{3.1.1.2} H, C₁₋₄-Alkyl;
R^{3.2} ein Rest ausgewählt aus der Gruppe bestehend aus C₃₋₆-Cycloalkyl, Het, Hetaryl und Spiro der gegebenenfalls substituiert ist mit einem oder mehreren R^{3.2.1}
R^{3.2.1} C₁₋₆-Alkyl, C₃₋₆-Cycloalkyl, OH, NR^{3.2.1.1}R^{3.2.1.2}, NHCOR^{3.2.1.3} oder Het, gegebenenfalls substituiert mit einem oder mehreren Resten ausgewählt aus der Gruppe bestehend aus C₁₋₄-Alkyl, SO₂R^{3.2.1.1}, CH₂-C₃₋₆-Cycloalkyl und Aryl;
R^{3.2.1.1} H, C₁₋₄-Alkyl oder C₇₋₁₁- Aralkyl;
R^{3.2.1.2} H, C₁₋₄-Alkyl oder C₇₋₁₁- Aralkyl;
R^{3.2.1.2} Aryl, C₇₋₁₁-Aralkyl; oder C₁₋₆-alkyl, das gegebenenfalls substituiert ist mit einem oder zwei R^{3.2.2};
R^{3.2.2} C₁₋₆-Alkyl, C₂₋₆-Alkenyl, C₂₋₆-Alkinyl, COOR^{3.2.2.1}, CONR^{3.2.2.1}R^{3.2.2.2}, NR^{3.2.2.1}R^{3.2.2.2} NHCOR^{3.2.2.1}, C₁₋₆-Haloalkyl, CN, OR^{3.2.2.1}, SO₂R^{3.2.2.1}, C₃₋₆-Cycloalkyl, CO-Het, C₂₋₄-Alkinyl-Hetaryl, Guanidin oder ein Rest ausgewählt aus der Gruppe bestehend aus Het, Hetaryl und Aryl, der gegebenenfalls substituiert ist mit einem oder mehreren Resten ausgewählt aus der Gruppe Halogen, C₁₋₆-Alkyl, CONR^{3.2.2.1}R^{3.2.2.2}, OH, Imidazolidinon;
R^{3.2.2.1} H oder C₁₋₆-Alkyl, Aryl, C₇₋₁₁-Aralkyl
R^{3.2.2.2.} H oder C₁₋₆-Alkyl; oder Aryl, das gegebenenfalls substituiert ist mit einem oder zwei R^{3.2.3}
R^{3.2.3} ein Rest ausgewählt aus der Gruppe bestehend aus NH-C₁₋₆-alkyl-N(C₁₋₆-alkyl)₂ oder Het, wobei Het gegebenenfalls substituiert mit einem Rest C₁₋₆-Alkyl sein kann;
R^{3.3} H oder ein Rest ausgewählt aus der Gruppe bestehend aus C₁₋₆-Alkyl, C₂₋₆-Alkenyl, C₂₋₆-Alkinyl, C₁₋₆-Haloalkyl und Aryl, der gegebenenfalls substituiert sein kann mit einem oder mehreren Resten R^{3.3.1};
R^{3.3.1} C₃₋₆-Cycloalkyl, d₃₋₆-Cycloalkenyl, OR^{3.3.1.1}, NR^{3.3.1.1}R^{3.3.1.2}, CONR^{3.3.1.1}R^{3.3.1.2}, COOR^{3.3.1.1}, NR^{3.3.1.1}COR^{3.3.1.2},SOR^{3.3.1.1}, SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het, Gegebenenfalls substituiert mit einem oder mehreren Resten ausgewählt aus der Gruppe bestehend aus C₁₋₄-Alkyl, SO₂H, SO₂-C₁₋₄-Alkyl, SO₂C₇₋₁₁-Aralkyl, CH₂-C₃₋₆-Cycloalkyl und Aryl;
R^{3.3.1.1}, R^{3.3.1.2} und R^{3.3.1.3} ein Rest ausgewählt aus der Gruppe bestehend aus C₁₋₆-Alkyl, C₂₋₆-Alkenyl, C₂₋₆-Alkinyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, COC₁₋₄-Alkyl-Hetaryl, COC₂₋₄-Alkenyl-Hetaryl, COC₂₋₄-Alkinyl-Hetaryl; oder zwei der Gruppen R^{3.3.1.1}, R^{3.3.1.2} und R^{3.3.1.3} bilden gemeinsam einen Ring, bestehend aus Kohlenstoffatomen und gegebenenfalls einem Heteroatom ausgewählt aus der Gruppe bestehend aus Sauerstoff, Stickstoff und Schwefel;
**R^{a}** H, C₁₋₆-Alkyl, C₂₋₆-Alkenyl, C₃₋₈-Cycloalkyl, C₃₋₈-Cycloalkenyl, C₁₋₆-Haloalkyl, COR⁸, NR⁹R¹⁰, NO₂, OR⁸, SR¹¹, SOR¹¹, SO₂R¹¹, NHCO-C₁₋₆-Alkyl-NH₂, Spiro oder ein Rest ausgewählt aus der Gruppe bestehend aus C₇₋₁₁- Aralkyl, CH₂-O-Aryl und Het der gegebenenfalls substituiert sein kann mit einem oder mehreren Halogen, C₁₋₆-Alkyl, CO-C₁₋₄-Haloalkyl, C₁₋₄-Alkyl-NH₂ oder CH₂NHCOOR¹²;
R⁸ C₁₋₆-Alkyl, C₃₋₆-Cycloalkyl, NH₂, Hetaryl oder Aryl, gegebenenfalls substituiert mit einem oder mehreren Halogen oder C₁₋₄-Alkyl;
R⁹ H, COOR¹², CONR¹² oder C₁₋₆-Alkyl, gegebenenfalls substituiert mit einem oder mehreren COOH, N(C₁₋₆-Alkyl)₂ oder Het, gegebenenfalls substituiert mit einem oder mehreren C₁₋₆-Alkyl; oder R⁹ bedeutet Het, gegebenenfalls substituiert mit einem oder mehreren C₁₋₄-Alkyl;
R¹⁰ H, C₁₋₆-Alkyl, CO-C₁₋₆-Alkyl oder C₂₋₆-Alkinyl;
R¹¹ C₁₋₆-Alkyl, gegebenenfalls substituiert mit einem oder mehreren N(C₁₋₄-Alkyl)₂;
R¹² H, C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Bevorzugt sind die oben genannten Verbindungen der Formel 1; worin
- **R^{a}**: ein Rest ausgewählt aus der Gruppe bestehend aus Aryl, C₇₋₁₁-Aralkyl und Hetaryl, der gegebenenfalls substituiert durch einen oder mehrere Reste ausgewählt aus der Gruppe bestehend aus R¹, R² und R³ sein kann;
- R¹ und R²: unabhängig voneinander C₁₋₆-Alkyl, C₂₋₆-Alkenyl C₂₋₆-Alkinyl, C₃₋₆-Cycloalkyl, C₃₋₆-Cycloalkenyl, C₁₋₆-Haloalkyl, C₁₋₆-Alkylen-COOH, C₁₋₆-Alkoxy, Halogen, OH, CN, COR^{1.1}, O-C₁₋₄-Haloalkyl, NO₂ oder SR^{1.1}, SOR^{1.1}, SO₂R^{1.1}, Het oder Hetaryl,
R^{1.1} OH, C₁₋₆-Alkyl, C₂₋₆-Alkenyl C₂₋₆-Alkinyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 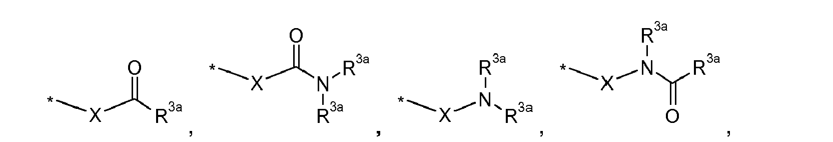 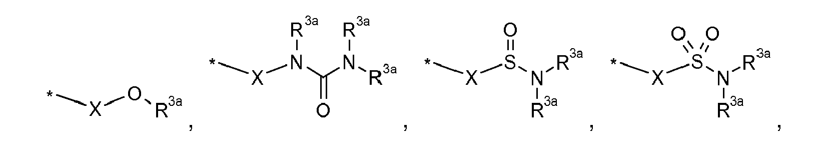 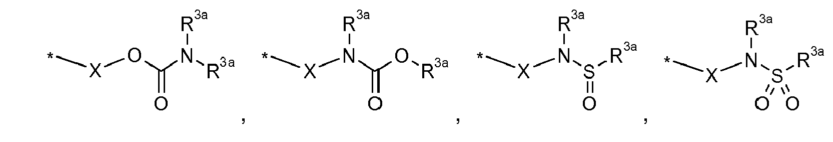 oder 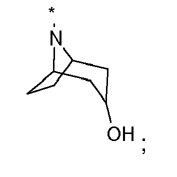 worin
X Bindung oder C₁₋₄-alkylen;
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} C₁₋₆-Alkyl, C₂₋₆-Alkenyl, OH, C₁₋₄-Alkylen-OH, C₁₋₄-Alkylen-NR^{3.1.1.1}R^{3.1.1.2,}, COR^{3.1.1.1}, COOR^{3.1.1.1}, CONR^{3.1.1.1}R^{3.1.1.2}, NR^{3.1.1.1}R^{3.1.1.2}, Het, Hetaryl, NHCOR^{3.1.1.1}
R^{3.1.1.1} ein Rest ausgewählt aus der Gruppe bestehend aus H, C₁₋₄-Alkyl, Aryl und C₇₋₁₁-Aralkyl; gegebenenfalls substituiert mit einem Rest ausgewählt aus der Gruppe Halogen, OH und CN;
R^{3.1.1.2} H, C₁₋₄-Alkyl;
R^{3.2} ein Rest ausgewählt aus der Gruppe bestehend aus C₃₋₆-Cycloalkyl, Het, Hetaryl und Spiro der gegebenenfalls substituiert ist mit einem oder mehreren R^{3.2.1}
R^{3.2.1} C₁₋₆-Alkyl, C₃₋₆-Cycloalkyl, OH, NR^{3.2.1.1}R^{3.2.1.2}, NHCOR^{3.2.1.3} oder Het, gegebenenfalls substituiert mit einem oder mehreren Resten ausgewählt aus der Gruppe bestehend aus C₁₋₄-Alkyl, SO₂R^{3.2.1.1}, CH₂-C₃₋₆-Cycloalkyl und Aryl;
R^{3.2.1.1} H, C₁₋₄-Alkyl oder C₇₋₁₁- Aralkyl;
R^{3.2.1.2} H, C₁₋₄-Alkyl oder C₇₋₁₁- Aralkyl;
R^{3.2.1.2} Aryl, C₇₋₁₁Aralkyl; oder C₁₋₆-alkyl, das gegebenenfalls substituiert ist mit einem oder zwei R^{3.2.2};
R^{3.2.2} C₁₋₆-Alkyl, C₂₋₆-Alkenyl, C₂₋₆-Alkinyl, COOR^{3.2.2.1}, CONR^{3.2.2.1}R^{3.2.2.2}, NR^{3.2.2.1}R^{3.2.2.2}, NHCOR^{3.2.2.1}, C₁₋₆-Haloalkyl, CN, OR^{3.2.2.1}, SO₂R^{3.2.2.1}, C₃₋₆-Cycloalkyl, CO-Het, C₂₋₄-Alkinyl-Hetaryl, Guanidin oder ein Rest ausgewählt aus der Gruppe bestehend aus Het, Hetaryl und Aryl, der gegebenenfalls substituiert ist mit einem oder mehreren Resten ausgewählt aus der Gruppe Halogen, C₁₋₆-Alkyl, CONR^{3.2.2.1}R^{3.2.2.2}, OH, Imidazolidinon;
R^{3.2.2.1} H oder C₁₋₆-Alkyl, Aryl, C₇₋₁₁-Aralkyl
R^{3.2.2.2.} H oder C₁₋₆-Alkyl; oder Aryl, das gegebenenfalls substituiert ist mit einem oder zwei R^{3.2.3}
R^{3.2.3} ein Rest ausgewählt aus der Gruppe bestehend aus NH-C₁₋₆-alkyl-N(C₁₋₆-alkyl)₂ oder Het, wobei Het gegebenenfalls substituiert mit einem Rest C₁₋₆-Alkyl sein kann;
R^{3.3} H oder ein Rest ausgewählt aus der Gruppe bestehend aus C₁₋₆-Alkyl, C₂₋₆-Alkenyl, C₂₋₆-Alkinyl, C₁₋₆-Haloalkyl und Aryl, der gegebenenfalls substituiert sein kann mit einem oder mehreren Resten R^{3.3.1};
R^{3.3.1} C₃₋₆-Cycloalkyl, C₃₋₆-Cycloalkenyl, OR^{3.3.1.1}, NR^{3.3.1.1}R^{3.3.1.2}, CONR^{3.3.1.1}R^{3.3.1.2} COOR^{3.3.1.1}, NR^{3.3.1.1}COR^{3.3.1.2}SOR^{3.3.1.1}, SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het, gegebenenfalls substituiert mit einem oder mehreren Resten ausgewählt aus der Gruppe bestehend aus C₁₋₄-Alkyl, SO₂H, SO₂-C₁₋₄-Alkyl, SO₂C₇₋₁₁-Aralkyl, CH₂-C₃₋₆-Cycloalkyl und Aryl;
R^{3.3.1.1}, R^{3.3.1.2} und R^{3.3.1.3} ein Rest ausgewählt aus der Gruppe bestehend aus C₁₋₆-Alkyl, C₂₋₆-Alkenyl, C₂₋₆-Alkinyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, COC₁₋₄-Alkyl-Hetaryl, COC₂₋₄-Alkenyl-Hetaryl, COC₂₋₄-Alkinyl-Hetaryl, oder zwei der Gruppen R^{3.3.1.1}, R^{3.3.1.2} und R^{3.3.1.3} bilden gemeinsam einen Ring, bestehend aus Kohlenstoffatomen und gegebenenfalls einem Heteroatom ausgewählt aus der Gruppe bestehend aus Sauerstoff, Stickstoff und Schwefel;
**R^{a}** H, C₁₋₆-Alkyl, C₂₋₆-Alkenyl, C₃₋₈-Cycloalkyl, C₃₋₈-Cycloalkenyl, C₁₋₆-Haloalkyl, COR⁸, NR⁹R¹⁰, NO₂, OR⁸, SR¹¹, SOR¹¹, SO₂R¹¹, NHCO-C₁₋₆-Alkyl-NH₂, Spiro oder ein Rest ausgewählt aus der Gruppe bestehend aus C₇₋₁₁- Aralkyl, CH₂-O-Aryl und Het der gegebenenfalls substituiert sein kann mit einem oder mehreren Halogen, C₁₋₆-Alkyl, CO-C₁₋₄-Haloalkyl, C₁₋₄-Alkyl-NH₂ oder CH₂NHCOOR¹²;
R⁸ C₁₋₆-Alkyl, C₃₋₆-Cycloalkyl, NH₂, Hetaryl oder Aryl, gegebenenfalls substituiert mit einem oder mehreren Halogen oder C₁₋₄-Alkyl;
R⁹ H, COOR¹², CONR¹² oder C₁₋₆-Alkyl, gegebenenfalls substituiert mit einem oder mehreren COOH, N(C₁₋₆-Alkyl)₂ oder Het, gegebenenfalls substituiert mit einem oder mehreren C₁₋₆-Alkyl; oder R⁹ bedeutet Het, gegebenenfalls substituiert mit einem oder mehreren C₁₋₄-Alkyl;
R¹⁰ H, C₁₋₆-Alkyl, CO-C₁₋₆-Alkyl oder C₂₋₆-Alkinyl;
R¹¹ C₁₋₆-Alkyl, gegebenenfalls substituiert mit einem oder mehreren N(C₁₋₄-Alkyl)₂;
R¹² H, C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}** = NHCONH-Et bedeutet.

Bevorzugt sind die oben genannten Verbindungen der Formel **1**; worin
- **R^{a}**: ein Rest ausgewählt aus der Gruppe bestehend aus Aryl, C₇₋₁₁-Aralkyl und Hetaryl, der gegebenenfalls substituiert durch einen oder mehrere Reste ausgewählt aus der Gruppe bestehend aus R¹, R² und R³ sein kann;
- R¹ und R²: unabhängig voneinander C₁₋₆-Alkyl, C₂₋₆-Alkenyl C₂₋₆-Alkinyl, C₃₋₆-Cycloalkyl, C₃₋₆-Cycloalkenyl, C₁₋₆-Haloalkyl, C₁₋₆-Alkylen-COOH, C₁₋₆-Alkoxy, Halogen, OH, CN, COR^{1.1}, O-C₁₋₄-Haloalkyl, NO₂ oder SR^{1.1}, SOR¹.¹, SO₂R¹¹, Het oder Hetaryl,
R^{1.1} OH, C₁₋₆-Alkyl, C₂₋₆-Alkenyl C₂₋₆-Alkinyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 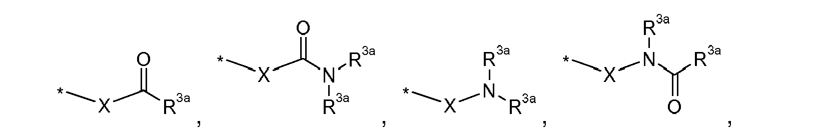 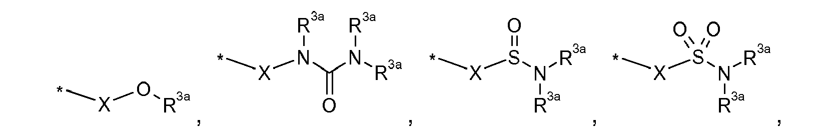 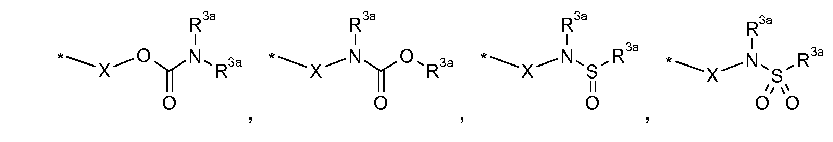 oder 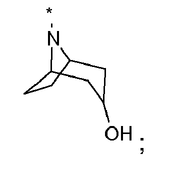 worin
X Bindung oder C₁₋₄-alkylen;
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} C₁₋₆-Alkyl, C₂₋₆-Alkenyl, OH, C₁₋₄-Alkylen-OH, C₁₋₄-Alkylen-NR^{3.1.1.1}R^{3.1.1.2}, COR^{3.1.1.1}, COOR^{3.1.1.1,} CONR^{3.1.1.1}R^{3.1.1.2}, NR^{3.1.1.1}R^{3.1.1.2}, Het, Hetaryl, NHCOR^{3.1.1.1}
R^{3.1.1.1} ein Rest ausgewählt aus der Gruppe bestehend aus H, C₁₋₄-Alkyl, Aryl und C₇₋₁₁-Aralkyl; gegebenenfalls substituiert mit einem Rest ausgewählt aus der Gruppe Halogen, OH und CN;
R^{3.1.1.2} H, C₁₋₄-Alkyl;
R^{3.2} ein Rest ausgewählt aus der Gruppe bestehend aus C₃₋₆-Cycloalkyl, Het, Hetaryl und Spiro der gegebenenfalls substituiert ist mit einem oder mehreren R^{3.2.1}
R^{3.2.1} C₁₋₆-Alkyl, C₃₋₆-Cycloalkyl, OH, -NR^{3.2.1.1}R^{3.2.1.2}, NHCOR^{3.2.1.3} oder Het, gegebenenfalls substituiert mit einem oder mehreren Resten ausgewählt aus der Gruppe bestehend aus C₁₋₄-Alkyl, SO₂R^{3.2.1.1}, CH₂-C₃₋₆-Cycloalkyl und Aryl;
R^{3.2.1.1} H, C₁₋₄-Alkyl oder C₇₋₁₁- Aralkyl;
R^{3.2.1.2} H, C₁₋₄-Alkyl oder C₇₋₁₁- Aralkyl;
R^{3.2.1.2} Aryl, C₇₋₁₁-Aralkyl; oder -C₁₋₆-alkyl, das gegebenenfalls substituiert ist mit einem oder zwei R^{3.2.2};
R^{3.2.2} C₁₋₆-Alkyl, C₂₋₆-Alkenyl, C₂₋₆-Alkinyl, COOR^{3.2.2.1}, CONR^{3.2.2.1}R^{3.2.2.2}, NR^{3.2.2.1}R^{3.2.2.2}, NHCOR^{3.2.2.1}, C₁₋₆-Haloalkyl, CN, OR^{3.2.2.1}, SO₂R^{3.2.2.1,} C₃₋₆-Cycloalkyl, CO-Het, C₂₋₄-Alkinyl-Hetaryl, Guanidin oder ein Rest ausgewählt aus der Gruppe bestehend aus Het, Hetaryl und Aryl, der gegebenenfalls substituiert ist mit einem oder mehreren Resten ausgewählt aus der Gruppe Halogen, C₁₋₆-Alkyl, CONR^{3.2.2.1}R^{3.2.2.2}, OH, Imidazolidinon;
R^{3.2.2.1} H oder C₁₋₆-Alkyl, Aryl, C₇₋₁₁-Aralkyl
R^{3.2.2.2.} H oder C₁₋₆-Alkyl; oder Aryl, das gegebenenfalls substituiert ist mit einem oder zwei R^{3.2.3}
R^{3.2.3} ein Rest ausgewählt aus der Gruppe bestehend aus NH-C₁₋₆-alkyl-N(C₁₋₆-alkyl)₂ oder Het, wobei Het gegebenenfalls substituiert mit einem Rest C₁₋₆-Alkyl sein kann;
R^{3.3} H oder ein Rest ausgewählt aus der Gruppe bestehend aus C₁₋₆-Alkyl, C₂₋₄-Alkenyl, C₂₋₄-Alkinyl, C₁₋₄-Haloalkyl und Aryl, der gegebenenfalls substituiert sein kann mit einem oder mehreren Resten R^{3.3.1};
R^{3.3.1} C₅₋₆-Cycloalkyl, C₅₋₆-Cycloalkenyl, OR^{3.3.1.1}, NR^{3.3.1.1}R^{3.3.1.2}, CONR^{3.3.1.1}R^{3.3.1.2}, COOR^{3.3.1.1}, NR^{3.3.1.1}COR^{3.3.1.2},SOR^{3.3.1.1}, SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het, Gegebenenfalls substituiert mit einem oder mehreren Resten ausgewählt aus der Gruppe bestehend aus C₁₋₄-Alkyl, SO₂H, SO₂-C₁₋₄-Alkyl, SO₂C₇₋₁₁-Aralkyl, CH₂-C₃₋₆-Cycloalkyl und Aryl;
R^{3.3.1.1}, R^{3.3.1.2} und R^{3.3.1.3} ein Rest ausgewählt aus der Gruppe bestehend aus C₁₋₄-Alkyl, C₂₋₄-Alkenyl, C₂₋₄-Alkinyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, COC₁₋₄-Alkyl-Hetaryl, COC₂₋₄-Alkenyl-Hetaryl, COC₂₋₄-Alkinyl-Hetaryl; oder zwei der Gruppen R^{3.3.1.1}, R^{3.3.1.2} und R^{3.3.1.3} bilden gemeinsam einen fünf-, sechs oder siebengliedrigen Ring, bestehend aus Kohlenstoffatomen und gegebenenfalls einem Heteroatom ausgewählt aus der Gruppe bestehend aus Sauerstoff, Stickstoff und Schwefel;
**R^{a}** H, C₁₋₆-Alkyl, C₂₋₆-Alkenyl, C₃₋₈-Cycloalkyl, C₃₋₈-Cycloalkenyl, C₁₋₆-Haloalkyl, COR⁸, NR⁹R¹⁰, NO₂, OR⁸, SR¹¹, SOR¹¹, SO₂R¹¹, NHCO-C₁₋₆-Alkyl-NH₂, Spiro oder ein Rest ausgewählt aus der Gruppe bestehend aus C₇₋₁₁- Aralkyl, CH₂-O-Aryl und Het der gegebenenfalls substituiert sein kann mit einem oder mehreren Halogen, C₁₋₆-Alkyl, CO-C₁₋₄-Haloalkyl, C₁₋₄-Alkyl-NH₂ oder CH₂NHCOOR¹²;
R⁸ C₁₋₆-Alkyl, C₃₋₆-Cycloalkyl, NH₂, Hetaryl oder Aryl, gegebenenfalls substituiert mit einem oder mehreren Halogen oder C₁₋₄-Alkyl;
R⁹ H, COOR¹² oder C₁₋₄-Alkyl, gegebenenfalls substituiert mit einem oder mehreren COOH, N(C₁₋₄-Alkyl)₂ oder Het, gegebenenfalls substituiert mit einem oder mehreren C₁₋₄-Alkyl; oder R⁹ bedeutet Het, gegebenenfalls substituiert mit einem oder mehreren C₁₋₄-Alkyl;
R¹⁰ H, C₁₋₆-Alkyl, CO-C₁₋₄-Alkyl oder C₂₋₆-Alkinyl;
R¹¹ C₁₋₆-Alkyl, gegebenenfalls substituiert mit einem oder mehreren N(C₁₋₄-Alkyl)₂;
R¹² C₁₋₆-Alkyl;
R^{b} R⁴, OR⁴, -CH₂OR⁴, COR⁴, COOR⁴, CONR⁴R⁵, NR⁴R⁵, NR⁵COR⁴, NR⁵COOR⁴, NR⁵CONR⁴R⁵, NR⁵SOR⁴ oder NR⁵SO₂R⁴;
R⁴ H, C₁₋₆-Alkyl, C₁₋₆-Haloalkyl, C₁₋₆-Alkylen-OH, C₂₋₆-Alkenyl, C₇₋₁₁Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl;
R⁵ H oder C₁₋₆-Alkyl; oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
**R^{c}** NHR⁶ oder ein Rest ausgewählt aus der Gruppe bestehend aus 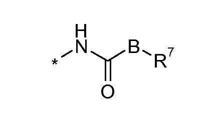 worin
B Bindung, C₁₋₄-Alkyl oder C₂₋₄-Alkinyl;
R⁶ H oder ein Rest ausgewählt aus der Gruppe C₁₋₄-Alkyl, C₂₋₄-Alkenyl, C₂₋₄-Alkinyl, C₃₋₆-Cycloalkyl, C₃-₆-Cycloalkenyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
R⁷ H, C₁₋₄-Alkyl, C₂₋₄-Alkenyl, C₂₋₄-Alkinyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₄-Alkyl, (CH₂)₂₋₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Bevorzugt sind die oben genannten Verbindungen der Formel **1**; worin
- **R^{a}**: ein Rest ausgewählt aus der Gruppe bestehend aus Aryl, C₇-₁₁-Aralkyl und Hetaryl, der gegebenenfalls substituiert durch einen oder mehrere Reste ausgewählt aus der Gruppe bestehend aus R¹, R² und R³ sein kann;
- R¹ und R²: unabhängig voneinander C₁-₆-Alkyl, C₂-₆-Alkenyl C₂-₆-Alkinyl, C₃₋₆-Cycloalkyl, C₃-₆-Cycloalkenyl, C₁-₆-Haloalkyl, C₁-₆-Alkylen-COOH, C₁-₆-Alkoxy, Halogen, OH, CN, COR^{1.1}, O-C₁-₄-Haloalkyl, NO₂ oder SR^{1.1}, SOR^{1.1}, SO₂R^{1.1}, Het oder Hetaryl,
R^{1.1} OH, C₁₋₆-Alkyl, C₂₋₆-Alkenyl C₂₋₆-Alkinyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 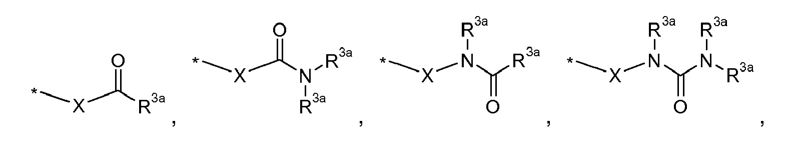 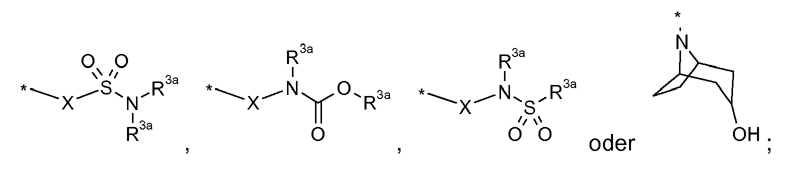 worin
X Bindung oder C₁₋₄-alkylen;
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} C₁₋₆-Alkyl, C₂₋₆-Alkenyl, OH, C₁₋₄-Alkylen-OH, C₁₋₄-Alkylen-NR^{3.1.1.1}R^{3.1.1.2}, COR^{3.1.1.1}, COOR^{3.1.1.1}, CONR^{3.1.1.1}R^{3.1.1.2}, NR^{3.1.1.1}R^{3.1.1.2}, Het, Hetaryl, NHCOR^{3.1.1.1}
R^{3.1.1.1} ein Rest ausgewählt aus der Gruppe bestehend aus H, C₁₋₄-Alkyl, Aryl und C₇₋₁₁-Aralkyl; gegebenenfalls substituiert mit einem Rest ausgewählt aus der Gruppe Halogen, OH und CN;
R^{3.1.1.2} H, C₁₋₄-Alkyl;
R^{3.2} ein Rest ausgewählt aus der Gruppe bestehend aus C₃₋₆-Cycloalkyl, Het, Hetaryl und Spiro der gegebenenfalls substituiert ist mit einem oder mehreren R^{3.2.1}
R^{3.2.1} C₁₋₆-Alkyl, C₃₋₆-Cycloalkyl, OH, -NR^{3.2.1.1}R^{3.2.1.2}, NHCOR^{3.2.1.3} oder Het, gegebenenfalls substituiert mit einem oder mehreren Resten ausgewählt aus der Gruppe bestehend aus C₁₋₄-Alkyl, SO₂R^{3.2.1.1}, CH₂-C₃₋₆-Cycloalkyl und Aryl;
R^{3.2.1.1} H, C₁₋₄-Alkyl oder C₇₋₁₁-Aralkyl;
R^{3.2.1.2} H, C₁₋₄-Alkyl oder C₇₋₁₁-Aralkyl;
R^{3.2.1.2} Aryl, C₇₋₁₁-Aralkyl; oder -C₁₋₆-alkyl, das gegebenenfalls substituiert ist mit einem oder zwei R^{3.2.2};
R^{3.2.2} C₁₋₆-Alkyl, C₂₋₆-Alkenyl, C₂₋₆-Alkinyl, COOR^{3.2.2.1}, CONR^{3.2.2.1}R^{3.2.2.2}, NR^{3.2.2.1}R^{3.2.2.2}, NHCOR^{3.2.2.1}, C₁₋₆-Haloalkyl, CN, OR^{3.2.2.1}, SO₂R^{3.2.2.1}, C₃₋₆-Cycloalkyl, CO-Het, C₂-₄-Alkinyl-Hetaryl, Guanidin oder ein Rest ausgewählt aus der Gruppe bestehend aus Het, Hetaryl und Aryl, der gegebenenfalls substituiert ist mit einem oder mehreren Resten ausgewählt aus der Gruppe Halogen, C₁₋₆-Alkyl, CONR^{3.2.2.1}R^{3.2.2.2}, OH, Imidazolidinon;
R^{3.2.2.1} H oder C₁₋₆-Alkyl, Aryl, C₇-₁₁-Aralkyl
R^{3.2.2.2.} H oder C₁₋₆-Alkyl; oder Aryl, das gegebenenfalls substituiert ist mit einem oder zwei R^{3.2.3}
R^{3.2.3} ein Rest ausgewählt aus der Gruppe bestehend aus NH-C₁₋₆-alkyl-N(C₁₋₆-alkyl)₂ oder Het, wobei Het gegebenenfalls substituiert mit einem Rest C₁₋₆-Alkyl sein kann;
R^{3.3} H oder ein Rest ausgewählt aus der Gruppe bestehend aus C₁₋₆-Alkyl, C₂₋₄-Alkenyl, C₂₋₄-Alkinyl und Aryl, der gegebenenfalls substituiert sein kann mit einem oder mehreren Resten R^{3.3.1} ;
R^{3.3.1} C₅₋₆-Cycloalkyl, C₅₋₆-Cycloalkenyl, OR^{3.3.1.1}, NR^{3.3.1.1}R^{3.3.1.2}, CONR^{3.3.1}R^{3.3.1.2}, COOR^{3.3.1.1} NR^{3.3.1.1}COR^{3.3.1.2},SOR^{3.3.1.1} SO₂R^{3.3.1.1} C(NR^{3.3.1.1}R^{3.3.1.2})NR^{3.3.1.3} NR^{3.3.1.1}CONR^{3.3.1.2}R^{3.3.1.3} OH, CN, Halogen oder Het, Gegebenenfalls substituiert mit einem oder mehreren Resten ausgewählt aus der Gruppe bestehend aus C₁₋₄-Alkyl, SO₂H, SO₂-C₁₋₄-Alkyl, SO₂C₇₋₁₁-Aralkyl, CH₂-C₃₋₆-Cycloalkyl und Aryl;
R^{3.3.1.1}, R^{3.3.1.2} und R^{3.3.1.3} ein Rest ausgewählt aus der Gruppe bestehend aus C₁₋₄-Alkyl, C₇-₁₁-Aralkyl, C₁-₄-Alkyl-Hetaryl, COC₁₋₄-Alkyl-Hetaryl; oder zwei der Gruppen R^{3.3.1.1}, R^{3.3.1.2} und R^{3.3.1.3} bilden gemeinsam einen fünf-, sechs oder siebengliedrigen Ring, bestehend aus Kohlenstoffatomen und gegebenenfalls einem Heteroatom ausgewählt aus der Gruppe bestehend aus Sauerstoff, Stickstoff und Schwefel;
**R^{a}** H, C₁₋₆-Alkyl, C₂₋₆-Alkenyl, C₃₋₆-Cycloalkyl, C₃₋₆-Cycloalkenyl, C₁₋₆-Haloalkyl, COR⁸, NR⁹R¹⁰, NO₂, OR⁸, SR¹¹, SOR¹¹, SO₂R¹¹, NHCO-C₁₋₆-Alkyl-NH₂, Spiro oder ein Rest ausgewählt aus der Gruppe bestehend aus C₇₋₁₁- Aralkyl, CH₂-O-Aryl und Het der gegebenenfalls substituiert sein kann mit einem oder mehreren Halogen, C₁₋₆-Alkyl, CO-C₁₋₄-Haloalkyl, C₁₋₄-Alkyl-NH₂ oder CH₂NHCOOR¹²;
R⁸ C₁₋₆-Alkyl, C₃₋₆-Cycloalkyl, NH₂, Hetaryl oder Aryl, gegebenenfalls substituiert mit einem oder mehreren Halogen oder C₁₋₄-Alkyl;
R⁹ H, COOR¹² oder C₁₋₄-Alkyl, gegebenenfalls substituiert mit einem oder mehreren COOH, N(C₁₋₄-Alkyl)₂ oder Het, gegebenenfalls substituiert mit einem oder mehreren C₁₋₄-Alkyl; oder R⁹ bedeutet Het, gegebenenfalls substituiert mit einem oder mehreren C₁₋₄-Alkyl;
R¹⁰ H, C₁₋₆-Alkyl, CO-C₁₋₄-Alkyl oder C₂₋₆-Alkinyl;
R¹¹ C₁₋₆-Alkyl, gegebenenfalls substituiert mit einem oder mehreren N(C₁-₄-Alkyl)₂;
R¹² C₁₋₆-Alkyl;
R^{b} R⁴, OR⁴, -CH₂OR⁴, COR⁴, COOR⁴, CONR⁴R⁵, NH₂, NR⁵COOR⁴, NR⁵CONR⁴R⁵ oder NR⁵SOR⁴;
R⁴ H, C₁₋₆-Alkyl, C₁₋₆-Haloalkyl, C₁₋₆-Alkylen-OH, C₂₋₆-Alkenyl, C₇₋₁₁Aralkyl, C₁₋₄-Alkyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl;
R⁵ H oder C₁₋₆-Alkyl;
oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
- **R^{c}**: NHR⁶ oder ein Rest ausgewählt aus der Gruppe bestehend aus 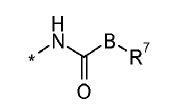 worin
B Bindung, C₁₋₄-Alkyl oder C₂₋₄-Alkinyl;
R⁶ H oder ein Rest ausgewählt aus der Gruppe C₁₋₄-Alkyl, C₃₋₆-Cycloalkyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
R⁷ H, C₁₋₄-Alkyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₄-Alkyl, (CH₂)₂-₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Bevorzugt sind die oben genannten Verbindungen der Formel 1; worin
- **R^{a}**: ein Rest ausgewählt aus der Gruppe bestehend aus Aryl und C₇₋₁₁-Aralkyl, der gegebenenfalls substituiert durch einen oder mehrere Reste ausgewählt aus der Gruppe bestehend aus R¹, R² und R³ sein kann; oder Hetaryl gegebenenfalls substituiert durch ein oder mehrere C₁₋₄-Alkyl;
- R¹: C₁₋₄-Alkyl, C₁₋₄-Haloalkyl, C₁₋₄-Alkylen-COOH, C₁₋₄-Alkoxy, Halogen, OH, CN, COR^{1.1}, O-C₁₋₄-Haloalkyl, NO₂ oder SO₂R^{1.1};
R^{1.1} OH, Methyl, NH₂, NHMe, NMe₂, 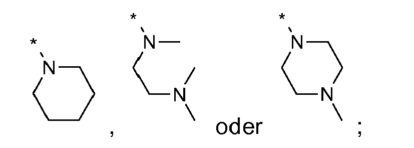
- R²: C₁₋₄-Alkyl, C₁₋₄-Alkoxy oder Halogen;
- R³: ein Rest ausgewählt aus der Gruppe bestehend aus 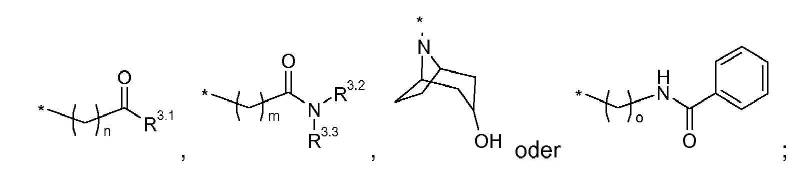 worin
n 0 oder 1;
m 0 oder 1;
o 2;
R^{3.1} Spiro oder Het, wobei Het gegebenenfalls substituiert mit einem oder mehreren R^{3.1.1} sein kann;
R^{3.1.1} C₁₋₄-Alkyl, C₂₋₄-Alkenyl, OH, C₁₋₄-Alkylen-OH, CH₂NEt₂, COMe, COOH, CONH₂, NH₂, Het, Hetaryl, 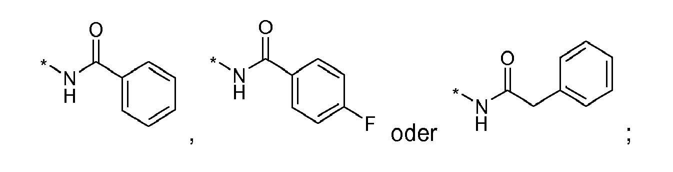 R^{3.2} ein Rest ausgewählt aus der Gruppe bestehend aus C₃₋₆-Cycloalkyl, Het, Hetaryl und Spiro der gegebenenfalls substituiert ist mit einem oder zwei R^{3.2.1}
R^{3.2.1} C₁₋₄-Alkyl, Cyclopentyl, OH, -NR^{3.2.1.1}R^{3.2.1.2} oder 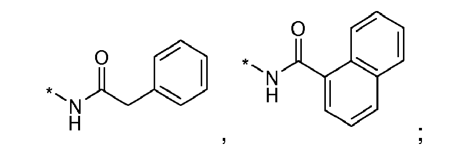 oder Het, gegebenenfalls substituiert ist mit einem oder mehreren Resten ausgewählt aus der Gruppe bestehend aus Methyl, SO₂R^{3.2.1.1}, 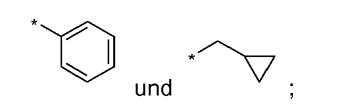 R^{3.2.1.1} H, Methyl oder Benzyl; R^{3.2.1.2} H, Methyl oder Benzyl; oder
-C₁₋₆-alkyl, das gegebenenfalls substituiert ist mit einem oder zwei R^{3.2.2};
R^{3.2.2} C₂₋₄-Alkenyl, C₂₋₄-Alkinyl, COOR^{3.2.2.1}, CONR^{3.2.2.1}R^{3.2.2.2}, NR^{3.2.2.1}R^{3.2.2.2}, NHCOR^{3.2.2.1}, C₁₋₄-Haloalkyl, CN, OH, SO₂R^{3.2.2.1}, C₃₋₆-Cycloalkyl oder ein Rest ausgewählt aus der Gruppe bestehend aus 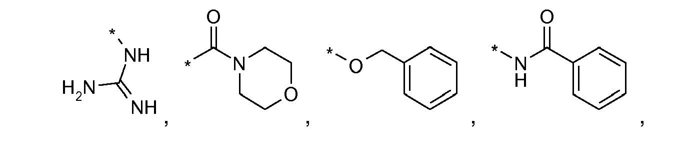 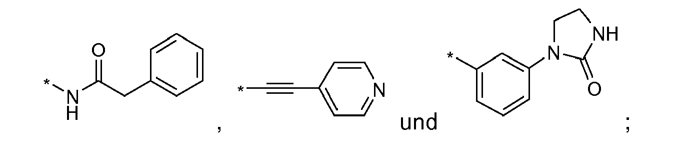 oder ein Rest ausgewählt aus der Gruppe bestehend aus Het, Hetaryl und Aryl, der gegebenenfalls substituiert ist mit einem oder mehreren Resten ausgewählt aus der Gruppe CI, Methyl, CONR^{3.2.2.1}R^{3.2.2.2}, OH;
R^{3.2.2.1} H oder Methyl;
R^{3.2.2.2.} H oder Methyl; oder Aryl, das gegebenenfalls substituiert ist mit einem oder zwei R^{3.2.3}
R^{3.2.3} ein Rest ausgewählt aus der Gruppe bestehend aus 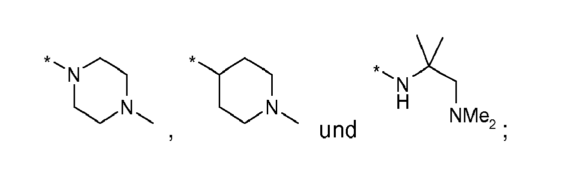 R^{3.3} H oder ein Rest ausgewählt aus der Gruppe bestehend aus C₁₋₆-Alkyl und Aryl, der gegebenenfalls substituiert sein kann mit einem oder mehreren Resten R^{3.3.1};
R^{3.3.1} C₅₋₆-Cycloalkyl, C₅₋₆-Cycloalkenyl, OR^{3.3.1.1}, NR^{3.3.1.1}R^{3.3.1.2}, CONR^{3.3.1.1}R^{3.3.1.2}, COOR^{3.3.1.1}, NR^{3.3.1.1}COR^{3.3.1.2},SOR^{3.3.1.1}, SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het, gegebenenfalls substituiert ist mit einem oder mehreren Resten ausgewählt aus der Gruppe bestehend aus Methyl, SO₂H, SO₂-C₁₋₄-Alkyl, SO₂C₇₋₁₁-Aralkyl, 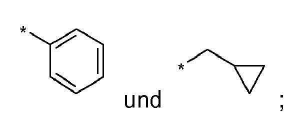 R^{3.3.1.1} R^{3.3.1.2} und R^{3.3.1.3} ein Rest ausgewählt aus der Gruppe bestehend aus C₁₋₄-Alkyl, C₇₋₁₁-Aralkyl, C₁₋₄-Alkyl-Hetaryl, COC₁₋₄-Alkyl-Hetaryl;
- **R^{a}**: H, C₁₋₆-Alklyl, C₂₋₆-Alkenyl, C₃₋₆-Cycloalkyl, CF₃, COR⁸, NR⁹R¹⁰, NO₂, S(O)ₙR¹¹, oder ein Rest ausgewählt aus der Gruppe bestehend aus 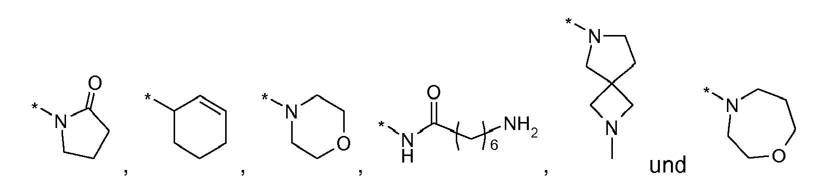 oder ein Rest ausgewählt aus der Gruppe bestehend aus 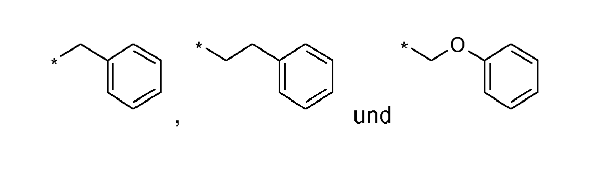 der gegebenenfalls substituiert sein kann mit einem oder mehreren CI;
oder ein Rest ausgewählt aus der Gruppe bestehend aus 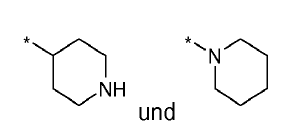 der gegebenenfalls substituiert sein kann mit einem oder mehreren CH₃, COCF₃, CH₂NH₂ oder CH₂NHCOOR¹²;
- R⁸: C₁₋₄-Alkyl, C₃₋₆-Cycloalkyl, NH₂, Furanyl oder Phenyl, gegebenenfalls substituiert mit einem oder mehreren Chlor;
- R⁹: H, COOR¹² oder Piperidino, gegebenenfalls substituiert mit einem oder mehreren CH₃, oder ein Rest ausgewählt aus der Gruppe bestehend aus C₁₋₄-Alkyl, der gegebenenfalls substituiert sein kann mit einem oder mehreren COOH, NMe₂ oder 4-Methylpiperazin;
- R¹⁰: H, C₁₋₄-Alkyl, C₂₋₄-Alkinyl oder COCH₃;
- R¹¹: C₁₋₄-Alkyl, gegebenenfalls substituiert mit einem oder mehreren NMe₂,
- R¹²: C₁₋₄-Alkyl;
- R^{b}: R⁴, CH₂OR⁴, COR⁴, COOR⁴, CONR⁴R⁵, NH₂, NHCOOR⁴, NHCONR⁴R⁵ oder OH;
- R⁴: H, C₁₋₄-Alkyl, C₁₋₄-Alkylen-OH, C₂₋₄-Alkinyl, C₁₋₆-Haloalkyl, Aryl, Het, Hetaryl, 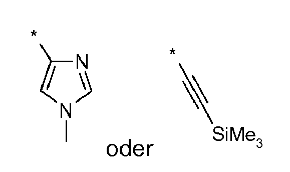
- R⁵: H oder C₁₋₄-Alkyl;
- **R^{c}**: NHR⁶ oder ein Rest ausgewählt aus der Gruppe bestehend aus 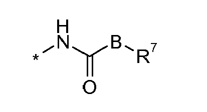 worin
B Bindung, C₁₋₄-Alkyl oder C₂₋₄-Alkinyl;
R⁶ H, C₁₋₄-Alkyl, C₃₋₆-Cycloalkyl, C₇₋₁₁- Aralkyl, Aryl, gegebenenfalls substituiert mit SO₂CH₃;
R⁷ H, NR^{7.1}R^{7.2}, OR^{7.2}, SR^{7.2}, Hetaryl, Het, gegebenenfalls substituiert mit C₁₋₄-Alkyl oder CONH₂, oder ein Rest ausgewählt aus der Gruppe bestehend aus 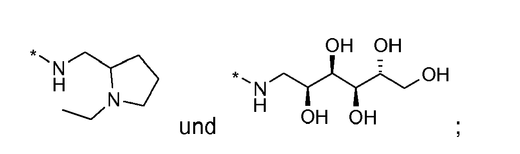 R^{7.1} H, C₁₋₄-Alkyl, (CH₂)₂R^{7.1.1} oder COOButyl;
R^{7.2} H, C₁₋₄-Alkyl;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Bevorzugt sind die oben genannten Verbindungen der Formel 1; worin
- **R^{a}**: Phenyl oder Benzyl, jeweils gegebenenfalls substituiert durch einen oder mehrere Reste ausgewählt aus der Gruppe bestehend aus R¹, R² und R³; oder 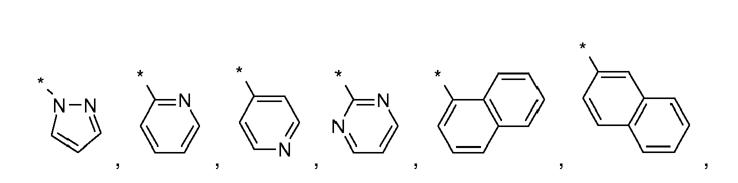 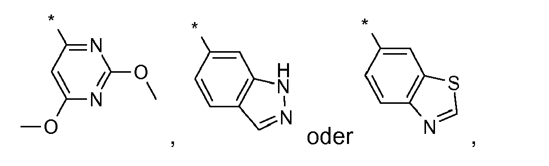
- R¹: Methyl, Ethyl, Propyl, Butyl, CF₃, CH₂COOH, Methoxy, F, CI, Br, OH, CN, COR^{1.1}, OCF₃, NO₂ oder SO₂R^{1.1};
R^{1.1} OH, Methyl, NH₂, NHMe, NMe₂, 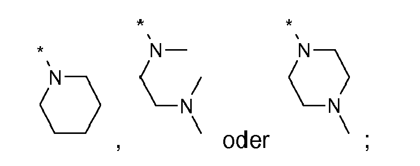
- R²: Methyl, Methoxy, F, CI oder Br;
- R³: ein Rest ausgewählt aus der Gruppe bestehend aus 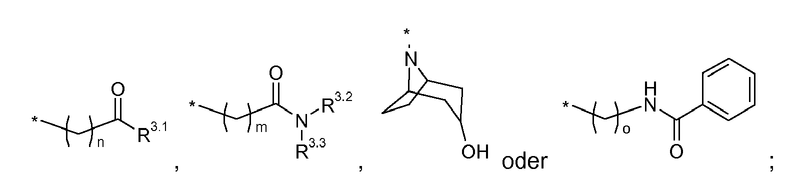 worin
n 0 oder 1;
m 0 oder 1;
o 2;
R^{3.1} ein Rest ausgewählt aus der Gruppe bestehend aus 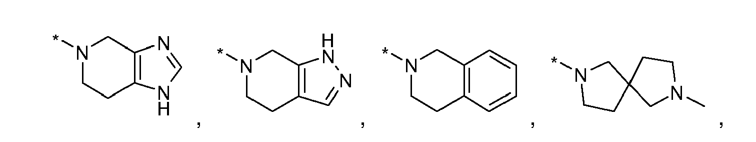 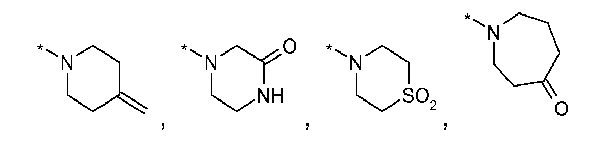 und 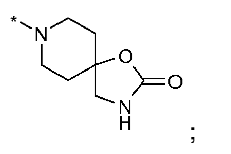 oder ein Rest ausgewählt aus der Gruppe bestehend aus 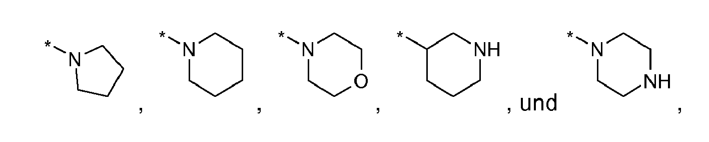 der gegebenenfalls substituiert sein kann mit einem oder mehreren R^{3.1.1};
R^{3.1.1} Methyl, Ethyl, OH, CH₂OH, CH₂CH₂OH, CH₂NEt₂, COMe, COOH, CONH₂, NH₂, 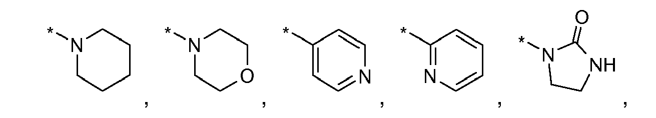 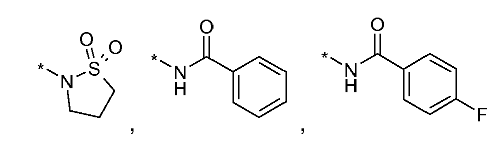 oder 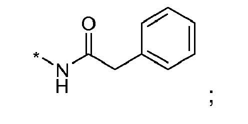 R^{3.2} ein Rest ausgewählt aus der Gruppe bestehend aus 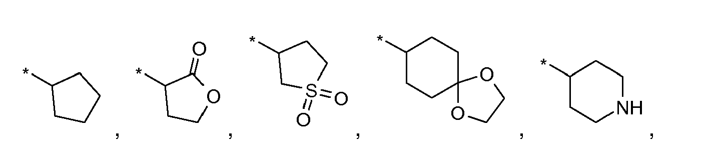 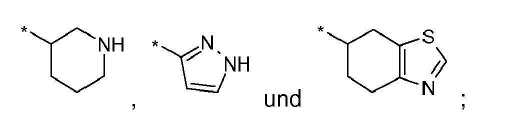 der gegebenenfalls substituiert ist mit einem oder mehreren Resten ausgewählt aus der Gruppe bestehend aus Methyl, Ethyl, Cyclopentyl, OH, NH₂; oder
Cyclohexyl, das gegebenenfalls substituiert ist mit einem oder zwei R^{3.2.1}
R^{3.2.1} -NR^{3.2.1.1}R^{3.2.1.2} oder ein Rest ausgewählt aus der Gruppe bestehend aus 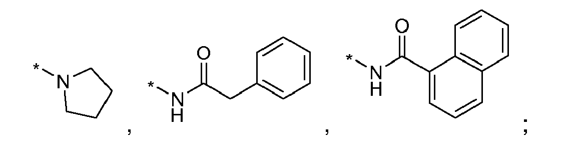 oder ein Rest ausgewählt aus der Gruppe bestehend aus 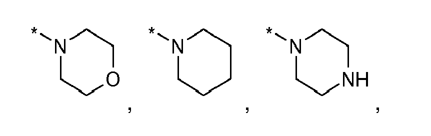 der gegebenenfalls substituiert ist mit einem oder mehreren Resten ausgewählt aus der Gruppe bestehend aus Methyl, SO₂R^{3.2.1.1}, 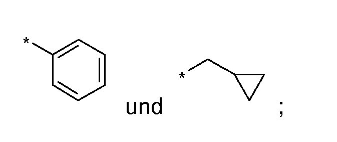 R^{3.2.1.1} H, Methyl oder Benzyl;
R^{3.2.1.2} H, Methyl oder Benzyl; oder -C₁₋₆-alkyl, gerade oder verzweigt, das gegebenenfalls substituiert ist mit einem oder zwei R^{3.2.2};
R^{3.2.2} C=CH₂, C≡CH, COOR^{3.2.2.1}, CONR^{3.2.2.1}R^{3.2.2.2}, NR^{3.2.2.1}R^{3.2.2.2}, NHCOR^{3.2.2.1}, CF₃, CN, OH, SO₂R^{3.2.2.1} oder ein Rest ausgewählt aus der Gruppe bestehend aus 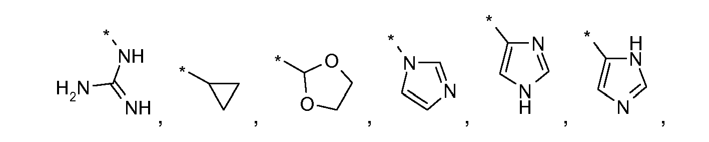 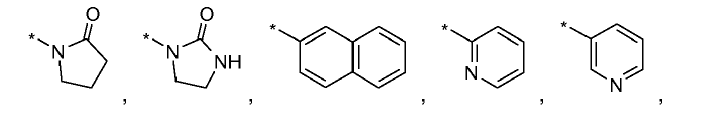 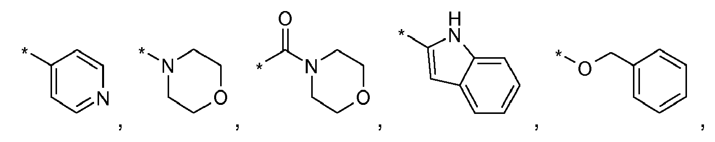 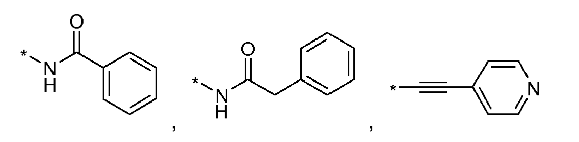 und 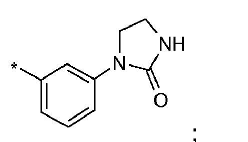 oder ein Rest ausgewählt aus der Gruppe bestehend aus 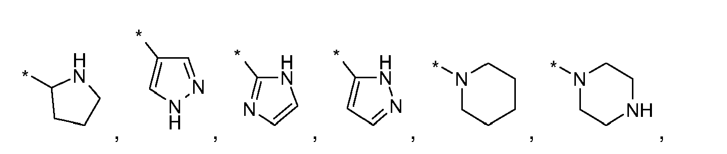 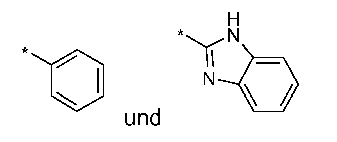 der gegebenenfalls substituiert ist mit einem oder mehreren Resten ausgewählt aus der Gruppe CI, Methyl, CONR^{3.2.2.1}R^{3.2.2.2}, OH;
R^{3.2.2.1} H oder Methyl;
R^{3.2.2.2.} H oder Methyl; oder Phenyl, das gegebenenfalls substituiert ist mit einem oder zwei R^{3.2.3}
R^{3.2.3} ein Rest ausgewählt aus der Gruppe bestehend aus 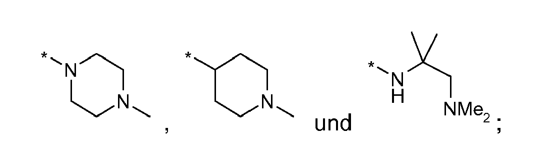 R^{3.3} H, C₁₋₆-Alkyl, gegebenenfalls substituiert mit einem oder mehreren R^{3.3.1};
R^{3.3.1} C₅₋₆-Cycloalkyl, C₅₋₆-Cycloalkenyl, OR^{3.3.1.1}, NR^{3.3.1.1}R^{3.3.1.2}, CONR^{3.3.1.1}R^{3.3.1.2}, COOR^{3.3.1.1}, NR^{3.3.1.1}COR^{3.3.1.2},SOR^{3.3.1.1}, SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het der gegebenenfalls substituiert ist mit einem oder mehreren Resten ausgewählt aus der Gruppe bestehend aus Methyl, SO₂H, SO₂Me, SO₂CH₂Phenyl; 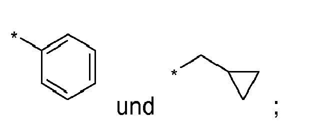 R^{3.3.1.1}, R^{3.3.1.2} und R^{3.3.1.3} ein Rest ausgewählt aus der Gruppe bestehend aus C₁-₄-Alkyl, C₇₋₁₁-Aralkyl, C₁₋₄-Alkyl-Hetaryl, COC₁₋₄-Alkyl-Hetaryl;
- **R^{a}**: H, Methyl, Ethyl, Propyl, Butyl, 3-Methyl-butyl, Propenyl, Cyclopropyl, Cyclohexyl, CF₃, COR⁸, NR⁹R¹⁰, NO₂, OR⁸, SR¹¹, SOR¹¹, SO₂R¹¹ oder ein Rest ausgewählt aus der Gruppe bestehend aus 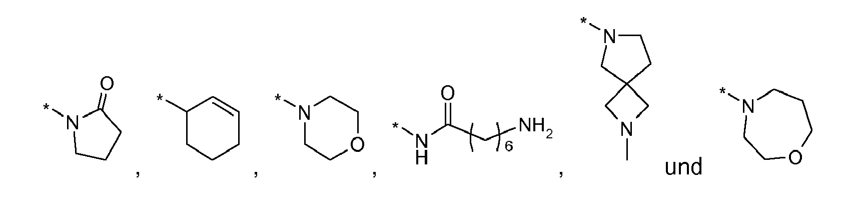 oder ein Rest ausgewählt aus der Gruppe bestehend aus 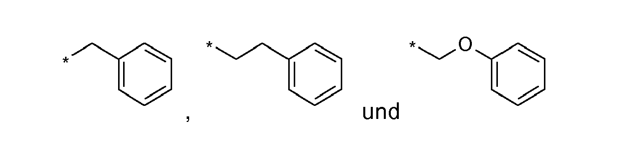 der gegebenenfalls substituiert sein kann mit einem oder mehreren CI,
oder ein Rest ausgewählt aus der Gruppe bestehend aus 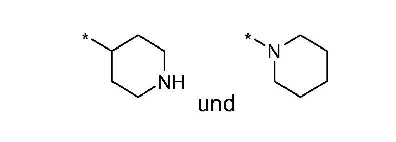 der gegebenenfalls substituiert sein kann mit einem oder mehreren CH₃, COCF₃, CH₂NH₂ oder CH₂NHCOOR¹²;
- R⁸: Methyl, Propyl, Cyclopropyl, NH₂, Furanyl oder Phenyl, gegebenenfalls substituiert mit einem oder mehreren Chlor;
- R⁹: H, COOR¹² oder Piperidino, gegebenenfalls substituiert mit einem oder mehreren CH₃, oder ein Rest ausgewählt aus der Gruppe bestehend aus Methyl, Ethyl und Propyl, der gegebenenfalls substituiert sein kann mit einem oder mehreren COOH, NMe₂ oder 4-Methylpiperazin;
- R¹⁰: H, Methyl, COCH₃, C≡CH oder CH₂C≡CH;
- R¹¹: Ethyl oder Propyl, gegebenenfalls substituiert mit einem oder mehreren NMe₂,
- R¹²: Butyl
- **R^{b}**: R⁴, CH₂OR⁴, COR⁴, COOR⁴, CONR⁴R⁵, NH₂, NHCOOR⁴, NHCONR⁴R⁵ oder OH;
- R⁴: H, Methyl, Ethyl, 2-Hydroxyethyl, Propyl, C≡CH, CF₃, Phenyl, 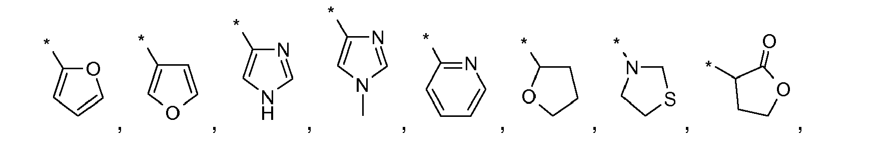 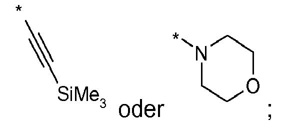
- R⁵: H, Methyl oder Ethyl;
- **R^{c}**: NHR⁶ oder ein Rest ausgewählt aus der Gruppe bestehend aus 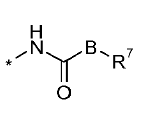 worin
B Bindung, Methylen, Ethylen, Propylen oder Butinylen;
R⁶ H, C₁₋₄-Alkyl, Aryl, gegebenenfalls substituiert mit SO₂CH₃;
R⁷ H, NR^{7.1}R^{7.2}, OR^{7.2}, SR^{7.2} oder ein Rest ausgewählt aus der Gruppe bestehend aus 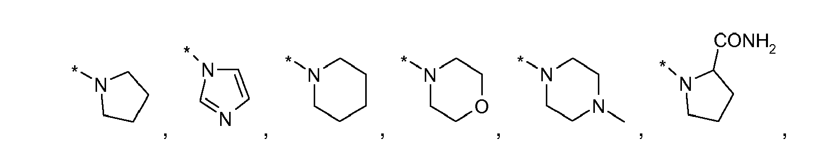 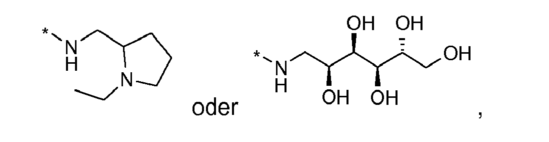 R^{7.1} H, Methyl, Ethyl, (CH₂)₂R^{7.1.1} oder COOButyl;
R^{7.2} H, Methyl oder Ethyl;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Bevorzugt sind die oben genannten Verbindungen der Formel 1; worin
- **R^{a}**: ein Rest ausgewählt aus der Gruppe bestehend aus Aryl, C₇₋₁₁-Aralkyl und Hetaryl, der gegebenenfalls substituiert durch einen oder mehrere Reste ausgewählt aus der Gruppe bestehend aus R¹, R² und R³ sein kann;
- R¹ und R²: unabhängig voneinander C₁₋₆-Alkyl, C₂₋₆-Alkenyl C₂₋₆-Alkinyl, C₃₋₆-Cycloalkyl, C₃₋₆-Cycloalkenyl, C₁₋₆-Haloalkyl, C₁₋₆-Alkylen-COOH, C₁₋₆-Alkoxy, Halogen, OH, CN, COR^{1.1}, O-C₁₋₄-Haloalkyl, NO₂ oder SR^{1.1}, SOR^{1.1}, SO2R^{1.1}, Het oder Hetaryl,
R^{1.1} OH, C₁₋₆-Alkyl, C₂₋₆-Alkenyl C₂₋₆-Alkinyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 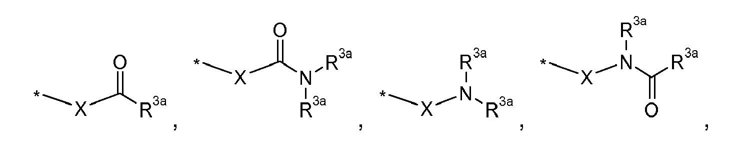 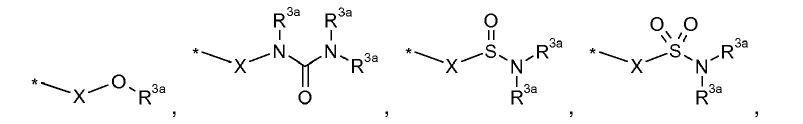 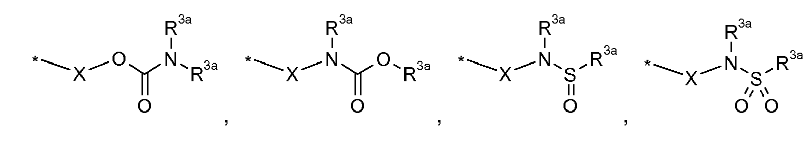 oder 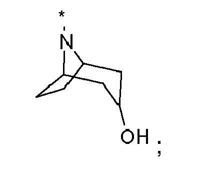 worin
X Bindung oder C₁₋₄-alkylen;
R^{3a} ein Rest, gleich oder verschieden, ausgewählt aus der Gruppe bestehend aus R^{3.1} , R^{3.2} und R^{3.3};
R^{3.1} Spiro oder Het, wobei Het gegebenenfalls substituiert mit einem oder mehreren R^{3.1.1} sein kann;
R^{3.1.1} C₁₋₆-Alkyl, C₂₋₆-Alkenyl, OH, C₁₋₄-Alkylen-OH, C₁₋₄-Alkylen-NR^{3.1.1.1}R^{3.1.1.2}, COR^{3.1.1.1} COOR^{3.1.1.1}, CONR^{3.1.1.1}R^{3.1.1.2}, NR^{3.1.1.1}R^{3.1.1.2}, Het, Hetaryl, NHCOR^{3.1.1.1}
R^{3.1.1.1} ein Rest ausgewählt aus der Gruppe bestehend aus H, C₁₋₄-Alkyl, Aryl und C₇₋₁₁-Aralkyl; gegebenenfalls substituiert mit einem Rest ausgewählt aus der Gruppe Halogen, OH und CN;
R^{3.1.1.2} H C₁₋₄-Alkyl;
R^{3.2} ein Rest ausgewählt aus der Gruppe bestehend aus C₃₋₆-Cycloalkyl, Het, Hetaryl und Spiro der gegebenenfalls substituiert ist mit einem oder mehreren R^{3.2.1}
R^{3.2.1} C₁₋₆-Alkyl, C₃₋₆-Cycloalkyl, OH, NR^{3.2.1.1}R^{3.2.1.2}, NHCOR^{3.2.1.3} oder Het, gegebenenfalls substituiert mit einem oder mehreren Resten ausgewählt aus der Gruppe bestehend aus C₁₋₄-Alkyl, SO₂R^{3.2.1.1}, CH₂-C₃₋₆-Cycloalkyl und Aryl;
R^{3.2.1.1} H, C₁₋₄-Alkyl oder C₇₋₁₁- Aralkyl;
R^{3.2.1.2} H, C₁₋₄-Alkyl oder C₇₋₁₁- Aralkyl;
R^{3.2.1.2} Aryl, C₇₋₁₁-Aralkyl; oder C₁₋₆-alkyl, das gegebenenfalls substituiert ist mit einem oder zwei R^{3.2.2};
R^{3.2.2} C₁₋₆-Alkyl, C₂₋₆-Alkenyl, C₂₋₆-Alkinyl, COOR^{3.2.2.1}, CONR^{3.2.2.1}R^{3.2.2.2}, NR^{3.2.2.1}R^{3.2.2.2} NHCOR^{3.2.2.1}, C₁₋₆-Haloalkyl, CN, OR^{3.2.2.1}, SO₂R^{3.2.2.1}, C₃₋₆-Cycloalkyl, CO-Het, C₂₋₄-Alkinyl-Hetaryl, Guanidin oder ein Rest ausgewählt aus der Gruppe bestehend aus Het, Hetaryl und Aryl, der gegebenenfalls substituiert ist mit einem oder mehreren Resten ausgewählt aus der Gruppe Halogen, C₁₋₆-Alkyl, CONR^{3.2.2.1}R^{3.2.2.2}, OH, Imidazolidinon;
R^{3.2.2.1} H oder C₁₋₆-Alkyl, Aryl, C₇₋₁₁-Aralkyl
R^{3.2.2.2.} H oder C₁₋₆-Alkyl; oder Aryl, das gegebenenfalls substituiert ist mit einem oder zwei R^{3.2.3}
R^{3.2.3} ein Rest ausgewählt aus der Gruppe bestehend aus NH-C₁₋₆-alkyl-N(C₁₋₆-alkyl)₂ oder Het, wobei Het gegebenenfalls substituiert mit einem Rest C₁₋₆-Alkyl sein kann;
R^{3.3} H oder ein Rest ausgewählt aus der Gruppe bestehend aus C₁₋₆-Alkyl, C₂₋₆-Alkenyl, C₂₋₆-Alkinyl, C₁₋₆-Haloalkyl und Aryl, der gegebenenfalls substituiert sein kann mit einem oder mehreren Resten R^{3.3.1} ;
R^{3.3.1} C₃₋₆-Cycloalkyl, C₃₋₆-Cycloalkenyl, OR^{3.3.1.1}, NR^{3.3.1.1}R^{3.3.1.2}, CONR^{3.3.1.}1R^{3.3.1.2}, COOR^{3.3.1.1}, NR^{3.3.1.}1COR^{3.3.1.2}, SOR^{3.3.1.1} SO₂R^{3.3.1.1}, C(NR^{3.3.1.1}R^{3.3.1.2})NR^{3.3.1.3}, NR^{3.3.1.1}CONR^{3.3.1.2}R^{3.3.1.3} OH, CN, Halogen oder Het, gegebenenfalls substituiert mit einem oder mehreren Resten ausgewählt aus der Gruppe bestehend aus C₁₋₄-Alkyl, SO₂R^{3.2.1.1} , SO₂C₁₋₄-Alkyl, SO₂C₇₋₁₁-Aralkyl, CH₂-C₃₋₆-Cycloalkyl und Aryl;
R^{3.3.1.1} R^{3.3.1.2} und R^{3.3.1.3} ein Rest ausgewählt aus der Gruppe bestehend aus C₁₋₆-Alkyl, C₂₋₆-Alkenyl, C₂₋₆-Alkinyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, COC₁₋₄-Alkyl-Hetaryl, COC₂₋₄-Alkenyl-Hetaryl, COC₂₋₄-Alkinyl-Hetaryl; oder zwei der Gruppen R^{3.3.1.1} , R^{3.3.1.2} und R^{3.3.1.3} bilden gemeinsam einen Ring, bestehend aus Kohlenstoffatomen und gegebenenfalls einem Heteroatom ausgewählt aus der Gruppe bestehend aus Sauerstoff, Stickstoff und Schwefel;
**R^{b}** R⁴, OR⁴, -CH₂OR⁴, COR⁴, COOR⁴, CONR⁴R⁵, NR⁴R⁵, NR⁵COR⁴, NR⁵COOR⁴, NR⁵CONR⁴R⁵, NR⁵SOR⁴ oder NR⁵SO₂R⁴;
R⁴, R⁵ H, C₁₋₆-Alkyl, C₁₋₆-Haloalkyl, C₁₋₆-Alkylen-OH, C₂₋₆-Alkenyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl;
oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
- **R^{c}**: NHR⁶ oder ein Rest ausgewählt aus der Gruppe bestehend aus 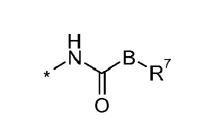 worin
B Bindung, C₁₋₆-Alkyl, C₂₋₆-Alkenyl oder C₂₋₆-Alkinyl;
R⁶ H oder ein Rest ausgewählt aus der Gruppe C₁₋₄-Alkyl, C₂₋₄-Alkenyl, C₂₋₄-Alkinyl, C₃₋₆-Cycloalkyl, C₃₋₆-Cycloalkenyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
R⁷ H, C₁₋₄-Alkyl, C₂₋₄-Alkenyl, C₂₋₄-Alkinyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₆-Alkyl, (CH₂)₂₋₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon.

Bevorzugt sind die oben genannten Verbindungen der Formel 1; worin
- **R^{a}**: ein Rest ausgewählt aus der Gruppe bestehend aus Aryl, C₇₋₁₁-Aralkyl und Hetaryl, der gegebenenfalls substituiert durch einen oder mehrere Reste ausgewählt aus der Gruppe bestehend aus R¹, R² und R³ sein kann;
- R¹ und R²: unabhängig voneinander C₁₋₆-Alkyl, C₂₋₆-Alkenyl C₂₋₆-Alkinyl, C₃₋₆-Cycloalkyl, C₃₋₆-Cycloalkenyl, C₁₋₆-Haloalkyl, C₁₋₆-Alkylen-COOH, C₁₋₆-Alkoxy, Halogen, OH, CN, COR^{1.1}, O-C₁₋₄-Haloalkyl, NO₂ oder SR^{1.1}, SOR^{1.1}, SO₂R^{1.1}, Het oder Hetaryl,
R^{1.1} OH, C₁₋₆-Alkyl, C₂₋₆-Alkenyl C₂₋₆-Alkinyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 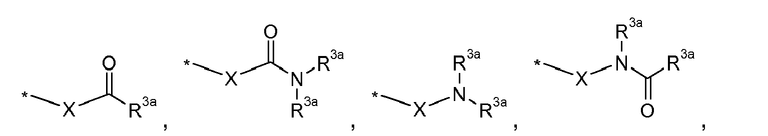 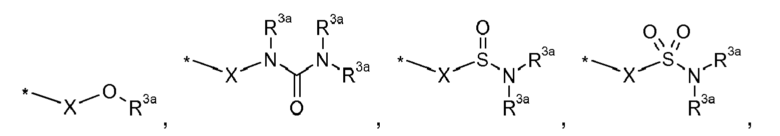 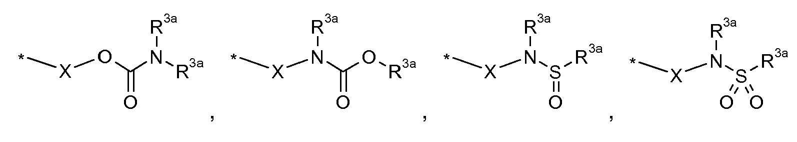 oder 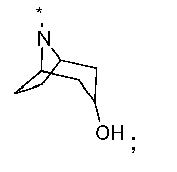 worin
X Bindung oder C₁₋₄-alkylen;
R^{3a} ein Rest, gleich oder verschieden, ausgewählt aus der Gruppe bestehend aus R^{3.1} , R^{3.2} und R^{3.3};
R^{3.1} Spiro oder Het, wobei Het gegebenenfalls substituiert mit einem oder mehreren R^{3.1.1} sein kann;
R^{3.1.1} C₁-₆-Alkyl, C₂₋₆-Alkenyl, OH, C₁₋₄-Alkylen-OH, C₁₋₄-Alkylen-NR^{3.1.1.1}R^{3.1.1.2}, COR^{3.1.1.1}, COOR^{3.1.1.1} , CONR^{3.1.1.1}R^{3.1.1.2}, NR^{3.1.1.1}R^{3.1.1.2}, Het, Hetaryl, NHCOR^{3.1.1.1}
R^{3.1.1.1} ein Rest ausgewählt aus der Gruppe bestehend aus H, C₁₋₄-Alkyl, Aryl und C₇₋₁₁-Aralkyl; gegebenenfalls substituiert mit einem Rest ausgewählt aus der Gruppe Halogen, OH und CN;
R^{3.1.1.2} H C₁-₄-Alkyl;
R^{3.2} ein Rest ausgewählt aus der Gruppe bestehend aus C₃₋₆-Cycloalkyl, Het, Hetaryl und Spiro der gegebenenfalls substituiert ist mit einem oder mehreren R^{3.2.1}
R^{3.2.1} C₁₋₆-Alkyl, C₃₋₆-Cycloalkyl, OH, -NR^{3.2.1.1}R^{3.2.1.2}, NHCOR^{3.2.1.3} oder Het, gegebenenfalls substituiert mit einem oder mehreren Resten ausgewählt aus der Gruppe bestehend aus C₁₋₄-Alkyl, SO₂R^{3.2.1.1}, CH₂-C₃₋₆-Cycloalkyl und Aryl;
R^{3.2.1.1} H, C₁₋₄-Alkyl oder C₇₋₁₁- Aralkyl;
R^{3.2.1.2} H, C₁₋₄-Alkyl oder C₇₋₁₁- Aralkyl;
R^{3.2.1.2} Aryl, C₇₋₁₁Aralkyl; oder -C₁₋₆-alkyl, das gegebenenfalls substituiert ist mit einem oder zwei R^{3.2.2};
R^{3.2.2} C₁₋₆-Alkyl, C₂₋₆-Alkenyl, C₂₋₆-Alkinyl, COOR^{3.2.2.1}, CONR^{3.2.2.1}R^{3.2.2.2}, NR^{3.2.2.1}R^{3.2.2.2}, NHCOR^{3.2.2.1}, C₁₋₆-Haloalkyl, CN, OR^{3.2.2.1}, SO₂R^{3.2.2.1}, C₃₋₆-Cycloalkyl, CO-Het, C₂₋₄-Alkinyl-Hetaryl, Guanidin oder ein Rest ausgewählt aus der Gruppe bestehend aus Het, Hetaryl und Aryl, der gegebenenfalls substituiert ist mit einem oder mehreren Resten ausgewählt aus der Gruppe Halogen, C₁₋₆-Alkyl, CONR^{3.2.2.1}R^{3.2.2.2}, OH, Imidazolidinon;
R^{3.2.2.1} H oder C₁₋₆-Alkyl, Aryl, C₇₋₁₁-Aralkyl
R^{3.2.2.2.} H oder C₁₋₆-Alkyl; oder Aryl, das gegebenenfalls substituiert ist mit einem oder zwei R^{3.2.3}
R^{3.2.3} ein Rest ausgewählt aus der Gruppe bestehend aus NH-C₁₋₆-alkyl-N(C₁₋₆-alkyl)₂ oder Het, wobei Het gegebenenfalls substituiert mit einem Rest C₁₋₆-Alkyl sein kann;
R^{3.3} H oder ein Rest ausgewählt aus der Gruppe bestehend aus C₁₋₆-Alkyl, C₂₋₄-Alkenyl, C₂₋₄-Alkinyl, C₁₋₄-Haloalkyl und Aryl, der gegebenenfalls substituiert sein kann mit einem oder mehreren Resten R^{3.3.1};
R^{3.3.1} C₅₋₆-Cycloalkyl, C₅₋₆-Cycloalkenyl, OR^{3.3.1.1}, NR^{3.3.1.1}R^{3.3.1.2}, CONR^{3.3.1.1}R^{3.3.1.2}, COOR^{3.3.1.1}, NR^{3.3.1.1}COR^{3.3.1.2}SOR^{3.3.1.1}, SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het, Gegebenenfalls substituiert mit einem oder mehreren Resten ausgewählt aus der Gruppe bestehend aus C₁₋₄-Alkyl, SO₂R^{3.2.1.1}, SO₂C₁₋₄-Alkyl, SO₂C₇₋₁₁- Aralkyl, CH₂-C₃₋₆-Cycloalkyl und Aryl;
R^{3.3.1.1}, R^{3.3.1.2} und R^{3.3.1.3} ein Rest ausgewählt aus der Gruppe bestehend aus C₁₋₄-Alkyl, C₂₋₄-Alkenyl, C₂₋₄-Alkinyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, COC₁₋₄-Alkyl-Hetaryl, COC₂₋₄-Alkenyl-Hetaryl, COC₂₋₄-Alkinyl-Hetaryl; oder zwei der Gruppen R^{3.3.1.1}, R^{3.3.1.2} und R^{3.3.1.3} bilden gemeinsam einen fünf-, sechs oder siebengliedrigen Ring, bestehend aus Kohlenstoffatomen und gegebenenfalls einem Heteroatom ausgewählt aus der Gruppe bestehend aus Sauerstoff, Stickstoff und Schwefel;
- **R^{b}**: R⁴, OR⁴, -CH₂OR⁴, COR⁴, COOR⁴, CONR⁴R⁵, NR⁴R⁵, NR⁵COR⁴, NR⁵COOR⁴, NR⁵CONR⁴R⁵, NR⁵SOR⁴ oder NR⁵SO₂R⁴;
- R⁴: H, C₁₋₆-Alkyl, C₁₋₆-Haloalkyl, C₁₋₆-Alkylen-OH, C₂₋₆-Alkenyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl;
- R⁵: H oder C₁₋₆-Alkyl;
oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
- **R^{c}**: NHR⁶ oder ein Rest ausgewählt aus der Gruppe bestehend aus 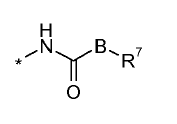 worin
B Bindung, C₁₋₄-Alkyl oder C₂₋₄-Alkinyl;
R⁶ H oder ein Rest ausgewählt aus der Gruppe C₁₋₄-Alkyl, C₂₋₄-Alkenyl, C₂₋₄-Alkinyl, C₃₋₆-Cycloalkyl, C₃₋₆-Cycloalkenyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
R⁷ H, C₁₋₄-Alkyl, C₂₋₄-Alkenyl, C₂₋₄-Alkinyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₄-Alkyl, (CH₂)₂₋₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon.

Bevorzugt sind die oben genannten Verbindungen der Formel 1; worin
- **R^{a}**: ein Rest ausgewählt aus der Gruppe bestehend aus Aryl, C₇₋₁₁-Aralkyl und Hetaryl, der gegebenenfalls substituiert durch einen oder mehrere Reste ausgewählt aus der Gruppe bestehend aus R¹, R² und R³ sein kann;
- R¹ und R²: unabhängig voneinander C₁₋₆-Alkyl, C₂₋₆-Alkenyl C₂₋₆-Alkinyl, C₃₋₆-Cycloalkyl, C₃₋₆-Cycloalkenyl, C₁₋₆-Haloalkyl, C₁₋₆-Alkylen-COOH, C₁₋₆-Alkoxy, Halogen, OH, CN, COR^{1.1}, O-C₁₋₄-Haloalkyl, NO₂ oder SR^{1.1}, SOR^{1.1}, SO₂R^{1.1}, Het oder Hetaryl,
R^{1.1} OH, C₁₋₆-Alkyl, C₂₋₆-Alkenyl C₂₋₆-Alkinyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 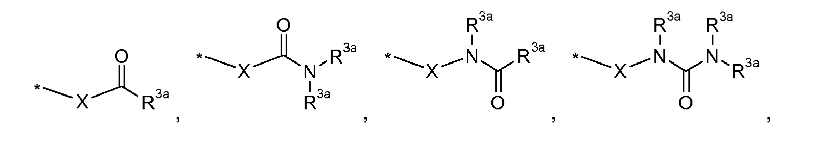 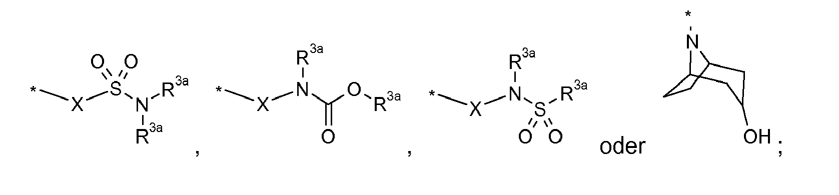 worin
X Bindung oder C₁₋₄-alkylen;
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} C₁₋₆-Alkyl, C₂₋₆-Alkenyl, OH, C₁₋₄-Alkylen-OH, C₁₋₄-Alkylen-NR^{3.1.1.1}R^{3.1.1.2}, COR^{3.1.1.1}, COOR^{3.1.1.1}, CONR^{3.1.1.1}R^{3.1.1.2}, NR^{3.1.1.1}R^{3.1.1.2}, Het, Hetaryl, NHCOR^{3.1.1.1}
R^{3.1.1.1} ein Rest ausgewählt aus der Gruppe bestehend aus H, C₁₋₄-Alkyl, Aryl und C₇₋₁₁-Aralkyl; gegebenenfalls substituiert mit einem Rest ausgewählt aus der Gruppe Halogen, OH und CN;
R^{3.1.1.2} H, C₁₋₄-Alkyl;
R^{3.2} ein Rest ausgewählt aus der Gruppe bestehend aus C₃₋₆-Cycloalkyl, Het, Hetaryl und Spiro der gegebenenfalls substituiert ist mit einem oder mehreren R^{3.2.1}
R^{3.2.1} C₁₋₆-Alkyl, C₃₋₆-Cycloalkyl, OH, -NR^{3.2.1.1}R^{3.2.1.2}, NHCOR^{3.2.1.3} oder Het, gegebenenfalls substituiert mit einem oder mehreren Resten ausgewählt aus der Gruppe bestehend aus C₁₋₄-Alkyl, SO₂R^{3.2.1.1}, CH₂-C₃₋₆-Cycloalkyl und Aryl;
R^{3.2.1.1} H, C₁₋₄-Alkyl oder C₇₋₁₁- Aralkyl;
R^{3.2.1.2} H, C₁₋₄-Alkyl oder C₇₋₁₁- Aralkyl;
R^{3.2.1.2} Aryl, C₇₋₁₁-Aralkyl; oder -C₁₋₆-alkyl, das gegebenenfalls substituiert ist mit einem oder zwei R^{3.2.2};
R^{3.2.2} C₁₋₆-Alkyl, C₂₋₆-Alkenyl, C₂₋₆-Alkinyl, COOR^{3.2.2.1}, CONR^{3.2.2.1}R^{3.2.2.2}, NR^{3.2.2.1}R^{3.2.2.2} NHCOR^{3.2.2.1}, C₁₋₆-Haloalkyl, CN, OR^{3.2.2.1}, SO₂R^{3.2.2.1}, C₃₋₆-Cycloalkyl, CO-Het, C₂₋₄-Alkinyl-Hetaryl, Guanidin oder ein Rest ausgewählt aus der Gruppe bestehend aus Het, Hetaryl und Aryl, der gegebenenfalls substituiert ist mit einem oder mehreren Resten ausgewählt aus der Gruppe Halogen, C₁₋₆-Alkyl, CONR^{3.2.2.1}R^{3.2.2.2}, OH, Imidazolidinon;
R^{3.2.2.1} H oder C₁₋₆-Alkyl, Aryl, C₇₋₁₁-Aralkyl
R^{3.2.2.2.} H oder C₁₋₆-Alkyl; oder Aryl, das gegebenenfalls substituiert ist mit einem oder zwei R^{3.2.3}
R^{3.2.3} ein Rest ausgewählt aus der Gruppe bestehend aus NH-C₁₋₆-alkyl-N(C₁₋₆-alkyl)₂ oder Het, wobei Het gegebenenfalls substituiert mit einem Rest C₁₋₆-Alkyl sein kann;
R^{3.3} H oder ein Rest ausgewählt aus der Gruppe bestehend aus C₁₋₆-Alkyl, C₂₋₄-Alkenyl, C₂₋₄-Alkinyl und Aryl, der gegebenenfalls substituiert sein kann mit einem oder mehreren Resten R^{3.3.1};
R^{3.3.1} C₅₋₆-Cycloalkyl, C₅₋₆-Cycloalkenyl, OR^{3.3.1.1}, NR^{3.3.1.1}R^{3.3.1.2}, CONR^{3.3.1.1}R^{3.3.1.2},COOR^{3.3.1.1}, NR^{3.3.1.1}COR^{3.3.1.2},SOR^{3.3.1.1}, SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het, Gegebenenfalls substituiert mit einem oder mehreren Resten ausgewählt aus der Gruppe bestehend aus C₁₋₄-Alkyl, SO₂R^{3.2.1.1}, SO₂C₁₋₄-Alkyl, SO₂C₇₋₁₁-Aralkyl, CH₂-C₃₋₆-Cycloalkyl und Aryl;;
R^{3.3.1.1}, R^{3.3.1.2} und R^{3.3.1.3} ein Rest ausgewählt aus der Gruppe bestehend aus C₁₋₄-Alkyl, C₇₋₁₁-Aralkyl, C₁₋₄-Alkyl-Hetaryl, COC₁₋₄-Alkyl-Hetaryl; oder zwei der Gruppen R^{3.3.1.1}, R^{3.3.1.2} und R^{3.3.1.3} bilden gemeinsam einen fünf-, sechs oder siebengliedrigen Ring, bestehend aus Kohlenstoffatomen und gegebenenfalls einem Heteroatom ausgewählt aus der Gruppe bestehend aus Sauerstoff, Stickstoff und Schwefel;
- **R^{b}**: R⁴, OR⁴, -CH₂OR⁴, COR⁴, COOR⁴, CONR⁴R⁵, NH₂, NR⁵COOR⁴, NR⁵CONR⁴R⁵ oder NR⁵SOR⁴;
- R⁴: H, C₁₋₆-Alkyl, C₁₋₆-Haloalkyl, C₁₋₆-Alkylen-OH, C₂₋₆-Alkenyl, C₇₋₁₁-Aralkyl, C₁₋₄-Alkyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl;
- R⁵: H oder C₁₋₆-Alkyl;
oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
- **R^{c}**: NHR⁶ oder ein Rest ausgewählt aus der Gruppe bestehend aus 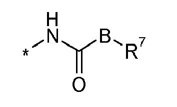 worin
- B: Bindung, C₁₋₄-Alkyl oder C₂₋₄-Alkinyl;
- R⁶: H oder ein Rest ausgewählt aus der Gruppe C₁₋₄-Alkyl, C₃₋₆-Cycloalkyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
- R⁷: H, C₁₋₄-Alkyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₄-Alkyl, (CH₂)₂₋₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon.

Bevorzugt sind die oben genannten Verbindungen der Formel 1; worin
- **R^{a}**: ein Rest ausgewählt aus der Gruppe bestehend aus Aryl und C₇₋₁₁-Aralkyl, der gegebenenfalls substituiert durch einen oder mehrere Reste ausgewählt aus der Gruppe bestehend aus R¹, R² und R³ sein kann; oder Hetaryl gegebenenfalls substituiert durch ein oder mehrere C₁₋₄-Alkyl;
- R¹: C₁₋₄-Alkyl, C₁₋₄-Haloalkyl, C₁₋₄-Alkylen-COOH, C₁₋₄-Alkoxy, Halogen, OH, CN, COR^{1.1}, O-C₁₋₄-Haloalkyl, NO₂ oder SO₂R^{1.1};
R^{1.1} OH, Methyl, NH₂, NHMe, NMe₂, 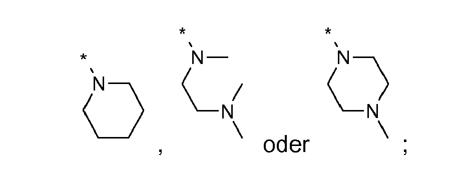
- R²: C₁₋₄-Alkyl, C₁₋₄-Alkoxy oder Halogen;
- R³: ein Rest ausgewählt aus der Gruppe bestehend aus 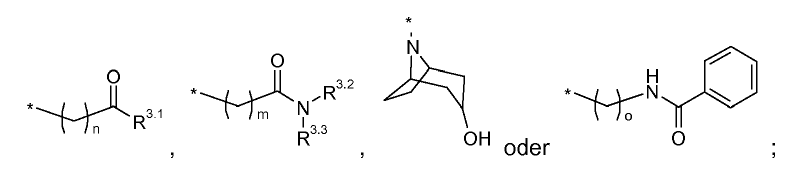 worin
n 0 oder 1;
m 0 oder 1;
o 2;
R^{3.1} Spiro oder Het, wobei Het gegebenenfalls substituiert mit einem oder mehreren R^{3.1.1} sein kann;
R^{3.1.1} C₁₋₄-Alkyl, C₂₋₄-Alkenyl, OH, C₁₋₄-Alkylen-OH, CH₂NEt₂, COMe, COOH, CONH₂, NH₂, Het, Hetaryl, 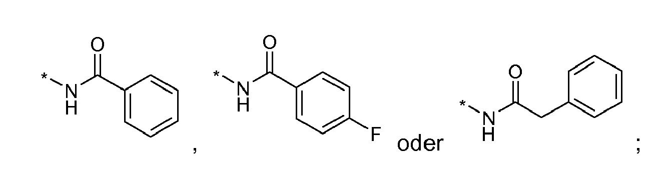 R^{3.2} ein Rest ausgewählt aus der Gruppe bestehend aus C₃₋₆-Cycloalkyl, Het, Hetaryl und Spiro der gegebenenfalls substituiert ist mit einem oder zwei R^{3.2.1}
R^{3.2.1} C₁₋₄-Alkyl, Cyclopentyl, OH, -NR^{3.2.1.1}R^{3.2.1.2} oder 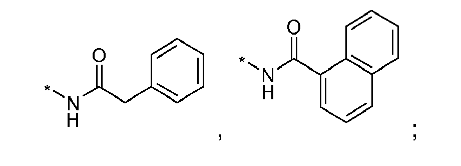 oder Het, gegebenenfalls substituiert ist mit einem oder mehreren Resten ausgewählt aus der Gruppe bestehend aus Methyl, SO₂R^{3.2.1.1}, 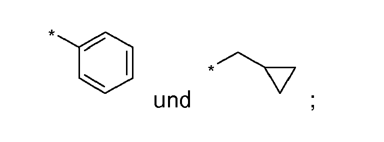 R^{3.2.1.1} H, Methyl oder Benzyl;
R^{3.2.1.2} H, Methyl oder Benzyl; oder -C₁₋₆-alkyl, das gegebenenfalls substituiert ist mit einem oder zwei R^{3.2.2};
R^{3.2.2} C₂₋₄-Alkenyl, C₂₋₄-Alkinyl, COOR^{3.2.2.1}, CONR^{3.2.2.1}R^{3.2.2.2}, NR^{3.2.2.1}R^{3.2.2.2}, NHCOR^{3.2.2.1}, C₁₋₄-Haloalkyl, CN, OH, SO₂R^{3.2.2.1}, C₃₋₆-Cycloalkyl oder ein Rest ausgewählt aus der Gruppe bestehend aus 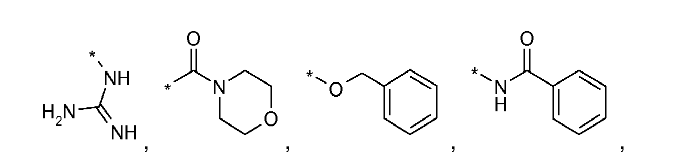 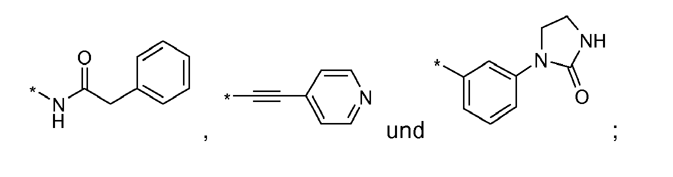 oder ein Rest ausgewählt aus der Gruppe bestehend aus Het, Hetaryl und Aryl, der gegebenenfalls substituiert ist mit einem oder mehreren Resten ausgewählt aus der Gruppe Cl, Methyl, CONR^{3.2.2.1}R^{3.2.2.2}, OH;
R^{3.2.2.1} H oder Methyl;
R^{3.2.2.2.} H oder Methyl; oder Aryl, das gegebenenfalls substituiert ist mit einem oder zwei R^{3.2.3}
R^{3.2.3} ein Rest ausgewählt aus der Gruppe bestehend aus 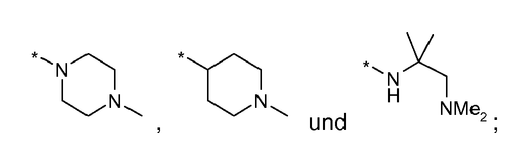 R^{3.3} H oder ein Rest ausgewählt aus der Gruppe bestehend aus C₁₋₆-Alkyl und Aryl, der gegebenenfalls substituiert sein kann mit einem oder mehreren Resten R^{3.3.1};
R^{3.3.1} C₅₋₆-Cycloalkyl, C₅₋₆-Cycloalkenyl, OR^{3.3.1.1}, NR^{3.3.1.1}R^{3.3.1.2}, CONR^{3.3.1.1}R^{3.3.1.2}, COOR^{3.3.1.1}, NR^{3.3.1.1}COR^{3.3.1.2},SOR^{3.3.1.1}, SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het, Gegebenenfalls substituiert mit einem oder mehreren Resten ausgewählt aus der Gruppe bestehend aus C₁₋₄-Alkyl, SO₂R^{3.2.1.1}, SO₂C₁₋₄-Alkyl, SO₂C₇₋₁₁-Aralkyl, 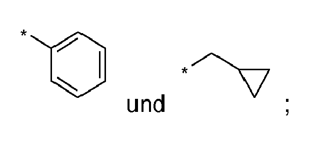 R^{3.3.1.1}, R^{3.3.1.2} und R^{3.3.1.3} ein Rest ausgewählt aus der Gruppe bestehend aus C₁₋₄-Alkyl, C₇₋₁₁-Aralkyl, C₁₋₄-Alkyl-Hetaryl, COC₁₋₄-Alkyl-Hetaryl;
- **R^{b}**: R⁴, CH₂O_{R}⁴, COR⁴, COOR⁴, CONR⁴R⁵, NH₂, NHCOOR⁴, NHCONR⁴R⁵ oder OH;
- R⁴: H, C₁₋₄-Alkyl, C₁₋₄-Alkylen-OH, C₂₋₄-Alkinyl, C₁₋₆-Haloalkyl, Aryl, Het, Hetaryl, 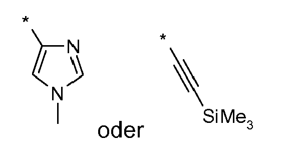
- R⁵: H oder C₁₋₄-Alkyl;
- **R^{c}**: NHR⁶ oder ein Rest ausgewählt aus der Gruppe bestehend aus 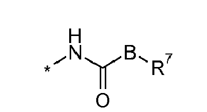 worin
B Bindung, C₁₋₄-Alkyl oder C₂₋₄-Alkinyl;
R⁶ H, C₁₋₄-Alkyl, C₃₋₆-Cycloalkyl, C₇₋₁₁-Aralkyl, Aryl, gegebenenfalls substituiert mit SO₂CH₃;
R⁷ H, NR^{7.1}R^{7.2}, OR^{7.2}, SR^{7.2}, Hetaryl, Het, gegebenenfalls substituiert mit C₁₋₄-Alkyl oder CONH₂, oder ein Rest ausgewählt aus der Gruppe bestehend aus 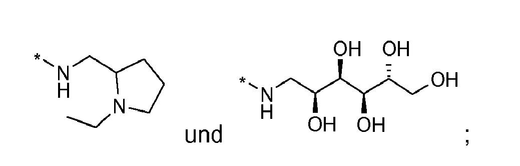 R^{7.1} H, C₁₋₄-Alkyl, (CH₂)₂R^{7.1.1} oder COOButyl;
R^{7.2} H, C₁₋₄-Alkyl;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon.

Besonders bevorzugt sind die oben genannten Verbindungen der Formel 1; worin
- **R^{a}**: Phenyl oder Benzyl, jeweils gegebenenfalls substituiert durch einen oder mehrere Reste ausgewählt aus der Gruppe bestehend aus R¹, R² und R³; oder 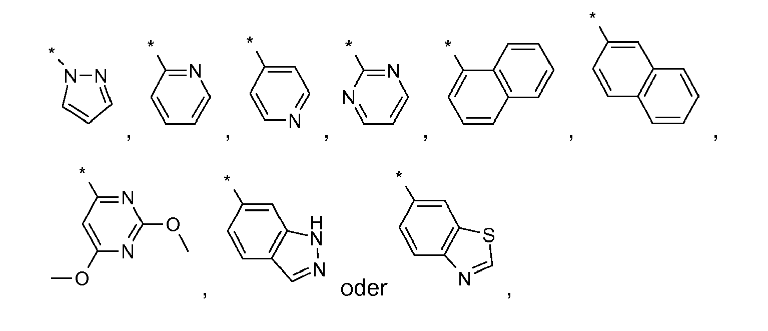
- R¹: Methyl, Ethyl, Propyl, Butyl, CF₃, CH₂COOH, Methoxy, F, Cl, Br, OH, CN, COR^{1.1}, OCF₃, NO₂ oder SO₂R^{1.1};
R^{1.1} OH, Methyl, NH₂, NHMe, NMe₂, 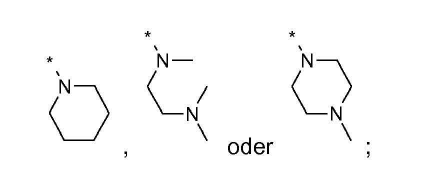
- R²: Methyl, Methoxy, F, Cl oder Br;
- R³: ein Rest ausgewählt aus der Gruppe bestehend aus 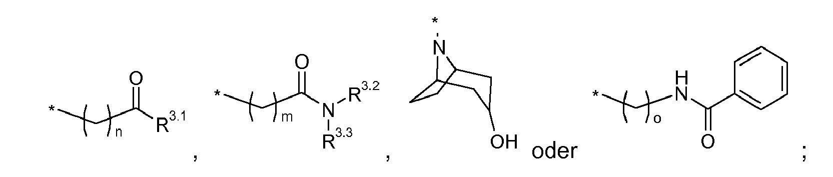 worin
n 0 oder 1;
m 0 oder 1;
o 2;
R^{3.1} ein Rest ausgewählt aus der Gruppe bestehend aus 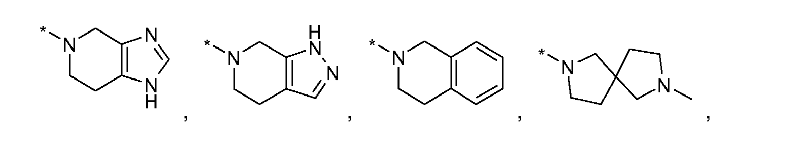 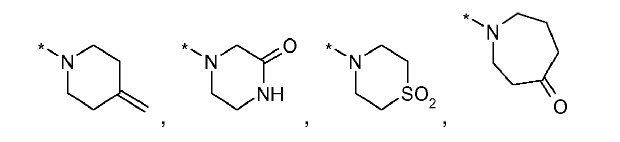 und 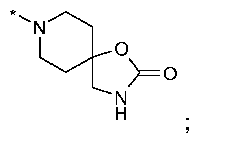 oder ein Rest ausgewählt aus der Gruppe bestehend aus 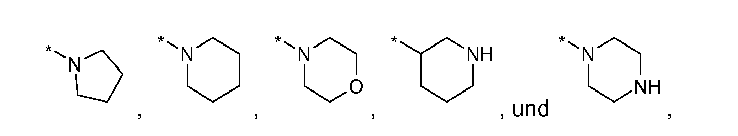 der gegebenenfalls substituiert sein kann mit einem oder mehreren R^{3.1.1};
R^{3.1.1} Methyl, Ethyl, OH, CH₂OH, CH₂CH₂OH, CH₂NEt₂, COMe, COOH, CONH₂, NH₂, 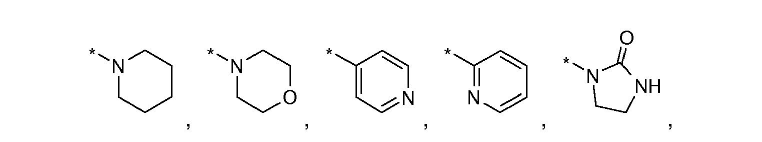 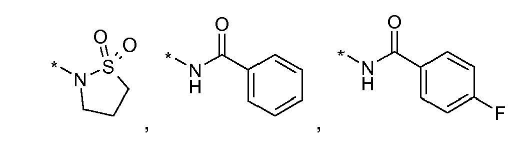 oder 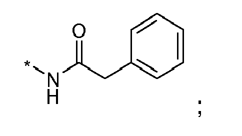 R^{3.2} ein Rest ausgewählt aus der Gruppe bestehend aus 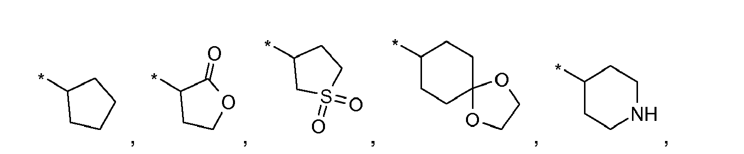 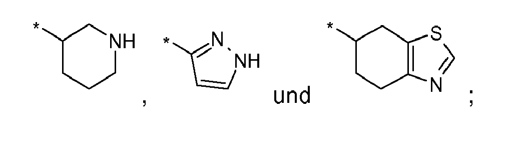 der gegebenenfalls substituiert ist mit einem oder mehreren Resten ausgewählt aus der Gruppe bestehend aus Methyl, Ethyl, Cyclopentyl, OH, NH₂; oder
Cyclohexyl, das gegebenenfalls substituiert ist mit einem oder zwei R^{3.2.1}
R^{3.2.1} -NR^{3.2.1.1}R^{3.2.1.2} oder ein Rest ausgewählt aus der Gruppe bestehend aus 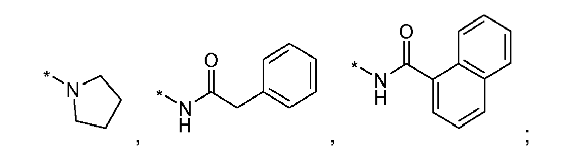 oder ein Rest ausgewählt aus der Gruppe bestehend aus 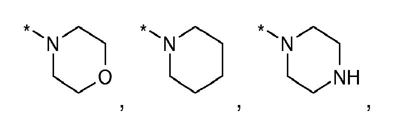 der gegebenenfalls substituiert ist mit einem oder mehreren Resten ausgewählt aus der Gruppe bestehend aus Methyl, SO₂R^{3.2.1.1}, 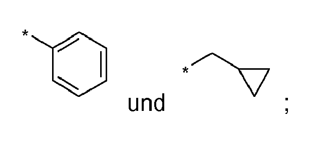 R^{3.2.1.1} H, Methyl oder Benzyl;
R^{3.2.1.2} H, Methyl oder Benzyl; oder -C₁₋₆-alkyl, gerade oder verzweigt, das gegebenenfalls substituiert ist mit einem oder zwei R^{3.2.2};
R^{3.2.2} C=CH₂, C=CH, COOR^{3.2.2.1}, CONR^{3.2.2.1}R^{3.2.2.2}, NR^{3.2.2.1}R^{3.2.2.2}, NHCOR^{3.2.2.1}, CF₃, CN, OH, SO₂R^{3.2.2.1} oder ein Rest ausgewählt aus der Gruppe bestehend aus 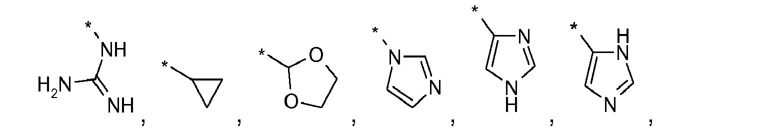 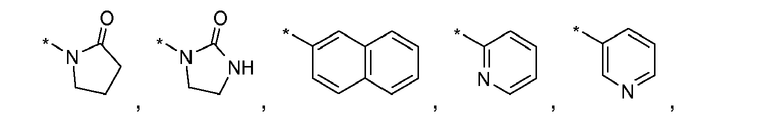 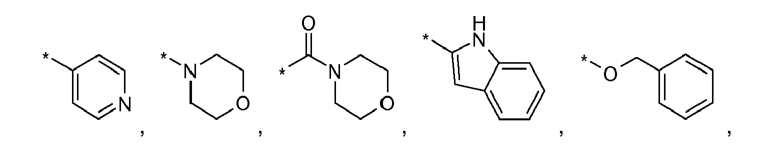 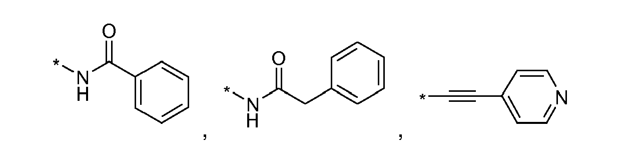 und 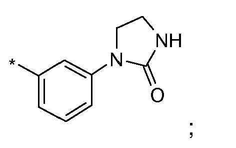 oder ein Rest ausgewählt aus der Gruppe bestehend aus 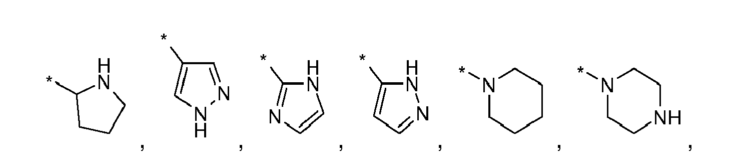 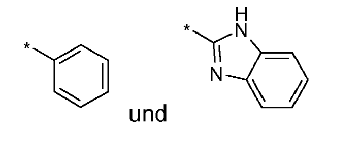 der gegebenenfalls substituiert ist mit einem oder mehreren Resten ausgewählt aus der Gruppe Cl, Methyl, CONR^{3.2.2.1}R^{3.2.2.2}, OH;
R^{3.2.2.1} H oder Methyl;
R^{3.2.2.2.} H oder Methyl; oder Phenyl, das gegebenenfalls substituiert ist mit einem oder zwei R^{3.2.3}
R^{3.2.3} ein Rest ausgewählt aus der Gruppe bestehend aus 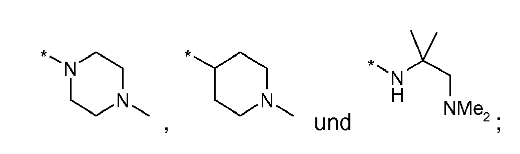 R^{3.3} H, Methyl oder Ethyl;
- **R^{b}**: R⁴, CH₂OR⁴, COR⁴, COOR⁴, CONR⁴R⁵, NH₂, NHCOOR⁴, NHCONR⁴R⁵ oder OH;
- R⁴: H, Methyl, Ethyl, 2-Hydroxyethyl, Propyl, C≡CH, CF₃, Phenyl, 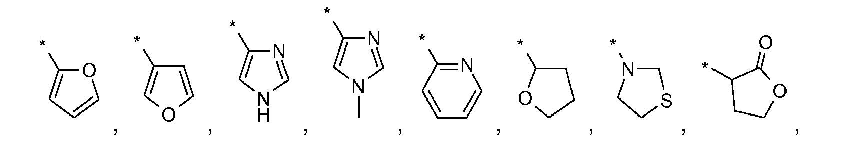 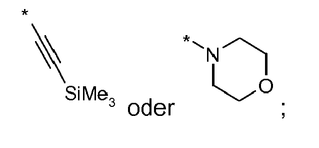
- R⁵: H, Methyl oder Ethyl;
- **R^{c}**: NH₂ oder ein Rest ausgewählt aus der Gruppe bestehend aus 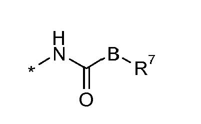 worin
B Bindung, Methylen, Ethylen, Propylen oder Butinylen;
R⁶ H, Phenyl, gegebenenfalls substituiert mit SO₂CH₃;
R⁷ H, NR^{7.1}R^{7.2}, OR^{7.2}, SR^{7.2} oder ein Rest ausgewählt aus der Gruppe bestehend aus 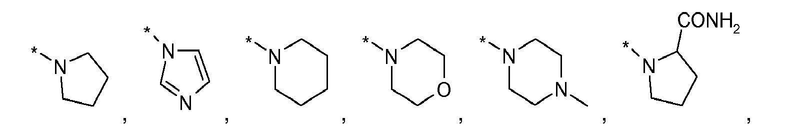 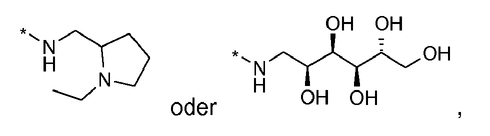 R^{7.1} H, Methyl, Ethyl, (CH₂)₂R^{7.1.1} oder COOButyl;
R^{7.2} H, Methyl oder Ethyl;
R^{7.1.1} NMe₂ oder 1-Imidazolyl
bedeuten, sowie pharmakologisch verträgliche Salz, Diastereomere, Enantiomere, Racemate, Hydrate oder Solvate davon.

Besonders bevorzugt sind die oben genannten Verbindungen der Formel 1; worin
- **R^{c}**: ein Rest 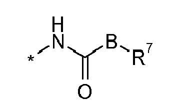 worin
B Methylen, Propylen;
R⁷ H, NR^{7.1}R^{7.2} oder 1-Imidazolyl;
R^{7.1} H oder Methyl;
R^{7.2} H oder Methyl;
bedeuten, sowie pharmakologisch verträgliche Salz, Diastereomere, Enantiomere, Racemate, Hydrate oder Solvate davon.

Besonders bevorzugt sind die oben genannten Verbindungen der Formel **1**; worin
- **R^{a}**: Phenyl, gegebenenfalls substituiert durch einen oder mehrere Reste ausgewählt aus der Gruppe bestehend aus R¹, R² und R³;
bedeuten, sowie pharmakologisch verträgliche Salz, Diastereomere, Enantiomere, Racemate, Hydrate oder Solvate davon.

Besonders bevorzugt sind die oben genannten Verbindungen der Formel 1; worin
- **R^{a}**: Phenyl, gegebenenfalls substituiert durch einen oder mehrere Reste ausgewählt aus der Gruppe bestehend aus R¹ und R³;
- R¹: Methyl, Ethyl, Propyl, CF₃, Methoxy, F, Cl oder Br;
- R³: ein Rest 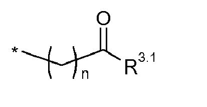
bedeuten, sowie pharmakologisch verträgliche Salz, Diastereomere, Enantiomere, Racemate, Hydrate oder Solvate davon.

Besonders bevorzugt sind die oben genannten Verbindungen der Formel 1; worin
- **R^{a}**: Phenyl, gegebenenfalls substituiert durch einen oder mehrere Reste ausgewählt aus der Gruppe bestehend aus R¹ und R³;
- R¹: Methyl, Ethyl, Propyl, CF₃, Methoxy, F, Cl oder Br;
- R³: ein Rest 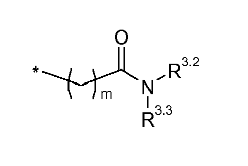
bedeuten, sowie pharmakologisch verträgliche Salz, Diastereomere, Enantiomere, Racemate, Hydrate oder Solvate davon.

Besonders bevorzugt von den oben genannten Verbindungen der Formel **1** sind die Verbindungen der Formel **1.1;** worin 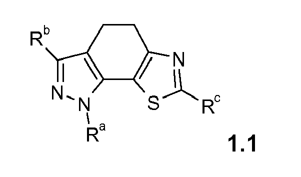 worin **R^{a}, R^{b}** und **R^{c}** die oben genannte Bedeutung haben, sowie pharmakologisch verträgliche Salz, Diastereomere, Enantiomere, Racemate, Hydrate oder Solvate davon.

Besonders bevorzugt sind die oben genannten Verbindungen der Formel 1.1; worin
- **R^{a}**: Phenyl, gegebenenfalls substituiert durch einen oder mehrere Reste ausgewählt aus der Gruppe bestehend aus R¹, R² und R³; oder 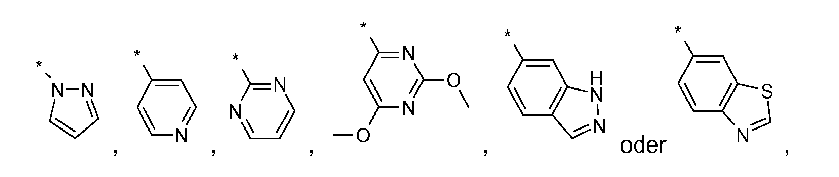
- R¹: Methyl, Ethyl, Propyl, CF₃, CH₂COOH, Methoxy, F, Cl, Br, CN, COR^{1.1} oder SO₂R^{1.1}_{;}
R^{1.1} OH, Methyl, NH₂, NHMe, NMe₂ oder 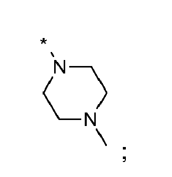
- R²: Methyl, F, Cl oder Br;
- R³: ein Rest ausgewählt aus der Gruppe bestehend aus 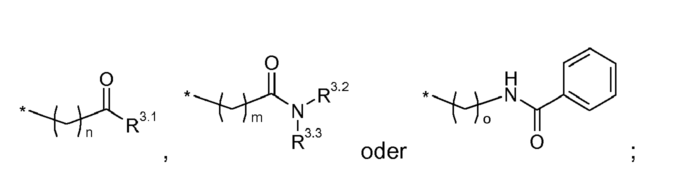 worin
n 0 oder 1;
m 0 oder 1;
o 2;
R^{3.1} ein Rest ausgewählt aus der Gruppe bestehend aus 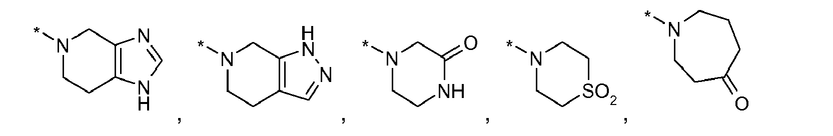 und 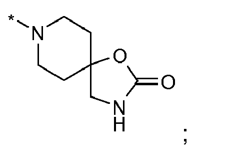 oder ein Rest ausgewählt aus der Gruppe bestehend aus 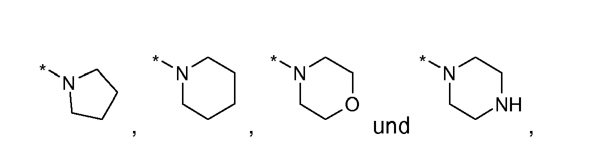 der gegebenenfalls substituiert sein kann mit einem oder mehreren R^{3.1.1};
R^{3.1.1} Methyl, OH, CH₂OH, CH₂CH₂OH, CH₂NEt₂, COMe, COOH, CONH₂, 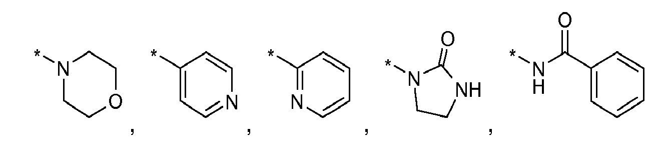 oder 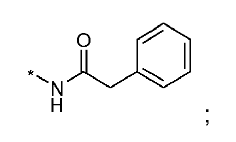 R^{3.2} ein Rest ausgewählt aus der Gruppe bestehend aus 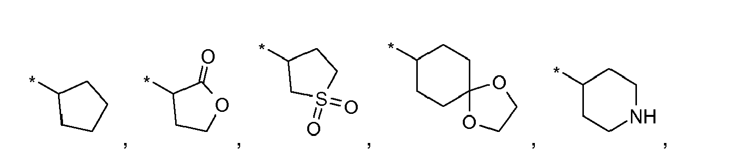 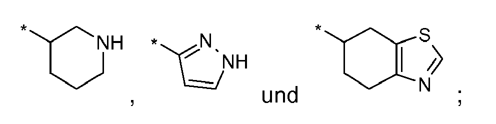 der gegebenenfalls substituiert ist mit einem oder mehreren Resten ausgewählt aus der Gruppe bestehend aus Methyl, Ethyl, Cyclopentyl, OH, NH₂; oder
Cyclohexyl, das gegebenenfalls substituiert ist mit einem oder zwei R^{3.2.1}
R^{3.2.1} -NR^{3.2.1.1}R^{3.2.1.2} oder ein Rest ausgewählt aus der Gruppe bestehend aus 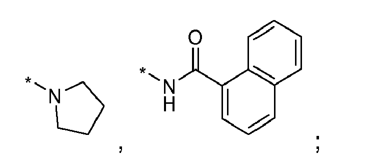 oder ein Rest ausgewählt aus der Gruppe bestehend aus 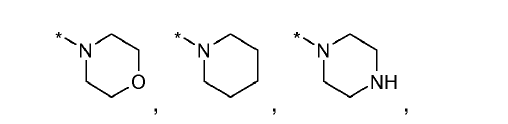 der gegebenenfalls substituiert ist mit einer oder mehreren Methylgruppen
R^{3.2.1.1} H oder Methyl;
R^{3.2.1.2} H oder Methyl; oder -C₁₋₆-alkyl, gerade oder verzweigt, das gegebenenfalls substituiert ist mit einem oder zwei R^{3.2.2};
R^{3.2.2} C=CH₂, C≡CH, COOR^{3.2.2.1}, CONR^{3.2.2.1}R^{3.2.2.2}, NR^{3.2.2.1}R^{3.2.2.2}, CN, OH oder ein Rest ausgewählt aus der Gruppe bestehend aus 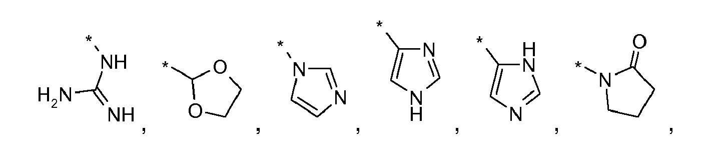 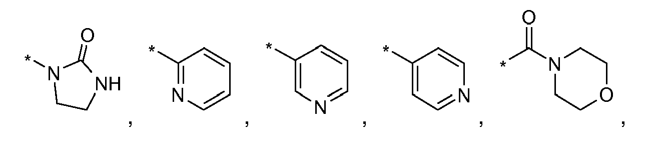 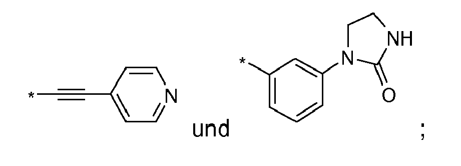 oder ein Rest ausgewählt aus der Gruppe bestehend aus 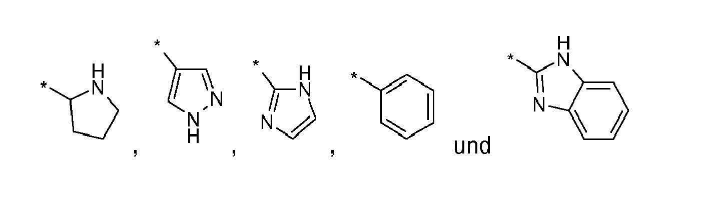 der gegebenenfalls substituiert ist mit einem oder mehreren Resten ausgewählt aus der Gruppe Methyl, CONR^{3.2.2.1}R^{3.2.2.2}, OH;
R^{3.2.2.1} H oder Methyl;
R^{3.2.2.2.} H oder Methyl; oder Phenyl, das gegebenenfalls substituiert ist mit einem oder zwei R^{3.2.3}
R^{3.2.3} ein Rest ausgewählt aus der Gruppe bestehend aus 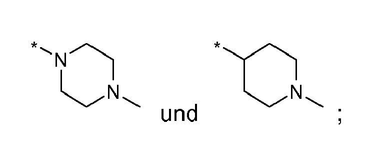 R^{3.3} H, Methyl oder Ethyl;
- **R^{b}**: R⁴, CH₂OCH₃, COR⁴, COOH, COOCH₃ CONR⁴R⁵, NH₂, NHCOOR⁴ oder OH;
- R⁴: H, Methyl, Propyl, C≡CH, Phenyl, 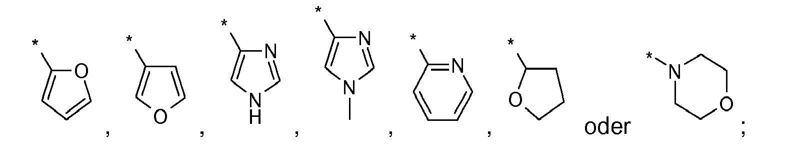
- R⁵: H oder Methyl;
- **R^{c}**: ein Rest 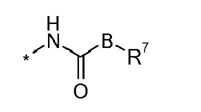 worin
B Methylen, Propylen;
R⁷ H, NR^{7.1}R^{7.2} oder 1-Imidazolyl;
R^{7.1} H oder Methyl;
R^{7.2} H oder Methyl;
bedeuten, sowie pharmakologisch verträgliche Salz, Diastereomere, Enantiomere, Racemate, Hydrate oder Solvate davon.

Am meisten bevorzugt sind die oben genannten Verbindungen der Formel **1.1**; worin
- **R^{a}**: Phenyl, gegebenenfalls substituiert durch einen oder mehrere Reste ausgewählt aus der Gruppe bestehend aus R¹, R² und R³; oder 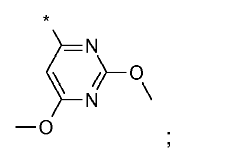
- R¹: Methyl, CF₃, Methoxy, F, Cl, Br, COR^{1.1} oder SO₂R^{1.1};
R^{1.1} OH, NH₂, NHMe oder NMe₂;
- R²: Cl;
- R³: ein Rest ausgewählt aus der Gruppe bestehend aus 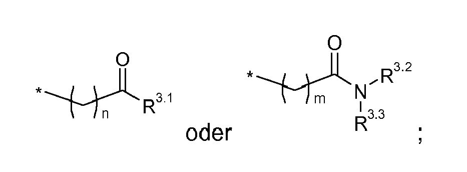 worin
n 0 oder 1;
m 0 oder 1;
R^{3.1} ein Rest ausgewählt aus der Gruppe bestehend aus 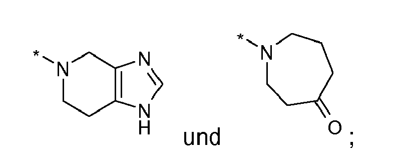 oder ein Rest ausgewählt aus der Gruppe bestehend aus 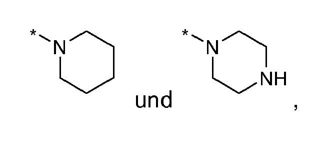 der gegebenenfalls substituiert sein kann mit einem oder mehreren R^{3.1.1};
R^{3.1.1} OH, CONH₂ oder 4-Pyridinyl,
R^{3.2} ein Rest ausgewählt aus der Gruppe bestehend aus 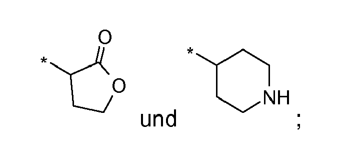 der gegebenenfalls substituiert ist mit einer oder mehreren Methylgruppen; oder
Cyclohexyl, das gegebenenfalls substituiert ist mit einem oder zwei R^{3.2.1}
R^{3.2.1} ein Rest ausgewählt aus der Gruppe bestehend aus 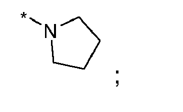 -C₁₋₆-alkyl, gerade oder verzweigt, das gegebenenfalls substituiert ist mit einem oder zwei R^{3.2.2};
R^{3.2.2} COOR^{3.2.2.1}, CONR^{3.2.2.1}R^{3.2.2.2}, 4-Pyrdinyl oder ein Rest ausgewählt aus der Gruppe bestehend aus 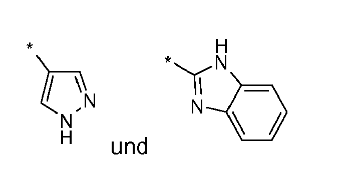 der gegebenenfalls substituiert ist mit einem oder mehreren Resten ausgewählt aus der Gruppe Methyl;
R^{3.2.2.1} H oder Methyl;
R^{3.2.2.2.} H oder Methyl; oder
R^{3.3} H, Methyl oder Ethyl;
- **R^{b}**: R⁴, CH₂OCH₃ oder OH;
- R⁴: H, C≡CH, 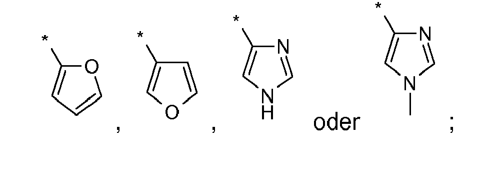
- **R^{c}**: ein Rest 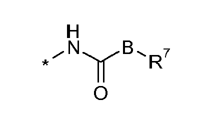 worin
B Methylen, Propylen;
R⁷ H oder NR^{7.1}R^{7.2};
R^{7.1} H oder Methyl;
R^{7.2} H oder Methyl;
bedeuten, sowie pharmakologisch verträgliche Salz, Diastereomere, Enantiomere, Racemate, Hydrate oder Solvate davon.

Besonders bevorzugt von den oben genannten Verbindungen der Formel 1 sind die Verbindungen der Formel 1.2; worin 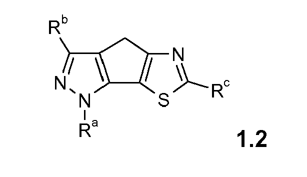 worin **R^{a}, R^{b}** und **R^{c}** die oben genannte Bedeutung haben, sowie pharmakologisch verträgliche Salz, Diastereomere, Enantiomere, Racemate, Hydrate oder Solvate davon.

Besonders bevorzugt sind die oben genannten Verbindungen der Formel **1.2;** worin **R^{b}** und **R^{c}** die oben genannte Bedeutung haben und
- **R^{a}**: Aryl, gegebenenfalls substituiert mit einem oder mehreren Reste ausgewählt aus der Gruppe bestehend aus R¹, R² und R³;
- R¹ und R²: unabhängig voneinander C₁₋₆-Alkyl, C₁₋₆-Haloalkyl, C₁₋₆-Alkoxy, Halogen, COR^{1.1}, SO₂R^{1.1},
R^{1.1} C₁₋₆-Alkyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 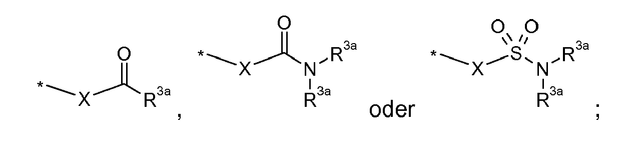 worin
X Bindung oder C₁₋₄-alkylen;
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} NR^{3.1.1.1}R^{3.1.1.2};
R^{3.1.1.1} H C₁₋₄-Alkyl;
R^{3.1.1.2} H C₁₋₄-Alkyl;
bedeutet, sowie pharmakologisch verträgliche Salz, Diastereomere, Enantiomere, Racemate, Hydrate oder Solvate davon.

Besonders bevorzugt sind die oben genannten Verbindungen der Formel **1.2**; worin
- **R^{a}**: Phenyl, gegebenenfalls substituiert mit einem oder mehreren Reste ausgewählt aus der Gruppe bestehend aus R¹, R² und R³;
- R¹ und R²: unabhängig voneinander C₁₋₆-Alkyl, C₁₋₆-Haloalkyl, C₁₋₆-Alkoxy, Halogen, COR^{1.1}, SO₂R^{1.1},
R^{1.1} Methyl Ethyl, Propyl, NR^{1.1.1}R^{1.1.2}
R^{1.1.1} H, Methyl Ethyl, Propyl;
R^{1.1.2} H, Methyl Ethyl, Propyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 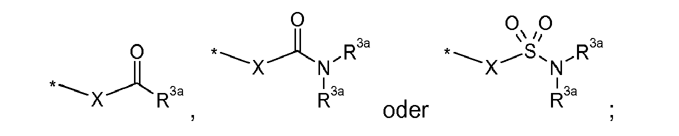 worin
X Bindung oder Methylen, Ethylen, Propylen;
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} NR^{3.1.1.1}R^{3.1.1.2};
R^{3.1.1.1} H, Methyl Ethyl, Propyl;
R^{3.1.1.2} H, Methyl Ethyl, Propyl;
- **R^{b}**: R⁴;
- R⁴: H;
- **R^{c}**: NHR⁶ oder ein Rest 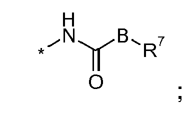 worin
B Bindung, Methylen, Ethylen oder Propylen;
R⁶ H;
R⁷ H oder NR^{7.1}R^{7.2}, 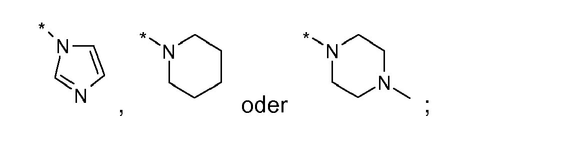 R^{7.1} H, Methyl, Ethyl, (CH₂)₂R^{7.1.1} oder COOButyl;
R^{7.2} H, Methyl oder Ethyl;
R^{7.1.1} NMe₂ oder 1-Imidazolyl
bedeutet, sowie pharmakologisch verträgliche Salz, Diastereomere, Enantiomere, Racemate, Hydrate oder Solvate davon.

Besonders bevorzugt sind die oben genannten Verbindungen der Formel **1.2;** worin
- **R^{a}**: Phenyl, gegebenenfalls substituiert durch einen oder mehrere Reste ausgewählt aus der Gruppe bestehend aus R¹ und R²;
- R¹: Methyl, Ethyl, Propyl, CF₃, F, Cl, COR^{1.1} oder SO₂R^{1.1};
R^{1.1} Methyl;
- R²: Methyl, F oder Cl;
- **R^{b}**: R⁴;
- R⁴: H;
- **R^{c}**: NHR⁶ oder ein Rest 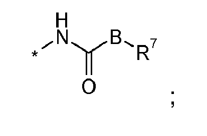 worin
B Bindung, Methylen, Ethylen oder Propylen;
R⁶ H;
R⁷ H oder NR^{7.1}R^{7.2}, 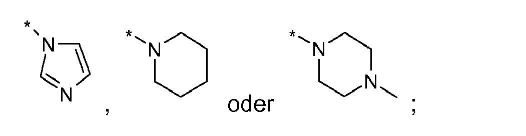 R^{7.1} H, Methyl, Ethyl, (CH₂)₂R^{7.1.1} oder COOButyl;
R^{7.2} H, Methyl oder Ethyl;
R^{7.1.1} NMe₂ oder 1-Imidazolyl
bedeuten, sowie pharmakologisch verträgliche Salz, Diastereomere, Enantiomere, Racemate, Hydrate oder Solvate davon.

Am meisten bevorzugt sind die oben genannten Verbindungen der Formel 1.2; worin **R^{a}** Phenyl, gegebenenfalls substituiert mit R¹;
- R¹: Methyl, F, Cl, Br oder COR^{1.1};
R^{1.1} Methyl;
- **R^{b}**: R⁴;
- R⁴: H;
- **R^{c}**: ein Rest 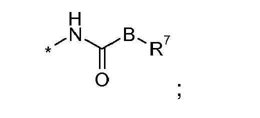 worin
B Bindung, Methylen oder Propylen;
R⁷ H oder NR^{7.1}R^{7.2},
R^{7.1} H oder Methyl;
R^{7.2} Methyl;
bedeuten, sowie pharmakologisch verträgliche Salz, Diastereomere, Enantiomere, Racemate, Hydrate oder Solvate davon.

Besonders bevorzugt von den oben genannten Verbindungen der Formel 1 sind die Verbindungen der Formel 1.3; worin 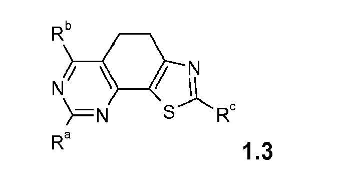 worin **R^{a}, R^{b}** und **R^{c}** die oben genannte Bedeutung haben, sowie pharmakologisch verträgliche Salz, Diastereomere, Enantiomere, Racemate, Hydrate oder Solvate davon.

Besonders bevorzugt sind die oben genannten Verbindungen der Formel **1.3;** worin **R^{b}** und **R^{c}** die oben genannte Bedeutung haben und
- **R^{a}**: Aryl, gegebenenfalls substituiert mit einem oder mehreren Reste ausgewählt aus der Gruppe bestehend aus R¹, R² und R³, oder Hetaryl;
- R¹ und R²: unabhängig voneinander C₁₋₆-Alkyl, C₁₋₆-Haloalkyl, C₁₋₆-Alkoxy, Halogen, COR^{1.1};
R^{1.1} OH, C₁₋₆-Alkyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 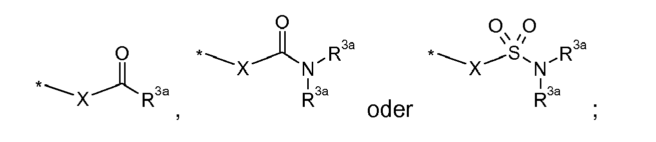 worin
X Bindung oder C₁₋₄-alkylen;
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} NR^{3.1.1.1}R^{3.1.1.2};
R^{3.1.1.1} H, C₁₋₄-Alkyl;
R^{3.1.1.2} H, C₁₋₄-Alkyl;
bedeutet, sowie pharmakologisch verträgliche Salz, Diastereomere, Enantiomere, Racemate, Hydrate oder Solvate davon.

Besonders bevorzugt sind die oben genannten Verbindungen der Formel 1.3; worin
- R^{a}: Phenyl, gegebenenfalls substituiert mit einem oder mehreren Reste ausgewählt aus der Gruppe bestehend aus R¹, R² und R³, Pyrazolyl oder Pyridinyl;
- R¹ und R²: unabhängig voneinander C₁₋₆-Alkyl, C₁₋₆-Haloalkyl, C₁₋₆-Alkoxy, Halogen, COR^{1.1},
R^{1.1} Methyl Ethyl, Propyl, NR^{1.1.1}R^{1.1.2}
R^{1.1.1} H, Methyl Ethyl, Propyl;
R^{1.1.2} H, Methyl Ethyl, Propyl; oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
- R³: ein Rest ausgewählt aus der Gruppe bestehend aus 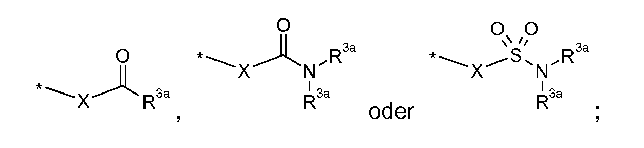 worin
X Bindung oder C₁₋₄-alkylen;
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} NR^{31.1.1}R^{3.1.1.2};
R^{3.1.1.1} H, Methyl Ethyl, Propyl;
R^{3.1.1.2} H, Methyl Ethyl, Propyl;
- **R^{b}**: R⁴ oder OH;
- R⁴: H;
- **R^{c}**: NHR⁶ oder ein Rest 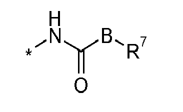 worin
B Bindung, Methylen, Ethylen oder Propylen;
R⁶ H;
R⁷ H, NR^{7.1}R^{7.2} oder ein Rest ausgewählt aus der Gruppe bestehend aus 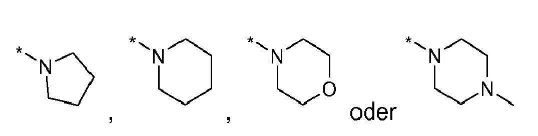 R^{7.1} H, Methyl oder (CH₂)₂R^{7.1.1};
R^{7.2} H, Methyl oder Ethyl;
R^{7.1.1} NMe₂;
bedeuten, sowie pharmakologisch verträgliche Salz, Diastereomere, Enantiomere, Racemate, Hydrate oder Solvate davon.

Besonders bevorzugt sind die oben genannten Verbindungen der Formel **1.3;** worin
- **R^{a}**: Phenyl, gegebenenfalls substituiert durch einen oder mehrere Reste ausgewählt aus der Gruppe bestehend aus R¹ und R²; oder 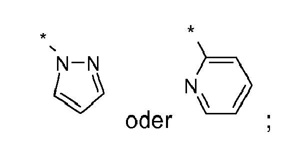
- R¹: Methyl, Methoxy, Cl, OH oder COR^{1.1};
R^{1.1} NH₂, NHMe oder NMe₂;
- R²: Methoxy oder Cl;
- **R^{b}**: R⁴ oder OH;
- R⁴: H;
- **R^{c}**: NHR⁶ oder ein Rest 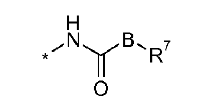 worin
B Bindung, Methylen, Ethylen oder Propylen;
R⁶ H;
R⁷ H, NR^{7.1}R^{7.2} oder ein Rest ausgewählt aus der Gruppe bestehend aus 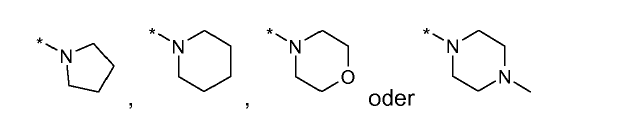 R^{7.1} H, Methyl oder (CH₂)₂R^{7.1.1};
R^{7.2} H, Methyl oder Ethyl;
R^{7.1.1} NMe₂;
bedeuten, sowie pharmakologisch verträgliche Salz, Diastereomere, Enantiomere, Racemate, Hydrate oder Solvate davon.

Am meisten bevorzugt sind die oben genannten Verbindungen der Formel **1.3;** worin
- **R^{a}**: Phenyl, gegebenenfalls substituiert durch einen oder mehrere Reste ausgewählt aus der Gruppe bestehend aus R¹, R² und R³; oder
- R¹: Cl oder COR^{1.1};
R^{1.1} NH₂;
- R²: Cl;
- **R^{b}**: R⁴ oder OH;
- R⁴: H;
- **R^{c}**: NHR⁶ oder ein Rest 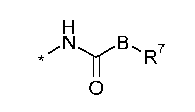 worin
B Methylen;
R⁶ H;
R⁷ H;
bedeuten, sowie pharmakologisch verträgliche Salz, Diastereomere, Enantiomere, Racemate, Hydrate oder Solvate davon.

Besonders bevorzugt von den oben genannten Verbindungen der Formel **1** sind die oben genannten Verbindungen der Formel **1.4;** worin 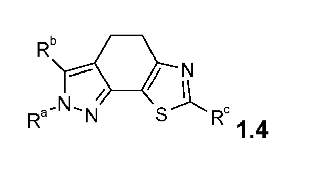 worin **R^{a}, R^{b}** und **R^{c}** die oben genannte Bedeutung haben, sowie pharmakologisch verträgliche Salz, Diastereomere, Enantiomere, Racemate, Hydrate oder Solvate davon.

Besonders bevorzugt sind die oben genannten Verbindungen der Formel **1.4;** worin
- **R^{a}**: Phenyl, gegebenenfalls substituiert durch einen oder mehrere Reste ausgewählt aus der Gruppe bestehend aus R¹ und R³; oder
- R¹: Methyl, F, Cl oder Br;
- R³: ein Rest ausgewählt aus der Gruppe bestehend aus 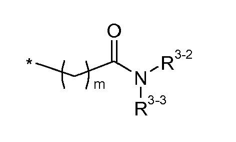 worin
m 0;
R^{3.2} ein Rest 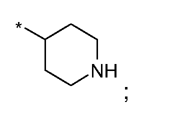 der gegebenenfalls substituiert ist mit einem Rest ausgewählt aus der Gruppe bestehend aus Methyl und Cyclopentyl;
Cyclohexyl, das gegebenenfalls substituiert ist mit einem R^{3.2.1}
R^{3.2.1} ein Rest 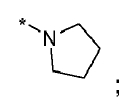 R^{3.3} H;
- **R^{b}**: R⁴;
- R⁴: ein Rest ausgewählt aus der Gruppe bestehend aus 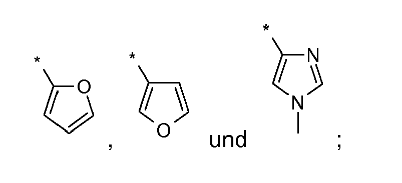
- **R^{c}**: ein Rest 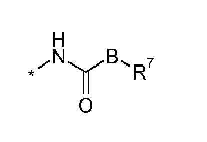 worin
B Methylen;
R⁷ H;
bedeuten, sowie pharmakologisch verträgliche Salz, Diastereomere, Enantiomere, Racemate, Hydrate oder Solvate davon.

Am meisten bevorzugt sind die oben genannten Verbindungen der Formel **1.4;** worin
- **R^{a}**: Phenyl, gegebenenfalls substituiert durch einen oder mehrere Reste ausgewählt aus der Gruppe bestehend aus R¹ und R³; oder
- R¹: Cl;
- R³: ein Rest ausgewählt aus der Gruppe bestehend aus 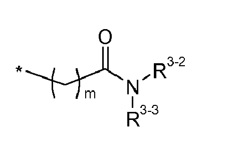 worin
m 0;
R^{3.2} ein Rest 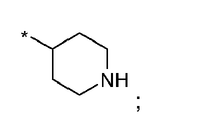 der gegebenenfalls substituiert ist mit Methyl; oder
Cyclohexyl, das gegebenenfalls substituiert ist mit einem R^{3.2.1}
R^{3.2.1} ein Rest 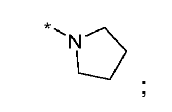 R^{3.3} H;
- **R^{b}**: R⁴;
- R⁴: ein Rest 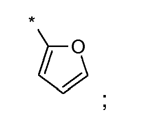
- **R^{c}**: ein Rest 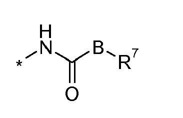 worin
B Methylen;
R⁷ H;
bedeuten, sowie pharmakologisch verträgliche Salz, Diastereomere, Enantiomere, Racemate, Hydrate oder Solvate davon.

Ebenfalls bevorzugt sind die oben genannten Verbindungen der Formel **1;** worin
- **R^{a}**: H, C₁₋₆-Alkyl, C₂₋₆-Alkenyl, C₃₋₈-Cycloalkyl, C₃₋₈-Cycloalkenyl, C₁₋₆-Haloalkyl, COR⁸, NR⁹R¹⁰, N0₂, OR⁸, SR¹¹, SOR¹¹, SO₂R¹¹, NHCO-C₁₋₆-Alkyl-NH₂, Spiro oder ein Rest ausgewählt aus der Gruppe bestehend aus C₇₋₁₁-Aralkyl, CH₂-O-Aryl und Het der gegebenenfalls substituiert sein kann mit einem oder mehreren Halogen, C₁₋₆-Alkyl, CO-C₁₋₄-Haloalkyl, C₁₋₄-Alkyl-NH₂ oder CH₂NHCOOR¹²;
- R⁸: C₁₋₆-Alkyl, C₃₋₆-Cycloalkyl, NH₂, Hetaryl oder Aryl, gegebenenfalls substituiert mit einem oder mehreren Halogen oder C₁₋₄-Alkyl;
- R⁹: H, COOR¹², CONR¹² oder C₁₋₆-Alkyl, gegebenenfalls substituiert mit einem oder mehreren COOH, N(C₁₋₆-Alkyl)₂ oder Het, gegebenenfalls substituiert mit einem oder mehreren C₁₋₆-Alkyl; oder R⁹ bedeutet Het, gegebenenfalls substituiert mit einem oder mehreren C₁₋₄-Alkyl;
- R¹⁰: H, C₁₋₆-Alkyl, CO-C₁₋₆-Alkyl oder C₂₋₆-Alkinyl;
- R¹¹: C₁₋₆-Alkyl, gegebenenfalls substituiert mit einem oder mehreren N(C₁₋₄-Alkyl)₂;
- R¹²: H, C₁₋₆-Alkyl;
- **R^{b}**: R⁴, OR⁴, -CH₂OR⁴, COR⁴, COOR⁴, CONR⁴R⁵, NR⁴R⁵, NR⁵COR⁴, NR⁵COOR⁴, NR⁵CONR⁴R⁵, NR⁵SOR⁴ oder NR⁵SO₂R⁴;
- R⁴, R⁵: H, C₁₋₆-Alkyl, C₁₋₆-Haloalkyl, C₁₋₆-Alkylen-OH, C₂₋₆-Alkenyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl;
oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
- **R^{c}**: NHR⁶ oder ein Rest ausgewählt aus der Gruppe bestehend aus 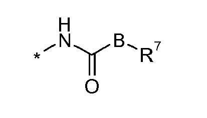 worin
B Bindung, C₁₋₆-Alkyl, C₂₋₆-Alkenyl oder C₂₋₆-Alkinyl;
R⁶ H oder ein Rest ausgewählt aus der Gruppe C₁₋₄-Alkyl, C₂₋₄-Alkenyl, C₂₋₄-Alkinyl, C₃₋₆-Cycloalkyl, C₃₋₆-Cycloalkenyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
R⁷ H, C₁₋₄-Alkyl, C₂₋₄-Alkenyl, C₂₋₄-Alkinyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₆-Alkyl, (CH₂)₂₋₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Bevorzugt sind die oben genannten Verbindungen der Formel 1; worin
- **R^{a}**: H, C₁₋₆-Alkyl, C₂₋₆-Alkenyl, C₃₋₈-Cycloalkyl, C₃₋₈-Cycloalkenyl, C₁₋₆-Haloalkyl, COR⁸, NR⁹R¹⁰, NO₂, OR⁸, SR¹¹, SOR¹¹, SO₂R¹¹, NHCO-C₁₋₆-Alkyl-NH₂, Spiro oder ein Rest ausgewählt aus der Gruppe bestehend aus C₇₋₁₁- Aralkyl, CH₂-O-Aryl und Het der gegebenenfalls substituiert sein kann mit einem oder mehreren Halogen, C₁₋₆-Alkyl, CO-C₁₋₄-Haloalkyl, C₁₋₄-Alkyl-NH₂ oder CH₂NHCOOR¹²;
- R⁸: C₁₋₆-Alkyl, C₃₋₆-Cycloalkyl, NH₂, Hetaryl oder Aryl, gegebenenfalls substituiert mit einem oder mehreren Halogen oder C₁₋₄-Alkyl;
- R⁹: H, COOR¹² oder C₁₋₄-Alkyl, gegebenenfalls substituiert mit einem oder mehreren COOH, N(C₁₋₄-Alkyl)₂ oder Het, gegebenenfalls substituiert mit einem oder mehreren C₁₋₄-Alkyl; oder R⁹ bedeutet Het, gegebenenfalls substituiert mit einem oder mehreren C₁₋₄-Alkyl;
- R¹⁰: H, C₁₋₆-Alkyl, CO-C₁₋₄-Alkyl oder C₂₋₆-Alkinyl;
- R¹¹: C₁₋₆-Alkyl, gegebenenfalls substituiert mit einem oder mehreren N(C₁₋₄-Alkyl)₂;
- R¹²: C₁₋₆-Alkyl;
- **R^{b}**: R⁴, OR⁴, -CH₂OR⁴, COR⁴, COOR⁴, CONR⁴R⁵, NR⁴R⁵, NR⁵COR⁴, NR⁵COOR⁴, NR⁵CONR⁴R⁵, NR⁵SOR⁴ oder NR⁵SO₂R⁴;
- R⁴: H, C₁₋₆-Alkyl, C₁₋₆-Haloalkyl, C₁₋₆-Alkylen-OH, C₂₋₆-Alkenyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl;
- R⁵: H oder C₁₋₆-Alkyl;
oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
- **R^{c}**: NHR⁶ oder ein Rest ausgewählt aus der Gruppe bestehend aus 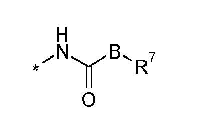 worin
B Bindung, C₁₋₄-Alkyl oder C₂₋₄-Alkinyl;
R⁶ H oder ein Rest ausgewählt aus der Gruppe C₁₋₄-Alkyl, C₂₋₄-Alkenyl, C₂₋₄-Alkinyl, C₃₋₆-Cycloalkyl, C₃₋₆-Cycloalkenyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
R⁷ H, C₁₋₄-Alkyl, C₂₋₄-Alkenyl, C₂₋₄-Alkinyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₄-Alkyl, (CH₂)₂₋₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Bevorzugt sind die oben genannten Verbindungen der Formel 1; worin
- **R^{a}**: H, C₁₋₆-Alkyl, C₂₋₆-Alkenyl, C₃₋₆-Cycloalkyl, C₃₋₆-Cycloalkenyl, C₁₋₆-Haloalkyl, COR⁸, NR⁹R¹⁰, NO₂, OR⁸, SR¹¹, SOR¹¹, SO₂R¹¹, NHCO-C₁₋₆-Alkyl-NH₂, Spiro oder ein Rest ausgewählt aus der Gruppe bestehend aus C₇₋₁₁-Aralkyl, CH₂-O-Aryl und Het der gegebenenfalls substituiert sein kann mit einem oder mehreren Halogen, C₁₋₆-Alkyl, CO-C₁₋₄-Haloalkyl, C₁₋₄-Alkyl-NH₂ oder CH₂NHCOOR¹²;
- R⁸: C₁₋₆-Alkyl, C₃₋₆-Cycloalkyl, NH₂, Hetaryl oder Aryl, gegebenenfalls substituiert mit einem oder mehreren Halogen oder C₁₋₄-Alkyl;
- R⁹: H, COOR¹² oder C₁₋₄-Alkyl, gegebenenfalls substituiert mit einem oder mehreren COOH, N(C₁₋₄-Alkyl)₂ oder Het, gegebenenfalls substituiert mit einem oder mehreren C₁₋₄-Alkyl; oder R⁹ bedeutet Het, gegebenenfalls substituiert mit einem oder mehreren C₁₋₄-Alkyl;
- R¹⁰: H, C₁₋₆-Alkyl, CO-C₁₋₄-Alkyl oder C₂₋₆-Alkinyl;
- R¹¹: C₁₋₆-Alkyl, gegebenenfalls substituiert mit einem oder mehreren N(C₁₋₄-Alkyl)₂;
- R¹²: C₁₋₆-Alkyl;
- **R^{b}**: R⁴, OR⁴, -CH₂OR⁴, COR⁴, COOR⁴, CONR⁴R⁵, NH₂, NR⁵COOR⁴, NR⁵CONR⁴R⁵ oder NR⁵SOR⁴_{;}
- R⁴: H, C₁₋₆-Alkyl, C₁₋₆-Haloalkyl, C₁₋₆-Alkylen-OH, C₂₋₆-Alkenyl, C₇₋₁₁-Aralkyl, C₁₋₄-Alkyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl;
- R⁵: H oder C₁₋₆-Alkyl;
oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
- **R^{c}**: NHR⁶ oder ein Rest ausgewählt aus der Gruppe bestehend aus 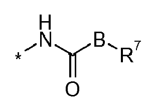 worin
B Bindung, C₁₋₄-Alkyl oder C₂₋₄-Alkinyl;
R⁶ H oder ein Rest ausgewählt aus der Gruppe C₁₋₄-Alkyl, C₃₋₆-Cycloalkyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
R⁷ H, C₁₋₄-Alkyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₄-Alkyl, (CH₂)₂₋₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Bevorzugt sind die oben genannten Verbindungen der Formel 1; worin
- **R^{a}**: H, C₁₋₆-Alklyl, C₂₋₆-Alkenyl, C₃₋₆-Cycloalkyl, CF₃, COR⁸, NR⁹R¹⁰, NO₂, S(O)ₙR¹¹, oder ein Rest ausgewählt aus der Gruppe bestehend aus 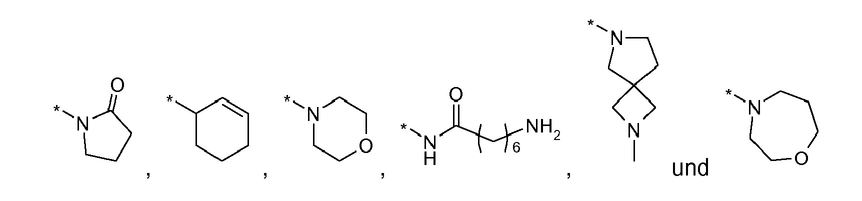 oder ein Rest ausgewählt aus der Gruppe bestehend aus 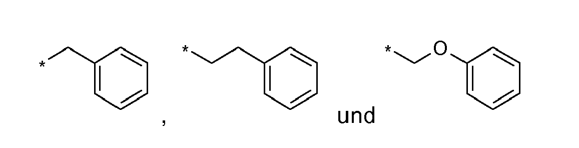 der gegebenenfalls substituiert sein kann mit einem oder mehreren Cl;
oder ein Rest ausgewählt aus der Gruppe bestehend aus 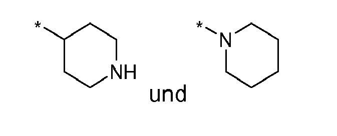 der gegebenenfalls substituiert sein kann mit einem oder mehreren CH₃, COCF₃, CH₂NH₂ oder CH₂NHCOOR¹²;
- R⁸: C₁₋₄-Alkyl, C₃₋₆-Cycloalkyl, NH₂, Furanyl oder Phenyl, gegebenenfalls substituiert mit einem oder mehreren Chlor;
- R⁹: H, COOR¹² oder Piperidino, gegebenenfalls substituiert mit einem oder mehreren CH₃, oder ein Rest ausgewählt aus der Gruppe bestehend aus C₁₋₄-Alkyl, der gegebenenfalls substituiert sein kann mit einem oder mehreren COOH, NMe₂ oder 4-Methylpiperazin;
- R¹⁰: H, C₁₋₄-Alkyl, C₂₋₄-Alkinyl oder COCH₃;
- R¹¹: C₁₋₄-Alkyl, gegebenenfalls substituiert mit einem oder mehreren NMe₂,
- R¹²: C₁₋₄-Alkyl;
- **R^{b}**: R⁴, CH₂OR⁴, COR⁴, COOR⁴, CONR⁴R⁵, NH₂, NHCOOR⁴, NHCONR⁴R⁵ oder OH;
- R⁴: H, C₁₋₄-Alkyl, C₁₋₄-Alkylen-OH, C₂₋₄-Alkinyl, C₁₋₆-Haloalkyl, Aryl, Het, Hetaryl, 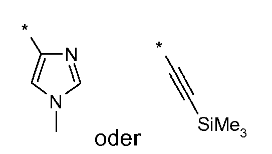
- R⁵: H oder C₁₋₄-Alkyl;
- **R^{c}**: NHR⁶ oder ein Rest ausgewählt aus der Gruppe bestehend aus 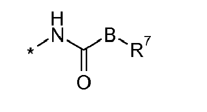 worin
B Bindung, C₁₋₄-Alkyl oder C₂₋₄-Alkinyl;
R⁶ H, C₁₋₄-Alkyl, C₃₋₆-Cycloalkyl, C₇₋₁₁-Aralkyl, Aryl, gegebenenfalls substituiert mit SO₂CH₃;
R⁷ H, NR^{7.1}R^{7.2}, OR^{7.2}, SR^{7.2}, Hetaryl, Het, gegebenenfalls substituiert mit C₁₋₄-Alkyl oder CONH₂, oder ein Rest ausgewählt aus der Gruppe bestehend aus 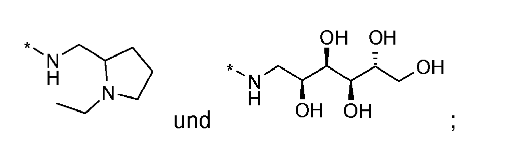 R^{7.1} H, C₁₋₄-Alkyl, (CH₂)₂R^{7.1.1} oder COOButyl;
R^{7.2} H, C₁₋₄-Alkyl;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Besonders bevorzugt sind die oben genannten Verbindungen der Formel 1; worin
- **R^{a}**: H, C₁₋₆-Alklyl, C₂₋₆-Alkenyl, C₃₋₆-Cycloalkyl, C₃₋₆-Cycloalkenyl, CF₃, COR⁸, NR⁹R¹⁰, NO₂, S(O)ₙR¹¹, Spiro, NHCO-C₁₋₆-Alkyl-NH₂ oder ein Rest ausgewählt aus der Gruppe bestehend aus C₇₋₁₁-Aralkyl und CH₂O-Aryl, der gegebenenfalls substituiert sein kann mit einem oder mehreren Cl; oder ein Rest ausgewählt aus der Gruppe bestehend aus Het, der gegebenenfalls substituiert sein kann mit einem oder mehreren C₁₋₄-Alkyl, COCF₃, CH₂NH₂ oder CH₂NHCOOR¹²;
- R⁸: C₁₋₄-Alkyl, C₃₋₆-Cycloalkyl, NH₂, Hetaryl, Aryl, gegebenenfalls substituiert mit einem oder mehreren Chlor;
- R⁹: H, COOR¹² oder Het, gegebenenfalls substituiert mit einem oder mehreren C₁₋₄-Alkyl, oder ein Rest ausgewählt aus der Gruppe bestehend aus C₁₋₄-Alkyl, der gegebenenfalls substituiert sein kann mit einem oder mehreren COOH, N(C₁₋₄-Alkyl)₂ oder 4-Methylpiperazin;
- R¹⁰: H, C₁₋₄-Alkyl, C₂₋₄-Alkinyl oder COCH₃;
- R¹¹: C₁₋₄-Alkyl, gegebenenfalls substituiert mit einem oder mehreren N(C₁₋₄-Alkyl)₂,
- R¹²: C₁₋₄-Alkyl;
- n: 0 oder 2;
- **R^{b}**: H, OH oder COOEt;
- **R^{c}**: NH₂ oder NHCOR¹³;
- R¹³: C₁₋₄-Alkyl, oder NR^{13.1}R^{13.2},
R^{13.1} H oder C₁₋₄-Alkyl;
R^{13.2} H oder C₁₋₄-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Besonders bevorzugt sind die oben genannten Verbindungen der Formel **1**; worin
- **R^{a}**: H, C₁₋₆-Alklyl, C₂₋₆-Alkenyl, C₃₋₆-Cycloalkyl, CF₃, COR⁸, NR⁹R¹⁰, NO₂, S(O)ₙR¹¹, oder ein Rest ausgewählt aus der Gruppe bestehend aus 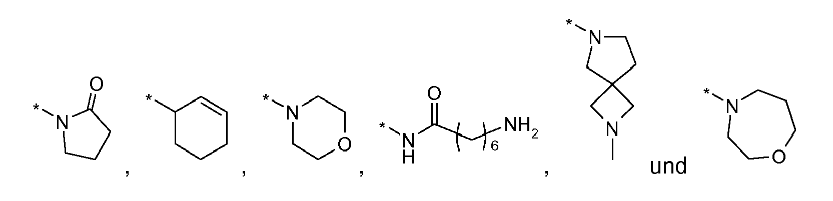 oder ein Rest ausgewählt aus der Gruppe bestehend aus 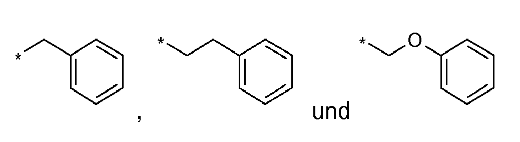 der gegebenenfalls substituiert sein kann mit einem oder mehreren Cl;
oder ein Rest ausgewählt aus der Gruppe bestehend aus 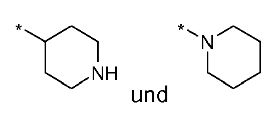 der gegebenenfalls substituiert sein kann mit einem oder mehreren CH₃, COCF₃, CH₂NH₂ oder CH₂NHCOOR¹²;
- R⁸: C₁₋₄-Alkyl, C₃₋₆-Cycloalkyl, NH₂, Furanyl oder Phenyl, gegebenenfalls substituiert mit einem oder mehreren Chlor;
- R⁹: H, COOR¹² oder Piperidino, gegebenenfalls substituiert mit einem oder mehreren CH₃, oder ein Rest ausgewählt aus der Gruppe bestehend aus C₁₋₄-Alkyl, der gegebenenfalls substituiert sein kann mit einem oder mehreren COOH, NMe₂ oder 4-Methylpiperazin;
- R¹⁰: H, C₁₋₄-Alkyl, C₂₋₄-Alkinyl oder COCH₃;
- R¹¹: C₁₋₄-Alkyl, gegebenenfalls substituiert mit einem oder mehreren NMe₂,
- R¹²: C₁₋₄-Alkyl;
- **R^{b}**: H, OH oder COOEt;
- **R^{c}**: NH₂ oder NHCOR¹³;
- R¹³: C₁₋₄-Alkyl, oder NR^{13.1}R^{13.2},
R^{13.1} H oder C₁₋₄-Alkyl;
R^{13.2} H oder C₁₋₄-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Besonders bevorzugt sind die oben genannten Verbindungen der Formel **1**; worin
- **R^{a}**: H, Methyl, Ethyl, Propyl, Butyl, 3-Methyl-butyl, Propenyl, Cyclopropyl, Cyclohexyl, CF₃, COR⁸, NR⁹R¹⁰, NO₂, S(O)ₙR¹¹, oder ein Rest ausgewählt aus der Gruppe bestehend aus 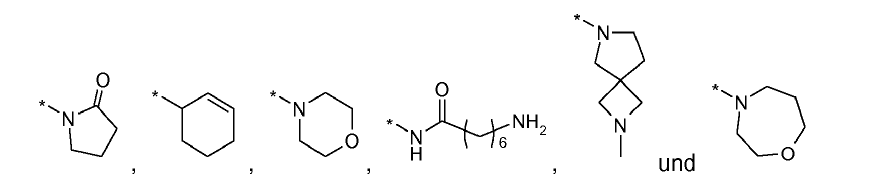 oder ein Rest ausgewählt aus der Gruppe bestehend aus 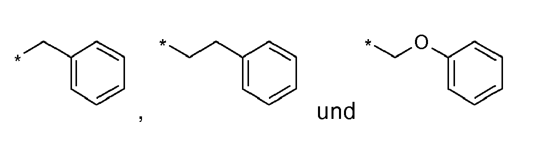 der gegebenenfalls substituiert sein kann mit einem oder mehreren Cl;
oder ein Rest ausgewählt aus der Gruppe bestehend aus 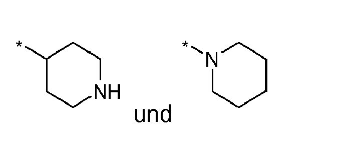 der gegebenenfalls substituiert sein kann mit einem oder mehreren CH₃, COCF₃, CH₂NH₂ oder CH₂NHCOOR¹²;
- R⁸: Methyl, Propyl, Cyclopropyl, NH₂, Furanyl oder Phenyl, gegebenenfalls substituiert mit einem oder mehreren Chlor;
- R⁹: H, COOR¹² oder Piperidino, gegebenenfalls substituiert mit einem oder mehreren CH₃, oder ein Rest ausgewählt aus der Gruppe bestehend aus Methyl, Ethyl und Propyl, der gegebenenfalls substituiert sein kann mit einem oder mehreren COOH, NMe₂ oder 4-Methylpiperazin;
- R¹⁰: H, Methyl, COCH₃, C≡CH oder CH₂C≡CH;
- R¹¹: Ethyl oder Propyl, gegebenenfalls substituiert mit einem oder mehreren NMe₂,
- R¹²: Butyl
- **R^{b}**: H, OH oder COOEt;
- **R^{c}**: NH₂ oder NHCOR¹³;
- R¹³: Methyl oder NR^{13.1}R^{13.2},
R^{13.1} H oder Methyl;
R^{13.2} H oder Methyl.
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

Am meisten bevorzugt sind die oben genannten Verbindungen der Formel **1**; worin
- **R^{a}**: Propyl, COR⁸, NR⁹R¹⁰, S(O)ₙR¹¹ und 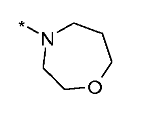
- R⁸: Furanyl;
- R⁹: Methyl;
- R¹⁰: Methyl;
- R¹¹: Ethyl;
- n: 0;
- **R^{b}**: H oder OH;
- **R^{c}**: NH₂ oder NHCOCH₃;
bedeuten, sowie pharmakologisch verträgliche Salz, Diastereomere, Enantiomere, Racemate, Hydrate oder Solvate davon.

Inbesondere bevorzugt sind die obigen Verbindungen; sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** und **Z** = Stickstoff; **j** = 2; **k** = 0; **R^{b}**= H und **R^{c}**= NHCONH-Et bedeutet und dass **R^{c}** nicht NH₂ sein kann, wenn **Y** und **Z** = Stickstoff; **j** = 2; **k** = 1; **R^{b}**= H und **R^{a}**= 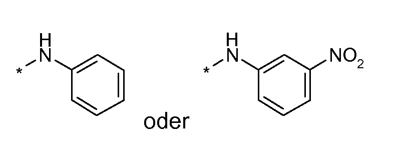 bedeutet.

### VERWENDETE BEGRIFFE UND DEFINTIONEN

Unter dem Begriff "C₁₋₆-Alkyl" (auch soweit sie Bestandteil anderer Reste sind) werden verzweigte und unverzweigte Alkylgruppen mit 1 bis 6 Kohlenstoffatomen verstanden und unter dem Begriff "C₁₋₄-Alkyl" verzweigte und unverzweigte Alkylgruppen mit 1 bis 4 Kohlenstoffatomen verstanden. Bevorzugt sind Alkylgruppen mit 1 bis 4 Kohlenstoffatome. Beispielsweise werden hierfür genannt: Methyl, Ethyl, *n-*Propyl, *iso-*Propyl, *n-*Butyl, *iso-*Butyl, *sec*-Butyl, *tert*-Butyl, *n-*Pentyl, *iso-*Pentyl, neo-Pentyl oder Hexyl. Gegebenenfalls werden für vorstehend genannten Gruppen auch die Abkürzungen Me, Et, *n-*Pr, *i-*Pr, *n-*Bu, *i-*Bu, *t-*Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n-*Propyl und *iso-*Propyl, Butyl umfasst *iso-*Butyl, *sec-*Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen verstanden und unter dem Begriff "C₁₋₄-Alkylen" verzweigte und unverzweigte Alkylengruppen mit 1 bis 4 Kohlenstoffatomen verstanden. Bevorzugt sind Alkylengruppen mit 1 bis 4 Kohlenstoffatome. Beispielsweise werden hierfür genannt: Methylen, Ethylen, Propylen, 1-Methylethylen, Butylen, 1-Methylpropylen, 1,1-Dimethylethylen, 1,2-Dimethylethylen, Pentylen, 1,1-Dimethylpropylen, 2,2,-Dimethylpropylen, 1,2-Dimethylpropylen, 1,3-Dimethylpropylen oder Hexylen. Sofern nicht anders beschrieben, umfassen die Definitionen Propylen, Butylen, Pentylen und Hexylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1,2-Dimethylethylen.

Unter dem Begriff "C₂-₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂-₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatome. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc.

Unter dem Begriff "C₂₋₆-Alkenylen" (auch soweit sie Bestandteil anderer Reste sind) werden verzweigte und unverzweigte Alkenylengruppen mit 2 bis 6 Kohlenstoffatomen verstanden und unter dem Begriff "C₂₋₄-Alkenylen" verzweigte und unverzweigte Alkylengruppen mit 2 bis 4 Kohlenstoffatomen verstanden. Bevorzugt sind Alkenylengruppen mit 2 bis 4 Kohlenstoffatome. Beispielsweise werden hierfür genannt: Ethenylen, Propenylen, 1-Methylethenylen, Butenylen, 1-Methylpropenylen, 1,1-Dimethylethenylen, 1,2-Dimethylethenylen, Pentenylen, 1,1-Dimethylpropenylen, 2,2,-Dimethylpropenylen, 1,2-Dimethylpropenylen, 1,3-Dimethylpropenylen oder Hexenylen. Sofern nicht anders beschrieben, umfassen die Definitionen Propenylen, Butenylen, Pentenylen und Hexenylen alle denkbaren isomeren Formen der jeweiligen Reste gleicher Kohlenstoffanzahl. So umfasst beispielsweise Propenyl auch 1-Methylethenylen und Butenylen umfasst 1-Methylpropenylen, 1,1-Dimethylethenylen, 1,2-Dimethylethenylen.

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Bevorzugt sind Alkinylgruppen mit 2 bis 4 Kohlenstoffatome. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc.

Unter dem Begriff "C₂₋₆-Alkinylen" (auch soweit sie Bestandteil anderer Reste sind) werden verzweigte und unverzweigte Alkinylengruppen mit 2 bis 6 Kohlenstoffatomen verstanden und unter dem Begriff "C₂₋₄-Alkinylen" verzweigte und unverzweigte Alkylengruppen mit 2 bis 4 Kohlenstoffatomen verstanden. Bevorzugt sind Alkinylengruppen mit 2 bis 4 Kohlenstoffatome. Beispielsweise werden hierfür genannt: Ethinylen, Propinylen, 1-Methylethinylen, Butinylen, 1-Methylpropinylen, 1,1-Dimethylethinylen, 1,2-Dimethylethinylen, Pentinylen, 1,1-Dimethylpropinylen, 2,2,-Dimethylpropinylen, 1,2-Dimethylpropinylen, 1,3-Dimethylpropinylen oder Hexinylen. Sofern nicht anders beschrieben, umfassen die Definitionen Propinylen, Butinylen, Pentinylen und Hexinylen alle denkbaren isomeren Formen der jeweiligen Reste gleicher Kohlenstoffanzahl. So umfasst beispielsweise Propinyl auch 1-Methylethinylen und Butinylen umfasst 1-Methylpropinylen, 1,1-Dimethylethinylen, 1,2-Dimethylethinylen.

Unter dem Begriff "C₁₋₆-Alkoxy" (auch soweit sie Bestandteil anderer Reste sind) werden verzweigte und unverzweigte Alkoxygruppen mit 1 bis 6 Kohlenstoffatomen verstanden und unter dem Begriff "C₁₋₄-Alkoxy" verzweigte und unverzweigte Alkoxygruppen mit 1 bis 4 Kohlenstoffatomen verstanden. Bevorzugt sind Alkoxygruppen mit 1 bis 4 Kohlenstoffatome. Beispielsweise werden hierfür genannt: Methoxy, Ethoxy, Propoxy, Butoxy oder Pentoxy. Gegebenenfalls werden für vorstehend genannte Gruppen auch die Abkürzungen OMe, OEt, OPr, etc. verwendet. Sofern nicht anders beschrieben, umfassen die Definitionen Propoxy, Butoxy und Pentoxy alle denkbaren isomeren Formen der jeweiligen Reste. So umfasst beispielsweise Propoxy n-Propoxy und *iso-*Propoxy, Butoxy umfasst *iso-*Butoxy, *sec*-Butoxy und *tert-*Butoxy etc.

Unter dem Begriff "C₃₋₈-Cycloalkyl" (auch soweit sie Bestandteil anderer Reste sind) werden cyclische Alkylgruppen mit 3 bis 8 Kohlenstoffatomen verstanden, unter dem Begriff "C₃₋₆-Cycloalkyl" cyclische Alkylgruppen mit 3 bis 8 Kohlenstoffatomen und unter dem Begriff "C₅₋₆-Cycloalkyl" cyclische Alkylgruppen mit 5 bis 6 Kohlenstoffatomen verstanden. Beispielsweise werden hierfür genannt: Cyclopropyl, Cyclobutyl, Cyclopentyl, Cyclohexyl, Cycloheptyl oder Cyclooctyl.

Unter dem Begriff "C₃₋₆-Cycloalkenyl" (auch soweit sie Bestandteil anderer Reste sind) werden cyclische Alkylgruppen mit 5 oder 6 Kohlenstoffatomen verstanden, die eine oder zwei Doppelbindungen enthalten. Beispielsweise werden hierfür genannt: Cyclopentenyl, Cyclopentadienyl, Cyclohexenyl oder Cyclohexadienyl.

Unter dem Begriff "C₁₋₆-Haloalkyl" (auch soweit sie Bestandteil anderer Reste sind) werden verzweigte und unverzweigte Alkylgruppen mit 1 bis 6 Kohlenstoffatomen verstanden bei denen ein oder mehrere Wasserstoffatome durch ein Halogenatom ausgewählt aus der Gruppe Fluor, Chlor oder Brom, bevorzugt Fluor und Chlor, besonders bevorzugt Fluor ausgetauscht sind. Unter dem Begriff "C₁₋₄-Haloalkyl" werden entsprechend verzweigte und unverzweigte Alkylgruppen mit 1 bis 4 Kohlenstoffatomen verstanden, bei denen analog oben beschrieben ein oder mehrere Wasserstoffatome ausgetauscht sind. Bevorzugt ist C₁₋₄-Haloalkyl. Beispielsweise werden hierfür genannt: CH₂F, CHF₂, CF₃,

Unter dem Begriff "C₇₋₁₁-Aralkyl" (auch soweit sie Bestandteil anderer Reste sind) werden verzweigte und unverzweigte Alkylgruppen mit 1 bis 4 Kohlenstoffatomen verstanden, die mit einem aromatischen Ringsystem mit 6 Kohlenstoffatomen substituiert sind. Beispielsweise werden hierfür genannt: Benzyl, 1- oder 2-Phenylethyl. Soweit nicht anders beschreiben, können die Aromaten substituiert sein mit einem oder mehreren Resten ausgewählt aus der Gruppe bestehend aus Methyl, Ethyl, *iso-*Propyl, *tert*-Butyl, Hydroxy, Fluor, Chlor, Brom und Jod.

Unter dem Begriff "Aryl" (auch soweit sie Bestandteil anderer Reste sind) werden aromatische Ringsysteme mit 6 oder 10 Kohlenstoffatomen verstanden. Beispielsweise werden hierfür genannt: Phenyl oder Naphthyl, bevorzugter Arylrest ist Phenyl.

Unter dem Begriff heterocyclische Ringe ("Het") werden fünf-, sechs- oder siebengliedrige, gesättigte oder ungesättigte heterocyclische Ringe oder 5-10 gliedrige, bicyclische Heteroringe verstanden die ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Als Beispiele für fünf-, sechs- oder siebengliedrige, gesättigte oder ungesättigte heterocyclische Ringe, werden genannt:

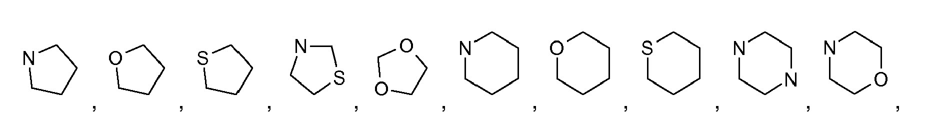

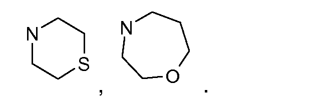

Soweit nicht anders erwähnt, kann ein heterocyclischer Ring mit einer Ketogruppe versehen sein. Als Beispiel hierfür werden genannt.

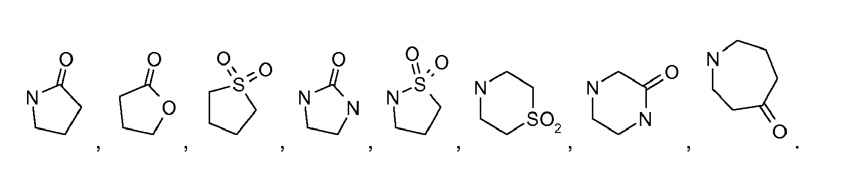

Als Beispiel für 5-1 0-gliedrige bicyclische Heteroringe werden genannt Pyrrolizin, Indol, Indolizin, Isoindol, Indazol, Purin, Chinolin, Isochinolin, Benzimdiazol, Benzofuran, Benzopyran, Benzothiazol, Benzothiazol, Benzoisothiazol, Pyridopyrimidin, Pteridin, Pyrimidopyrimidin,

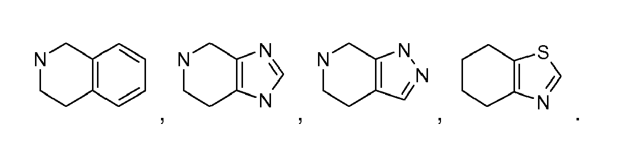

Obwohl unter dem Begriff heterocyclische Ringe ("Hetaryl") umfasst, definiert der Begriff heterocyclische Aromaten fünf- oder sechsgliedrige heterocyclische Aromaten oder 5-10 gliedrige, bicyclische Hetarylringe die ein, zwei oder drei Heteroatome, ausgewählt aus der Gruppe Sauerstoff, Schwefel und Stickstoff, enthalten können und so viele konjugierte Doppelbindungen enthalten, dass ein aromatisches gebildet Systeme wird. Der Ring kann über ein Kohlenstoffatom oder falls vorhanden über ein Stickstoffatom mit dem Molekül verknüpft sein. Als Beispiele für fünf- oder sechsgliedrige heterocyclische Aromaten, werden genannt:

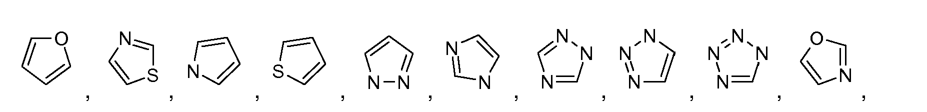

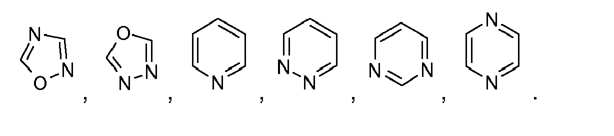

Als Beispiel für 5-1 0-gliedrige bicyclische Hetarylringe werden genannt Pyrrolizin, Indol, Indolizin, Isoindol, Indazol, Purin, Chinolin, Isochinolin, Benzimdiazol, Benzofuran, Benzopyran, Benzothiazol, Benzothiazol, Benzoisothiazol, Pyridopyrimidin, Pteridin, Pyrimidopyrimidin.

Unter dem Begriff heterocyclische Spiroringe ("Spiro") werden 5-10 gliedrige, spirocyclische Ringe verstanden die gegebenenfalls ein, zwei oder drei Heteroatome, ausgewählt aus der Gruppe Sauerstoff, Schwefel und Stickstoff enthalten können, dabei kann der Ring über ein Kohlenstoffatom oder falls vorhanden über ein Stickstoffatom mit dem Molekül verknüpft sein. Soweit nicht anders erwähnt, kann ein spirocyclischer Ring mit einer Ketogruppe versehen sein. Als Beispiele hierfür werden genannt:

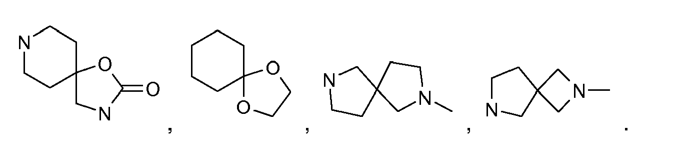

Unter dem Begriff "gegebenenfalls substituiert" wird im Rahmen der Erfindung die genannte Gruppe verstanden, die gegebenenfalls mit einem niedermolekularen Rest substituiert ist. Als niedermolekulare Reste werden als chemisch sinnvoll anzusehende Gruppen verstanden, bestehende aus 1-200 Atomen. Bevorzugt haben solche Gruppen keinen negativen Effekt auf die pharmakologische Wirksamkeit der Verbindungen.

Beispielsweise können die Gruppen umfassen:
● Gerade oder verzweigte Kohlenstoffketten, gegebenenfalls unterbrochen durch Heteroatome, gegebenenfalls substituiert mit Ringen, Heteroatomen oder anderen gängigen funktionellen Gruppen.
● Aromatische oder nicht-aromatische Ringsysteme bestehend aus Kohlenstoffatomen und gegebenenfalls Heteroatomen, die wiederum substituiert sein können mit funktionellen Gruppen.
● Mehrere aromatische oder nicht-aromatische Ringsysteme bestehend aus Kohlenstoffatomen und gegebenenfalls Heteroatomen, die durch eine oder mehrere Kohlenstoffketten, gegebenenfalls unterbrochen durch Heteroatome, gegebenenfalls substituiert mit Heteroatomen oder anderen gängigen funktionellen Gruppen verknüpft sein können.

### SYNTHESE DER REAGENZIEN

### IMIDAZOL-1-YL-[1-(2-TRIMETHYLSILANYL-ETHOXYMETHYL)-1H-IMIDAZOL-4-YL]-METHANON

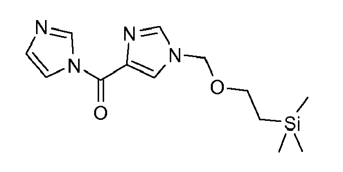

Eine Suspension von 1.5 g (63 mmol) Natriumhydrid (60%ige Suspension in Mineralöl) in 80 ml DMF wird portionsweise mit 8 g (63 mmol) Imidazol-4-carbonsäuremethylester versetzt und die entstandene Lösung 1 Stunde gerührt. Die Reaktionsmischung wird auf 5 °C gekühlt und 12 ml (70 mmol) [2-(Trimethylsilyl)-ethoxy]methylchlorid zugegeben. Nach 12 Stunden wird die Suspension mit 100 ml Wasser versetzt und mit Essigsäureethylester extrahiert. Die vereinten organischen Phasen werden mit Natriumchlorid-Lösung gewaschen, getrocknet und eingeengt. Ausbeute: 16 g

16 g (62 mmol) der zuvor beschriebenen Zwischenstufe wird in 20 ml Dioxan und 66 ml 2 N Natronlauge gelöst und 1.5 Stunden unter Rückfluss gekocht. Die Reaktionsmischung wird mit 2 N Salzsäure sauer gestellt und der ausgefallene Feststoff abgesaugt, mit Wasser und Diethylether gewaschen und getrocknet.
Ausbeute: 13 g

13 g (55 mmol) der zuvor beschriebenen Zwischenstufe werden in 150 ml Dichlormethan vorgelegt und mit 22.4 g (138 mmol) Carbonyldiimidazol versetzt. Das Reaktionsgemisch wird 1 Stunde bei Raumtemperatur gerührt und dann mit halbgesättigter Natriumchlorid-Lösung gewaschen. Die organische Phase wird getrocknet und zur Trockene eingedampft. Ausbeute: 15 g

### IMIDAZOL-1-YL(1-METHYL-1H-IMIDAZOL-4-YL)-METHANON

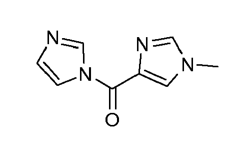

39.5 g (0.31 mol) 1-Methyl-1 H-imidazol-4-carbonsäure werden in 400 ml Dichlormethan vorgelegt und 115.6 g (0.71 mol) Carbonyldiimidazol zugegeben. Die Suspension wird 3 Stunden bei Raumtemperatur gerührt, anschließend mit gesättigter Natriumchloridlösung extrahiert. Die Wasserphase wird mit Dichlormethan extrahiert, die vereinten organischen Phasen getrocknet und zur Trockene eingedampft. Ausbeute: 60.6 g

### 1-IMIDAZOL-1-YL-2-METHYL-PROPAN-1-ON

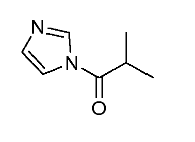

12.0 g (0.18 mol) Imidazol werden bei 0 °C in 200 ml Chloroform vorgelegt und dann mit 10.8 ml (0.10 mol) Isobutylchlorid versetzt. Es wird 1 Stunde bei Raumtemperatur gerührt. Anschließend wird das Reaktionsgemisch mit Wasser gewaschen, die organische Phase getrocknet und zur Trockene eingedampft. Ausbeute: 10.7 g

### (R)-IMIDAZOL-1-YL-(TETRAHYDROFURAN-2-YL)-METHANON

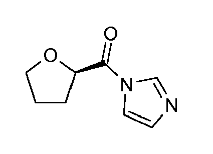

21.8 g (32 mmol) Imidazol werden in 400 ml Chloroform vorgelegt und auf 0 °C gekühlt. 21.3 g (15.0 mol) (*R)*-Tetrahydrofuran-2-carbonylchlorid werden zugetropft, dann 1.5 Stunden bei Raumtemperatur gerührt. Nach erneutem Abkühlen auf 0 °C wird das Reaktionsgemisch mit halbgesättigter Natriumchloridlösung extrahiert. Die organische Phase wird getrocknet und zur Trockene eingedampft. Ausbeute: 24.0 g

### (S)-IMIDAZOL-1-YL-(TETRAHYDROFURAN-2-YL)-METHANON

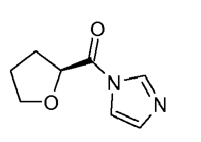

21.8 g (32.0 mmol) Imidazol werden in 400 ml Chloroform vorgelegt und auf 0 °C gekühlt. 21.5 g (15.0 mmol) (S)-Tetrahydrofuran-2-carbonylchlorid werden zugetropft, dann 1.5 Stunden bei Raumtemperatur gerührt. Nach erneutem Abkühlen auf 0 °C wird das Reaktionsgemisch mit halbgesättigter Natriumchloridlösung extrahiert. Die organische Phase wird getrocknet und zur Trockene eingedampft. Ausbeute: 23.5 g

### 1-IMIDAZOL-1-YL-2-METHOXY-ETHANON

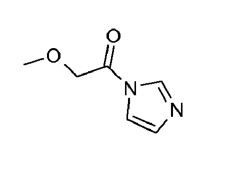

Analog zu der oben beschriebenen Vorgehensweise werden aus 14.9 g (219 mmol) Imidazol und 10 ml (109 mmol) Methoxyessigsäurechlorid 5.6 g des gewünschten Produkts erhalten.

### 1-IMIDAZOL-1-YL-3-TRIMETHYLSILANYL-PROPINON

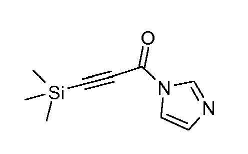

0.5 g (3.5 mmol) 3-Trimethylsilylpropinsäure werden in 10 ml THF gelöst und mit 1.7 g (10.6 mmol) Carbonyldiimidazol versetzt. Die Reaktionsmischung wird über Nacht bei Raumtemperatur gerührt und anschließend filtriert. Das Filtrat wird mit Diethylether verdünnt und 2 Mal mit kaltem Wasser gewaschen. Die organische Phase wird über Magnesiumsulfat getrocknet und eingeengt. Ausbeute: 0.4 g

### (4-HYDRAZINO-PHENYL)-ESSIGSÄURE HYDROCHLORID

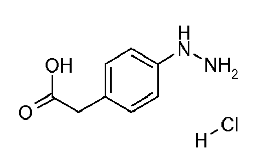

15.1 g (10.0 mmol) 4-Aminophenylessigsäure werden in einer Lösung aus 10.6 g (10.0 mmol) Natriumcarbonat in 100 ml Wasser vorgelegt. Es wird auf 0 °C gekühlt, dann 6.9 g (10.0 mmol) Natriumnitrit in 50 ml Wasser zugegeben. Diese Mischung wird unter Kühlung zu 100 ml konz. Salzsäure getropft, dann 0.1 Stunden nachgerührt. 45.1 g (20.0 mmol) Zinn(II)-chlorid in 40 ml konz. Salzsäure werden unter kräftigem Rühren zugetropft, dabei fällt ein Niederschlag aus. Das Reaktionsgemisch wird 1 Stunde bei Raumtemperatur gerührt, dann abgesaugt. Der Niederschlag wird mit Wasser gewaschen und getrocknet. Ausbeute: 22.0 g

### 3-CHLORO-5-HYDRAZINO-PHENOL

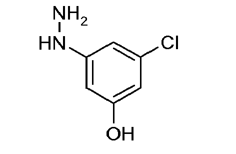

2.1 g (14.3 mmol) 3-Amino-5-chlor-phenol werden in 20 ml konz. Salzsäure und 20 ml Wasser gelöst und auf 0 °C abgekühlt. 1.0 g (15.0 mmol) Natriumnitrit in 4 ml Wasser werden zugegeben. Anschließend wird eine Lösung aus 12.1 g (53.5 mmol) Zinn(II)-chlorid in 16 ml Salzsäure bei -5 °C langsam zugetropft. Es wird 1 Stunde bei 0 °C nachgerührt, dann mit Natriumhydrogencarbonat auf pH7 gestellt. Der ausgefallene Niederschlag wird abgesaugt und mit Wasser gewaschen. Das Filtrat wird mit Diethylether extrahiert. Die organische Phase wird getrocknet und zur Trockene eingedampft. Der Rückstand wird mit Hexan ausgerührt. Ausbeute: 1.2 g

### 2-CHLORO-4-HYDRAZINO-PHENOL

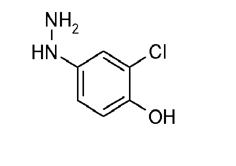

Analog zu oben beschriebenem Verfahren werden aus 2.2 g (15 mmol) 4-Amino-2-chlorphenol 2.3 g Hydrazin erhalten.

### 3-CHLOR-4-HYDRAZINO-BENZOESÄUREMETHYLESTER

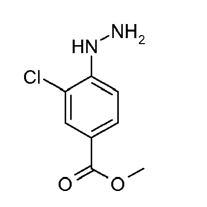

3.0 g (16.0 mmol) 4-Amino-3-chlorbenzoesäuremethylester werden in 15 ml konz. Salzsäure suspendiert und auf -10 °C abgekühlt. Erst wird eine Lösung aus 1.1 g (16.0 mmol) Natriumnitrit in 15 ml Wasser zugetropft, danach eine Lösung aus 16.1 g (71.0 mmol) Zinn(II)-chlorid in 13.5 ml Salzsäure. Ein Niederschlag fällt aus. Das Reaktionsgemisch wird mit 3 molarer Natronlauge basisch gestellt, dann abgesaugt. Der Niederschlag wird mit Dichlormethan und Wasser extrahiert, die organische Phase getrocknet, mit Aktivkohle versetzt und zur Trockene eingedampft. Der Rückstand wird chromatographisch gereinigt. Ausbeute: 1.4 g (Schmp: 120 °C)

### 3-CHLOR-4-HYDRAZINO-BENZOESÄURE

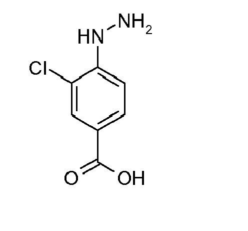

4.0 g (20.0 mmol) 3-Chlor-4-hydrazino-benzoesäuremethylester werden vorgelegt und 75 ml 3 molare Natronlauge zugegeben. Es wird 16 Stunden bei Raumtemperatur gerührt. Anschließend wird das Reaktionsgemisch mit Eisessig auf pH 6 gestellt und der ausgefallene Feststoff abgesaugt. Dieser wird in Diethylether ausgerührt. Ausbeute: 2.4 g

### (4-BROM-2-TRIFLUOMETHYL-PHENYL)-HYDRAZIN

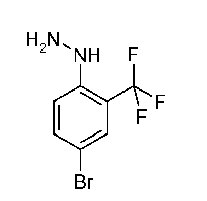

Eine Suspension aus 25.6 g (0.11 mol) 4-Brom-2-(trifluormethyl)-anilin in 125 ml konz. Salzsäure wird auf -10 °C gekühlt und eine Lösung aus 7.7 g (0.11 mol) Natriumnitrit in 125 ml Wasser wird zugegeben. Das Reaktionsgemisch wird 3 Stunden bei -10 bis -5 °C gerührt, dann wird eine Lösung aus 103 g (0.46 mol) Zinn-(II)-chlorid-dihydrat in 125 ml konz. Salzsäure zugetropft. Es wird 1 Stunde bei -5 °C nachgerührt. Der ausgefallene Niederschlag wird abgesaugt und mit Wasser gewaschen. Die wässrige Mutterlauge wird mit Petrolether extrahiert, dann mit Natriumhydroxid basisch gestellt. Der hierbei ausfallende Niederschlag wird mit Diethylether extrahiert. Die organische Phase wird getrocknet und zur Trockene eingedampft. Ausbeute: 12.9 g

### 2,2,2-TRIFLUORO-1-(4-HYDRAZINO-PIPERIDIN-1-YL)-ETHANON-TRIFLUORACETAT

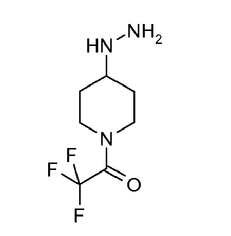

100 g (0.65 mol) 4-Piperdinon-hydrat-hydrochlorid und 274.6 ml (1.97 mol) Triethylamin werden in 1000 ml Dichlormethan vorgelegt und dann auf 0 °C abgekühlt. 182.1 ml (1.31 mol) Trifluoressigsäureanhydrid werden langsam zugegeben und 0.5 Stunden nachgerührt. Das Reaktionsgemisch wird mit Wasser und Natriumbicarbonatlösung gewaschen, die organische Phase getrocknet und zur Trockene eingedampft. Der Rückstand wird mit Methyl-tert-butylether verrührt, der Niederschlag abgesaugt, gewaschen und getrocknet. Ausbeute: 106.0 g
106.0 g (0.54 mol) 1-(2,2,2-Trifluor-acetyl)-piperidin-4-on werden in 1000 ml Ethanol gelöst und mit 71.8 g (0.54 mol) Hydrazinoameisensäure-tert-butylester versetzt. Das Reaktionsgemisch wird 3.5 Stunden bei Raumtemperatur gerührt, anschließend zur Trockene eingedampft. Ausbeute: 172.0 g

172.0 g (0.56 mol) N'-[1-(2,2,2-Trifluor-acetyl)-piperidin-4-yliden]-hydrazin-carbonsäure-*tert*-butylester werden in 2000 ml Methanol gelöst. 17.0 g Palladium auf Kohle (10%) werden zugegeben und bei 1 bar Wasserstoff hydriert. Nach beendeter WasserstoffAufnahme wird der Katalysator abgesaugt und die Mutterlauge eingedampft. Der Rückstand kristallisiert über Nacht. Ausbeute: 168.0 g

5.0 g (16.1 mmol) N'-[1-(2,2,2-Trifluor-acetyl)-piperidin-4-yl]-hydrazin-carbonsäure-tert-butylester werden in 50 ml Dichlormethan gelöst und mit 6.0 ml (77.9 mmol) Trifluoressigsäure versetzt. Es wird 5 Stunden bei Raumtemperatur gerührt, anschließend wird das Reaktionsgemisch eingedampft. Ausbeute: 6.70 g

### 4-(4-METHYL-PIPERAZIN-1-YL)-PHENYLAMIN

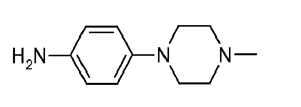

18.1 g (0.18 mol) 1-Methyl-piperazin, 25.5 g (0.18 mol) 1-Fluor-4-nitrobenzol und 50 g (0.36 mol) Kaliumcarbonat werden in 200 ml Dimethylformamid vorgelegt und 15 Stunden bei 130 °C gerührt. Nach dem Abkühlen werden 300 ml Wasser zugegeben, dabei fällt ein Niederschlag aus. Die wässrige Phase mit dem Niederschlag wird gekühlt, dann abgesaugt und mit Wasser gewaschen. Der Niederschlag wird aus Ethanol umkristallisiert. Ausbeute: 23.2 g

10.0 g (45 mmol) 1-Methyl-4-(4-nitro-phenyl)-piperazin werden in 250 ml Ethanol gelöst, dann mit 2 g Palladium/Kohle (10%) versetzt und bei 5 bar Wasserstoff 2 Stunden hydriert. Anschließend wird das Reaktionsgemisch abgesaugt, das Filtrat eingedampft. Der Rückstand wird aus Cyclohexan umkristallisiert. Ausbeute: 7.7 g

### 4-(1-METHYL-PIPERIDIN-4-YL)-PHENYLAMIN

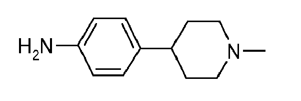

125 g (0.78 mol) 4-Phenylpiperidin werden in 1300 ml Dichlormethan gelöst und mit 149 ml (0.85 mol) Diisopropylethylamin versetzt. Das Reaktionsgemisch wird auf -5 °C gekühlt, dann werden innerhalb von 2 Stunden 120 ml (0.85 mol) Trifluoressigsäureanhydrid zugetropft. Anschließend wird noch 1 Stunde unter Eiskühlung und 16 Stunden bei Raumtemperatur nachgerührt. Es werden 400 ml Wasser zugegeben, die Phasen getrennt. Die organische Phase wird mit Wasser gewaschen, getrocknet und zur Trockene eingedampft. Ausbeute: 193 g
80 g (0.31 mol) 2,2,2-Trifluor-1-(4-phenyl-piperidin-1-yl)-ethanon werden in 400 ml Eisessig und 200 ml Acetanhydrid gelöst und auf 0 °C gekühlt. 1.6 g Natriumnitrit werden zugegeben, dann 52 ml (1.24 mol) rauchende Salpetersäure zugetropft. Das Reaktionsgemisch wird 16 Stunden bei Raumtemperatur gerührt. Anschließend wird das Reaktionsgemisch auf 1200 ml Eiswasser gegossen und mit 8 N Natronlauge auf pH 8 eingestellt (Temp. <20° C). Nach Zugabe von Dichlormethan werden die Phasen getrennt, die wässrige Phase mit Dichlormethan extrahiert. Die vereinten organischen Phasen werden mit 0.1 N Natronlauge und Wasser gewaschen, getrocknet und zur Trockene eingedampft. Das Produkt wird aus Cyclohexan/Ethylacetat umkristallisiert. Ausbeute: 47 g
20 g (66.2 mmol) 2,2,2-Trifluor-1-[4-(4-nitro-phenyl)-piperidin-1yl]-ethanon und 32 g (23.2 mmol) Kaliumcarbonat werden in 400 ml Methanol vorgelegt und 48 Stunden bei Raumtemperatur gerührt. Anschließend wird das Reaktionsgemisch eingedampft, der Rückstand mit Wasser und Dichlormethan extrahiert. Die organische Phase wird getrocknet und zur Trockene eingedampft. Es wird das Hydrochlorid gefällt. Ausbeute: 15g

6.0 g (24.7 mmol) 4-(4-Nitro-phenyl)-piperidin-hydrochlorid werden in 300 ml Tetrahydrofuran vorgelegt und nacheinander mit 11 ml (61.8 mmol) Diisopropylethylamin, 5 ml (49.4 mmol) Formaldehyd (37% in Wasser) und 13 g (61.8 mmol) Natriumtriacetoxyborhydrid versetzt. Das Reaktionsgemisch wird 16 Stunden bei Raumtemperatur gerührt, dann auf Wasser gegossen und eingedampft. Der wässrige Rückstand wird mit 2 N Natronlauge basisch gestellt und mit Dichlormethan extrahiert. Die vereinten organischen Phasen werden getrocknet und zur Trockene eingedampft. Ausbeute: 4.5 g

4.3 g (19.5 mmol) 1-Methyl-4-(4-nitro-phenyl)-piperidin werden in einer Mischung aus 60 ml Ethanol und 60 ml THF gelöst, mit 2 g Palladium/Kohle (10%) versetzt und bei 1 bar Wasserstoff hydriert. Anschließend wird das Reaktionsgemisch abgesaugt und das Filtrat eingedampft. Ausbeute: 3.5 g

### 4-(1-CYCLOPROPYLMETHYL-PIPERIDIN-4-YL)-PHENYLAMIN

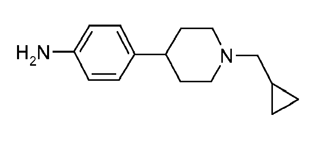

6.0 g (24.7 mmol) der zuvor bereits beschriebenen Zwischenstufe 4-(4-Nitro-phenyl)-piperidin-hydrochlorid werden in 300 ml Tetrahydrofuran vorgelegt und nacheinander mit 11 ml (61.8 mmol) Diisopropylethylamin, 3.7 ml (49.4 mmol) Cyclopropancarboxaldehyd und 13 g (61.8 mmol) Natriumtriacetoxyborhydrid versetzt. Das Reaktionsgemisch wird 3 Stunden bei Raumtemperatur gerührt, dann auf Wasser gegossen und eingedampft. Der wässrige Rückstand wird mit Dichlormethan extrahiert, die organische Phase getrocknet und zur Trockene eingedampft. Die wässrige Phase wird mit 2 N Natronlauge basisch gestellt, dann mit Dichlormethan extrahiert. Die organische Phase wird getrocknet und zur Trockene eingedampft. Ausbeute: 5.6 g
3.3 g (12.5 mmol) 1-Cyclopropylmethyl-4-(4-nitro-phenyl)-piperidin werden in einer Mischung aus 40 ml Ethanol und 40 ml THF gelöst, mit 1.3 g Palladium/Kohle (10%) versetzt und bei 1 bar Wasserstoff hydriert. Anschließend wird das Reaktionsgemisch abgesaugt und das Filtrat eingedampft. Ausbeute: 2.7 g

### 1,1-DIMETHYL-2-DIMETHYLAMINO-1-YL-ETHYLAMIN UND 1,1-DIMETHYL-2-PIPERIDIN-1-YL-ETHYLAMIN

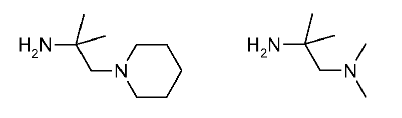

Die Verbindungen wurden hergestellt nach folgenden Literaturstellen: a) S. Schuetz et al. Arzneimittel-Forschung 1971, 21, 739-763 b) V. M. Belikov et al.Tetrahedron 1970, 26, 1199-1216. c) E.B. Butler and McMillan J. Amer. Chem. Soc. 1950, 72, 2978.

Weitere Amine wurden in zu der oben beschriebenen Literatur modifizierter Art und Weise wie folgt hergestellt.

### 1,1-DIMETHYL-2-MORPHOLIN-1-YL-ETHYLAMIN

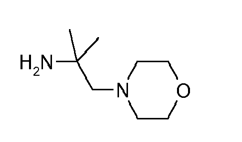

8.7 ml Morpholin und 9.3 ml 2-Nitropropan werden unter Eiskühlung vorgelegt, 7.5 ml Formaldehyd (37% in Wasser) und 4 ml einer 0.5 mol/L NaOH-Lsg. werden langsam zugetropft (<10 °C). Danach wird 1 h bei 25 °C und 1 h bei 50 °C gerührt. Die Lösung wird mit Wasser und Ether behandelt und die wässrige Phase 3x mit Ether extrahiert. Die vereinten organischen Phasen werden über Natriumsulfat getrocknet und mit Salzsäure in Dioxan (4mol/L) versetzt, der entstandene Niederschlag wird abgesaugt. Ausbeute: 21.7 g farbloses Pulver
5 g des weißen Pulvers werden in 80 ml Methanol gelöst und unter Zusatz von 2 g RaNi mit Wasserstoff bei 35 °C und 50 psi für 40 Minuten behandelt. Dies ergibt 3.6 g 1,1-Dimethyl-2-morpholin-1-yl-ethylamin.

Analog zu dieser Vorschrift wird das folgende Amin hergestellt:

### 1,1- DIMETHYL-N-METHYLPIPERAZIN-1-YL-ETHYLAMIN

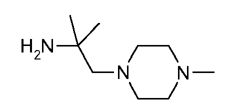

### 1,3 DIMORPHOLIN-2-AMINO-PROPAN

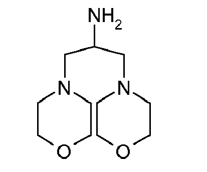

5 g 1,3 Dimorpholin-2-nitropropan werden in 80 ml Methanol gelöst und unter Zusatz von 2 g RaNi mit Wasserstoff bei 30 °C und 50 psi für 5.5 h behandelt. Dies ergibt 4.2 g 1,3 Dimorpholin-2-amino-propan.

### TRANS-N,N-DIBENZYL-CYCLOHEXAN-1,4-DIAMIN

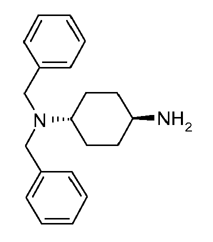

33 g (112 mmol) 4-Dibenzylcyclohexanon werden in 300 ml Methanol gelöst, mit 17.4 g (250 mmol) Hydroxylaminhydrochlorid versetzt und 4 h bei 60 °C gerührt. Das Lösungsmittel wird im Vakuum eingeengt, mit 500 ml Wasser und 50 g Kaliumcarbonat versetzt und zweimal mit je 300 ml Dichlormethan extrahiert. Die organische Phase wird getrocknet, im Vakuum eingeengt, der Rückstand aus Petrolether kristallisiert, in 1.5 L Ethanol gelöst und auf 70 °C erwärmt. Es werden 166 g Natrium portionsweise hinzugefügt und bis zur Auflösung des Natriums unter Rückfluss gekocht. Das Lösungsmittel wird im Vakuum abgezogen, der Rückstand mit 100 ml Wasser versetzt und zweimal mit je 400 ml Ether extrahiert. Die org. Phase wird mit Wasser gewaschen, getrocknet, im Vakuum eingeengt und über eine Säule das trans-Isomer isoliert (ca .1,5 L Kieselgel; ca. 2 L Essigester 80/ Methanol 20 + 2 % konz. Ammoniak). Ausbeute: 12.6 g

### TRANS-N,N-DIMETHYL-CYCLOHEXAN-1,4-DIAMIN (DIMETHANSULFONAT)

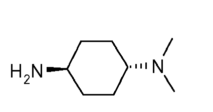

4 g (13.6 mmol) trans-N,N-Dibenzyl-cyclohexan-1,4-diamin werden in 8.1 g (100 mmol) Formalinlösung (37% in Wasser) und 20 ml (43.4 mmol) Ameisensäure vorgelegt und 2 Stunden unter Rückfluss gerührt. Das Reaktionsgemisch wird auf Eiswasser gegeben und mit konz. Ammoniak versetzt. Es wird mit Ethylacetat extrahiert. Die organische Phase wird mit Wasser gewaschen, getrocknet und zur Trockene eingedampft. Der Rückstand wird aus Aceton und Methansulfonsäure kristallisiert. Ausbeute: 6 g (Schmp.: 203 - 204°C
6 g (11,7 mmol) *trans*-N,N-Dibenzyl-N',N'-dimethyl-cyclohexan-1,4-diamindimethansulfonat werden in 120 ml Methanol vorgelegt, 1.2 g Palladium/Kohle (10%) werden zugegeben und dann bei 50 psi und 20 °C hydriert. Das Reaktionsgemisch wird über Kieselgur abgesaugt, die Mutterlauge eingedampft. Der Rückstand wird aus Aceton kristallisiert. Ausbeute: 3.5 g

### CIS- UND TRANS-4-MORPHOLINO-CYCLOHEXYLAMIN

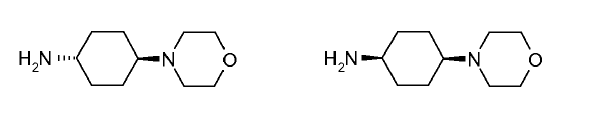

3.9 g (30 mmol) 4-Dibenzylcyclohexanon werden in 100 ml Dichlormethan gelöst und mit 3.9 g (45 mmol) Morpholin und 9.5 g (45 mmol) Natriumtriacetoxyborhydrid 12 h bei RT gerührt. Anschließend wird mit Wasser und Kaliumcarbonat versetzt, die organische Phase abgetrennt, getrocknet und im Vakuum das Lösungsmittel abgezogen. Der Rückstand wird über eine Kieselgelsäule gereinigt (ca 20 ml Kieselgel; ca 500 ml Essigester 90/ Methanol 10 + 1% konz. Ammoniak). Die geeigneten Fraktionen werden im Vakuum eingeengt. Ausbeute: 6.6 g cis-Isomer und 2 g trans-Isomer.

Alternativ kann das trans-Dibenzyl-4-morpholino-cyclohexylamin nach folgendem Weg dargestellt werden: 6.8 g (23 mmol) trans-N,N-Dibenzyl-cyclohexan-1,4-diamin werden in 90 ml DMF gelöst und mit 5 ml (42 mmol) 2,2'-Dichlorethylether und 5 g Kaliumcarbonat 8 h bei 100 °C gerührt. Nach Abkühlung wird mit 30 ml Wasser versetzt, ausgefallene Kristalle abgesaugt und über eine kurze Säule (ca. 20 ml Kieselgel, ca. 100 ml Essigester) gereinigt. Der Rückstand wird aus Methanol und konz. HCl als Dihydrochlorid kristallisiert. Ausbeute: 7.3 g
7.2 g (16.4 mmol) trans-Dibenzyl-4-morpholino-cyclohexylamin werden in 100 ml Methanol gelöst und an 1.4 g Pd/C (10%) bei 30-50 °C hydriert. Das Lösungsmittel wird im Vakuum abgezogen und der Rückstand aus Ethanol und konz. HCl kristallisiert. Ausbeute: 3.9 g (Schmp. 312 °C). Das cis-Isomer kann analog dargestellt werden.

### CIS- UND TRANS-4-PIPERIDINO-CYCLOHEXYLAMIN

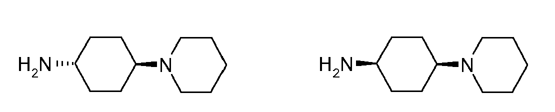

2.0 g (6.8 mmol) trans-1Amino-4-dibenzylaminocyclohexan (siehe Beispiel zuvor) werden in 50 ml DMF gelöst und mit 1.6 g (7 mmol) 1,5-Dibrompentan und 2 g Kaliumcarbonat 48 h bei RT gerührt. Es wird abgekühlt, mit Wasser versetzt, zweimal mit je 100 ml Dichlormethan extrahiert, getrocknet und das Lösungsmittel im Vakuum abgezogen. Der Rückstand wird über eine Säule gereinigt (ca. 100 ml Kieselgel, ca. 500 ml Essigester 80/Methanol 20 +1% konz. Ammoniak). Die geeigneten Fraktionen werden im Vakuum eingeengt und aus Petrolether kristallisiert. Ausbeute: 1.2 g

1.7 g (4.8 mmol) trans-Dibenzyl-4-piperidino-cyclohexylamin werden in 35 ml Methanol gelöst und an 350 mg Pd/C (10%) bei 20 °C hydriert. Das Lösungsmittel wird im Vakuum abgezogen und der Rückstand aus Ethanol und konz. HCl kristallisiert. Ausbeute: 1.1 g. Das cis-Isomer kann analog dargestellt werden.

### CIS- UND TRANS-4-(4-PHENYL-PIPERAZIN-1-YL)-CYCLOHEXYLAMIN

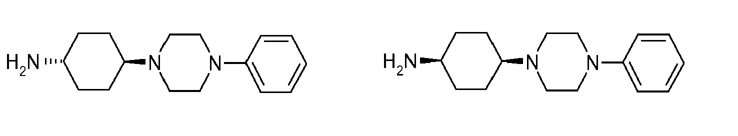

4.1 g (25.3 mmol) 4-Dibenzylcyclohexanon werden in 50 ml Dichlormethan gelöst und mit 7.4 g (25.3 mmol) N-Phenylpiperazin und 7.4 g (35 mmol) Natriumtriacetoxyborhydrid 12 h bei RT gerührt. Anschließend wird mit Wasser und Kaliumcarbonat versetzt, die organische Phase abgetrennt, getrocknet und das Lösungsmittel im Vakuum abgezogen. Der Rückstand wird über eine Kieselgelsäule gereinigt (Essigester 80/ Methanol 20 + 0,5% konz. Ammoniak). Ausbeute: 1.7 g cis-Isomer und 0.27 g *trans*-Isomer.
270 mg (0.61 mmol) trans-Dibenzyl-[4-(4-phenyl-piperazin-1-yl)-cyclohexyl]-amin werden in 5 ml Methanol gelöst und an 40 mg Pd/C (10%) bei 20-30 °C hydriert. Das Lösungsmittel wird im Vakuum abgezogen und der Rückstand aus Ethanol und konz. HCl kristallisiert. Ausbeute: 110 mg. Das cis-Isomer kann analog dargestellt werden.

### CIS-4-(4-CYCLOPROPYLMETHYL-PIPERAZIN-1-YL)-CYCLOHEXYLAMIN

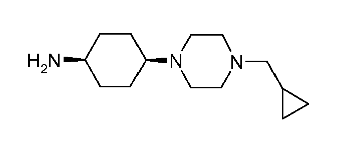

9.8 g (33.4 mmol) 4-Dibenzylcyclohexanon werden in 100 ml Dichlormethan gelöst und mit 5.6 g (40 mmol) N-Cyclopropylmethylpiperazin und 8.5 g (40 mmol) Natriumtriacetoxyborhydrid 12 h bei RT gerührt. Anschließend wird mit Wasser und Kaliumcarbonat versetzt, die organische Phase abgetrennt, getrocknet und das Lösungsmittel im Vakuum abgezogen. Der Rückstand wird über eine Kieselgelsäule gereinigt (ca. 50 ml Kieselgel, ca. 3 L Essigester 95/ Methanol 5 + 0,25% konz. Ammoniak). Die geeigneten Fraktionen werden im Vakuum eingeengt. Ausbeute: 8.5 g *cis*-Isomer und 2.2 *trans*-Isomer.
8.5 g (20 mmol) cis-Dibenzyl-[4-(4-cyclopropylmethyl-piperazin-1-yl)-cyclohexyl]-amin werden in 170 ml Methanol gelöst und an 1.7 g Pd/C (10%) bei 30-50 °C hydriert. Das Lösungsmittel wird im Vakuum abgezogen und der Rückstand aus Ethanol und konz. HCl kristallisiert. Ausbeute: 4.4 g

### 4-(4,4-DIMETHYL-PIPERIDIN-1YL)-CYCLOHEXYLAMIN

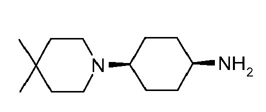

8.8 g (30 mmol) 4-Dibenzylamino-cyclohexanon, 6.7 g (45 mmol) 4,4-Dimethyl-piperidinhydrochlorid und 9.5 g (45 mmol) Natriumtriacetoxyborhydrid werden in 100 ml Dichlormethan 16 Stunden bei Raumtemperatur gerührt. Es werden 200 ml Wasser und 20 g Kaliumcarbonat zugegeben, die organische Phase abgetrennt und zur Trockene eingedampft. Der Rückstand wird chromatographisch gereinigt. Ausbeute: 0.7 g *cis-*Isomer (Schmp.: 125 - 126 °C) und 0.4 *trans*-Isomer.
0.7 g (1.8 mmol) **cis**-Dibenzyl-[4-(4,4-dimethyl-piperidin-1yl)-cyclohexyl]-amin werden in 14 ml Methanol vorgelegt, 0.14 g Palladium/Kohle (10%) zugegeben und bei 50 psi und 20 °C hydriert. Anschließend wird der Katalysator abgesaugt, die Mutterlauge eingedampft. Ausbeute: 0.3 g

### CIS- UND TRANS-4-PYRROLIDIN-1-YL-CYCLOHEXYLAMIN (HYDROCHLORID)

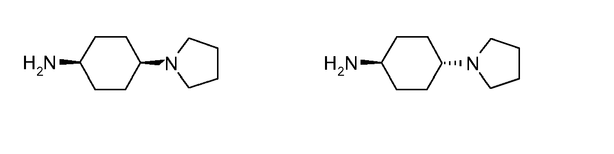

0.8 g (21 mmol) Lithiumaluminiumhydrid werden in 50 ml Tetrahydrofuran vorgelegt und 6.2 g (20 mmol) 4-Dibenzylamino-cyclohexanonoxim (Darstellung siehe oben) gelöst in 62 ml Tetrahydrofuran zugetropft. Anschließend wird das Reaktionsgemisch 3 Stunden unter Rückfluss gerührt. Nach dem Abkühlen werden 0.8 ml Wasser und 2.4 ml 15%ige Natronlauge zugetropft. Der ausgefallene Niederschlag wird abgesaugt und mit Tetrahydrofuran gewaschen. Die Mutterlauge wird zur Trockene eingedampft. Ausbeute: 4.9 g (*cis*/*trans*-Gemisch)
2.9 g (10 mmol) cis/trans-N,N-Dibenzyl-cyclohexan-1,4-diamin, 2.4 g (11 mmol)) 1,4-Dibrombutan und 2.7 g Kaliumcarbonat werden in 70 ml Dimethylformamid vorgelegt und 48 Stunden bei Raumtemperatur gerührt. Anschließend wird das Reaktionsgemisch eingedampft, der Rückstand mit Wasser und Dichlormethan extrahiert. Die organische Phase wird mit Wasser gewaschen, getrocknet und zur Trockene eingedampft. Der Rückstand wird chromatographisch gereinigt, entsprechende Fraktionen vereinigt, eingedampft und aus Petrolether kristallisiert. Ausbeute: 0.8 g cis-Isomer (Schmp.: 81-82 °C) und 0.9 g *trans*-Isomer (122 - 124 °C).
2.2 g (6.1 mmol) *trans*-Dibenzyl-(4-pyrrolidin-1-yl-cyclohexyl)-amin werden in 40 ml Methanol vorgelegt und 0.4 g Palladium/Kohle (10%) zugegeben. Es wird bei 5 bar und 20 °C hydriert. Anschließend wird der Katalysator abgesaugt, die Mutterlauge mit konz. Salzsäure versetzt und eingedampft. Der Rückstand wird mit Aceton ausgerührt. Ausbeute: 1.20 g. Analog kann das cis-Isomer hergestellt werden.

### TRANS-4-(4-METHANSULFONYL-PIPERAZIN-1YL)-CYCLOHEXYLAMIN

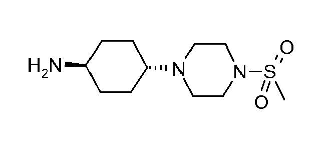

14 g (47 mmol) 4-Dibenzylamino-cyclohexanon, 8.5 g (51 mmol) 1-Methansulfonylpiperazin und 17.5 g (78 mmol) Natriumtriacetoxyborhydrid werden in 250 ml Dichlormethan 4 Stunden bei Raumtemperatur gerührt. Anschließend wird das Reaktionsgemisch mit 200 ml Wasser und 20 g Kaliumcarbonat versetzt. Die organische Phase wird abgetrennt, getrocknet und zur Trockene eingedampft. Der Rückstand wird chromatographisch gereinigt. Ausbeute: 4.8 g *trans*-Isomer
4.8 g (11 mmol) *trans*-Dibenzyl-[4-(4-methansulfonyl-piperazin-1yl)-cyclohexyl]-amin werden in 100 ml Methanol gelöst und 2 g Palladium/Kohle (10%) zugegeben. Es wird bei 50 psi und 50 °C hydriert. Anschließend wird der Katalysator abgesaugt, die Mutterlauge eingedampft. Der ausgefallene Niederschlag wird abgesaugt und mit Diethylether gewaschen. Ausbeute: 2.4 g

### CIS- UND TRANS-4-(2,6-DIMETHYL-MORPHOLIN-4-YL)-CYCLOHEXYLAMIN-DIHYDROCHLORID

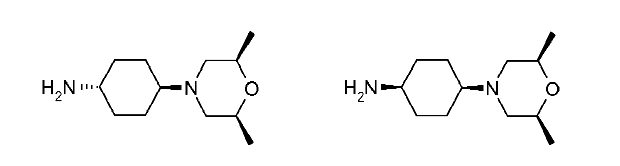

9 g (31 mmol) 4-Dibenzylamino-cyclohexanon, 7.5 g (65 mmol) *cis*-2,6-Dimethylmorpholin und 9.5 g (45 mmol) Natriumtriacetoxyborhydrid werden in 100 ml Dichlormethan 2 Stunden bei Raumtemperatur gerührt. Anschließend werden 200 ml Wasser zugegeben und mit Kaliumcarbonat basisch gestellt. Die organische Phase wird abgetrennt, getrocknet und zur Trockene eingedampft. Der Rückstand wird chromatographisch gereinigt. Entsprechende Fraktionen werden vereinigt und eingedampft, dann das Hydrochlorid gefällt. Ausbeute: 10 g *cis*-Isomer (Schmp.: 304-305 °C) und 3.5 g *trans*-Isomer (334 - 335 °C).
9.8 g (21 mmol) *cis*-Dibenzyl-[4-(2,6-dimethyl-morpholin-4-yl)-cyclohexyl]-amin-dihydrochlorid werden in 150 ml Methanol vorgelegt und 2 g Palladium/Kohle (10%) zugegeben. Es wird bei 50 psi und 50 °C hydriert. Anschließend wird der Katalysator abgesaugt, die Mutterlauge eingedampft. Der ausfallende Niederschlag wird abgesaugt und mit Diethylether gewaschen. Ausbeute: 5.5 g. Analog kann das *trans*-Isomer hergestellt werden.

### CIS- UND TRANS-4-(4-METHYL-PIPERAZIN-1YL)-CYCLOHEXYLAMIN

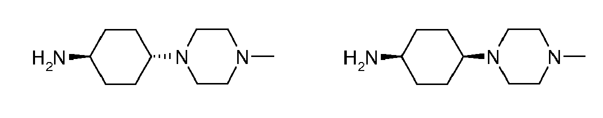

14.7 g (50 mmol) 4-Dibenzylamino-cyclohexanon, 15.8 g (100 mmol) Piperazin-N-carbonsäureethylester und 10.6 g (50 mmol) Natriumtriacetoxyborhydrid werden in 200 ml Dichlormethan 16 Stunden bei Raumtemperatur gerührt. Anschließend wird das Reaktionsgemisch mit 200 ml Wasser und 20 g Kaliumcarbonat versetzt. Die organische Phase wird abgetrennt und zur Trockene eingedampft. Der Rückstand wird chromatographisch gereinigt. Ausbeute: 12 g *cis*-Isomer (Schmp.: 143-144 °C) und 4 g *trans*-Isomer (281 - 282 °C).
12 g (27.5 mmol) cis-4-(4-Dibenzylamino-cyclohexyl)-piperazin-1-carbonsäure-ethylester werden in 50 ml Wasser und 50 ml konz. Salzsäure vorgelegt, dann 72 Stunden unter Rückfluss gerührt. Anschließend wird das Reaktionsgemisch eingedampft, der Rückstand mit 200 ml Wasser und 20 g Kaliumcarbonat versetzt und mit Dichlormethan extrahiert. Die organische Phase wird abgetrennt, getrocknet und zur Trockene eingedampft. Der Rückstand wird aus Ethylacetat kristallisiert. Ausbeute: 8.6 g (Schmp.: 100 - 101 °C) 4 g (11 mmol) *cis*-Dibenzyl-(4-piperazin-1-yl-cyclohexyl)-amin werden in 5 ml (16.7 mmol) Formalinlösung (37% in Wasser) und 10 ml (22 mmol) Ameisensäure vorgelegt, dann 2 Stunden unter Rückfluss gerührt. Das Reaktionsgemisch wird auf Eiswasser gegeben und mit konz. Ammoniak versetzt. Es wird mit Diethylether extrahiert. Die organische Phase wird mit Wasser gewaschen, getrocknet und zur Trockene eingedampft. Der Rückstand wird mit Petrolether ausgerührt, abgesaugt und getrocknet. Ausbeute: 4,10 g (Schmp: 93-94 °C)
4.1 g (11 mmol) *cis*-Dibenzyl-[4-(4-methyl-piperazin-1-yl)-cyclohexyl]-amin werden in 80 ml Methanol vorgelegt, 0.8 g Palladium/Kohle (10%) zugegeben und dann bei 50 psi und 20 °C hydriert. Das Reaktionsgemisch wird über Kieselgur abgesaugt, die Mutterlauge eingedampft. Ausbeute: 1.90 g. Das *trans*-Isomer kann analog hergestellt werden.

### SYNTHESE DER ZWISCHENVERBINDUNGEN

### ZWISCHENVERBINDUNG 1. N-(7-OXO-4,5,6,7-TETRAHYDRO-BENZOTHIAZOL-2-YL)-ACETAMID

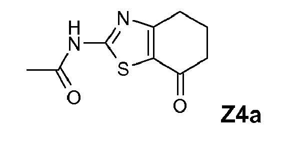

112 g (1.0 mol) 1,3-Cyclohexandion werden in 700 ml Eiswasser suspendiert und 51.6 ml (1.0 mol) Brom bei 0 °C innerhalb von 45 Minuten zugetropft. Die Suspension wird 3.5 Stunden bei max. 10 °C nachgerührt. Anschließend wird abgesaugt und der Feststoff in 800 ml Wasser ausgerührt, abgesaugt, mit 3 L Wasser gewaschen und getrocknet. Der erhaltene Feststoff wird aus Ethanol umkristallisiert. Ausbeute: 37 g **Z2a** (Smp.: 159-160 °C)

15.5 g (0.2 mol) Thioharnstoff werden bei Raumtemperatur in 200 ml Ethanol vorgelegt. Zu dieser Suspension werden 37.1 g (0.2 mol) **Z2a** portionsweise gegeben, dann wird mit 60 ml Ethanol nachgespült. Die allmählich entstehende Lösung wird 2 Stunden unter Rückfluss gerührt und anschließend eingedampft. Der Rückstand wird mit Wasser und Diethylether extrahiert, die Wasserphase mit Natriumcarbonatlösung basisch gestellt. Der hierbei entstandene Feststoff wird abgesaugt, mit Wasser gewaschen, dann mit Methanol ausgerührt und zur Trockene eingedampft. Ausbeute: 22 g **Z3a** (Smp.: 265-268 °C)

230 ml (2.4 mol) Essigsäureanhydrid werden bei Raumtemperatur vorgelegt, 22 g (0.13 mol) **Z3a** zugegeben und 3 Stunden unter Rückfluss gerührt. Die Suspension geht dabei teilweise in Lösung. Nach dem Abkühlen mittels Eis/Kochsalzbad wird der Feststoff abgesaugt, 2x in je 150 ml Aceton aufgekocht, abgesaugt und getrocknet. Ausbeute: 25 g **Z4a** (Smp.: 268-272 °C)

### ZWISCHENVERBINDUNG 2. N-(6-FORMYL-7-OXO-4,5,6,7-TETRAHYDRO-BENZOTHIAZOL-2-YL)-ACETAMID

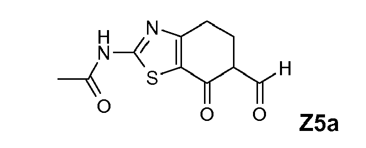

20 g (0.37 mol) Natriummethylat werden in 50 ml Dimethylformamid suspendiert, eine Suspension aus 21 g (0.1 mol) Zwischenverbindung 1 in 100 ml Dimethylformamid zugetropft. Es wird 15 Minuten nachgerührt, dann auf 0 °C gekühlt. Ein Gemisch aus 29.9 ml (0.37 mol) Ameisensäureethylester und 60 ml Benzol wird zugetropft und die Reaktionsmischung mit weiteren 100 ml Benzol verdünnt. Allmählich fällt ein Niederschlag aus und es wird bei 0 °C 3.5 Stunden weitergerührt. Die Suspension wird mit 370 ml 1 molarer Salzsäure hydrolysiert, der dabei ausgefallene Feststoff wird abgesaugt. Die zwei Phasen der Mutterlauge werden getrennt, die Wasserphase mit Dichlormethan extrahiert. Die daraus resultierende organische Phase wird getrocknet und zur Trockene eingedampft. Der Feststoff und der Rückstand aus der Extraktion werden aus Acetonitril umkristallisiert. Ausbeute: 20 g **Z5a**

### ZWISCHENVERBINDUNG 3. N-(6-DIMETHYLAMINOMETHYLEN-7-OXO-4,5,6,7-TETRAHYDRO-BENZOTHIAZOL-2-YL)-ACETAMID

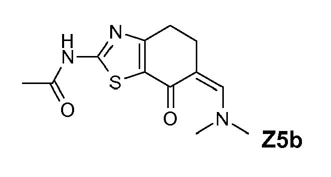

30 g (0.13 mol) Zwischenverbindung 2 werden in 750 ml Dichlormethan suspendiert und unter Eiskühlung mit 1 ml Eisessig und 30 ml Dimethylamin (33%ige Lösung in THF) versetzt. Die Reaktionsmischung wird über Nacht bei Raumtemperatur gerührt, eingeengt und der verbliebene Feststoff mit Cyclohexan extrahiert. Ausbeute: 33 g **Z5b**

### ZWISCHENVERBINDUNG 4. N-[6-(FURAN-2-CARBONYL)-7-OXO-4,5,6,7-TETRAHYDRO-BENZOTHIAZOL-2-YL]-ACETAMID

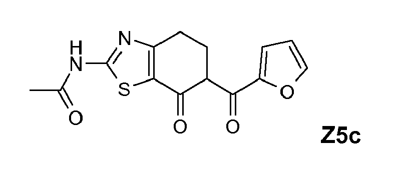

Analog zur Herstellung von Zwischenverbindung 2 werden aus 2 g (10 mmol) Zwischenverbindung 1, 1.6 g (30 mmol) Natriummethylat und 3.8 g (30 mmol) 2-Furansäuremethylester 1.7 g Produkt **Z5c** erhalten. (Smp.: 255-256 °C)

### ZWISCHENVERBINDUNG 5. N-(6-BENZOYL-7-OXO-4,5,6,7-TETRAHYDRO-BENZOTHIAZOL-2-YL)-ACETAMID

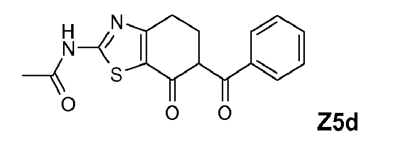

Analog zur Herstellung von Zwischenverbindung 2 werden aus 10 g (50 mmol) Zwischenverbindung 1, 7.8 g (140 mmol) Natriummethylat und 17.9 ml (140 mmol) Benzoesäuremethylester 3.6 g Produkt **Z5d** erhalten.

### ZWISCHENVERBINDUNG 6. N-[6-(FURAN-3-CARBONYL)-7-OXO-4,5,6,7-TETRAHYDRO-BENZOTHIAZOL-2-YL]-ACETAMID

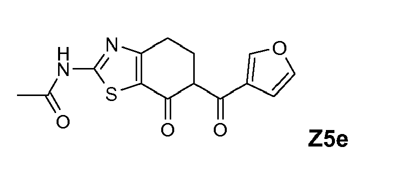

Analog zur Herstellung von Zwischenverbindung 2 werden aus 7.5 g (40 mmol) Zwischenverbindung 1, 7.7 g (110 mmol) Natriummethylat und 15.1 ml (110 mmol) Furan-3-carbonsäureethylester 4.8 g Produkt **Z5e** erhalten.

### ZWISCHENVERBINDUNG 7. (2-ACETYLAMINO-7-OXO-4,5,6,7-TETRAHYDRO-BENZOTHIAZOL-6-YL)-OXO-ESSIGSÄUREMETHYLESTER

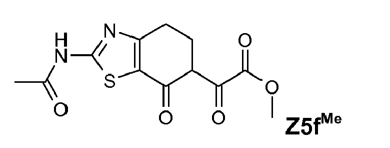

Analog zur Herstellung von Zwischenverbindung 2 werden aus 40 g (190 mmol) Zwischenverbindung 1, 38 g (0.7 mol) Natriummethylat und 84 g (0.7 mol) Dimethyloxalat 52 g Produkt **Z5f^{Me}** erhalten.

### ZWISCHENVERBINDUNG 8. (2-ACETYLAMINO-7-OXO-4,5,6,7-TETRAHYDRO-BENZOTHIAZOL-6-YL)-OXO-ESSIGSÄUREETHYLESTER

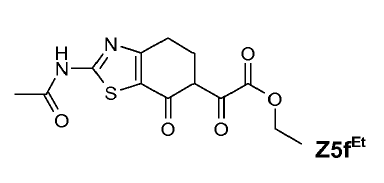

Analog zur Herstellung von Zwischenverbindung 2 werden aus 73 g (348 mmol) Zwischenverbindung 1, 54 g (1 mol) Natriummethylat und 152 g (1 mol) Diethyloxalat 78 g Produkt **Z5f^{Et}** erhalten.

### ZWISCHENVERBINDUNG 9. N-[7-OXO-6-(PYRIDIN-3-CARBONYL)-4,5,6,7-TETRAHYDRO-BENZOTHIAZOL-2-YL]-ACETAMID

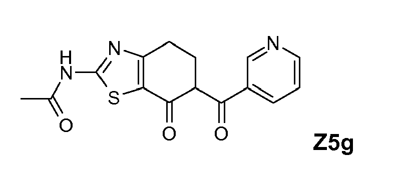

Analog zur Herstellung von Zwischenverbindung 2 werden aus 4 g (19 mmol) Zwischenverbindung 1, 3.9 g (57 mmol) Natriumethylat und 7.9 g (57 mmol) Nicotinsäuremethylester 3.1 g Produkt **Z5g** erhalten.

### ZWISCHENVERBINDUNG 10. N-(6-ACETYL-7-OXO-4,5,6,7-TETRAHYDRO-BENZOTHIAZOL-2-YL)-ACETAMID

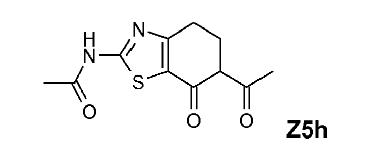

1 g (4.8 mmol) Zwischenverbindung 1 werden in 20 ml THF gelöst, auf -20 °C gekühlt und mit 12 ml (12 mmol) einer 1 N Lösung von Lithiumhexamethyldisilazid in Hexan versetzt. Nach 45 Minuten bei -20 °C werden 0.8 g (7.2 mmol) 1-Imidazol-1-yl-ethanon zugegeben und die Reaktionsmischung langsam auf Raumtemperatur erwärmt. Nach einer Stunde bei dieser Temperatur wird mit 2 N Salzsäure ein pH-Wert von 6 eingestellt und mit Essigsäureethylester extrahiert. Die vereinten organischen Phasen werden getrocknet und eingeengt. Ausbeute: 1.2 g **Z5h**

### ZWISCHENVERBINDUNG 11. N-[6-(1-METHYL-1H-IMIDAZOL-4-CARBONYL)-7-OXO-4,5,6,7-TETRAHYDRO-BENZOTHIAZOL-2-YL]-ACETAMID

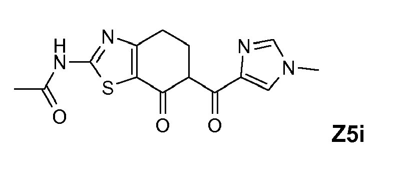

Analog zur Herstellung von Zwischenverbindung 10 werden aus 50 g (0.24 mol) Zwischenverbindung 1, 714 ml (0.71 mol) LiHMDS (1 M in THF) und 55 g (0.31 mol) Imidazol-1-yl-(1-methyl-1 H-imidazol-4-yl)-methanon 57 g Produkt **Z5i** erhalten.

### ZWISCHENVERBINDUNG 12. N-(6-ISOBUTYRYL-7-OXO-4,5,6,7-TETRAHYDRO-BENZOTHIAZOL-2-YL)-ACETAMID

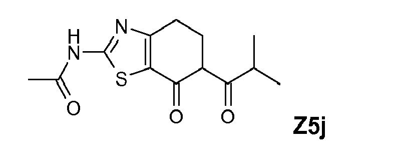

Analog zur Herstellung von Zwischenverbindung 10 werden aus 4.8 g (23 mmol) Zwischenverbindung 1, 71 ml (71 mmol) LiHMDS (1 M in Hexan) und 6.3 g (46 mmol) 1-Imidazol-1-yl-2-methyl-propan-1-on 4.4 g Produkt **Z5j** erhalten.

### ZWISCHENVERBINDUNG 13. N-[6-(2-METHOXY-ACETYL)-7-OXO-4,5,6,7-TETRAHYDRO-BENZOTHIAZOL-2-YL]-ACETAMID

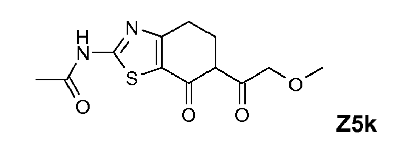

Analog zur Herstellung von Zwischenverbindung 10 werden aus 4.2 g (20 mmol) Zwischenverbindung 1, 60 ml (60 mmol) LiHMDS (1 M in Hexan) und 5.6 g (40 mmol) 1-Imidazol-1-yl-2-methoxy-ethanon 1.8 g Produkt **Z5k** erhalten.

### ZWISCHENVERBINDUNG 14. (R)-N-[7-OXO-6-(TETRAHYDRO-FURAN-2-CARBONYL)-4,5,6,7-TETRAHYDRO-BENZOTHIAZOL-2-YL]-ACETAMID

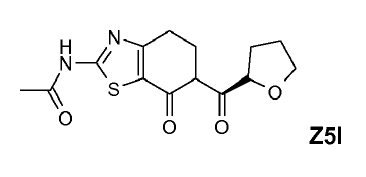

Analog zur Herstellung von Zwischenverbindung 10 werden aus 10 g (48 mmol) Zwischenverbindung 1, 145 ml (145 mmol) LiHMDS (1 M in Hexan) und 23.5 g (52 mmol) (R)-Imidazol-1-yl-(tetrahydro-furan-2-yl)-methanon 1 g Produkt **Z5l** erhalten.

### ZWISCHENVERBINDUNG 15. (S)-N-[7-OXO-6-(TETRAHYDRO-FURAN-2-CARBONYL)-4,5,6,7-TETRAHYDRO-BENZOTHIAZOL-2-YL]-ACETAMID

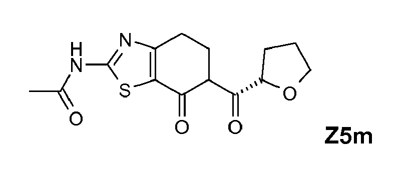

Analog zur Herstellung von Zwischenverbindung 10 werden aus 19.4 g (92 mmol) Zwischenverbindung 1, 277 ml (277 mmol) LiHMDS (1 M in Hexan) und 23.5 g (141 mmol) (S)-Imidazol-1-yl-(tetrahydro-furan-2-yl)-methanon 3.1 g Produkt **Z5m** erhalten.

### ZWISCHENVERBINDUNG 16. N-[7-OXO-6-(3-TRIMETHYLSILANYL-PROPYNOYL)-4,5,6,7-TETRAHYDRO-BENZOTHIAZOL-2-YL]-ACETAMID

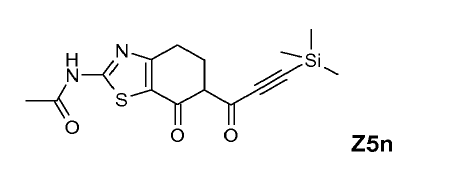

Analog zur Herstellung von Zwischenverbindung 10 werden aus 1.1 g (5.7 mmol) Zwischenverbindung 1, 17.5 ml (17.5 mmol) LiHMDS (1 M in Hexan) und 4.6 g (8.4 mmol) 1-Imidazol-1-yl-3-trimethylsilanyl-propynon 1.1 g Produkt **Z5n** erhalten.

### ZWISCHENVERBINDUNG 17. N-{7-OXO-6-[1-(2-TRIMETHYLSILANYL-ETHOXYMETHYL)-1 H-IMIDAZOL-4-CARBONYL]-4,5,6,7-TETRAHYDRO-BENZOTHIAZOL-2-YL}-ACETAMID

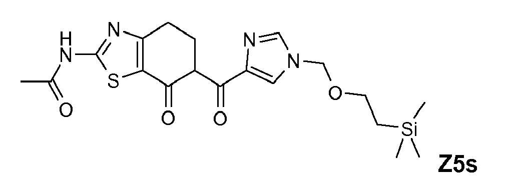

Eine Lösung von 125 ml (0.125 mol) LHMDS (Lithiumhexamethyldisilazid) in 100mL Tetrahydrofuran wird bei -20 °C mit 10.5 g (50 mmol) Zwischenverbindung 1 versetzt und 0.75 Stunden nachgerührt. 15.6 g (53 mmol) Imidazol-1-yl-[1-(2-trimethylsilanylethoxymethyl)-1 H-imidazol-4-yl]-methanon werden in 80 ml Tetrahydrofuran gelöst zugegeben. Die entstandene Suspension wird 16 Stunden bei Raumtemperatur gerührt. Anschließend wird mit 2 N Salzsäure hydrolysiert und mit Methyl-*tert*-butylether extrahiert. Die organische Phase wird getrocknet und zur Trockene eingedampft. Der Rückstand wird chromatographisch gereinigt. Ausbeute: 1.5 g

### ZWISCHENVERBINDUNG 18. 2-ACETYLAMINO-7-OXO-4,5,6,7-TETRAHYDRO-BENZOTHIAZOL-6-CARBONSÄUREETHYLESTER:

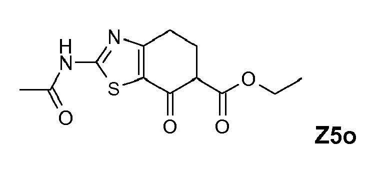

4.0 g (19 mmol) Zwischenverbindung 1 werden in 120 ml Tetrahydrofuran vorgelegt und auf -50 °C gekühlt. 80 ml (60 mmol) einer 1 molaren Lösung von Lithiumbis(trimethylsilylamid) in Tetrahydrofuran werden zugegeben und 5 Stunden bei - 30 °C bis -50 °C gerührt. Anschließend wird das Reaktionsgemisch mit 6.5 ml (80 mmol) Chlorameisensäureethylester versetzt, dann 1 Stunde nachgerührt. Es werden 50 ml Wasser zugegeben, mit 2 N Salzsäure sauer gestellt und mit Ethylacetat extrahiert. Die organische Phase wird getrocknet und zur Trockene eingedampft. Der Rückstand wird chromatographisch gereinigt, entsprechende Fraktionen vereinigt und eingedampft. Das Rohprodukt wird mit Diethylether verrieben und abgesaugt. Ausbeute: 2.3 g **Z5o**

### ZWISCHENVERBINDUNG 19. N-[7-OXO-6-(2,2,2-TRIFLUORO-ACETYL)-4,5,6,7-TETRAHYDRO-BENZOTHIAZOL-2-YL]-ACETAMID

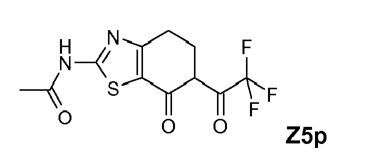

140 g (1.25 mol) 1,3-Cyclohexandion werden in 625 ml Chloroform gelöst, dann 145 ml (2.49 mol) Ethanol und 3.4 g (18 mmol) p-Toluolsulfonsäure zugegeben. Das Reaktionsgemisch wird 72 Stunden unter Rückfluss gerührt, wobei das entstehende Wasser mittels Wasserabscheider abgetrennt wird. Anschließend wird eingedampft und der Rückstand mit Diethylether versetzt, getrocknet und zur Trockene eingedampft. Der Rückstand wird durch Destillation gereinigt. Ausbeute: 166 g

4.6 g (0.115 mol) Natriumhydrid werden in 250 ml Diethylether suspendiert und auf 40 °C erhitzt. Dann wird eine Lösung aus 17 ml (0.14 mol) Trifluoressigsäureethylester und 10 g (71 mmol) der zuvor beschriebenen Zwischenstufe in 50 ml Diethylether zugetropft. Es wird 24 Stunden unter Rückfluss gerührt. Nach Abkühlen auf Raumtemperatur werden 150 ml Wasser zugesetzt und 0.1 Stunde bei Raumtemperatur nachgerührt. Die Phasen werden getrennt, die organische Phase mit 5%iger Natriumhydroxid-Lösung extrahiert. Die vereinigten basischen Wasserphasen werden sauer gestellt und mit Ethylacetat extrahiert. Die Ethylacetatphasen werden getrocknet und zur Trockene eingedampft. Das Rohprodukt wird chromatographisch gereinigt. Ausbeute: 4.1 g

0.4 g (1.7 mmol) der zuvor beschriebenen Zwischenstufe werden in 10 ml Dioxan und 10 ml Wasser gelöst, auf -10 °C gekühlt und dann mit 0.3 g (1.9 mmol) N-Bromsuccinimid versetzt. Es wird 1 Stunde bei Raumtemperatur gerührt und anschließend 0.13 g (1.7 mmol) Thioharnstoff zugegeben. Es wird 0.5 Stunden bei Raumtemperatur und 3 Stunden bei 80 °C gerührt. Nach Abkühlen wird das Reaktionsgemisch basisch gestellt und mit Dichlormethan extrahiert. Die organische Phase wird getrocknet und zur Trockene eingedampft. Ausbeute: 0.2 g

0.2 g (0.76 mmol) der zuvor beschriebenen Zwischenstufe werden in 10 ml (0.1 mol) Essigsäureanhydrid suspendiert und dann auf 100 °C erhitzt. Es wird 3 Stunden bei 100 °C und 16 Stunden bei Raumtemperatur gerührt. Anschließend wird das Reaktionsgemisch eingedampft, der Rückstand mit Eisessig versetzt und eingedampft.
Ausbeute: 0.3 g **Z5p**

### ZWISCHENVERBINDUNG 20. 1-PHENYL-4,5-DIHYDRO-1 H-PYRAZOLO[3',4':3,4]-BENZO[1,2-D]THIAZOL-7-YLAMIN

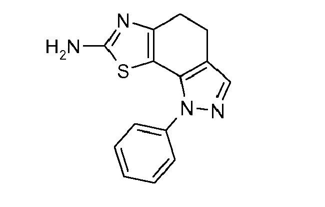

650 ml 37%ige Salzsäure werden in 650 ml Wasser vorgelegt und 99 g (0.27 mol) N-(1-Phenyl-4,5-dihydro-1H-pyrazolo [3',4':3,4]benzo[1,2-d]thiazol-7-yl)-acetamid (hergestellt analog zu Beispiel 1) darin gelöst. Die Lösung wird 2 Stunden unter Rückfluss gerührt. Nach Abkühlen auf Raumtemperatur wird mit Natronlauge vorsichtig basisch gestellt (pH 10-11). Der ausgefallene Niederschlag wird abgesaugt und mit Methanol ausgerührt.
Ausbeute: 66 g (Smp.: 307 - 308 °C)

### ZWISCHENVERBINDUNG 21. N-(6-BROM-7-OXO-4,5,6,7-TETRAHYDRO-BENZOTHIAZOL-2-YL)-ACETAMID

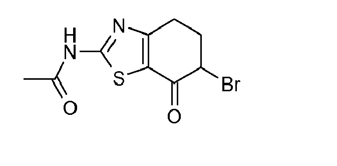

Eine Lösung von 2 g (9.5 mmol) Zwischenverbindung 1 in 60 ml Eisessig wird bei Raumtemperatur mit einer Lösung von 1.5 g (9.5 mmol) Brom in 10 ml Eisessig versetzt. Die Reaktionsmischung wird langsam auf 75 °C erwärmt, wobei eine rasche Entfärbung eintritt. Es wird zur Trockene eingedampft, der Rückstand in Methanol gelöst und das Produkt durch Zugabe von Wasser ausgefällt. Ausbeute: 2.2 g (Schmp.: 180-182 °C)

### ZWISCHENVERBINDUNG 22. N-(5-FORMYL-6-OXO-4,5,6,6A-TETRAHYDRO-3AH-CYCLOPENTATHIAZOL-2-YL)-ACETAMID

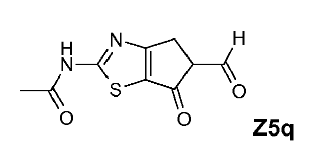

100 g (0.36 mol) 2-Brom-cyclopentan-1,3-dion (siehe M. Vanderwalle et al., Bull. Soc. Chim. Belg. 1966, 75, 648-654) werden in 370 ml Dimethylformamid gelöst und mit 43 g (0.36 mol) N-Acetylthioharnstoff versetzt. Es wird 3 Stunden bei 75 °C gerührt, dann bei 50 °C 15 g Aktivkohle zugesetzt. Nach Filtration über Kieselgur wird das Filtrat auf 10 °C gekühlt und mit 1200 ml Wasser versetzt. Der ausgefallene Niederschlag wird 16 Stunden bei Raumtemperatur nachgerührt, abgesaugt und getrocknet. Ausbeute: 20.4 g **Z4b** (Smp.: 270-272 °C)

27.6 g (0.51 mol) Natriummethylat werden in 50 ml Dimethylformamid suspendiert und bei Raumtemperatur eine Suspension aus 20.0 g (0.10 mol) **Z4b** in 350 ml Dimethylformamid innerhalb von 0.25 Stunden portionsweise zugetropft. Das Reaktionsgemisch wird 1 Stunde bei Raumtemperatur gerührt, anschließend auf 60 °C Innentemperatur erhitzt. Es wird eine Lösung aus 41 ml (0.51 mol) Ameisensäureethylester in 40 ml Benzol zugetropft und 2 Stunden gerührt. Nach Abkühlen auf 5 °C werden 100 ml halbkonzentrierte Salzsäure zugesetzt und mit Wasser auf 3000 ml verdünnt. Es fällt ein Niederschlag aus, dieser wird abgesaugt. Das Filtrat wird mit Dichlormethan extrahiert, die organische Phase getrocknet und zur Trockene eingedampft. Der Rückstand wird mit Dichlormethan/Diethylether 1:5 verrührt, abgesaugt und getrocknet. Ausbeute: 12.3 g **Z5q**

### ZWISCHENVERBINDUNG 23. N-(7-FORMYL-8-OXO-5,6,7,8-TETRAHYDRO-4H-CYCLOHEPTATHIAZOL-2-YL)-ACETAMID

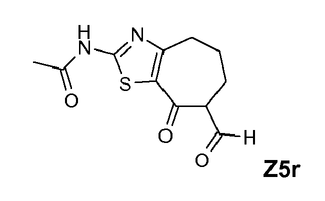

Eine Lösung von 7.4 g (90 mmol) Natriumacetat und 9.9 g (79 mmol) 1,3-Cycloheptadien in 300 ml Eisessig wird bei 15 °C mit 12.6 g (79 mmol) Brom versetzt und 30 min gerührt. Anschließend werden 6.0 g (79 mmol) Thioharnstoff zugegeben und die Suspension 5 Stunden unter Rückfluss gekocht. Die Essigsäure wird im Vakuum entfernt und der Rückstand in gesättigter Kochsalz-Lösung aufgenommen. Unlösliche Bestandteile werden abgesaugt und die wässrige Phase zunächst mit Ether extrahiert und dann mit Ammoniak alkalisch gestellt. Der ausgefallene Feststoff wird abgesaugt und getrocknet. Ausbeute: 3.7 g **Z3b**

3.7 g (20 mmol) der Verbindung **Z3b** werden in 50 ml Essigsäureanhydrid 1 Stunde unter Rückfluss erwärmt. Der nach dem Abkühlen ausgefallene Feststoff wird abgesaugt und mit Ether verrührt. Ausbeute: 2.4 g **Z4c**

1.7 g (31 mmol) Natriummethylat werden in 20 ml DMF suspendiert und portionsweise mit 2.4 g (11 mmol) der Verbindung **Z4c** versetzt. Nach 30 min wird auf -5 °C gekühlt, eine Lösung von 2.5 ml (31 mmol) Ameisensäureethylester in 10 ml Benzol zugetropft und bei Raumtemperatur über Nacht gerührt. Es wird mit 70 ml 1 N Salzsäure versetzt, der ausgefallene Niederschlag abgesaugt und mit Wasser gewaschen. Ausbeute: 2 g **Z5r**

### SYNTHESE VON VERBINDUNGEN DER FORMEL 1

### BEISPIEL 1: N-(1-P-TOLYL-4,5-DIHYDRO-1H-PYRAZOLO[3',4':3,4]BENZO[1,2-D]THIAZOL-7-YL)-ACETAMID

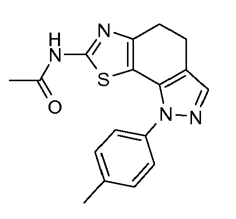

0.5 g (2 mmol) Zwischenverbindung 2 werden in 7.5 ml Eisessig vorgelegt, mit 0.32 g (2 mmol) p-Tolylhydrazin-Hydrochlorid versetzt und 3.5 Stunden auf 60 °C erwärmt. Der nach der Zugabe von 20 ml Wasser ausgefallene Niederschlag wird abgesaugt und aus Acetonitril unter Zusatz von Aktivkohle umkristallisiert. Ausbeute: 0.32 g (Smp.: 240-242 °C)

### BEISPIEL 2: N-[1-(5-FLUOR-2-METHYL-PHENYL)-4,5-DIHYDRO-1H-PYRAZOLO-[3',4':3,4]BENZO[1,2-D]THIAZOL-7-YL]-ACETAMID

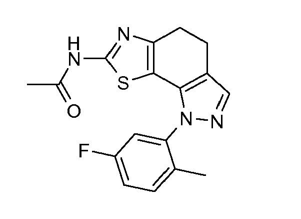

8.8 mg (0.05 mmol) 5-Fluoro-2-methyl-phenylhydrazin-Hydrochlorid werden in 2.5 ml Ethanol vorgelegt und mit 13.3 mg (0.05 mmol) Zwischenverbindung 3, gelöst in 2.5 ml Ethanol versetzt. Die Reaktionsmischung wird über Nacht auf 50 °C erwärmt, eingeengt und der Rückstand durch RP-HPLC gereinigt. Ausbeute: 13.3 mg

### BEISPIEL 3: 7-ACETYLAMINO-4,5-DIHYDRO-PYRAZOLO[3',4':3,4]BENZO[1 ,2-D]THIAZOL-1-CARBONSÄUREAMID

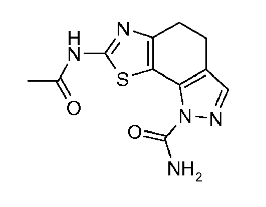

14 g (59 mmol) Zwischenverbindung 2 und 6.7 g (60 mmol) Semicarbazid werden in 130 ml Wasser suspendiert und 1 Stunde unter Rückfluss erhitzt. Der Feststoff wird abgesaugt und chromatographisch gereinigt. Ausbeute: 5.6 g (Schmp.: 265 - 270 °C)

### BEISPIEL 4: 1-(4-NITRO-PHENYL)-4,5-DIHYDRO-1 H-PYRAZOLO[3',4':3,4]BENZO[1,2-D]THIAZOL-7-YLAMIN

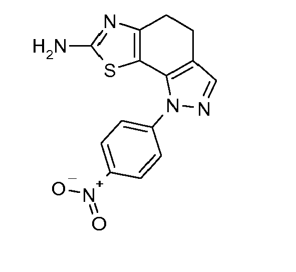

Analog zu Beispiel 1 werden aus 15 g (63 mmol) Zwischenverbindung 2 und 9.8 g (64 mmol) 4-Nitrophenylhydrazin 14 g Produkt erhalten (Schmp.: 300 - 310 °C).
7 g (20 mmol) dieser Zwischenstufe werden wie für Zwischenstufe 20 beschrieben mit 90 ml halbkonzentrierter Salzsäure verseift. Man erhält 5.2 g Produkt (Schmp.: 273 - 278 °C).

### BEISPIEL 5: N-(1-PHENYL-4,5-DIHYDRO-1H-PYRAZOLO [3',4':3,4]BENZO[1,2-D]THIAZOL-7-YL)-FORMAMID

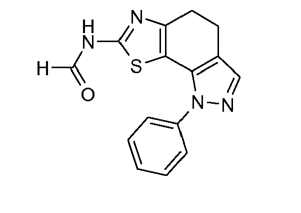

Eine Mischung aus 0.25 g (0.9 mmol) Zwischenverbindung 20 in 8 ml Ameisensäurephenylester wird 4 Stunden bei 60 °C gerührt. Nach Abkühlen auf Raumtemperatur wird der Niederschlag abgesaugt und mit wenig Aceton gewaschen. Das Rohprodukt wird aus Acetonitril unter Zusatz von Aktivkohle umkristallisiert. Ausbeute: 0.11 g (Smp.: 273 - 277 °C)

### BEISPIEL 6: (1-PHENYL-4,5-DIHYDRO-1 H-PYRAZOLO[3',4':3,4]BENZO[1,2-D]THIAZOL-7-YL)-CARBAMOYLSÄUREMETHYLESTER

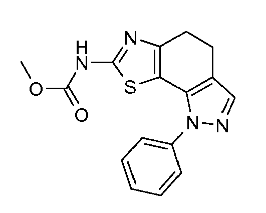

0.50 g (1.9 mmol) Zwischenverbindung 20 werden in 10 ml Pyridin suspendiert und auf 50 °C erhitzt. 0.6 ml (7.6 mmol) Chlorameisensäureethylester werden langsam zugegeben und die Reaktionsmischung dann 48 Stunden bei 50 °C gerührt. Im Verlauf dieser Zeit werden noch 2x je 0.6 ml des Chlorameisensäureethylesters zugegeben. Es wird 72 Stunden bei Raumtemperatur weitergerührt. Die Suspension wird filtriert, das Filtrat mit 130 ml Wasser versetzt. Der ausgefallene Niederschlag wird abgesaugt, gewaschen und getrocknet. Das Rohprodukt wird aus Methanol umkristallisiert. Ausbeute: 0.15 g (Smp.: 283-287 °C)

### BEISPIEL 7: 4-DIMETHYLAMINO-N-(1-PHENYL-4,5-DIHYDRO-1 H-PYRAZOLO-[3',4':3,4]BENZO[1, 2-D]THIAZOL-7-YL)-BUTYRAMID

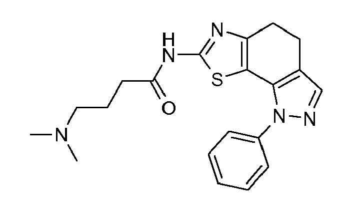

In einem verschließbaren Druckröhrchen werden 0.9 g (4.8 mmol) frisch hergestelltes 4-(Dimethylamino)-buttersäurechlorid-Hydrochlorid in 30 ml Tetrahydrofuran suspendiert und auf 50 °C erwärmt. Es werden 0.6 ml Triethylamin, danach portionsweise 0.5 g (1.9 mmol) Zwischenverbindung 20 zugegeben. Das Reaktionsgemisch wird 71 Stunden bei 70 °C gerührt. Nach Abkühlen auf Raumtemperatur wird der ausgefallene Niederschlag abgesaugt, gewaschen und in gesättigter Natriumhydrogencarbonatlösung und Chloroform aufgenommen. Die organische Phase wird mit Wasser gewaschen, getrocknet und zur Trockene eingedampft. Das Rohprodukt wird aus Acetonitril unter Zusatz von Aktivkohle umkristallisiert. Ausbeute: 0.2 g (Smp.: 165-166 °C)

### BEISPIEL 8: (1-PHENYL-4,5-DIHYDRO-1 H-PYRAZOLO[3',4':3,4]BENZO[1,2-D]THIAZOL-7-YL)-THIOCARBAMOYLSÄURE-S-ETHYLESTER

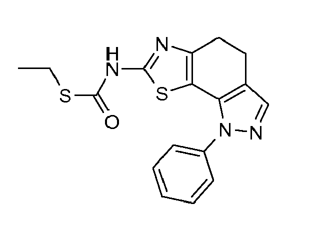

2.7 g (9.9 mmol) Zwischenverbindung 20 werden in 70 ml Pyridin vorgelegt und auf 50 °C erhitzt. Zu dieser Suspension werden 1.6 ml (15 mmol) Ethylthiochlorfomiat gegeben. Die entstandene Lösung wird 2 Stunden bei 50 °C gerührt. Nach Abkühlen auf Raumtemperatur wird die Lösung in 700 ml Wasser gegeben, der ausgefallene Niederschlag abgesaugt, gewaschen und getrocknet. Ausbeute: 2.2 g

### BEISPIEL 9: (1-PHENYL-4,5-DIHYDRO-1 H-PYRAZOLO[3',4':3,4]BENZO[1,2-D]THIAZOL-7-YL)-HARNSTOFF

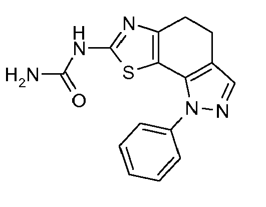

In einem verschließbarem Druckröhrchen werden zu einer Suspension aus 0.5 g (1.4 mmol) der in Beispiel 8 beschriebenen Verbindung in 9 ml Ethanol und 7 ml (14 mmol) 2 molare ethanolische Ammoniaklösung gegeben. Das Röhrchen wird verschlossen und das Reaktionsgemisch insgesamt 24 Stunden bei 80 °C gerührt. Nach 5 Stunden Reaktionszeit werden weitere 3 ml (6 mmol) der Ammoniaklösung zugegeben. Nach Abkühlen auf Raumtemperatur wird der ausgefallene Niederschlag abgesaugt, gewaschen und getrocknet. Das Rohprodukt wird aus Isopropanol unter Zusatz von Aktivkohle umkristallisiert. Ausbeute: 0.16 g (Smp.: 321-325 °C)

### BEISPIEL 10: 3-METHANESULFONYL-PHENYL)-(1-PHENYL-4,5-DIHYDRO-1H-PYRAZOLO[3',4':3,4]BENZO[1,2-D]THIAZOL-7-YL)-AMIN

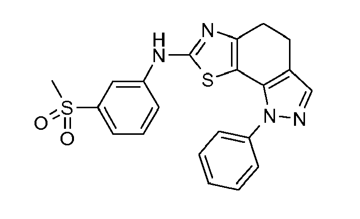

Eine Mischung aus 0.5 g (2 mmol) Zwischenverbindung 20, 0.5 g (2 mmol) 1-Brom-3-methansulfonylbenzol, 0.13 g (0.4 mmol) Tri-*tert*-butylphosphintetrafluoroborat, 0.2 g (0.2 mmol) Tris(dibenzylidenaceton)-dipalladium (0) und 0.2 g (2 mmol) Natriumcarbonat in 10 ml DMF wird 48 Stunden bei 90 °C gerührt. Die Reaktionsmischung wird mit Dichlormethan und Wasser versetzt und die wässrige Phase mit Dichlormethan extrahiert.

Die vereinten organischen Phasen werden getrocknet, eingeengt und der verbliebene Rückstand säulenchromatographisch gereinigt. Ausbeute: 25 mg

### BEISPIEL 11: 1-TERT-BUTYL-3-{4-[3-(1-PHENYL-4,5-DIHYDRO-1H-PYRAZOLO[3',4':3,4]BENZO [1,2-D]THIAZOL-7-YL)-UREIDO]-BUT-2-YNYL}-HARNSTOFF

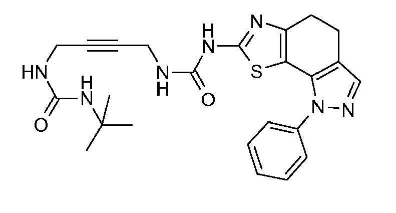

Eine Lösung von 0.4 g (1.5 mmol) Zwischenverbindung 20 in 0.5 ml Pyridin, 3 ml Dichlormethan und 0.5 ml THF wird mit 0.4 g (2 mmol) Chlorameisensäure-4-nitrophenylester versetzt und 2 Stunden bei Raumtemperatur gerührt. Anschließend wird die Reaktionsmischung mit 0.5 g (2.7 mmol) (4-Amino-but-2-ynyl)-carbamoylsäure *tert-*butylester und 1 ml Pyridin versetzt und weitere 24 Stunden gerührt. Nach Zugabe von Dichlormethan und Wasser wird die wässrige Phase mit Dichlormethan extrahiert. Die vereinten organischen Phasen werden eingeengt und der verbliebene Rückstand säulenchromatographisch gereinigt. Ausbeute: 0.2 g (Schmp.: 196 °C)

### BEISPIEL 12: 1-(4-AMINO-BUT-2-YNYL)-3-(1-PHENYL-4,5-DIHYDRO-1 H-PYRAZOLO[3',4':3,4]BENZO[1,2-D]THIAZOL-7-YL)-HARNSTOFF

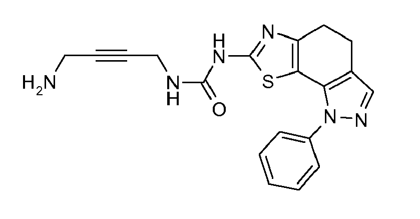

0.1 g (0.2 mmol) der in Beispiel 11 erhaltenen Verbindung werden in 0.5 ml Trifluoressigsäure und 2 ml Dichlormethan suspendiert und über Nacht bei Raumtemperatur gerührt. Die Reaktionsmischung wird eingeengt, der Rückstand in Aceton aufgenommen und das Produkt durch Zugabe von etherischer Salzsäure gefällt.
Ausbeute: 0.1 g (Schmp.: 232 °C)

### BEISPIEL 13: 2-[(1-ETHYL-PYRROLIDIN-2-YLMETHYL)-AMINO]-N-(1-PHENYL-4,5-DIHYDRO-1H-PYRAZOLO[3',4':3,4]BENZO[1,2-D]THIAZOL-7-YL)-ACETAMID

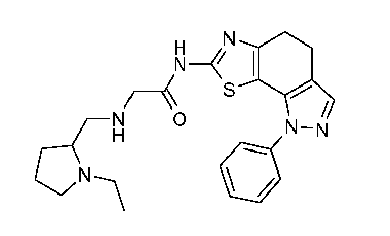

0.8 g (6.2 mmol) Bromessigsäure werden in 20 ml DMF gelöst, mit 4.9 g N-Cyclohexylcarbodiimid-N'-methyl-polystyrol HL (1.9 mmol/g) versetzt und 30 Minuten bei Raumtemperatur gerührt. Anschließend wird eine Lösung von 0.83 g (3.1 mmol) Zwischenverbindung 20 zugegeben und über Nacht gerührt. Das Polymer wird abgesaugt und mit DMF nachgewaschen. Das Filtrat wird eingeengt und der Rückstand in Essigsäureethylester/Diisopropylether verrieben. Ausbeute: 1 g

6 mg (0.048 mmol) C-(1-Ethyl-pyrrolidin-2-yl)-methylamin werden in 0.4 ml DMF gelöst und mit 0.02 ml (0.12 mmol) Triethylamin, gelöst in 0.1 ml DMF versetzt. Anschließend werden 16 mg (0.04 mmol) einer Lösung der zuvor beschriebenen Bromid-Zwischenstufe in 0.5 ml DMF zugegeben. Die Reaktionsmischung wird über Nacht gerührt und dann eingeengt. Ausbeute: 4 mg.

### BEISPIEL 14: 2-AMINO-N-(1-PHENYL-4,5-DIHYDRO-1 H-PYRAZOLO[3',4':3,4]BENZO-[1,2-D]THIAZOL-7-YL)-ACETAMID

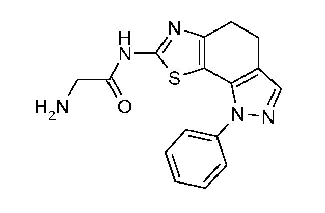

26 mg der im ersten Teil von Beispiel 13 erhaltenen Bromid-Zwischenstufe werden in 3 ml 33%ger wässriger Ammoniak-Lösung suspendiert und über Nacht bei Raumtemperatur gerührt. Die Reaktionsmischung wird mit Dichlormethan extrahiert und die vereinten organischen Phasen eingeengt. Der Rückstand wird durch RP-HPLC gereinigt. Ausbeute: 8 mg

### BEISPIEL 15: 2-HYDROXY-N-(1-PHENYL-4,5-DIHYDRO-1H-PYRAZOLO[3',4':3,4]-BENZO[1,2-D]THIAZOL-7-YL)-ACETAMID

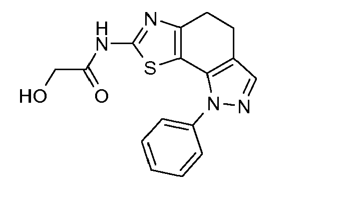

26 mg der im ersten Teil von Beispiel 13 erhaltenen Bromid-Zwischenstufe werden in 2 ml Wasser und 1 ml DMF suspendiert und 48 Stunden bei 100 °C gerührt. Die Reaktionsmischung wird durch RP-HPLC gereinigt. Ausbeute: 9 mg

### BEISPIEL 16: N-{1-[4-(MORPHOLIN-4-CARBONYL)-PHENYL]-4,5-DIHYDRO-1H-PYRAZOLO[3',4': 3,4]BENZO[1,2-D]THIAZOL-7-YL}-ACETAMID

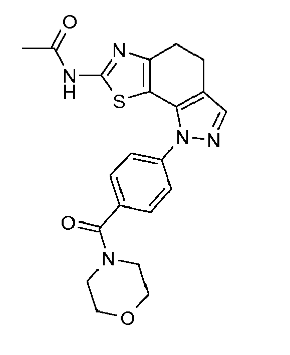

Eine Lösung von 3.5 mg (0.01 mmol) 4-(7-Acetylamino-4,5-dihydropyrazolo[3',4':3,4]benzo[1,2-d]thiazol-1-yl)-benzoesäure (hergestellt analog zu Beispiel 1) in 0.5 ml DMF wird mit 4 µL (0.03 mmol) Triethylamin und 4 mg (0.01 mmol) O-Pentafluorophenyl-1,1,3,3-tetramethyluroniumhexafluorophosphat (PFTU) versetzt und 15 Minuten gerührt. Zu diesem Gemisch wird eine Lösung von 1 mg (0.01 mmol) Morpholin in 0.5 ml DMF gegeben und 12 Stunden bei Raumtemperatur geschüttelt. Anschließend werden 3 mg Polyamin Harz HL (0.01 mmol, 200-400 mesh) zugegeben und weitere 8 Stunden geschüttelt. Das Harz wird abfiltriert und das Filtrat im Vakuum zur Trockene eingedampft.

### BEISPIEL 17: N-{1-[4-(4-AMINO-PIPERIDIN-1-CARBONYL)-PHENYL]-4,5-DIHYDRO-1 H-PYRAZOLO[3',4':3,4]BENZO[1,2-D]THIAZOL-7-YL}-ACETAMID

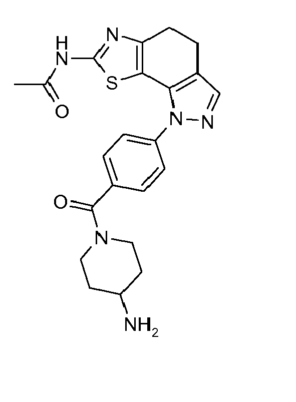

Eine Lösung aus 0.3 g (0.85 mmol) ) 4-(7-Acetylamino-4,5-dihydropyrazolo[3',4':3,4]benzo[1,2-d]thiazol-1-yl)-benzoesäure (hergestellt analog zu Beispiel 1), 0.32 g (1.00 mmol) TBTU und 0.75 ml (4.38 mmol) Diisopropylethylamin in 25 ml Dichlormethan wird 15 Minuten bei Raumtemperatur gerührt und dann mit 0.17 g (0.86 mmol) 4-N-Boc-aminopiperidin versetzt. Nach 2.5 Stunden wird die Reaktionsmischung auf 25 ml 5%ige Kaliumcarbonat-Lösung gegossen. Die organische Phase wird getrocknet und eingeengt. Der Rückstand wird mit Essigsäureethylester/Ether verrieben. Ausbeute: 0.43 mg (Schmp.: 230 °C)

0.40 g (0.75 mmol) der zuvor beschriebenen Verbindung werden in 10 ml etherischer Salzsäure suspendiert und 72 Stunden bei Raumtemperatur gerührt. Der Feststoff wird abgesaugt und mit Ether gewaschen. Ausbeute: 0.35 mg (Schmp.: 269 - 270 °C)

### BEISPIEL 18: N-{1-[4-(7-ACETYLAMINO-4,5-DIHYDRO-PYRAZOLO[3',4':3,4]-BENZO[1,2-D]THIAZOL-1-YL)-BENZOYL]-PIPERIDIN-4-YL}-2-PHENYL-ACETAMID

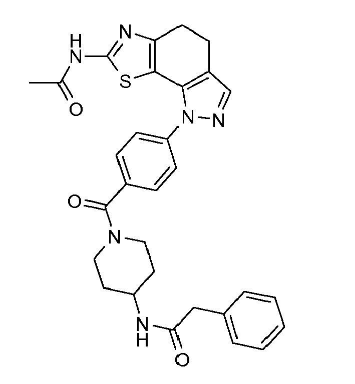

Eine Lösung aus 12 mg (0.09 mmol) Phenylessigsäure, 33 mg (0.09 mmol) TBTU und 80 µL (0.47 mmol) Diisopropylethylamin in 2 ml Dichlormethan wird 15 Minuten bei Raumtemperatur gerührt und dann mit 40 mg (0.09 mmol) der in Beispiel 17 beschriebenen Verbindung versetzt. Nach 2.5 Stunden wird die Reaktionsmischung auf 5%ige Kaliumcarbonat-Lösung gegossen. Die organische Phase wird getrocknet und eingeengt. Der Rückstand wird mit Essigsäureethylester verrieben. Ausbeute: 34 mg (Schmp.: 271-272 °C)

### BEISPIEL 19: N-[1-(4-METHYLSULFAMOYL-PHENYL)-4,5-DIHYDRO-PYRAZOLO[3',4':3,4]BENZO[1,2-D]THIAZOL-7-YL]-ACETAMID

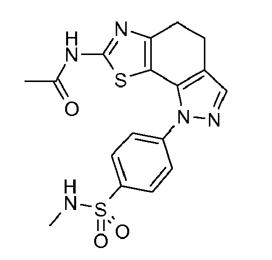

3.0 g (7.7 mmol) 4-(7-Acetylamino-4,5-dihydro-pyrazolo[3',4':3,4]benzo[1,2-d]thiazol-1-yl)-benzolsulfonsäure (hergestellt analog zu Beispiel 1) werden in 180 ml Phosphoroxychlorid vorgelegt, unter Kühlung werden 1.6 g (7.7 mmol) Phosphorpentachlorid zugegeben. Die Suspension wird 3 Stunden bei 90 °C und 16 Stunden bei Raumtemperatur gerührt, anschließend auf 5 °C gekühlt. Innerhalb von 0.75 Stunden wird die Reaktionslösung auf Eis getropft, dann mit Chloroform extrahiert. Die organische Phase wird getrocknet und zur Trockene eingedampft. Ausbeute: 1.3 g

In einem Druckreaktionsgefäß werden 0.15 g (0.37 mmol) des zuvor beschriebenen Sulfonsäurechlorids und 2 ml (4.0 mmol) Methylamin (2 M in THF) vorgelegt, dann wird 1 Stunde bei Raumtemperatur gerührt. Die Lösung wird eingedampft, der Rückstand aus Ethanol kristallisiert. Das Rohprodukt wird aus Methanol umkristallisiert. Ausbeute: 0.02 g (Smp.: >260 °C)

### BEISPIEL 20: N-(1-CYCLOHEX-2-ENYL-4,5-DIHYDRO-1H-PYRAZOLO[3',4':3,4]BENZO-[1,2-D]THIAZOL-7-YL)-ACETAMID

### BEISPIEL 21: N-(2-CYCLOHEX-2-ENYL-4,5-DIHYDRO-2H-PYRAZOLO[3',4':3,4]BENZO-[1,2-D]THIAZOL-7-YL)-ACETAMID

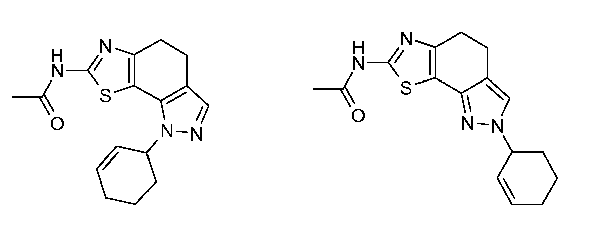

4.0 g (16.8 mmol) Zwischenverbindung 2 werden in 50 ml Eisessig suspendiert und mit 1.6 g (16.8 mol) Hydrazin-acetat versetzt. Es werden 3.5 Stunden bei 60 °C gerührt und anschließend 150 ml Wasser zugegeben. Der entstandene Niederschlag wird abgesaugt, gewaschen und getrocknet. Ausbeute: 2.8 g (Smp.: 261-264 °C)

0.2 g (0.85 mmol) der zuvor beschriebenen Zwischenstufe werden in 2 ml Dimethylacetamid gelöst und mit 0.1 g (2.5 mmol) Natriumhydroxid (gemörsert) versetzt. Es wird 0.5 Stunden bei Raumtemperatur gerührt. Anschließend werden 12 mg (0.035 mmol) Tetrabutylammoniumhydrogensulfat und 0.09 ml (0.9 mmol) 3-Bromcyclohexen zugegeben. Es wird 3 Stunden bei 120 °C gerührt. Das Reaktionsgemisch wird eingedampft, der Rückstand mit Wasser und Dichlormethan extrahiert. Die vereinten organischen Phasen werden getrocknet und zur Trockene eingedampft. Das Rohprodukt wird chromatographisch gereinigt. Ausbeute: 0.020 g (Smp.: 247-249 °C, Beispiel 21); Ausbeute: 0.060 g (Smp.: 213-214 °C, Beispiel 20)

### BEISPIEL 22: N-(1-BENZOYL-4,5-DIHYDRO-1 H-PYRAZOLO[3',4':3,4]BENZO[1,2-D]THIAZOL-7-YL)-ACETAMID

### BEISPIEL 23: N-(2-BENZOYL-4,5-DIHYDRO-2H-PYRAZOLO[3',4':3,4]BENZO[1,2-D]THIAZOL-7-YL)-ACETAMID

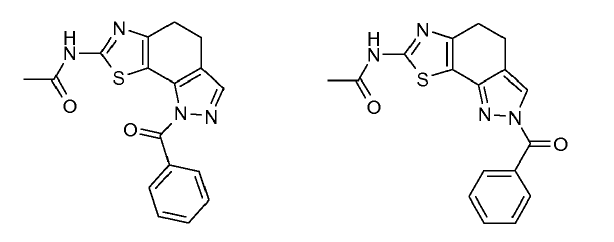

0.2 g (0.85 mmol) der in Beispiel 20/21 beschriebenen Zwischenstufe werden in 2 ml Benzol suspendiert und mit 0.25 ml (1.80 mmol) Triethylamin und 0.18 ml (1.55 mmol) Benzoylchlorid versetzt. Das Reaktionsgemisch wird 2 Stunden bei 75 °C gerührt. Anschließend wird die Suspension abgesaugt, der Niederschlag mit Ethylacetat und Wasser gewaschen. Ausbeute: 0.12 g (Smp.: 266-267 °C, Beispiel 23)
Die Mutterlauge wird eingedampft und der Rückstand chromatographisch gereinigt.
Ausbeute: 7 mg (Beispiel 22)

### BEISPIEL 24: N-[1-(2-CHLORO-PHENYL)-3-FURAN-2-YL-4,5-DIHYDRO-1H-PYRAZOLO[3',4':3,4] BENZO[1,2-D]THIAZOL-7-YL]-ACETAMID

### BEISPIEL 25: N-[2-(2-CHLORO-PHENYL)-3-FURAN-2-YL-4,5-DIHYDRO-2H-PYRAZOLO[3',4':3,4] BENZO[1,2-D]THIAZOL-7-YL]-ACETAMID

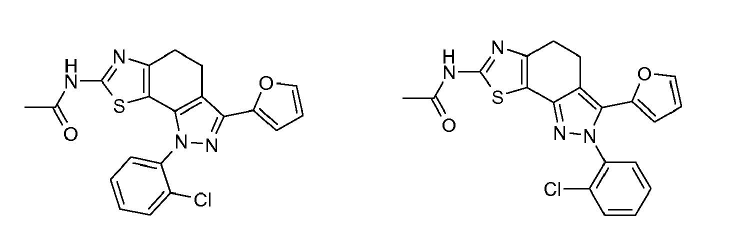

220 mg (0.7 mmol) Zwischenverbindung 4 werden in 3 ml Eisessig vorgelegt und mit 179 mg (0.7 mmol) 2-Chlor-phenylhydrazin-Hydrochlorid versetzt. Die Suspension wird über Nacht auf 50 °C erwärmt, dann mit 20 ml Wasser versetzt und der entstandene Niederschlag abgesaugt. Der Feststoff wird durch Säulenchromatographie (Laufmittel: Dichlormethan/Methanol 98:2) gereinigt. Ausbeute: 120 mg (gelber Feststoff, Smp.: 265-266 °C, Beispiel 24); 38 mg (Smp.: >300 °C, Beispiel 25)

### BEISPIEL 26: 4-(7-ACETYLAMINO-3-FURAN-2-YL-4,5-DIHYDRO-PYRAZOLO[3',4':3,4]BENZO[1,2-D]THIAZOL-1-YL)-3-CHLORO-N-(1-METHYLPIPERIDIN-4-YL)-BENZAMID

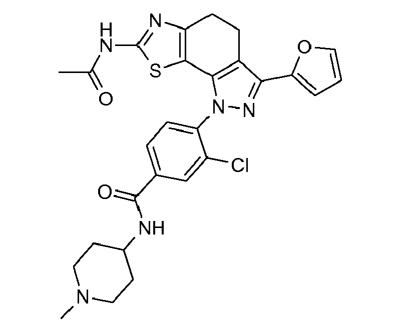

Analog zu Beispiel 1 erhält man aus 3.3 g (11 mmol) Zwischenverbindung 4 und 2.1 g (11 mmol) 3-Chloro-4-hydrazino-benzoesäuremethylester ein Gemisch der beiden möglichen Pyrazol-Isomere, die säulenchromatographisch getrennt werden. Ausbeute: 0.6 g Isomer A; 0.7 g Isomer B

0.7 g (1.5 mmol) des zuvor beschriebenen Isomers B werden in 8 ml Dioxan gelöst und mit einer Lösung von 0.1 g (4.4 mmol) Lithiumhydroxid in 1 ml Wasser versetzt. Nach 1.5 Stunden wird das Reaktionsgemisch mit 2 N Salzsäure sauer gestellt und der ausgefallene Feststoff wird abgesaugt. Das Produkt wird mit Ether verrührt. Ausbeute: 0.5 g

40 mg (0.09 mmol) der zuvor beschriebenen Säure, 33 mg (0.09 mmol) HATU und 46 µL Diisopropylethylamin werden in 3 ml DMF gelöst und 10 Minuten gerührt. Anschließend wird mit einer Lösung von 10 mg (0.09 mmol) Methylaminopiperidin in 2 ml DMF versetzt und das Gemisch 2 Stunden gerührt. Die Reaktionsmischung wird mit 15 ml 5%iger Kaliumhydrogencarbonat-Lösung verdünnt und mit Dichlormethan extrahiert. Die vereinten organischen Phasen werden mit Wasser gewaschen, getrocknet und eingeengt. Das Rohprodukt wird säulenchromatographisch gereinigt. Ausbeute: 24 mg

### BEISPIEL 27: N-[1-(2-CHLORO-PHENYL)-3-FURAN-3-YL-4,5-DIHYDRO-1H-PYRAZOLO[3',4':3,4] BENZO[1,2-D]THIAZOL-7-YL]-ACETAMID

### BEISPIEL 28: N-[2-(2-CHLORO-PHENYL)-3-FURAN-3-YL-4,5-DIHYDRO-2H-PYRAZOLO[3',4':3,4] BENZO[1,2-D]THIAZOL-7-YL]-ACETAMID

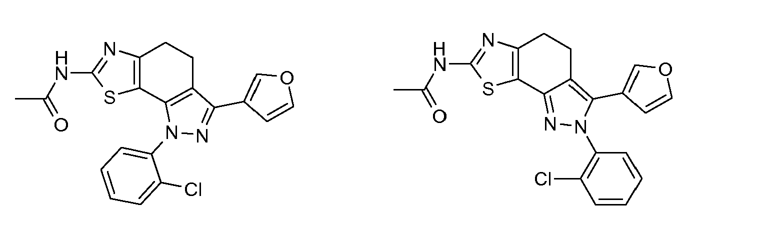

Analog zu Beispiel 1 werden aus 0.5 g (1.2 mmol) Zwischenverbindung 6 und 0.2 g (1.2 mmol) 2-Chlor-phenylhydrazin-Hydrochlorid nach säulenchromatographischer Reinigung 102 mg (Schmp. 265-266 °C, Beispiel 27); 12 mg (Smp.: >300 °C, Beispiel 28).

### BEISPIEL 29: N-[1-(2-CHLORO-PHENYL)-3-ISOPROPYL-4,5-DIHYDRO-1H-PYRAZOLO[3',4':3,4]BENZO[1,2-D]THIAZOL-7-YL]-ACETAMID

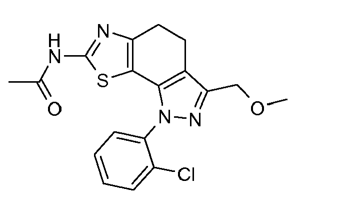

Analog zu Beispiel 1 werden aus 0.2 g (0.7 mmol) Zwischenverbindung 13 und 0.13 g (0.7 mmol) 2-Chlor-phenylhydrazin-Hydrochlorid 0.22 g Produkt erhalten.

### BEISPIEL 30: 4-(7-ACETYLAMINO-3-METHOXYMETHYL-4, 5-DIHYDRO-PYRAZOLO[3',4':3,4]BENZO[1,2-D]THIAZOL-1-YL)-3-CHLORO-N-(1-METHYLPIPERIDIN-4-YL)-BENZAMID

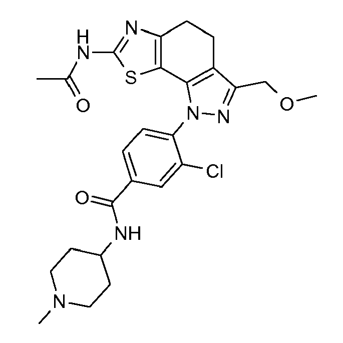

Analog zu Beispiel 1 erhält man aus 0.9 g (3.2 mmol) Zwischenverbindung 13 und 0.7 g (3.2 mmol) 3-Chloro-4-hydrazino-benzoesäuremethylester 1.2 g Produkt, dessen Ester-Funktion mit 0.2 g (8.4 mmol) Lithiumhydroxid analog zu Beispiel 26 verseift wird. Anschließend werden 50 mg der erhaltenen Säure mit Methylaminopiperidin wie in Beispiel 26 beschrieben einer Amid-Kupplung unterzogen.

### BEISPIEL 31: 7-ACETYLAMINO-1-(2-CHLORO-PHENYL)-4,5-DIHYDRO-1H-PYRAZOLO[3',4':3,4]BENZO[1,2-D]THIAZOL-3-CARBONSÄUREMETHYLESTER

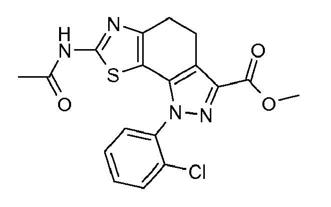

Analog zu Beispiel 1 werden aus 1.0 g (3.4 mmol) Zwischenverbindung 7 und 0.6 g (3.4 mmol) 2-Chlor-phenylhydrazin-Hydrochlorid 0.8 g Produkt erhalten (Schmp. 294 - 297 °C).

### BEISPIEL 32: 7-ACETYLAMINO-1-(2-CHLORO-PHENYL)-4,5-DIHYDRO-1 H-PYRAZOLO[3',4':3,4]BENZO[1,2-D]THIAZOL-3-CARBONSÄURE

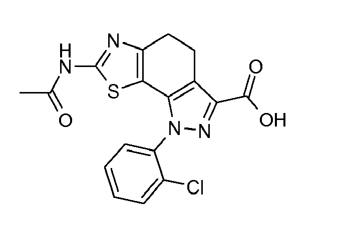

24 g (1 mol) der in Beispiel 31 beschriebenen Verbindung werden in 250 ml Dioxan vorgelegt und eine Lösung aus 4 g (10 mmol) Lithiumhydroxid in 35 ml Wasser zugegeben. Es wird 16 Stunden bei Raumtemperatur gerührt. Anschließend wird die Lösung leicht sauer gestellt und das Dioxan eingedampft. Die Suspension wird mit Wasser verdünnt, dann abgesaugt. Der Niederschlag wird getrocknet. Ausbeute: 23 g

### BEISPIEL 33: 7-ACETYLAMINO-1-PHENYL-4,5-DIHYDRO-1 H-PYRAZOLO[3',4':3,4]BENZO[1,2-D]THIAZOLE-3-CARBONSÄURE

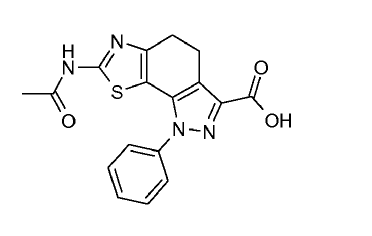

Analog zu Beispiel 1 werden aus 30 g (0.1 mol) Zwischenverbindung 7 und 10.3 ml (0.1 mol) Phenylhydrazin 27 g Produkt erhalten (Schmp.: 298 - 300 °C).
Von diesem werden 0.1 g (0.3 mmol) in 12 ml Methanol/Wasser (1:1) suspendiert und mit 0.4 ml 10%iger Kaliumhydroxid-Lösung versetzt. Nach 1.5 Stunden wird die Reaktionsmischung eingeengt und die Lösung mit verdünnter Salzsäure sauer gestellt. Der entstandene Niederschlag wird aus Acetonitril umkristallisiert. Ausbeute: 0.1 g (Schmp.: > 300 °C)

### BEISPIEL 34: N-[3-AMINO-1-(2-CHLORPHENYL)-4,5-DIHYDRO-1H-PYRAZOL[3',4':3,4]BENZO[1,2-D]THIAZO L-7-YL]-AC ETAM I D

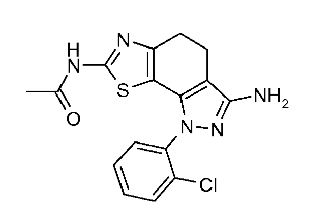

0.1 g (0.26 mmol) der in Beispiel 32 beschriebenen Verbindung, 0.06 ml (0.27 mmol) Phosphorsäure-diphenylester-azid (DPPA) und 0.04 ml (0.29 mmol) Triethylamin werden in 5 ml Dimethylacetamid vorgelegt und 2 Stunden bei 50 °C gerührt. Es werden 0.05 g (0.29 mmol) p-Toluolsulfonsäure und 0.10 ml Wasser zugesetzt und 16 Stunden bei 50 °C gerührt. Anschließend wird das Reaktionsgemisch chromatographisch gereinigt, entsprechende Fraktionen vereinigt und eingedampft. Der Rückstand wird aus Ethylacetat/Petrolether kristallisiert. Ausbeute: 0.02 g

### BEISPIEL 35: N-(3-AMINO-1-PHENYL-4,5-DIHYDRO-1 H-PYRAZOLO[3',4':3,4]-BENZO[1,2-D]THIAZOL-7-YL)-ACETAMID

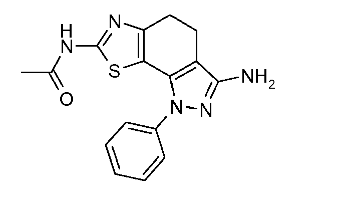

Analog zu Beispiel 34 wird aus 1.5 g der in Beispiel 33 beschriebenen Säure, 1 ml (4.6 mmol) DPPA und 0.6 ml (4.4 mmol) Triethylamin in 20 ml Dimethylacetamid und anschließender Umsetzung mit 2 g p-Toluolsulfonsäure und 10 ml Wasser 0.5 g Produkt erhalten.

### BEISPIEL 36: N-[1-(2-CHLORPHENYL)-3-(3-ISOPROPYL-UREIDO)-4,5-DIHYDRO-1H-PYRAZOL[3',4':3,4]BENZO[1,2-D]THIAZO L-7-YL]-AC ETAM I D

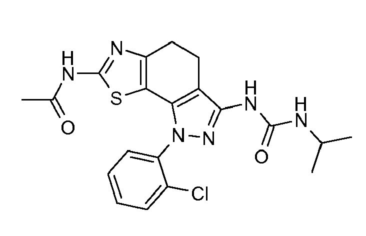

1.0 g (3.1 mmol) der in Beispiel 35 beschriebenen Verbindung und 1.0 ml (12.1 mmol) Pyridin werden in 10 ml Dichlormethan und 2 ml Tetrahydrofuran suspendiert und mit einer Lösung aus 0.8 g (4.0 mmol) Chlorameisensäure-4-nitrophenylester in 5 ml Dichlormethan versetzt. Es wird 0.5 Stunden bei Raumtemperatur gerührt. 1.8 ml dieser Lösung wird mit 0.05 ml (0.53 mmol) Isopropylamin versetzt. Es wird 16 Stunden bei Raumtemperatur gerührt, anschließend mit Dichlormethan und Wasser extrahiert. Die organische Phase wird getrocknet und zur Trockene eingedampft. Der Rückstand wird chromatographisch gereinigt, entsprechende Fraktionen vereinigt und eingedampft. Der Rückstand wird in Wasser gelöst, mit Natriumhydrogencarbonatlösung basisch gestellt und ausgefallene Kristalle abgesaugt. Ausbeute: 0.010 g

### BEISPIEL 37: N-[1-(2-CHLORO-PHENYL)-3-(3-METHYL-UREIDO)-4,5-DIHYDRO-1H-PYRAZOLO[3', 4':3,4]BENZO[1,2-D]THIAZOL-7-YL]-ACETAMID

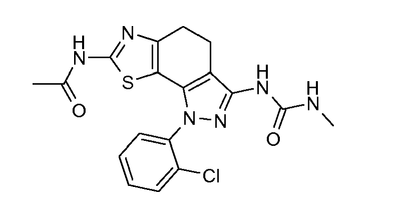

Analog zu Beispiel 36 wurde durch Verwendung von Methylamin das gewünschte Produkt erhalten.

### BEISPIEL 38: [7-ACETYLAMINO-1-(2-CHLORPHENYL)-4,5-DIHYDRO-1 H-PYRAZOL[3',4':3,4]BENZO[1,2-D]THIAZOL-73-YL]-CARBAMINSÄURE ISOPROPYLESTER

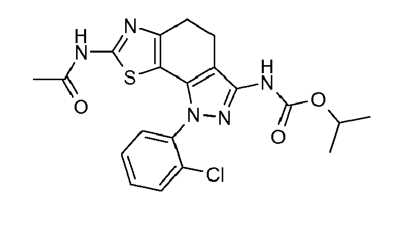

10 g (25 mmol) der in Beispiel 32 beschriebenen Verbindung, 6.0 ml (27 mmol) Phosphorsäure-diphenylester-azid (DPPA) und 4.0 ml (29 mmol) Triethylamin werden in 80 ml Dimethylacetamid vorgelegt und 2 Stunden bei 50 °C gerührt. 9 ml dieser Lösung werden mit 2 ml Isopropanol versetzt und 48 Stunden bei 50 °C gerührt. Anschließend wird mit Dichlormethan verdünnt, dann mit Kaliumhydrogensulfatlösung und Natriumhydrogencarbonatlösung gewaschen. Die organische Phase wird getrocknet und zur Trockene eingedampft. Der Rückstand wird in Dichlormethan gelöst und mit 0.3 g Anhydrid-Abfangharz (MP Anhydride Resin) 3 Stunden geschüttelt. Danach wird das Harz abgesaugt, mit Dichlormethan gewaschen und die organische Phase eingedampft. Der Rückstand wird chromatographisch gereinigt, entsprechende Fraktionen vereinigt und eingedampft. Ausbeute: 0.09 (Smp.: 196 °C)

### BEISPIEL 39: N-[1-(2-CHLORO-PHENYL)-3-(MORPHOLIN-4-CARBONYL)-4,5-DIHYDRO-1H-PYRAZOLO[3',4':3,4]BENZO[1,2-D]THIAZOL-7-YL]-ACETAMID

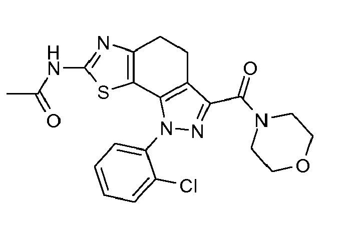

0.1 g (0.26 mmol) der in Beispiel 32 beschriebenen Verbindung werden in 5 ml Dichlormethan vorgelegt und 0.1 g (0.28 mmol) HATU (o-7-Azabenzotriazol-1-yl)-N,N,N',N'-tetramethyluronium-hexafluorophosphat) zugegeben. Anschließend wird mit 0.09 ml (0.53 mmol) Diisopropylethylamin und 0.03 ml (0.28 mmol) Morpholin versetzt. Das Reaktionsgemisch wird 4 Stunden bei Raumtemperatur gerührt, anschließend mit Dichlormethan und Wasser extrahiert. Die organische Phase wird getrocknet und zur Trockene eingedampft. Der Rückstand wird chromatographisch gereinigt. Entsprechende Fraktionen werden vereinigt, eingedampft, in Wasser gelöst und mit Natriumhydrogencarbonatlösung basisch gestellt. Der ausgefallene Feststoff wird abgesaugt und getrocknet. Ausbeute: 0.02 g

### BEISPIEL 40: 7-ACETYLAMINO-1-PHENYL-4,5-DIHYDRO-1 H-PYRAZOLO[3',4':3,4]BENZO[1,2-D]THIAZOL-3-CARBONSÄURE (2-HYDROXY-ETHYL)-METHYLAMID

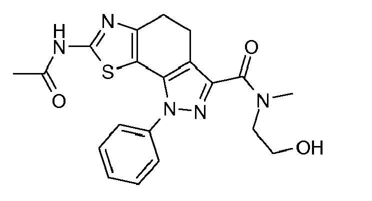

0.09 g (0.25 mmol) der in Beispiel 33 beschriebenen Verbindung werden in 5 ml Dimethylformamid vorgelegt, 0.1 ml (0.75 mmol) Triethylamin und 0.1 g (0.25 mmol) Dimethylamino-pentafluorphenyloxymethylen)-dimethyl-ammonium-hexafluorophosphat zugegeben. Es wird 0.1 Stunden bei Raumtemperatur gerührt, dann 0.02 g (0.25 mmol) 2-Methylamino-ethanol zugegeben. Das Reaktionsgemisch wird 16 Stunden bei Raumtemperatur und 24 Stunden bei 70° C gerührt. Anschließend wird eingedampft, der Rückstand chromatographisch gereinigt. Entsprechende Fraktionen werden vereinigt und gefriergetrocknet. Ausbeute: 0.04 g

### BEISPIEL 41: 7-ACETYLAMINO-4,5-DIHYDRO-1H-PYRAZOLO[3',4':3,4]BENZO[1,2-D]THIAZOLE-3-CARBONSÄUREETHYLESTER

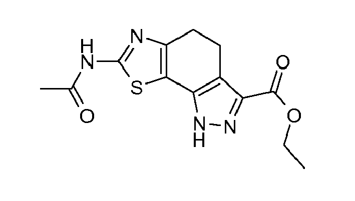

Analog zu Beispiel 1 werden aus 10 g (32 mmol) Zwischenverbindung 8 und 1.7 g (33 mmol) Hydrazin-Hydrat 6.4 g Produkt erhalten.

### BEISPIEL 42: N-[1-(2-CHLORO-PHENYL)-3-(1-METHYL-1 H-IMIDAZOL-4-YL)-4,5-DIHYDRO-1H-PYRAZOLO[3',4':3,4]BENZO[1,2-D]THIAZOL-7-YL]-ACETAMID

### BEISPIEL 43: N-[2-(2-CHLORO-PHENYL)-3-(1-METHYL-1 H-IMIDAZOL-4-YL)-4,5-DIHYDRO-2H-PYRAZOLO[3',4':3,4]BENZO[1,2-D]THIAZOL-7-YL]-ACETAMID

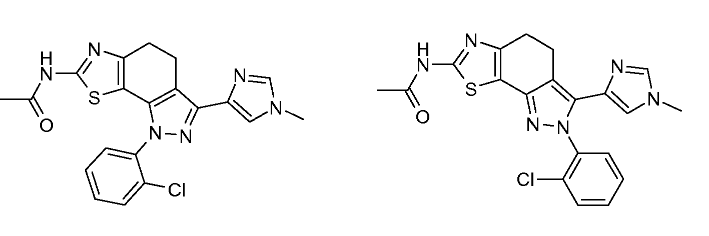

Analog zu Beispiel 1 werden aus 0.25 g (0.8 mmol) Zwischenverbindung 11 und 0.14 g (0.8 mmol) 2-Chlor-phenylhydrazin-Hydrochlorid nach säulenchromatographischer Reinigung 100 mg (Schmp. >300 °C, Beispiel 42) und 4 mg (Beispiel 43) erhalten.

### BEISPIEL 44: N-[1-(2-CHLORO-PHENYL)-3-ISOPROPYL-4,5-DIHYDRO-1H-PYRAZOLO[3',4':3,4]BENZO[1,2-D]THIAZOL-7-YL]-ACETAMID

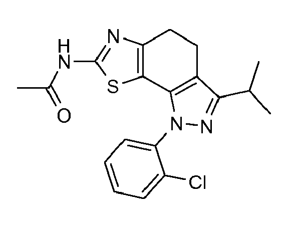

Analog zu Beispiel 1 werden aus 0.25 g (0.9 mmol) Zwischenverbindung 12 und 0.16 g (0.9 mmol) 2-Chlor-phenylhydrazin-Hydrochlorid nach säulenchromatographischer Reinigung 0.2 g Produkt erhalten.

### BEISPIEL 45: 4-(7-ACETYLAMINO-3-ISOPROPYL-4,5-DIHYDRO-PYRAZOLO-[3',4':3,4]BENZO[1,2-D]THIAZOL-1-YL)-3-CHLORO-N-(1-METHYL-PIPERIDIN-4-YL)-BENZAMID

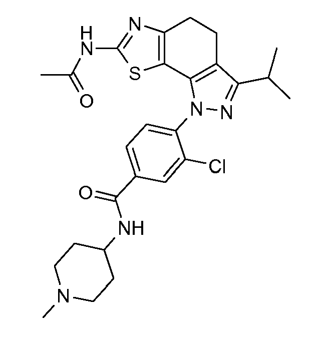

Analog zu Beispiel 1 erhält man aus 3.25 g (10.7 mmol) Zwischenverbindung 12 und 2.14 g (10.7 mmol) 3-Chloro-4-hydrazinbenzoesäuremethylester 0.68 g Produkt, dessen Ester-Funktion mit 0.1 g Lithiumhydroxid analog zu Beispiel 26 verseift wird. Anschließend werden 40 mg der erhaltenen Säure mit Methylaminopiperidin wie in Beispiel 26 beschrieben einer Amid-Kupplung unterzogen.

### BEISPIEL 46: N-[1-(2-CHLORO-PHENYL)-3-METHYL-4,5-DIHYDRO-1H-PYRAZOLO[3',4':3,4]BENZO[1,2-D]THIAZOL-7-YL]-ACETAMID

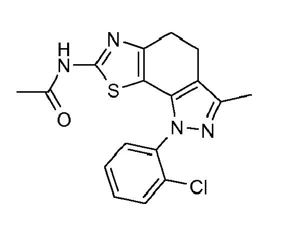

Analog zu Beispiel 1 werden aus 0.25 g (1.0 mmol) Zwischenverbindung 10 und 0.18 g (1.0 mmol) 2-Chlor-phenylhydrazin-Hydrochlorid 0.16 g Produkt erhalten.

### BEISPIEL 47: N-[1-(2-CHLORO-PHENYL)-3-PYRIDIN-3-YL-4,5-DIHYDRO-1H-PYRAZOLO[3',4':3, 4]BENZO[1,2-D]THIAZOL-7-YL]-ACETAMID

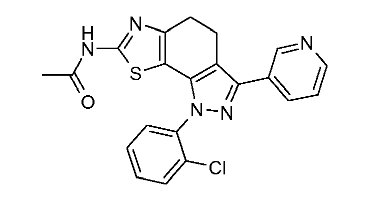

Analog zu Beispiel 1 werden aus 0.1 g (0.3 mmol) Zwischenverbindung 9 und 0.06 g (0.3 mmol) 2-Chlor-phenylhydrazin-Hydrochlorid nach säulenchromatographischer Reinigung 0.06 g Produkt erhalten.

### BEISPIEL 48: N-[1-(2-CHLORO-PHENYL)-3-PHENYL-4,5-DIHYDRO-1H-PYRAZOLO[3',4':3,4]BENZO[1,2-D]THIAZOL-7-YL]-ACETAMID

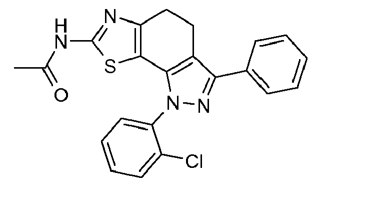

Analog zu Beispiel 1 werden aus 0.3 g (1.0 mmol) Zwischenverbindung 5 und 0.18 g (1.0 mmol) 2-Chlor-phenylhydrazin-Hydrochlorid 0.2 g Produkt erhalten (Schmp.: 232-234 °C).

### BEISPIEL 49: N-[1-(2-CHLORO-PHENYL)-3-PHENYL-4,5-DIHYDRO-1H-PYRAZOLO[3',4':3,4]BENZO[1,2-D]THIAZOL-7-YL]-4-DIMETHYLAMINO-BUTYRAMID

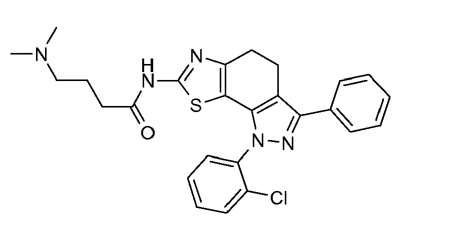

Eine Mischung von 0.5 g (1.2 mmol) der in Beispiel 48 erhaltenen Verbindung in 5 ml Wasser und 5 ml Salzsäure werden 2 Stunden unter Rückfluss gekocht. Der nach dem Abkühlen der Lösung ausgefallene Feststoff wird abgesaugt und getrocknet. Ausbeute: 0.4 g (Schmp.: 182 - 185 °C)

0.1 g der zuvor beschriebenen Zwischenverbindung werden in 5 ml Dichlormethan suspendiert, mit 0.16 ml (1.2 mmol) Triethylamin versetzt und 15 Minuten bei Raumtemperatur gerührt, wobei eine Lösung entsteht. Diese wird auf 40 °C erwärmt und 0.17 g (0.9 mmol) 4-Dimethylamino-butansäurechlorid zugegeben. Die Reaktionsmischung wird 3 Stunden bei 40 °C gerührt und anschließend mit 5%iger Natriumcarbonat-Lösung und Wasser gewaschen. Die organische Phase wird getrocknet, eingeengt und der Rückstand aus Isopropanol umkristallisiert. Ausbeute: 11 mg (Schmp. 290 - 291 °C)

### BEISPIEL 50: N-[3-(1 H-IMIDAZOL-4-YL)-1-(2-TRIFLUOROMETHYL-PHENYL)-4,5-DIHYDRO-1H-PYRAZOLO[3',4':3,4]BENZO[1,2-D]THIAZOL-7-YL]-ACETAMID

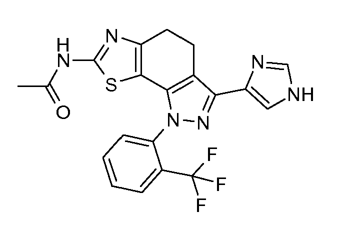

Analog zu Beispiel 1 werden aus 1.36 g (3.1 mmol) Zwischenverbindung 17 und 0.55 g (3.1 mmol) 2-Trifluormethyl-phenylhydrazin 1.26 g Zwischenprodukt erhalten. Von diesem werden 75 mg (0.13 mmol) in 0.65 ml (0.65 mmol) Tetrabutylammoniumfluorid-Lösung (1 M in THF) suspendiert und 5.5 Stunden unter Rückfluss gekocht. Die Lösung wird mit 5 ml pH 7-Pufferlösung versetzt, mit 10 ml Wasser verdünnt und mit Ethylacetat extrahiert. Die organische Phase wir mit Pufferlösung gewaschen, getrocknet und zur Trockene eingedampft. Der Rückstand wird chromatographisch gereinigt, entsprechende Fraktion mit Diethylether ausgerührt. Ausbeute: 5 mg (Schmp.: >300 °C)

### BEISPIEL 51: N-[1-(2-CHLORO-PHENYL)-3-(1H-IMIDAZOL-4-YL)-4,5-DIHYDRO-1H-PYRAZOLO[3',4':3,4]BENZO[1,2-D]THIAZOL-7-YL]-ACETAMID

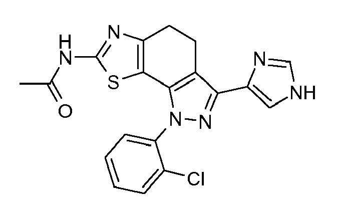

Analog zu Beispiel 1 werden aus 0.6 g (1.4 mmol) Zwischenverbindung 17 und 0.3 g (1.4 mmol) 2-Chlorphenylhydrazin-Hydrochlorid 0.5 g Zwischenprodukt erhalten. Dieses wird mit 1.7 ml (1.7 mmol) Tetrabutylammoniumfluorid-Lösung (1 M in THF) wir in Beispiel 50 beschrieben in das gewünschte Produkt überführt. Ausbeute: 35 mg (Schmp.: 287 - 288 °C)

### BEISPIEL 52: (R)-N-[1-(2-CHLORO-PHENYL)-3-(TETRAHYDRO-FURAN-2-YL)-4,5-DIHYDRO-1H-PYRAZOLO[3',4':3,4]BENZO[1,2-D]THIAZOL-7-YL]-ACETAMID

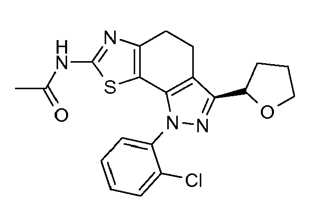

Analog zu Beispiel 1 werden aus 0.25 g (0.8 mmol) Zwischenverbindung 14 und 0.15 g (0.8 mmol) 2-Chlor-phenylhydrazin-Hydrochlorid nach säulenchromatographischer Reinigung 0.23 g Produkt erhalten.

### BEISPIEL 53: (R)-4-[7-ACETYLAMINO-3-(TETRAHYDRO-FURAN-2-YL)-4,5-DIHYDRO-PYRAZO LO[3',4': 3,4]BENZO[1,2-D]THIAZOL-1-YL]-3-CHLORO-N-PYRIDIN-4-YLMETHYL-BENZAMID

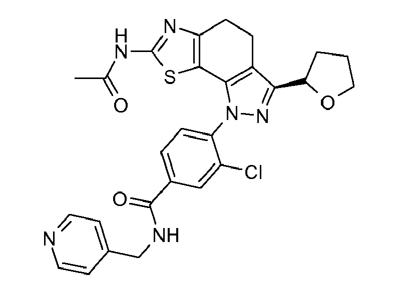

Analog zu Beispiel 1 erhält man aus 1.25 g (4.1 mmol) Zwischenverbindung 14 und 0.82 g (4.1 mmol) 3-Chloro-4-hydrazinbenzoesäuremethylester 1.16 g Produkt, dessen Ester-Funktion mit 0.2 g Lithiumhydroxid analog zu Beispiel 26 verseift wird. Anschließend werden 50 mg der erhaltenen Säure mit 4-Picolylamin wie in Beispiel 26 beschrieben einer Amid-Kupplung unterzogen.

### BEISPIEL 54: (S)-N-[1-(2-CHLORO-PHENYL)-3-(TETRAHYDRO-FURAN-2-YL)-4,5-DIHYDRO-1H-PYRAZOLO[3',4':3,4]BENZO[1,2-D]THIAZOL-7-YL]-ACETAMID

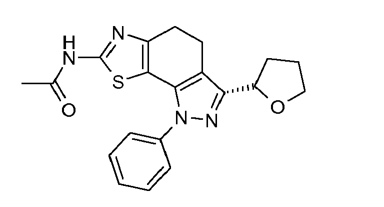

Analog zu Beispiel 1 werden aus 0.1 g (0.3 mmol) Zwischenverbindung 15 und 0.05 g (0.3 mmol) 2-Chlor-phenylhydrazin-Hydrochlorid nach säulenchromatographischer Reinigung 0.08 g Produkt erhalten.

### BEISPIEL 55: N-[1-(2-CHLORO-PHENYL)-3-TRIMETHYLSILANYLETHINYL-4,5-DIHYDRO-1H-PYRAZOLO[3',4':3,4]BENZO[1,2-D]THIAZOL-7-YL]-ACETAMID

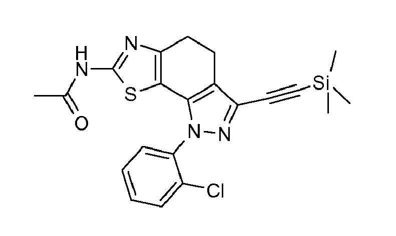

Analog zu Beispiel 1 werden aus 0.1 g (0.3 mmol) Zwischenverbindung 16 und 0.06 g (0.3 mmol) 2-Chlorphenylhydrazin-Hydrochlorid nach säulenchromatographischer Reinigung 0.02 g Produkt erhalten.

### BEISPIEL 56: N-[3-ETHINYL-1-(2-TRIFLUOROMETHYL-PHENYL)-4,5-DIHYDRO-1H-PYRAZOLO[3',4':3,4]BENZO[1,2-D]THIAZOL-7-YL]-ACETAMID

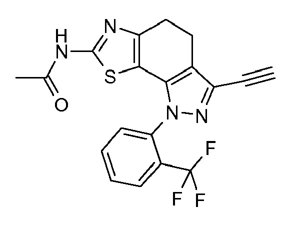

Analog zu Beispiel 1 werden aus 0.3 g (0.9 mmol) Zwischenverbindung 16 und 0.16 g (0.9 mmol) 2-(Trifluormethyl)phenylhydrazin nach säulenchromatographischer Reinigung 0.05 g Produkt erhalten. Von diesem werden 0.04 g in 5 ml THF vorgelegt und mit 0.1 ml (0.1 mmol) Tetrabutylammoniumfluorid-Lösung (1 M in THF) versetzt. Es wird 0.5 Stunden bei Raumtemperatur gerührt und dann Wasser zugegeben. Die wässrige Phase wird mit Ethylacetat extrahiert, die organische Phase mit Wasser und 1 N Salzsäure gewaschen. Die organische Phase wird getrocknet und zur Trockene eingedampft. Der Rückstand wird chromatographisch gereinigt. Ausbeute: 0.012 g

### BEISPIEL 57: N-(1-PHENYL-3-TRIFLUOROMETHYL-4,5-DIHYDRO-1 H-PYRAZOLO[3',4':3,4]BENZO[1,2-D]THIAZOL-7-YL)-ACETAMID

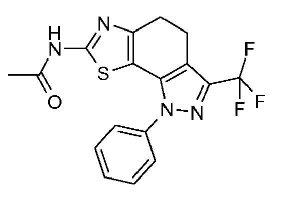

Analog zu Beispiel 1 werden aus 0.3 g (1.0 mmol) Zwischenverbindung 19 und 0.15 g (1.0 mmol) Phenylhydrazin-Hydrochlorid 0.04 g Produkt erhalten.

### BEISPIEL 58: N-[4-(4-CHLORO-PHENYL)-4,7-DIHYDRO-3-THIA-1,4,5-TRIAZA-CYCLOPENTA[A]PENTALEN-2-YL]-ACETAMID

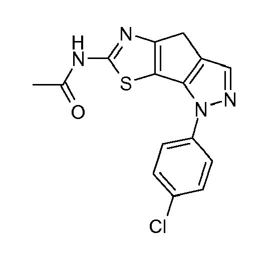

0.50 g (1.56 mmol) Zwischenverbindung 22 werden in 8 ml Eisessig suspendiert, mit 0.34 g (1.88 mmol) 4-Chlorphenylhydrazin-hydrochlorid versetzt. Es wird 1.5 Stunden bei 60 °C gerührt, dann auf Raumtemperatur abgekühlt. Nach Zugabe von 50 ml Wasser fällt ein Niederschlag aus. Dieser wird 0.1 Stunden bei 5 °C nachgerührt, abgesaugt und aus Methanol umkristallisiert. Ausbeute: 0.19 g (Smp.: 296-305 °C)

### BEISPIEL 59: 4 -PHENYL-4,7-DIHYDRO-3-THIA-1,4,5-TRIAZA-CYCLOPENTA[A]-PENTALEN-2-YL-AMIN

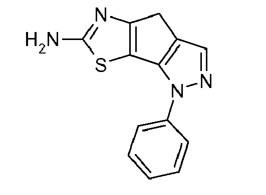

2.9 g (8.71 mmol) N-(4-Phenyl-4,7-dihydro-3-thia-1,4, 5-triaza-cyclopenta[a]pentalen-2-yl)-acetamid (hergestellt analog zu Beispiel 58 aus Zwischenverbindung 22) werden in 20 ml Wasser und 20 ml 32%iger Salzsäure suspendiert und 2 Stunden unter Rückfluss gerührt. Nach Abkühlen auf Raumtemperatur wird mit Diethylether extrahiert und die Wasserphase basisch gestellt. Der entstandene Niederschlag wird 0.25 Stunden bei 5 °C gerührt, abgesaugt und getrocknet. Das Rohprodukt wird in 150 ml Tetrahydrofuran suspendiert und mit 5 ml konz. Salzsäure versetzt, dann 16 Stunden bei 60 °C gerührt. Nach dem Abkühlen auf 5 °C wird der Niederschlag abgesaugt und getrocknet. Ausbeute: 1.8 g

### BEISPIEL 60) 4-O-TOLYL-3A,4,7,7A-TETRAHYDRO-3-THIA-1,4,5-TRIAZA-CYCLOPENTA[A]PENTALEN-2-YLAMIN

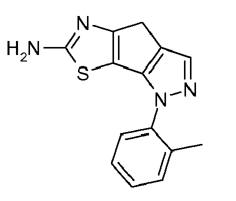

Analog zu Beispiel 59 werden aus 140 mg (0.5 mmol) N-(4-o-Tolyl-4,7-dihydro-3-thia-1,4,5-triaza-cyclopenta[a]pentalen-2-yl)-acetamid (hergestellt analog zu Beispiel 58) in 1 ml Wasser und 1 ml Salzsäure nach säulenchromatographischer Reinigung 14 mg Produkt erhalten.

### BEISPIEL 61: 4-DIMETHYLAMINO-N-(4-PHENYL-4,7-DIHYDRO-3-THIA-1,4,5-TRIAZA-CYCLOPENTA [A]PENTALEN-2-YL)-BUTANSÄUREAMID

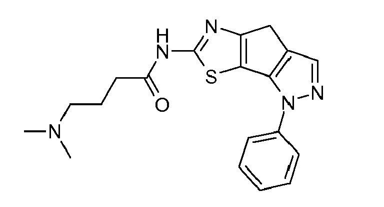

0.5 g (1.72 mmol) der unter Beispiel 59 beschriebenen Verbindung werden in 25 ml Dichlormethan suspendiert, mit 0.8 ml (6.02 mmol) Triethylamin versetzt und zum Rückfluss erhitzt. 0.8 g (4.30 mmol) 4-Dimethylamin-buttersäurechlorid-hydrochlorid werden in 5 ml Dichlormethan innerhalb 0.1 Stunden zugetropft und die Reaktionsmischung 16 Stunden unter Rückfluss gerührt. Nach Abkühlen auf Raumtemperatur wird der Niederschlag abgesaugt und das Filtrat mit 5%iger Natriumhydrogencarbonatlösung und Wasser gewaschen. Die organische Phase wird getrocknet und zur Trockene eingedampft. Der Rückstand wird mit Diethylether verrieben und abgesaugt. Ausbeute: 0.2 g (Smp.: 218-222 °C)

### BEISPIEL 62) N-[4-(2-CHLORO-PHENYL)-4,7-DIHYDRO-3-THIA-1,4,5-TRIAZA-CYCLOPENTA[A]PENTALEN-2-YL]-4-DIMETHYLAMINO-BUTANSÄUREAMID

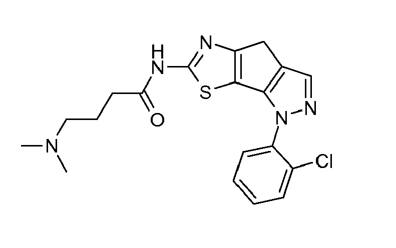

Analog zu Beispiel 59 werden aus 3.9 g (11.7 mmol) 4-(2-Chloro-phenyl)-4,7-dihydro-3-thia-1,4,5-triaza-cyclopenta[a]pentalen-2-ylamin (hergestellt analog zu Beispiel 58) 2.5 g einer Verbindung erhalten, von der 0.7 g (1.7 mmol) entsprechend dem Beispiel 61 zu 0.2 g Produkt umgesetzt werden (Smp.: 167-170 °C).

### BEISPIEL 63: PIPERIDIN-1-CARBONSÄURE-(4-PHENYL-4,7-DIHYDRO-3-THIA-1,4,5-TRIAZA-CYCLOPENTA[A]PENTALEN-2-YL)-AMID

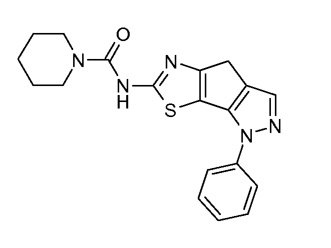

Eine Suspension aus 2.3 g (8 mol) der unter Beispiel 59 erhaltenen Verbindung in 60 ml Pyridin wird auf 50 °C erhitzt, dann mit 1.6 ml (10 mmol) Ethylchlorthioformiat versetzt. Das Reaktionsgemisch wird 12 Stunden bei 70 °C gerührt, anschließend in 600 ml Wasser gerührt. Der entstandene Niederschlag wird abgesaugt und mit Wasser und Ether gewaschen. Ausbeute: 2.3 g (Schmp.: 142-145 °C)

In einem verschließbaren Glasdruckröhrchen werden 0.3 g (0.7 mmol) der zuvor beschriebenen Verbindung in 8 ml Ethanol suspendiert, mit 1 ml (10.5 mmol) Piperidin versetzt und 16 Stunden unter Rückfluss gerührt. Nach Abkühlen auf Raumtemperatur wird das Reaktionsgemisch eingedampft. Das Rohprodukt wird über Kieselgel filtriert. Der Rückstand wird mit 20 ml Wasser und 5 Tropfen Acetonitril verrieben, abgesaugt und getrocknet. Ausbeute: 0.07 g (Smp.: 120-125 °C)

### BEISPIEL 64: N-[8-(4-HYDROXY-PHENYL)-4,5-DIHYDRO-THIAZOLO[4,5-H]CHINAZOLIN-2-YL]-ACETAMID

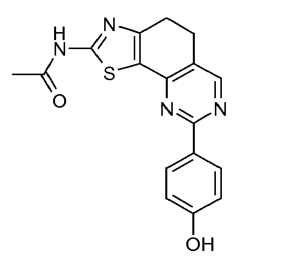

2.7 g (11.3 mmol) Zwischenverbindung 2 und 1.7 g (12.6 mmol) 4-Hydroxybenzamidin werden in 6 ml Pyridin suspendiert und 2 Stunden auf 100 °C erwärmt. Die Reaktionsmischung wird mit Ether versetzt und der ausgefallene Niederschlag mit Methanol verrührt. Ausbeute: 2.1 g

### BEISPIEL 65: 4,5-DIHYDRO-THIAZOLO[4,5-H]CHINAZOLIN-2,8-DIAMIN

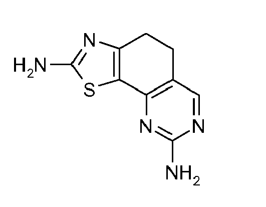

10 g (42 mmol) Zwischenverbindung 2, 4 g (42 mmol) Guanidin-Hydrochlorid und 2.2 g (21 mmol) Natriumcarbonat werden in 30 ml Amylalkohol suspendiert und 1 Stunde am Wasserabscheider unter Rückfluss erwärmt. Die Reaktionsmischung wird eingeengt, der Rückstand mehrmals mit Xylol abdestilliert und anschließend chromatographisch gereinigt. Das Produkt wird in Methanol aufgenommen und mit methanolischer Salzsäure als Hydrochlorid gefällt. Ausbeute: 1.2 g (Schmp.: 293 - 300 °C)

### BEISPIEL 66: N-(8-ACETYLAMINO-4,5-DIHYDRO-THIAZOLO[4,5-H]CHINAZOLIN-2-YL)-ACETAMID

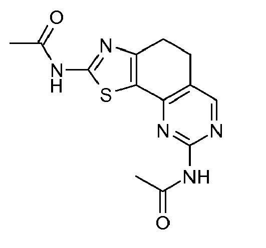

0.5 g (1.7 mmol) der in Beispiel 65 beschriebenen Verbindung und 0.4 g (4.6 mmol) Natriumacetat werden in 15 ml Essigsäureanhydrid suspendiert und 2 Stunden bei 100 °C gerührt. Es wird auf 5 °C gekühlt, der entstandene Feststoff abgesaugt und mit Wasser gewaschen. Das Rohprodukt wird mit Methanol verrührt. Ausbeute: 0.4 g (Schmp.: > 310 °C)

### BEISPIEL 67: N-(6-OXO-8-PHENYL-4,5,6,7-TETRAHYDRO-THIAZOLO[4,5-H]CHINAZOLIN-2YL)-ACETAMID

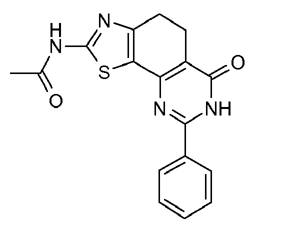

In einem Glasdruckröhrchen werden 0.1 g (0.35 mmol) Zwischenverbindung 18 und 0.1 g (0.83 mmol) Benzamidin in 1 ml Pyridin vorgelegt und 4 Stunden auf 160 °C erhitzt. Das Lösungsmittel wird eingedampft, der Rückstand wird mit heißem Ethanol verrührt und abgesaugt. Ausbeute: 14 mg

### BEISPIEL 68: N-(8-ETHYLSULFANYL-4,5-DIHYDRO-THIAZOLO[4,5-H]CHINAZOLIN-2YL)-ACETAMID

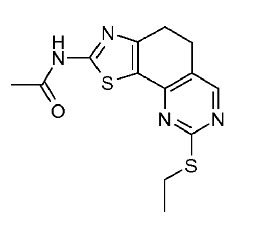

10.0 g (42 mmol) Zwischenverbindung 2 und 14.6 g (80 mmol) Ethylisothioharnstoffhydrobromid werden in 20 ml Pyridin suspendiert und dann 3 Stunden bei 110 °C gerührt. Nach Abkühlen auf Raumtemperatur wird das Reaktionsgemisch mit wenig Methanol verrieben, abgesaugt und getrocknet. Ausbeute: 10.2 g

### BEISPIEL 69: N-(8-ETHANSULFONYL-4,5-DIHYDRO-THIAZOLO[4,5-H]CHINAZOLIN-2YL)-ACETAMID

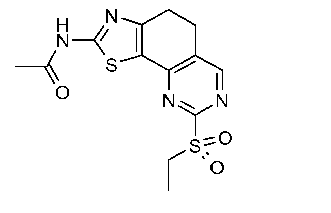

10.6 g (34.6 mmol) der unter Beispiel 68 hergestellten Verbindung werden in 100 ml Dichlormethan gelöst, 17.0 g (75.9 mmol) m-Chlorperbenzoesäure zugegeben und dann 24 Stunden bei Raumtemperatur gerührt. Das Reaktionsgemisch wird mit Dichlormethan verdünnt und mit Natriumcarbonatlösung extrahiert. Die organische Phase wird mit Wasser gewaschen, getrocknet und zur Trockene eingedampft. Ausbeute: 8.8 g

### BEISPIEL 70: [1-(2-ACETYLAMINO-4,5-DIHYDRO-THIAZOLO[4,5-H]CHINAZOLIN-8YL)-PIPERIDIN-3-YLMETHYL]-CARBAMINSÄURE-TERT-BUTYLESTER

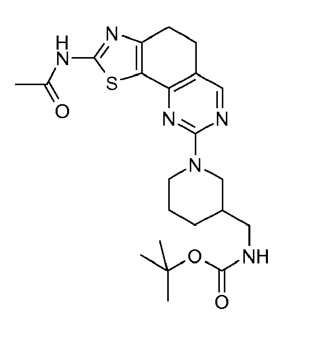

0.2 g (0.6 mmol) der unter Beispiel 69 hergestellten Verbindung und 0.25 g (1.2 mmol) Piperidin-3-ylmethyl-carbaminsäure-tert-butylester werden in 2 ml N-Methyl-pyrrolidin vorgelegt und 24 Stunden unter Rückfluss gerührt. Anschließend wird das Reaktionsgemisch mit verdünnter Kaliumcarbonatlösung und Dichlormethan versetzt und extrahiert. Die organische Phase wird mit Wasser gewaschen, getrocknet und zur Trockene eingedampft. Der Rückstand wird chromatographisch gereinigt. Ausbeute: 0.03 g (Smp.: 250-255 °C)

### BEISPIEL 71: 8-PHENYL-4,5-DIHYDRO-THIAZOLO[4,5-H]CHINAZOLIN-2YLAMIN

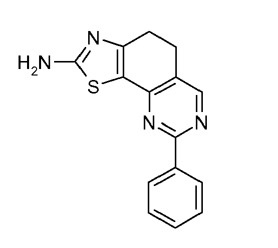

9.0 g (28 mmol) N-(8-Phenyl-4,5-dihydro-thiazolo[4, 5-h]quinazolin-2-yl)-acetamid (hergestellt analog zu Beispiel 64) werden in 130 ml halbkonzentrierter Salzsäure 2 Stunden unter Rückfluss erhitzt. Nach Abkühlen wird das Reaktionsgemisch basisch gestellt, der ausgefallene Niederschlag abgesaugt und getrocknet. Ausbeute: 7.3 g

### BEISPIEL 72: N*8*,N*8*-DIMETHYL-4,5-DIHYDRO-THIAZOLO[4,5-H]CHINAZOLIN-2,8-DIAMIN

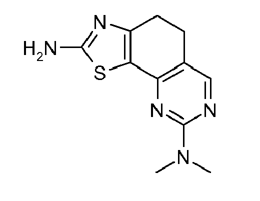

Analog zu Beispiel 71 können aus 0.20 g (0.7 mmol) N-(8-Dimethylamino-4,5-dihydrothiazolo[4,5-h]quinazolin-2-yl)-acetamid (erhalten analog zu Beispiel 58) 0.14 g Produkt erhalten werden (Schmp.: 292 - 295 °C)

### BEISPIEL 73: N-(8-PHENYL-4,5-DIHYDRO-THIAZOLO[4,5-H]CHINAZOLIN-2YL)-PROPIONSÄUREAMID

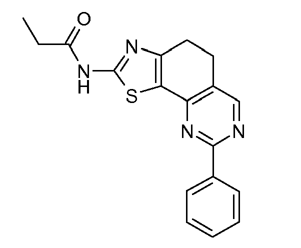

0.3 g (1.1 mmol) der in Beispiel 71 erhaltenen Verbindung werden in 15 ml Dichlormethan suspendiert, mit 0.4 ml (2.5 mmol) Triethylamin versetzt und zum leichten Rückfluss erhitzt. 0.2 ml (2.3 mmol) Propionsäurechlorid werden zugegeben und 5 Stunden unter Rückfluss gerührt. Nach Abkühlen auf Raumtemperatur wird die Reaktionslösung mit Natriumhydrogencarbonat und Wasser gewaschen, getrocknet und zur Trockene eingedampft. Der Rückstand wird mit Diethylether verrührt und abgesaugt. Ausbeute: 0.2 g (Smp.: 274-275 °C)

### BEISPIEL 74: 4-DIMETHYLAMINO-N-(8-PHENYL-4,5-DIHYDRO-THIAZOLO[4,5-H]CHINAZOLIN-2-YL)-BUTTERSÄUREAMID

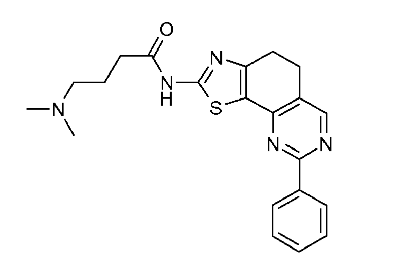

Analog zu Beispiel 73 werden aus 0.15 g (0.5 mmol) der in Beispiel 71 erhaltenen Verbindung, 1 ml Triethylamin und 0.2 g (1.3 mmol) 4-Dimethylamin-buttersäurechlorid-hydrochlorid 0.03 g Produkt erhalten.

### BEISPIEL 75: N-(8-PHENYL-4,5-DIHYDRO-THIAZOLO[4,5-H]CHINAZOLIN-2-YL)-FORMAMID

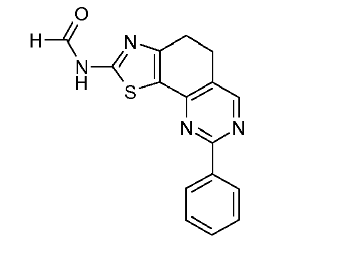

In einem Glasdruckröhrchen werden 0.2 g (0.71 mmol) der in Beispiel 71 beschriebenen Verbindung und 2.5 ml (22.7 mmol) Ameisensäurephenylester vorgelegt und 16 Stunden bei 80 °C gerührt. Anschließend wird das Reaktionsgemisch eingedampft und der Rückstand mit Diethylether/Ethanol verrieben. Ausbeute: 0.17g (Smp.: 304-306 °C)

### BEISPIEL 76: MORPHOLIN-4-CARBONSÄURE-(8-PHENYL-4,5-DIHYDRO-THIAZOLO[4,5-H]CHINAZOLIN-2-YL)-AMID

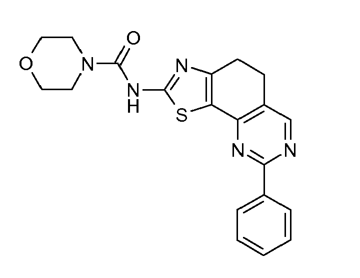

2.5 g (8.9 mmol) der in Beispiel 71 erhaltenen Verbindung werden in 50 ml Pyridin vorgelegt und auf 50 °C erhitzt. 1.5 ml (13.8 mmol) Ethylchlorthiolformiat werden zugegeben und 20 Stunden bei 50 °C gerührt. Nach Abkühlen auf Raumtemperatur wird die Reaktionslösung in Wasser gegeben und mit Dichlormethan extrahiert. Die organische Phase wird getrocknet und zur Trockene eingedampft. Der Rückstand wird mit Diethylether kristallisiert. Ausbeute: 1.0 g

In einem Glasdruckröhrchen werden 0.2 g (0.54 mmol) der zuvor beschriebenen Zwischenstufe und 0.1 g (1.15 mmol) Morpholin in 5 ml Ethanol vorgelegt und anschließend 16 Stunden bei 80 °C gerührt. Nach Abkühlen auf Raumtemperatur wird der ausgefallene Niederschlag abgesaugt und getrocknet. Ausbeute: 0.14 g (Smp.: 165-168 °C)

### BEISPIEL 77: 4-(2-ACETYLAMINO-4,5-DIHYDRO-THIAZOLO[4,5-H]CHINAZOLIN-8YL)-N,N-DIMETHYL-BENZAMID

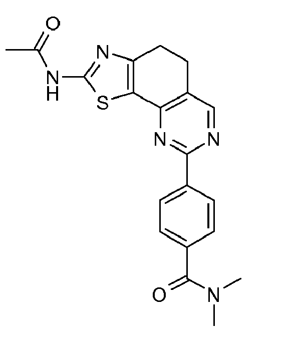

1.0 g (3.8 mmol) der Zwischenverbindung 2 und 0.8 g (3.1 mmol) Carbamimidoylbenzoesäure-methansulfonat werden in 5 ml Pyridin vorgelegt und 5 Stunden auf 180 °C erhitzt. Nach Abkühlen auf Raumtemperatur wird das Reaktionsgemisch mit Ethanol verrührt und abgesaugt. Ausbeute: 0.6 g

0.1 g (0.27 mmol) der zuvor hergestellten Verbindung und 0.1 g O-(1H-Benzotriazol-1-yl)-N,N,N',N'-tetramethyluronium-tetrafluorborat (TBTU) werden in 2 ml Dimethylformamid vorgelegt, mit 0.2 ml Diisopropylethylamin versetzt und 0.5 Stunden bei Raumtemperatur gerührt. Es werden 0.2 ml (4.0 mmol) Dimethylamin (2 M in THF) zugegeben und weitere 16 Stunden bei Raumtemperatur gerührt. Die entstandene Suspension wird mit Dimethylformamid gelöst, dann mittels RP-HPLC gereinigt. Entsprechende Fraktionen werden vereinigt und gefriergetrocknet. Ausbeute: 0.04 g (Smp.: >300° C)

### BEISPIEL 78: N-[8-(2-OXO-PYRROLIDIN-1-YL)-4,5-DIHYDRO-THIAZOLO[4,5-H]CHINAZOLIN-2-YL]-ACETAMID

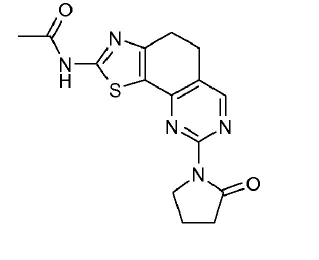

In einem Glasdruckröhrchen werden 2.0 g (8.4 mmol) Zwischenverbindung 2 und 2.4 g (16.8 mmol) 4-Guanidin-buttersäure in 30 ml Pyridin vorgelegt und dann 20 Stunden auf 190 °C erhitzt. Das Reaktionsgemisch wird eingedampft, der Rückstand mit Methanol aufgekocht und unlösliche Bestandteile abfiltriert. Aus der Mutterlauge wird das Rohprodukt als Hydrochlorid gefällt, abgesaugt und getrocknet. Der Feststoff wird säulenchromatographisch gereinigt. Ausbeute: 0.02g (Smp.: >160 °C)

### BEISPIEL 79: N-[8-(3-AMINOMETHYL-PIPERIDIN-1YL)-4,5-DIHYDRO-THIAZOLO[4,5-H]CHINAZOLIN-2YL]-ACETAMID

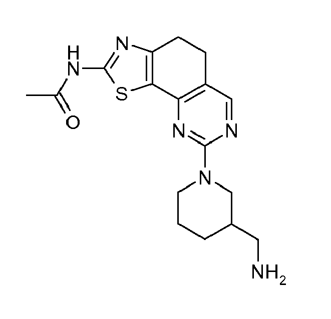

0.05 g (0.1 mmol) der unter Beispiel 70 beschriebenen Verbindung werden in 5 ml etherischer Salzsäure angelöst und 16 Stunden bei Raumtemperatur gerührt. Der entstandene Feststoff wird abgesaugt, mit Diethylether gewaschen und getrocknet. Ausbeute: 0.035 g

### BEISPIEL 80: N-{8-[3-(ISOPROPYLAMINO-METHYL)-PIPERIDIN-1YL]-4,5-DIHYDRO-THIAZOLO[4,5-H]CHINAZOLIN-2YL}-ACETAMID

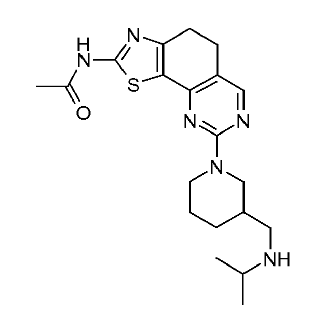

0.2 g (0.5 mmol) der in Beispiel 79 erhaltenen Verbindung, 0.05 ml (0.6 mmol) Aceton und 0.02 g (0,5 mol) Natriumborhydrid werden in Methanol gelöst und 0.5 Stunden bei 40 °C gerührt. Es werden nochmals die gleichen Mengen an Aceton und Natriumborhydrid zugegeben und weitere 16 Stunden bei Raumtemperatur gerührt. Anschließend wird das Reaktionsgemisch eingedampft, der Rückstand mit Dichlormethan und Wasser extrahiert. Die organische Phase wird getrocknet und zur Trockene eingedampft. Das Rohprodukt wird mit Diethylether verrieben und abgesaugt. Ausbeute: 0.040 g

### BEISPIEL 81: N-{8-[ACETYL-(2-DIMETHYLAMINO-ETHYL)-AMINO]-4,5-DIHYDRO-THIAZOLO[4,5-H]CHINAZOLIN-2YL}-ACETAMID

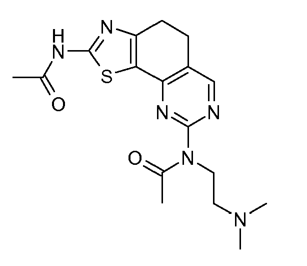

0.4 g (1.2 mmol) der unter Beispiel 69 erhaltenen Verbindung und 0.4 ml (4.5 mmol) N*1 *,N*1*-Dimethylethan-1,2-diamin werden in 5 ml N-Methylpyrollidin vorgelegt und 48 Stunden bei 90 °C gerührt. Das Reaktionsgemisch wird mit Dichlormethan und 10%-iger Kaliumcarbonatlösung extrahiert. Die organische Phase wird getrocknet und zur Trockene eingedampft. Ausbeute: 0.08 g

0.08 g (0.28 mmol) der zuvor beschriebenen Verbindung werden in 5 ml (49 mmol) Essigsäureanhydrid gelöst und 1 Stunde bei 140 °C gerührt. Das Reaktionsgemisch wird eingedampft, der Rückstand mit Acetonitril verrührt und abgesaugt. Ausbeute: 0.07 g

### BEISPIEL 82: (2-ACETYLAMINO-4,5-DIHYDRO-THIAZOLO[4,5-H]CHINAZOLIN-8YL)-PROP-2-INYL-CARBAMINSÄURE-TERT-BUTYLESTER

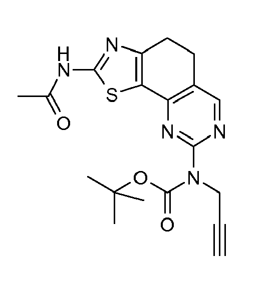

0.34 g (2.2 mmol) Prop-2-inyl-carbaminsäure-tert-butylester werden in 3 ml Tetrahydrofuran vorgelegt und auf -30 °C abgekühlt. 2.3 ml (4.4 mmol) 2 M Isopropylmethylmagnesiumchlorid-Lösung in Tetrahydrofuran werden langsam zugetropft und 0.5 Stunden gerührt. Danach wird eine Lösung aus 0.34 g (1,0 mmol) der in Beispiel 69 beschriebenen Verbindung in 4 ml Tetrahydrofuran bei -10 °C zugegeben und es wird 4 Stunden gerührt, wobei auf Raumtemperatur erwärmt wird. Das Reaktionsgemisch wird mit Wasser und Dichlormethan extrahiert, die organische Phase getrocknet und zur Trockene eingedampft. Der Rückstand wird mit Diethylether verrieben. Ausbeute: 0.18 g

### BEISPIEL 83: N-(8-PROP-2-INYLAMINO-4,5-DIHYDRO-THIAZOLO[4,5-H]CHINAZOLIN-2YL)-ACETAMID

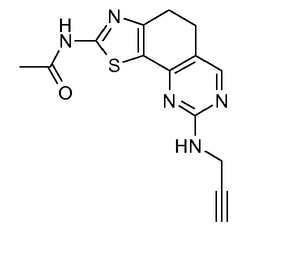

0.05 g (0.13 mmol) der in Beispiel 82 erhaltenen Verbindung werden in 5 ml etherischer Salzsäure gelöst und 16 Stunden bei Raumtemperatur gerührt. Der entstandene Feststoff wird abgesaugt, mit Diethylether/Aceton 9:1 verrieben und erneut abgesaugt. Ausbeute: 0.03g

### BEISPIEL 84: N-[1-(4-METHOXY-PHENYL)-1,4,5,6-TETRAHYDRO-9-THIA-1,2,7-TRIAZA-CYCLOPENTA[E]AZULEN-8-YL]-ACETAMID

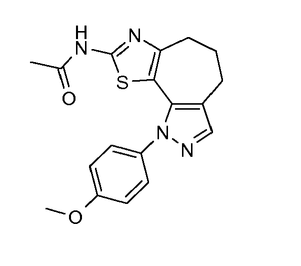

Eine Lösung von 0.25 g (1 mmol) Zwischenverbindung 23 und 0.18 mg (1 mmol) 4-Methoxyphenylhydrazin-Hydrochlorid in 5 ml Eisessig wird 2 h auf 60 °C erwärmt. Man versetzt mit Wasser und extrahiert dreimal mit Essigsäureethylester. Die vereinten organischen Phasen werden über Magnesiumsulfat getrocknet und eingeengt. Der Rückstand wird zunächst durch Säulenchromatographie (Laufmittel: Dichlormethan/Methanol 95:5), dann durch RP-HPLC gereinigt. Ausbeute: 0.07 g

### BEISPIEL 85: N-(8-NITRO-4,5-DIHYDRO-3H-1Λ*4*-NAPHTHO[2,1-D]THIAZOL-2-YL)-ACETAMID

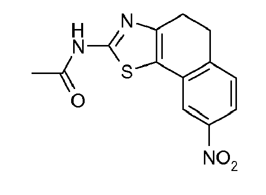

0.8 g (10.0 mmol) Natriumacetat und 1.9 g (9.40 mmol) 7-Nitro-3,4-dihydro-1H-naphthalen-2-on werden (hergestellt gemäß D.E. Nichols et al., J. Med. Chem. 1989, 32, 2128-2134) in 40 ml Essigsäure gelöst und auf 15 °C abgekühlt. Es werden 0.5 g (3.19 mmol) Brom zugegeben und 0.1 Stunden gerührt, bis das Brom vollständig abreagiert ist. Danach werden 0.8 g (10.5 mmol) Thioharnstoff zugegeben. Das Reaktionsgemisch wird 0.25 Stunden auf 60 °C erhitzt, anschließend abgesaugt und gewaschen. Die Kristalle werden aus Ethanol umkristallisiert. Ausbeute: 0.75 g (Schmp.: 247-248 °C)

0.75 g (3.0 mmol) der zuvor beschriebenen Verbindung werden in 5 ml (49 mmol) Essigsäureanhydrid vorgelegt und 0.1 Stunden unter Rückfluss gerührt. Nach Abkühlen auf Raumtemperatur wird der entstandene Niederschlag abgesaugt, mit Ethylacetat und Diethylether gewaschen und getrocknet. Ausbeute: 0.8 g (Schmp.: > 315 °C)

### BEISPIEL 86: N-(8-ACETYLAMINO-4,5-DIHYDRO-3H-1Λ*4*-NAPHTHO[2,1-D]THIAZOL-2-YL)-ACETAMI D

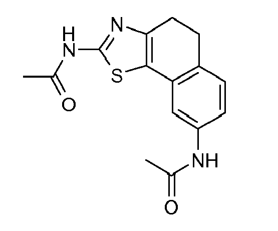

0.5 g (1.7 mmol) der in Beispiel 85 beschriebenen Verbindung werden in 10 ml Essigsäure suspendiert und mit 2 g Eisenpulver versetzt. Das Reaktionsgemisch wird auf 70 °C erwärmt und 0.1 Stunden gerührt. Anschließend wird abgesaugt und die Mutterlauge eingedampft. Der Rückstand wird mit Wasser versetzt, der Niederschlag abgesaugt, gewaschen und getrocknet. Ausbeute: 0.4 g

0.1 g (0.4 mmol) der zuvor beschriebenen Verbindung, 0.1 g (1,0 mmol) Essigsäureanhydrid und 0.1 ml Triethylamin werden in 5 ml Dichlormethan vorgelegt und 1 Stunde bei Raumtemperatur gerührt. Anschließend wird das Reaktionsgemisch mit 2 N Salzsäure und 2 N Natronlauge gewaschen. Die organische Phase wird getrocknet und zur Trockene eingedampft. Der Rückstand wird aus Ethylacetat kristallisiert. Ausbeute: 0.07 g (Smp.: 181-183 °C)

### BEISPIEL 87: N-(2-ACETYLAMINO-4,5-DIHYDRO-NAPHTHO[2,1-D]THIAZOL-8-YL)-ISOBUTTERSÄUREAMID

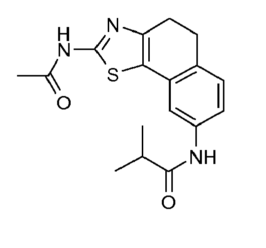

Analog zu Beispiel 86 können aus 0.1 g (0.4 mmol) der dort beschriebenen Zwischenstufe, 0.045 g (0.4 mmol) Isobuttersäurechlorid und 0.1 ml Triethylamin in 5 ml Dichlormethan 0.075 g Produkt erhalten werden (Schmp.: 272 - 274 °C)

### BEISPIEL 88: N-(8-ISOPROPYLAMINO-4,5-DIHYDRO-3H-1Λ*4*-NAPHTHO[2,1-D]THIAZOL-2-YL)-ACETAMID

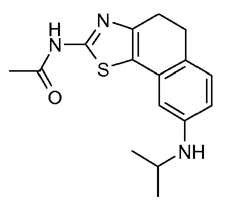

0.15 g (0.58 mmol) der in Beispiel 86 beschriebenen Zwischenstufe,1.0 g (17.2 mmol) Aceton und 0.4 g Natriumtriacetoxyborhydrid werden in 10 ml Dichlormethan vorgelegt und 4 Stunden bei Raumtemperatur gerührt. Das Reaktionsgemisch wird mit 50 ml Wasser und 2 g Kaliumcarbonat versetzt, die organische Phase abgetrennt, getrocknet und zur Trockene eingedampft. Der Rückstand wird chromatographisch gereinigt, anschließend aus Diethylether kristallisiert. Ausbeute: 0.11 g (Smp.: 183-184 °C)

### BEISPIEL 89: N-[5-(2-CHLORO-PHENYL)-8H-3-THIA-1,4,6-TRIAZA-CYCLOPENTA[A]INDEN-2-YL]-ACETAMID

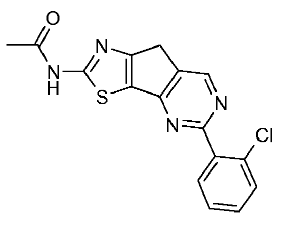

350 mg (1.56 mmol) Zwischenverbindung 22 und 298 mg (1.56 mmol) 2-Chlorbenzamidin werden in 1.5 mL Pyridin suspendiert und 1 Stunde auf 160 °C erwärmt. Das Reaktionsgemisch wird eingeengt und über RP-HPLC gereinigt. Ausbeute: 14 mg (Smp.: 257 °C)

### BEISPIEL 90: N-(5-MORPHOLIN-4-YL-8H-3-THIA-1,4,6-TRIAZA-CYCLOPENTA[A]-INDEN-2-YL)-ACETAMID

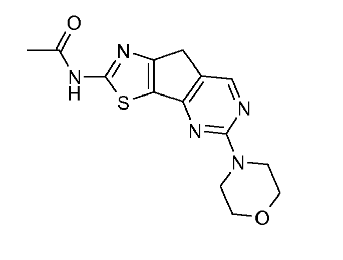

350 mg (1.56 mmol) Zwischenverbindung 22 und 260 mg (1.57 mmol) Morpholincarboxamidin werden in 1.5 mL Pyridin suspendiert und 1 Stunde auf 160 °C erwärmt. Das Reaktionsgemisch wird eingeengt und über RP-HPLC gereinigt. Ausbeute: 11 mg (Smp.: > 300 °C)

### BEISPIEL 91: N-(5-PHENYL-8H-3-THIA-1,4,6-TRIAZA-CYCLOPENTA[A]INDEN-2-YL)-ACETAMID

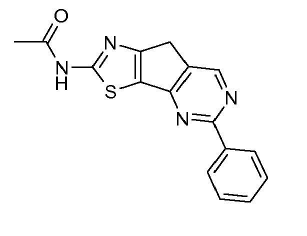

350 mg (1.56 mmol) Zwischenverbindung 22 und 190 mg (1.58 mmol) Benzamidin werden in 1.5 mL Pyridin suspendiert und 1 Stunde auf 160 °C erwärmt. Das Reaktionsgemisch wird eingeengt und über RP-HPLC gereinigt. Ausbeute: 16 mg (Smp.: > 300 °C)

Im Folgenden sind beispielhaft Verbindungen zusammengefasst, die nach einem der oben gezeigten Synthesewege dargestellt werden können. Alle Schmelzpunkte (mₚ) sind in °C angegeben. Um die inhibitorische Aktivität der Verbindungen auf die PI3Kγ ermitteln zu können, wurde ein *in-vitro* Kinase Assay etabliert, der auf der Übertragung des endständigen γ-Phosphats von ATP auf Phosphatidylinositol-4,5-bisphosphat (PIP₂) beruht. Als Enzym-Aktivität wurde die Gβ_{1γ2}-His stimulierte PI3K_{γ} eingesetzt. Die Expression und Reinigung von Gβ_{1γ2}-His und p101-GST/p110γ aus Sf9-Zellen (spodoptera frugiperda 9) wurde schon früher beschrieben (Maier et al., J. Biol. Chem. 1999 (274) 29311-29317).

Der Kinase-Assay wurde in weißen 384-well Flachboden-Platten durchgeführt. Jedes Well enthielt 5 µl der zu testenden Verbindung, die in Assay-Puffer (40 mM Hepes, pH 7,5, 100 mM NaCl, 1 mM EGTA, 1 mM β-Glycerophosphate, 1 mM DTT, 7 mM MgCl₂ and 0.1 % BSA; 6% DMSO) gelöst war. Dazu wurden jeweils 15 µl Lipidvesikel gegeben, die 10 ng PI3Kγ und 31.5 ng Gβ_{1γ2}-His enthielten. Die Lipidvesikel wiederum wurden generiert, indem PIP₂ (0.35µg/well), Phosphatidylethanolamin (3.75µg/well), Phosphatidylserin (3.5µg/well), Sphingomyelin (0.35µg/well) and Phosphatidylcholin (1.6µg/well) durch Ultraschallbehandlung in Lipid-Puffer (Assay-Puffer ohne DMSO) suspendiert wurden. Nach der Zugabe der Lipid-Vesikel wurde die Reaktion durch Zugabe von 10 µl Reaktions-Puffer (40 mM Hepes, pH 7,5, 100 mM NaCl, 1 mM EGTA, 1 mM β-Glycerophosphate, 1 mM DTT, 7 mM MgCl₂ and 0.1 % BSA; 1µM ATP und 0.2 µCi [γ-³³P]-ATP) gestartet. Die Reaktionsmischung wurde so für 1 h inkubiert und anschließend durch Zugabe einer Suspension von 0.12 mg LEADseeker beads (Amersham Biosciences) in Stop-Puffer (40 mM Hepes, pH 7,5, 100 mM NaCl, 1 mM EGTA, 12 mM EDTA, 1 mM β-Glycerophosphate, 1 mM DTT) gestoppt. Nach einer 1-minütigen Zentrifugation bei 500 x g wurden die Platten mit Hilfe eines LEADseeker-Gerätes ausgelesen und analysiert. Alle gezeigten Verbindungen weisen im Test einen IC₅₀-Wert unter 800 nM auf.

### BEISPIELE A

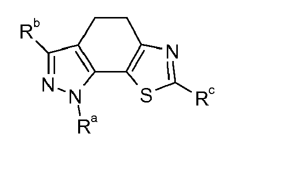

| | **R^{a}** | **R^{b}** | **R^{c}** | **mₚ** |
|---|---|---|---|---|
| 1. | 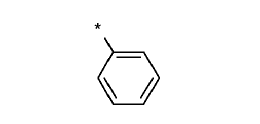 | H | 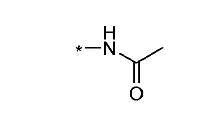 | |
| 2. | 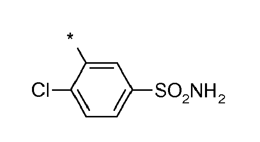 | H | 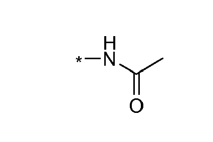 | |
| 3. | 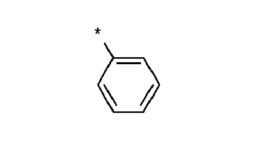 | -CH₃ | 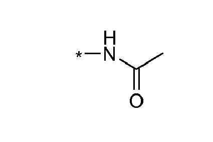 | >280 |
| 4. | 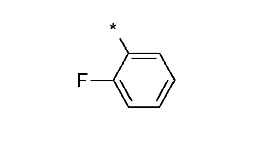 | 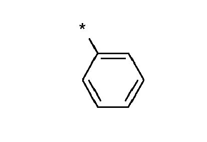 | 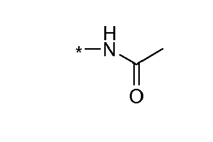 | 258-260 |
| 5. | 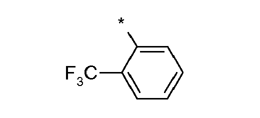 | 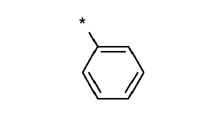 | 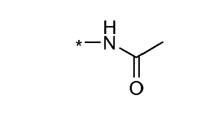 | 282-283 |
| 6. | 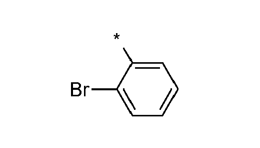 | 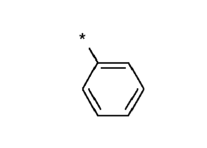 | 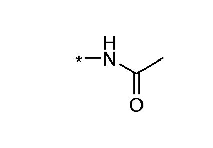 | 286-288 |
| 7. | 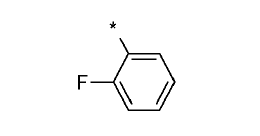 | 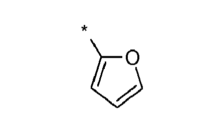 | 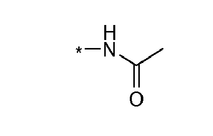 | 280-282 |
| 8. | 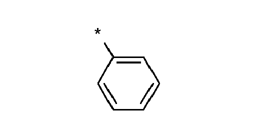 | 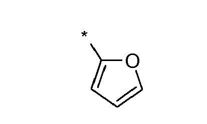 | 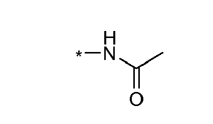 | > 300 |
| 9. | 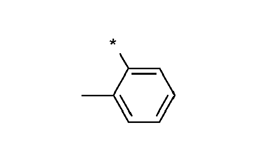 | 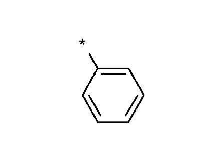 | 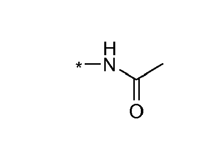 | 261-263 |
| 10. | 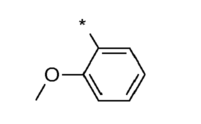 | 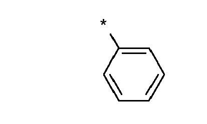 | 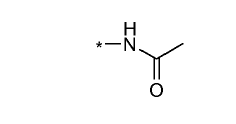 | 295-298 |
| 11. | 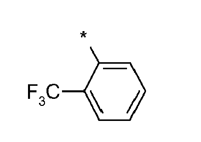 | 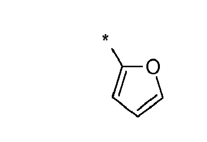 | 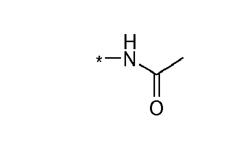 | > 300 |
| 12. | 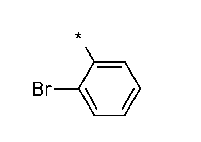 | 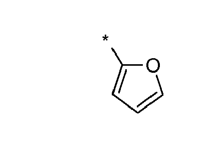 | 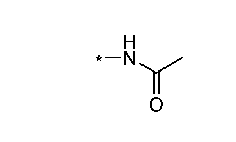 | > 300 |
| 13. | 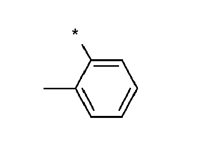 | 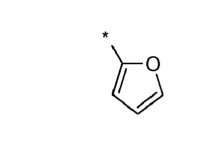 | 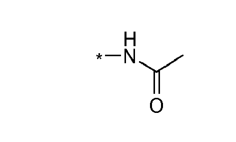 | 297-299 |
| 14. | 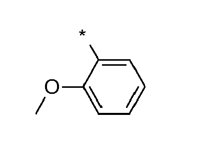 | 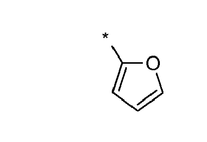 | 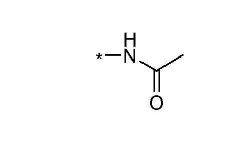 | > 300 |
| 15. | 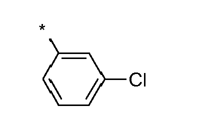 | 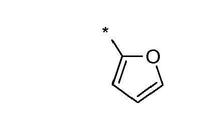 | 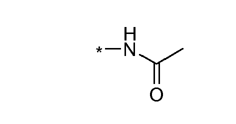 | > 300 |
| 16. | 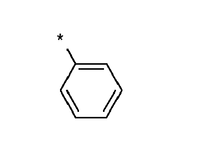 | 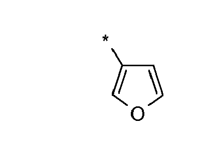 | 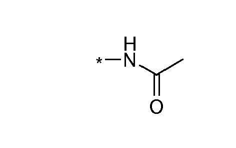 | > 300 |
| 17. | 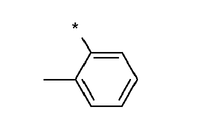 | 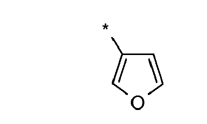 | 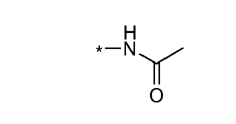 | 259-261 |
| 18. | 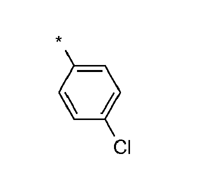 | 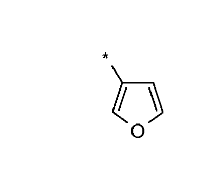 | 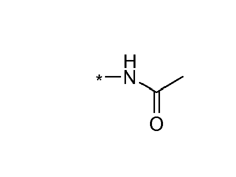 | >300 |
| 19. | 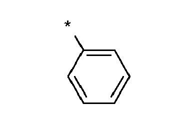 | H | 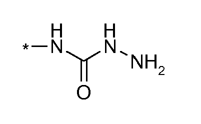 | |
| 20. | 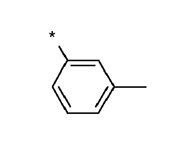 | H | 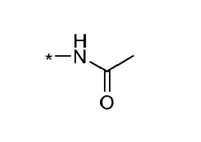 | 214-216 |
| 21. | 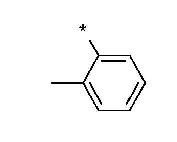 | H | 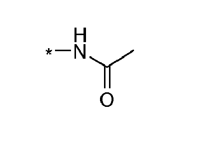 | 227-229 |
| 22. | 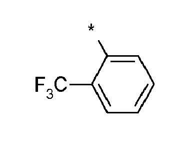 | H | 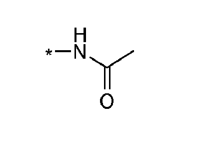 | 244-246 |
| 23. | 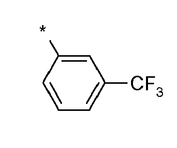 | H | 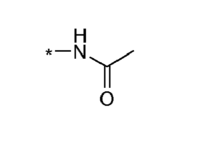 | 211-213 |
| 24. | 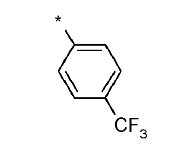 | H | 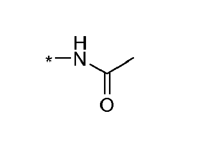 | 242-244 |
| 25. | 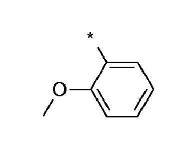 | H | 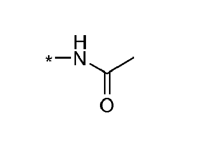 | 270-273 |
| 26. | 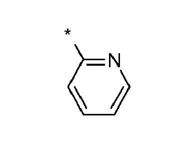 | H | 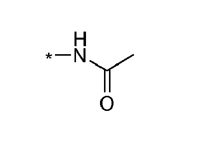 | 251-253 |
| 27. | 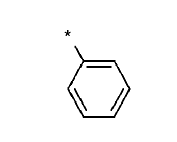 | H | 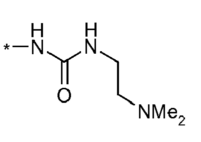 | |
| 28. | 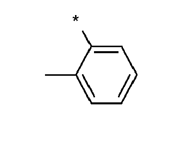 | 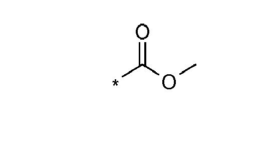 | 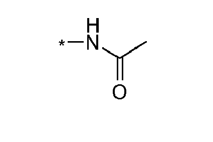 | 277-290 Zersetz. |
| 29. | 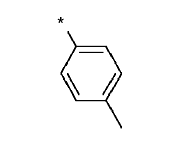 | 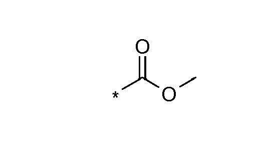 | 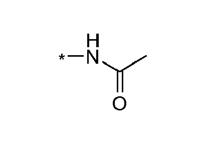 | 113-120 Zersetz. |
| 30. | 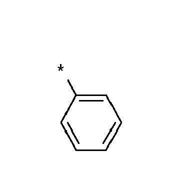 | H | 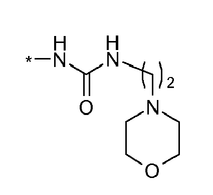 | |
| 31. | 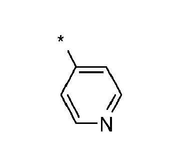 | H | 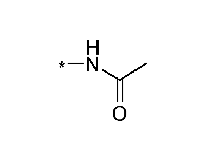 | |
| 32. | 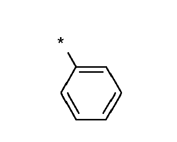 | H | 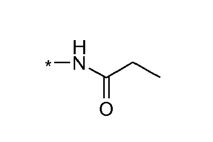 | 236-237,5 |
| 33. | 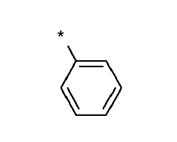 | H | 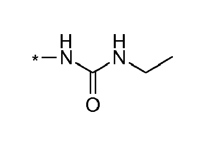 | 298-304 Zersetz. |
| 34. | 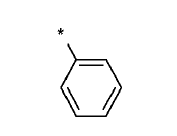 | H | 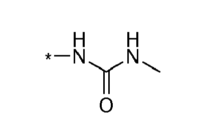 | 303,5-306 |
| 35. | 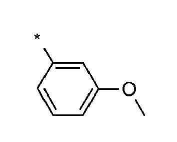 | H | 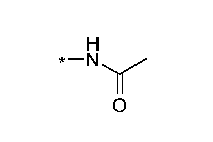 | |
| 36. | 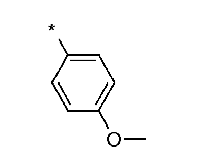 | H | 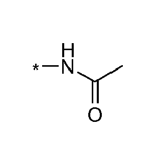 | |
| 37. | 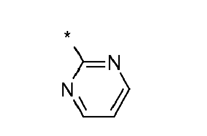 | H | 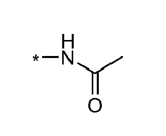 | > 300 |
| 38. | 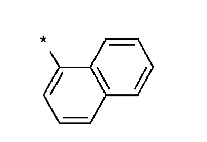 | H | 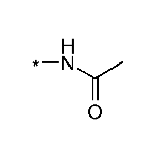 | |
| 39. | 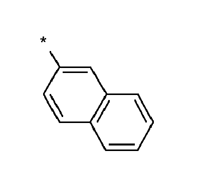 | H | 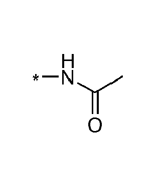 | 154-157 |
| 40. | 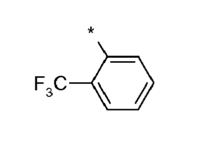 | 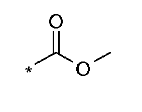 | 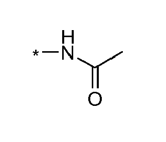 | 295-300 |
| 41. | 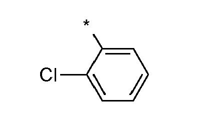 | H | 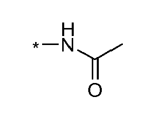 | 256-259 |
| 42. | 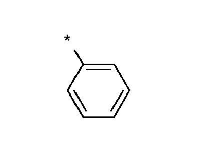 | H | 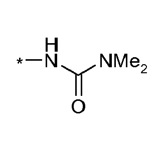 | 228-230 |
| 43. | 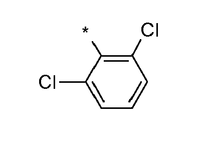 | H | 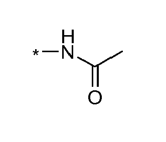 | 270-273 |
| 44. | 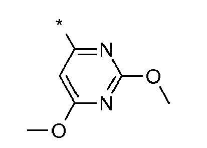 | H | 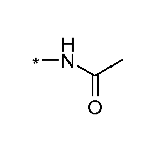 | 202-204 |
| 45. | 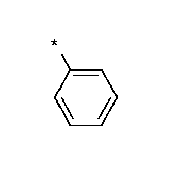 | H | 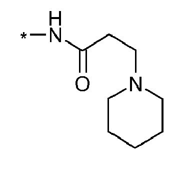 | 202-204 |
| 46. | 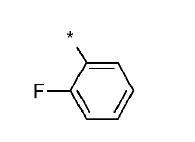 | H | 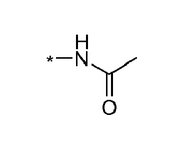 | > 300° |
| 47. | 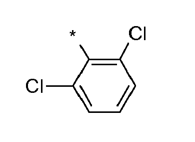 | 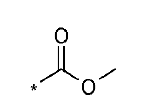 | 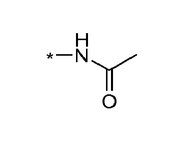 | 295-305 |
| 48. | 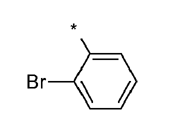 | H | 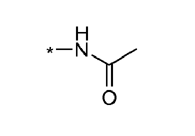 | 264-266 |
| 49. | 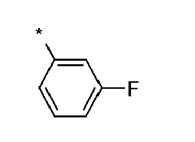 | H | 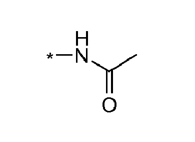 | 294-296 |
| 50. | 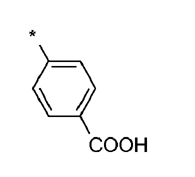 | H | 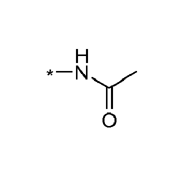 | > 300 |
| 51. | 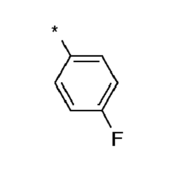 | H | 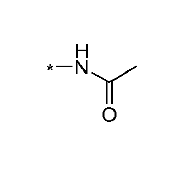 | |
| 52. | 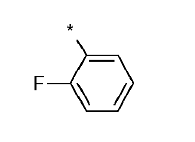 | O | 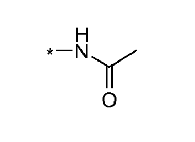 | 296->Zersetz. |
| 53. | 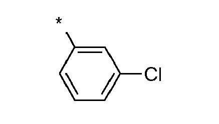 | H | 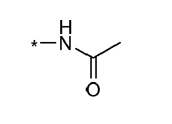 | 233-235 |
| 54. | 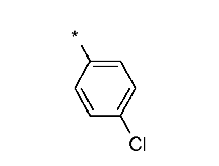 | H | 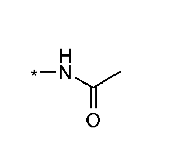 | 234-235 |
| 55. | 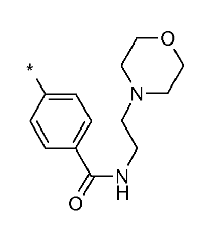 | H | 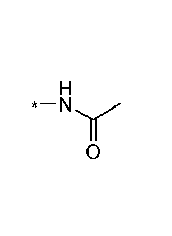 | |
| 56. | 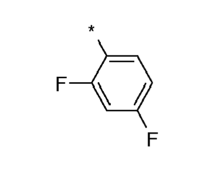 | H | 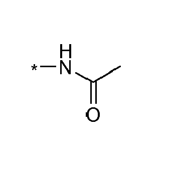 | 249-251 |
| 57. | 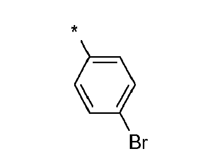 | H | 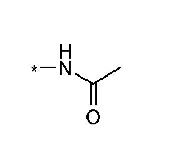 | 248-249 |
| 58. | 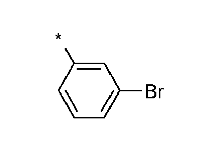 | H | 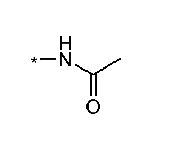 | 135-137 |
| 59. | 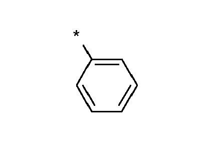 | H | 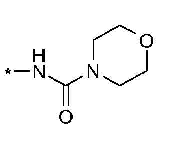 | 239 |
| 60. | 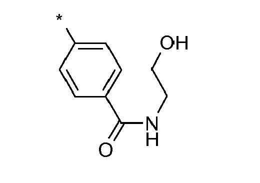 | H | 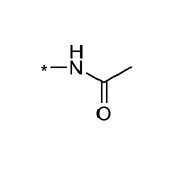 | |
| 61. | 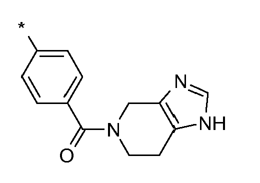 | H | 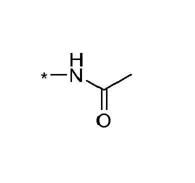 | |
| 62. | 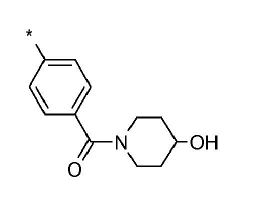 | H | 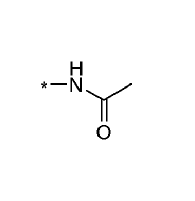 | |
| 63. | 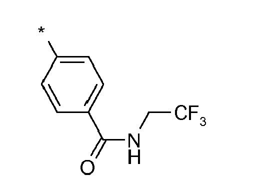 | H | 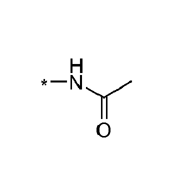 | |
| 64. | 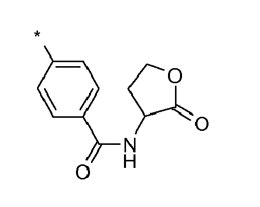 | H | 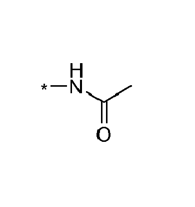 | |
| 65. | 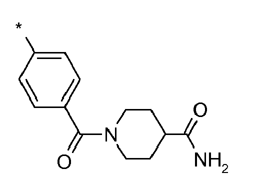 | H | 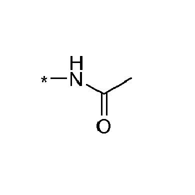 | |
| 66. | 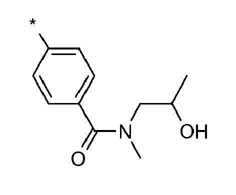 | H | 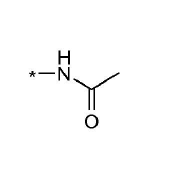 | |
| 67. | 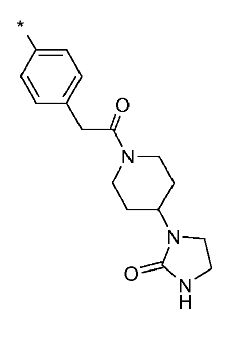 | H | 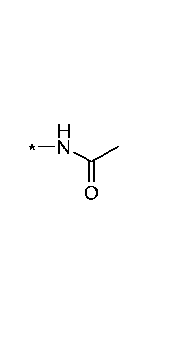 | |
| 68. | 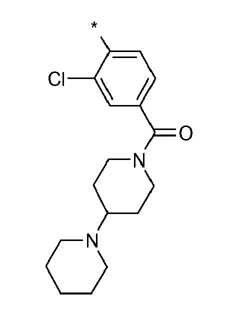 | H | 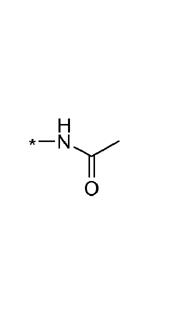 | |
| 69. | 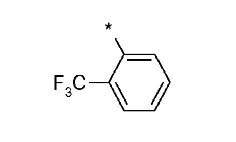 | 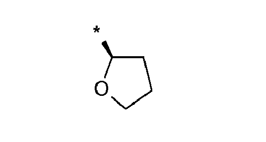 | 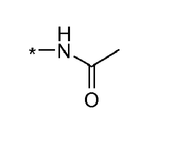 | |
| 70. | 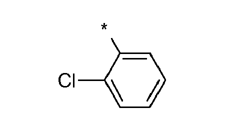 | 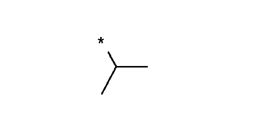 | 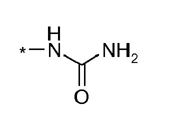 | |
| 71. | 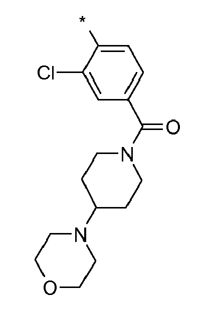 | H | 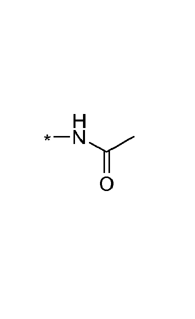 | |
| 72. | 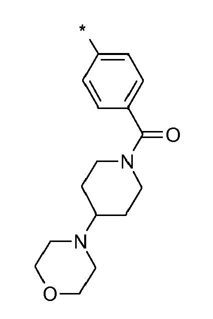 | H | 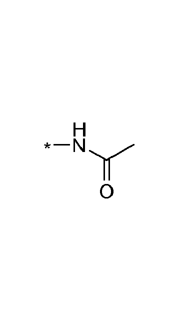 | 255-256 |
| 73. | 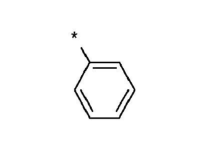 | 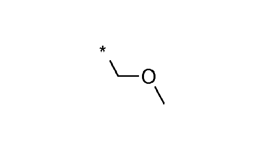 | 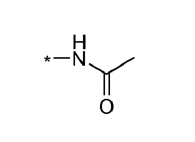 | |
| 74. | 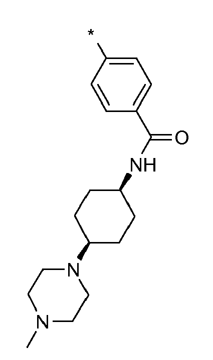 | H | 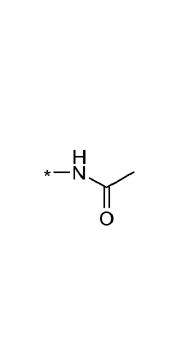 | |
| 75. | 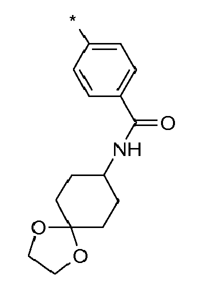 | H | 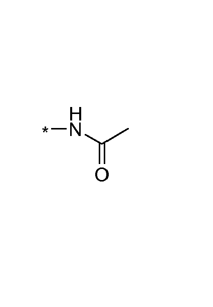 | |
| 76. | 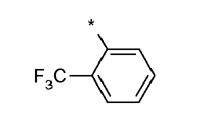 | 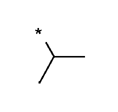 | 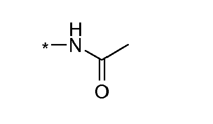 | |
| 77. | 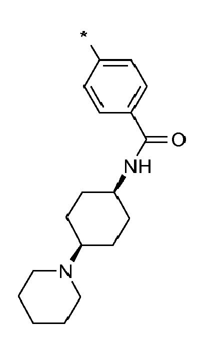 | H | 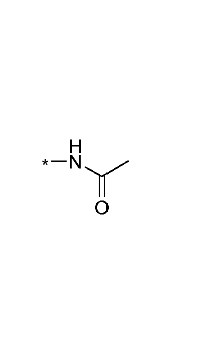 | |
| 78. | 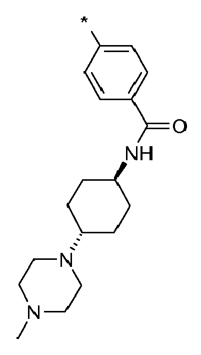 | H | 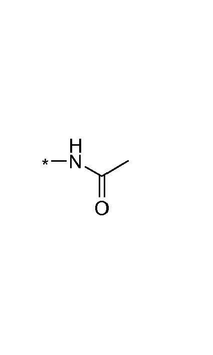 | |
| 79. | 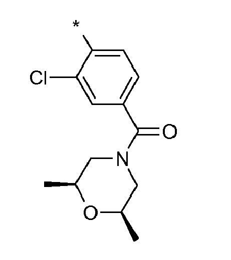 | H | 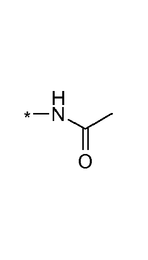 | |
| 80. | 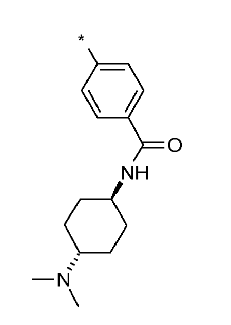 | H | 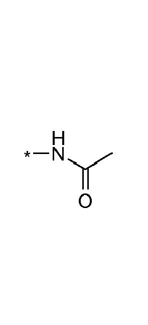 | |
| 81. | 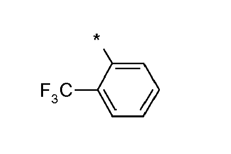 | 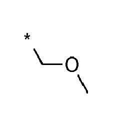 | 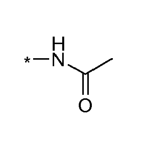 | |
| 82. | 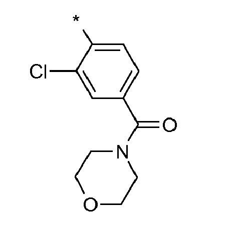 | H | 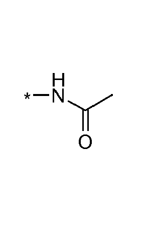 | |
| 83. | 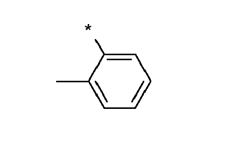 | 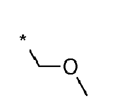 | 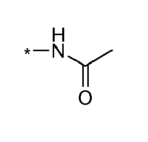 | |
| 84. | 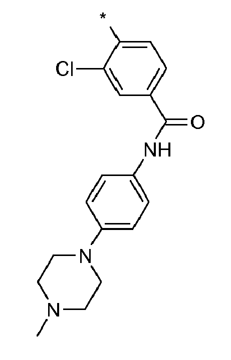 | H | 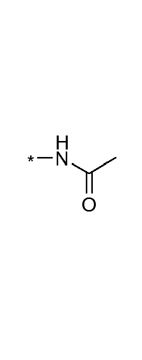 | |
| 85. | 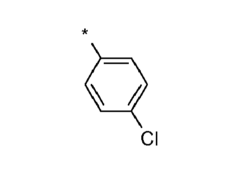 | -CH₃ | 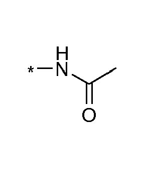 | |
| 86. | 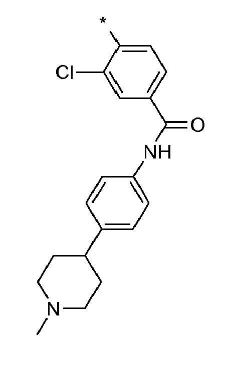 | H | 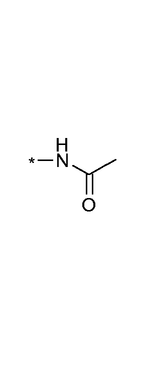 | |
| 87. | 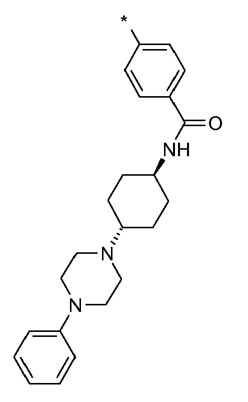 | H | 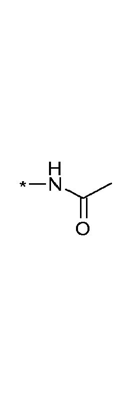 | |
| 88. | 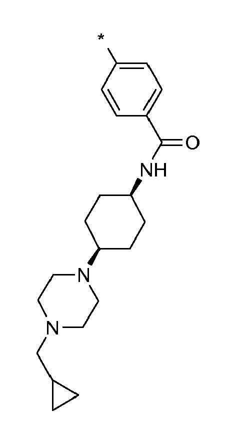 | H | 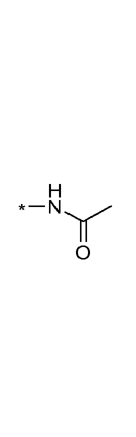 | |
| 89. | 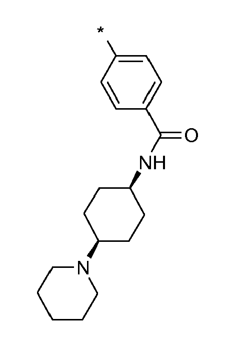 | H | 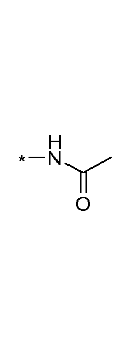 | 230 |
| 90. | 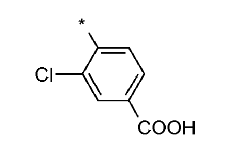 | 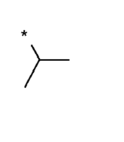 | 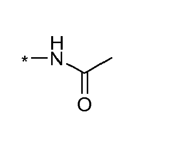 | |
| | 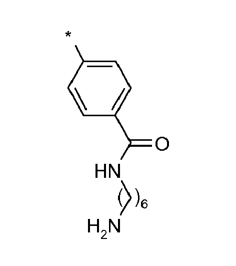 | H | 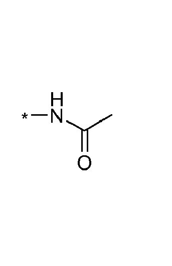 | 227,4 |
| 91. | 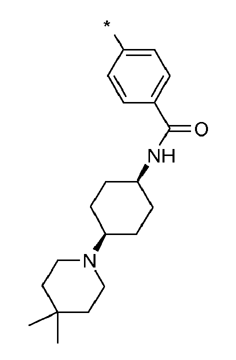 | H | 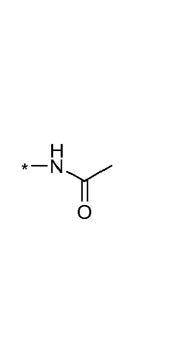 | |
| 92. | 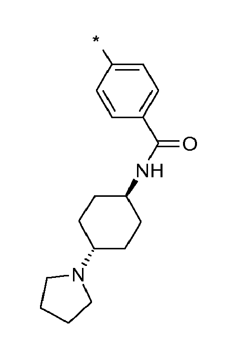 | H | 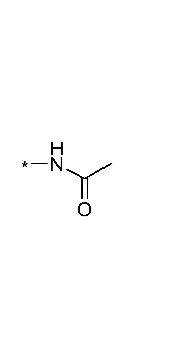 | |
| 93. | 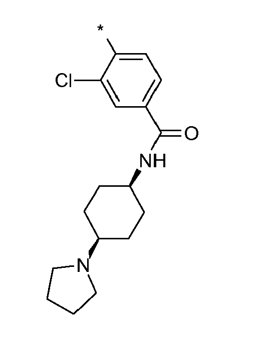 | 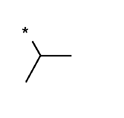 | 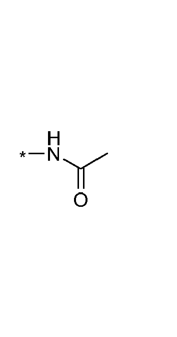 | |
| 94. | 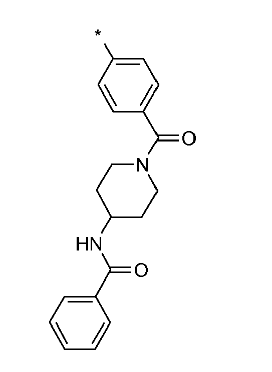 | H | 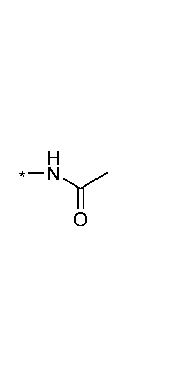 | |
| 95. | 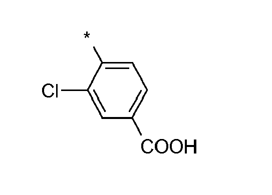 | H | 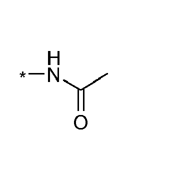 | >300 |
| 96. | 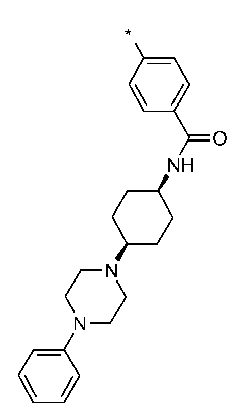 | H | 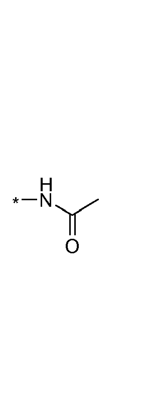 | |
| 97. | 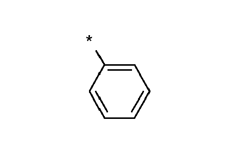 | 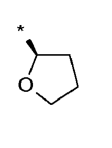 | 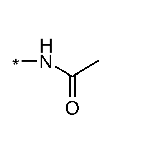 | |
| 98. | 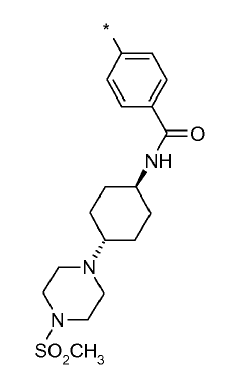 | H | 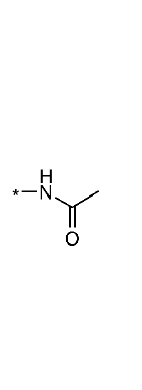 | |
| 99. | 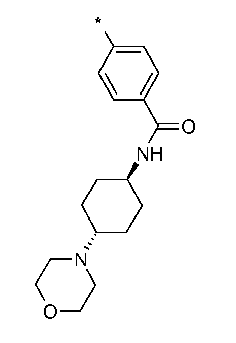 | H | 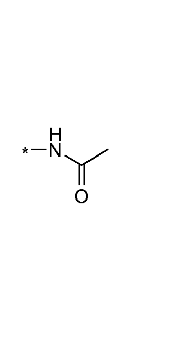 | |
| 100. | 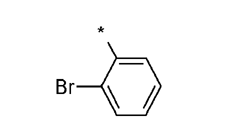 | 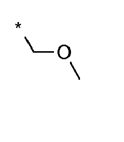 | 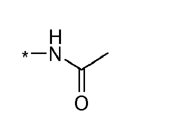 | |
| 101. | 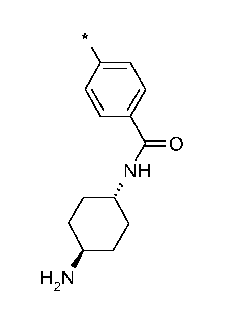 | H | 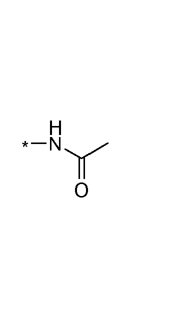 | |
| 102. | 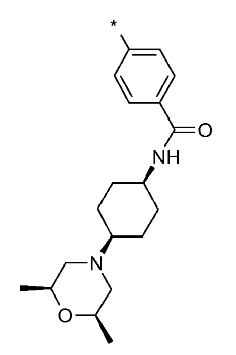 | H | 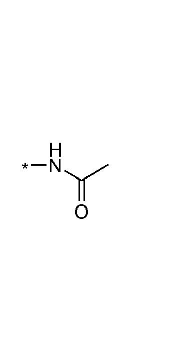 | |
| 103. | 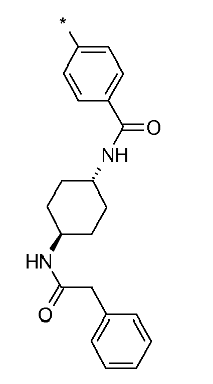 | H | 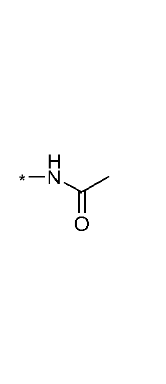 | |
| 104. | 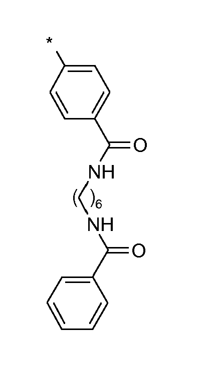 | H | 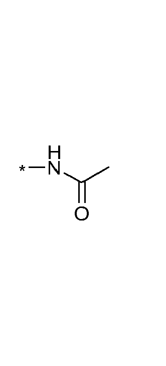 | |
| 105. | 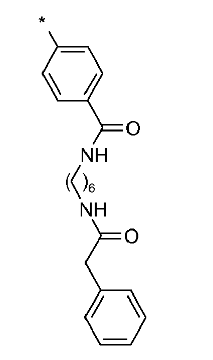 | H | 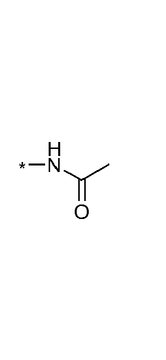 | |
| 106. | 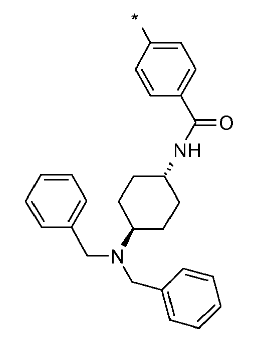 | H | 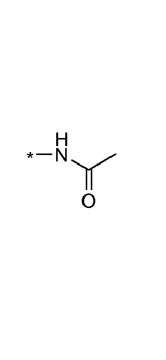 | |
| 107. | 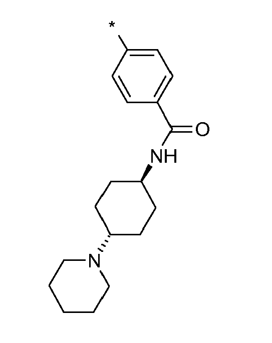 | H | 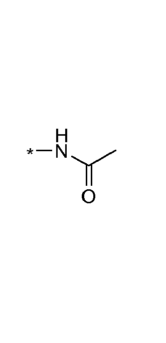 | |
| 108. | 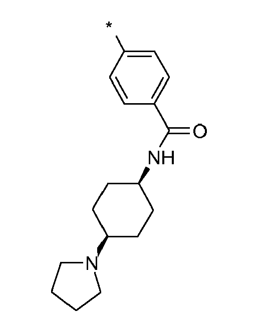 | H | 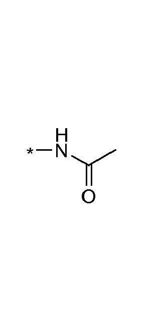 | |
| 109. | 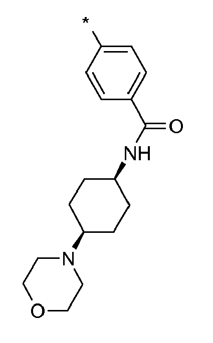 | H | 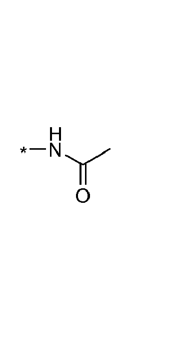 | |
| 110. | 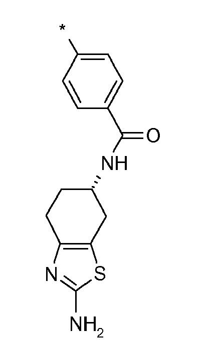 | H | 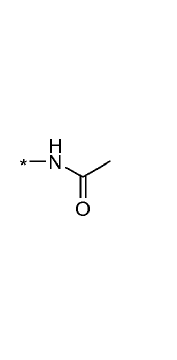 | |
| 111. | 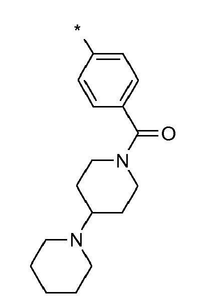 | H | 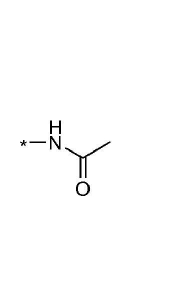 | |
| 112. | 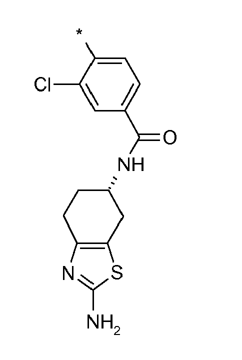 | H | 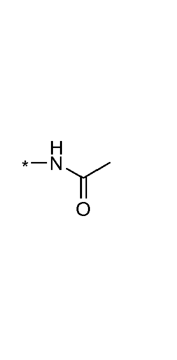 | |
| 113. | 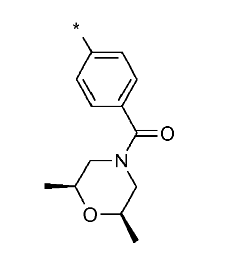 | H | 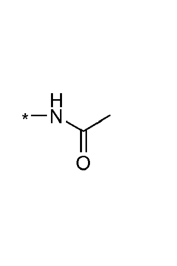 | |
| 114. | 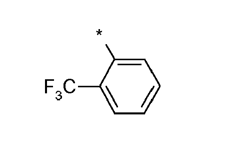 | -CH₃ | 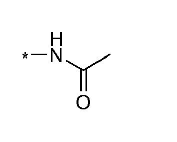 | |
| 115. | 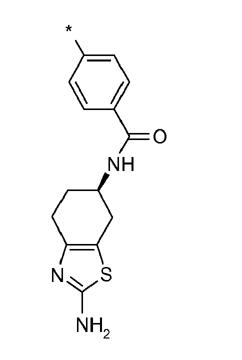 | H | 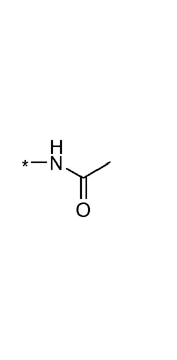 | |
| 116. | 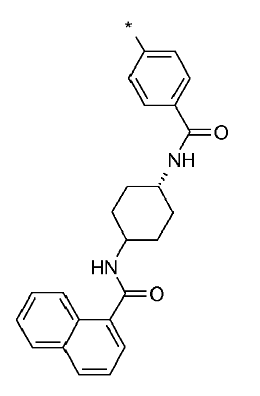 | H | 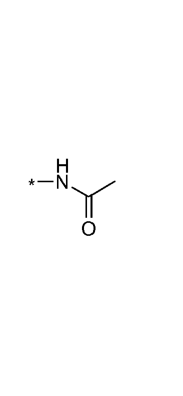 | |
| 117. | 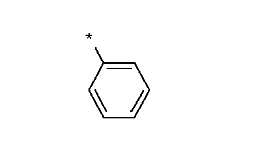 | 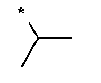 | 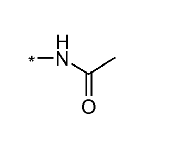 | |
| 118. | 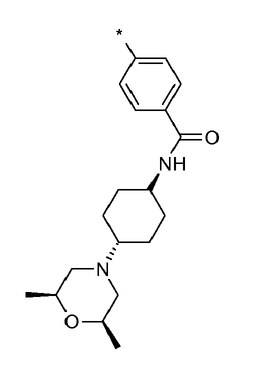 | H | 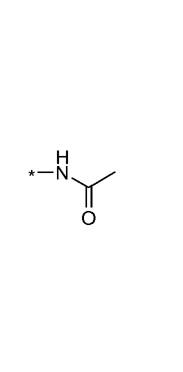 | |
| 119. | 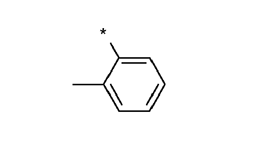 | 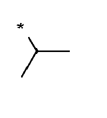 | 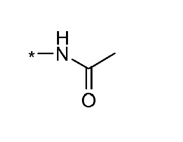 | |
| 120. | 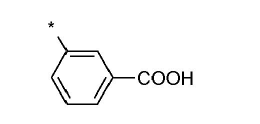 | H | 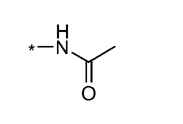 | |
| 121. | 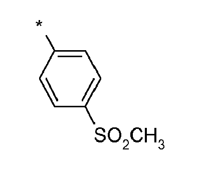 | H | 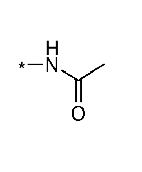 | |
| 122. | 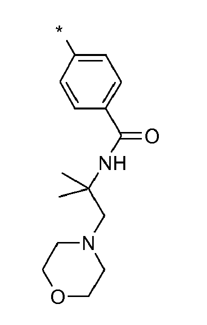 | H | 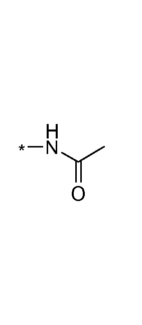 | 280-281 |
| 123. | 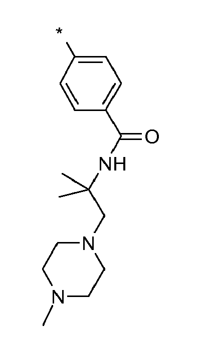 | H | 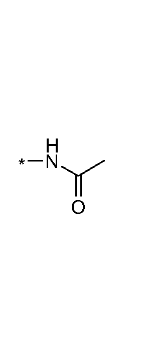 | 245 |
| 124. | 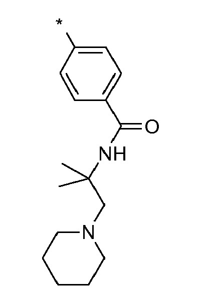 | H | 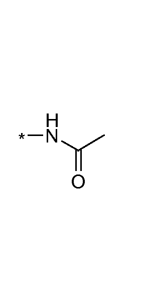 | 317-319 |
| 125. | 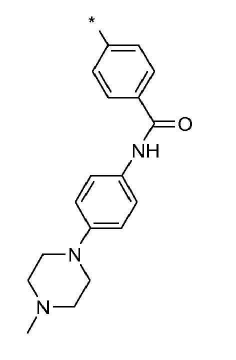 | H | 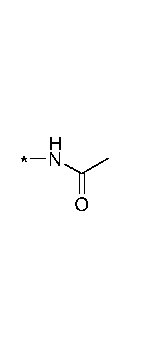 | 300-310 |
| 126. | 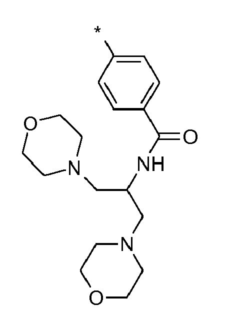 | H | 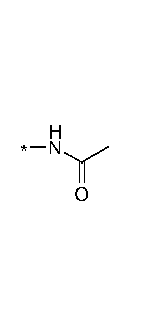 | 226-227 |
| 127. | 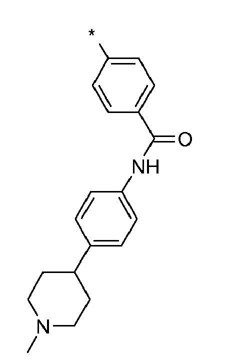 | H | 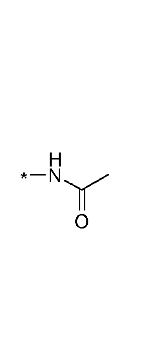 | 240-250 |
| 128. | 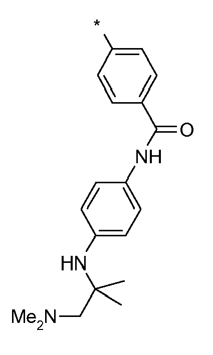 | H | 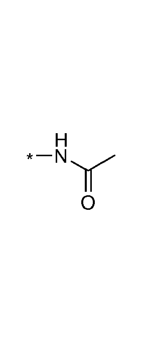 | 304-310 |
| 129. | 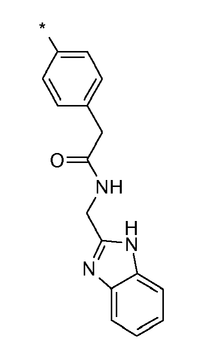 | H | 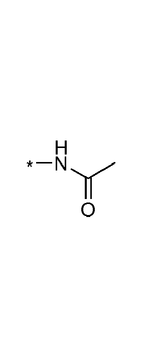 | |
| 130. | 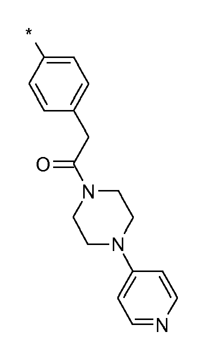 | H | 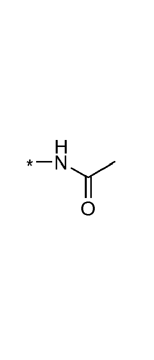 | |
| 131. | 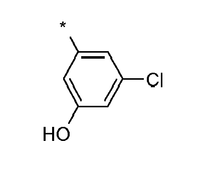 | H | 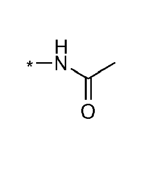 | |
| 132. | 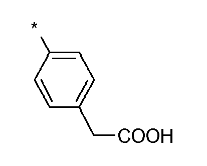 | H | 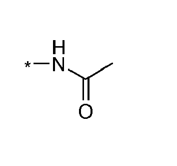 | |
| 133. | 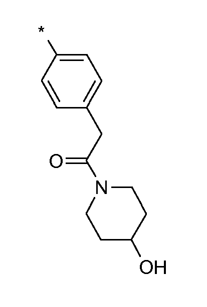 | H | 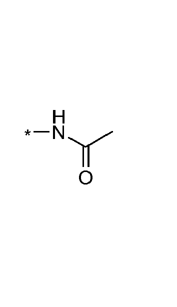 | |
| 134. | 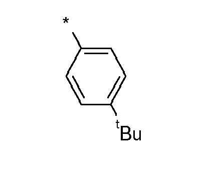 | H | 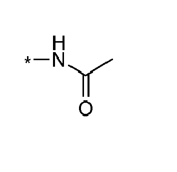 | |
| 135. | 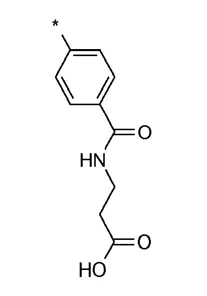 | H | 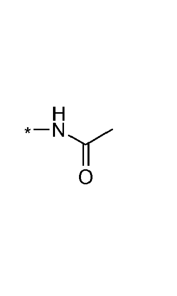 | |
| 136. | 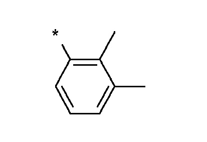 | H | 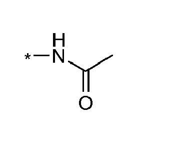 | |
| 137. | 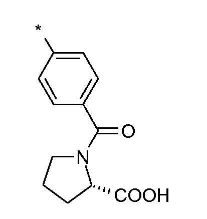 | H | 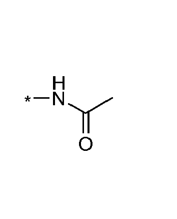 | |
| 138. | 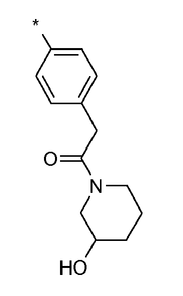 | H | 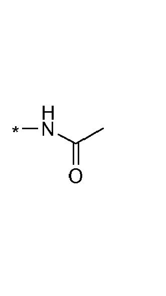 | |
| 139. | 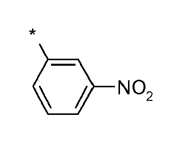 | H | 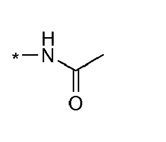 | |
| 140. | 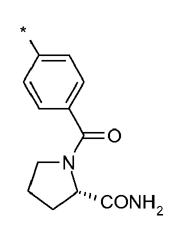 | H | 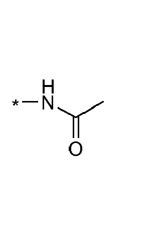 | |
| 141. | 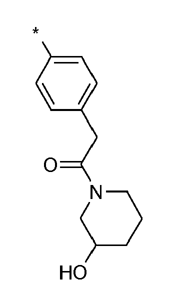 | H | 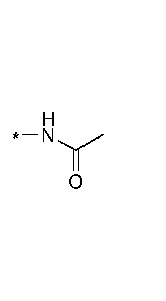 | |
| 142. | 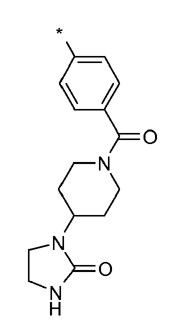 | H | 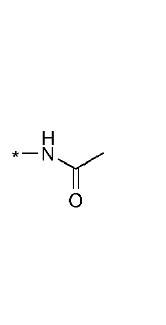 | |
| 143. | 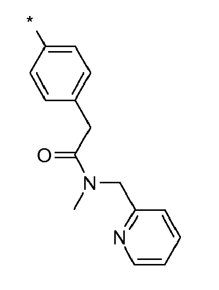 | H | 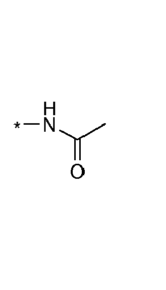 | |
| 144. | 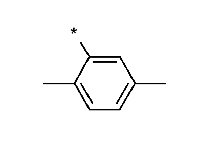 | H | 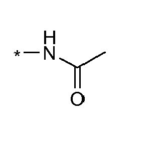 | |
| 145. | 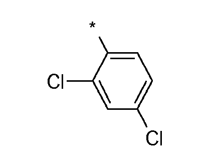 | H | 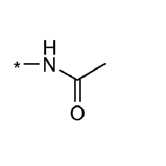 | |
| 146. | 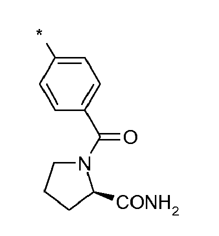 | H | 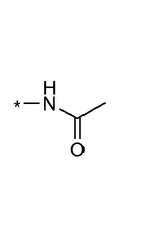 | |
| 147. | 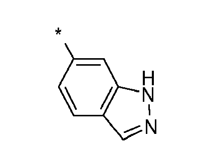 | H | 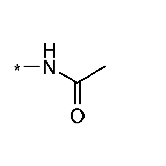 | |
| 148. | 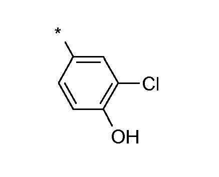 | H | 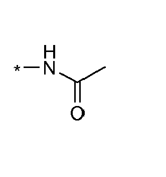 | |
| 149. | 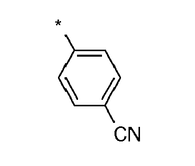 | H | 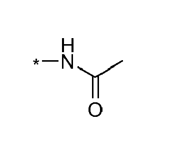 | |
| 150. | 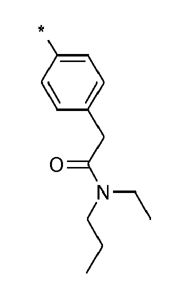 | H | 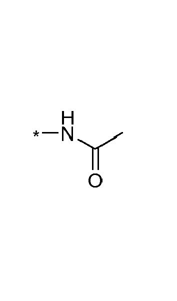 | |
| 151. | 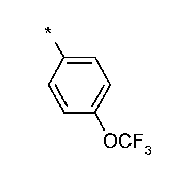 | H | 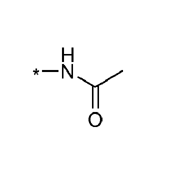 | |
| 152. | 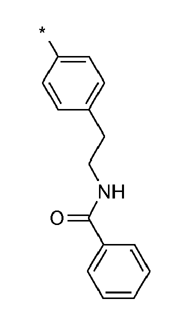 | H | 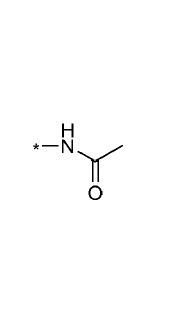 | |
| 153. | 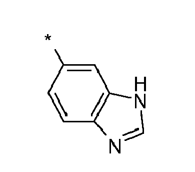 | H | 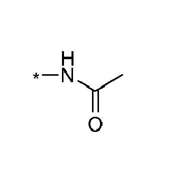 | |
| 154. | 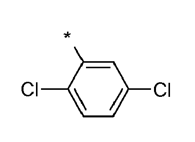 | H | 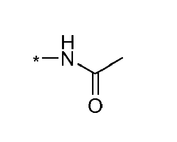 | |
| 155. | 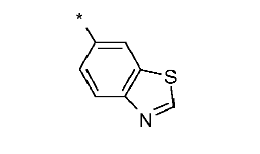 | H | 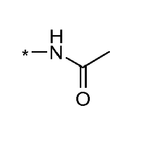 | |
| 156. | 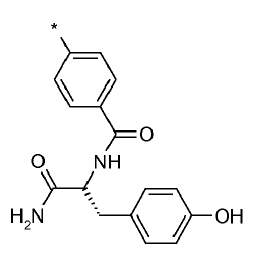 | H | 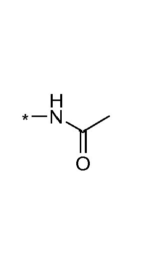 | |
| 157. | 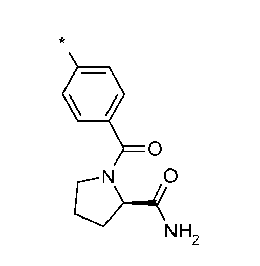 | H | 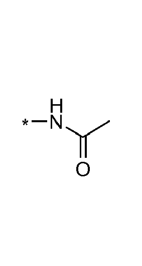 | |
| 158. | 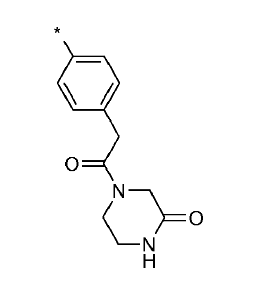 | H | 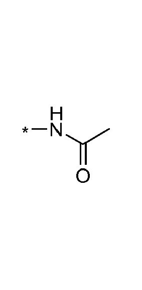 | |
| 159. | 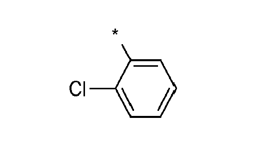 | 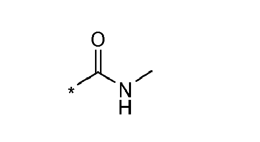 | 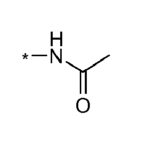 | 303,1 |
| 160. | 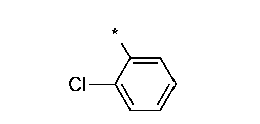 | 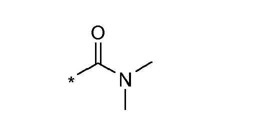 | 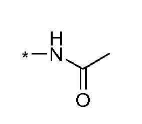 | 281,3 |
| 161. | 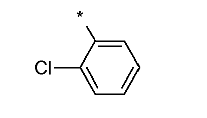 | 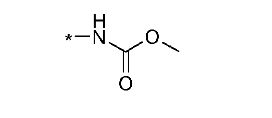 | 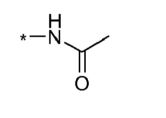 | 248,3 |
| 162. | 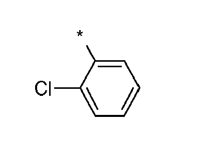 | 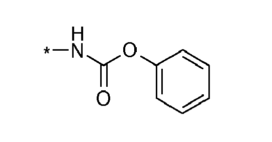 | 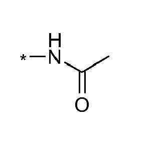 | |
| 163. | 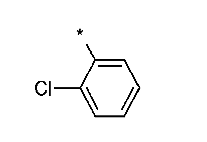 | 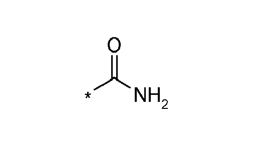 | 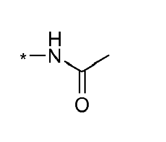 | |
| 164. | 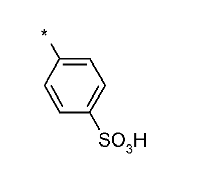 | H | 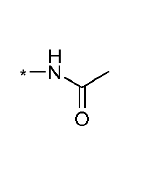 | >300 |
| 165. | 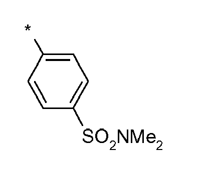 | H | 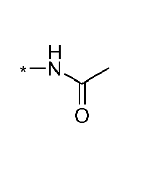 | >280 |
| 166. | 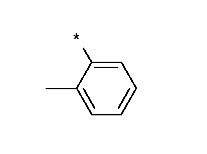 | 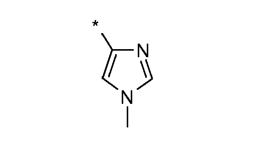 | 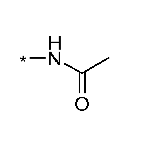 | > 300 |
| 167. | 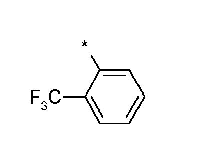 | 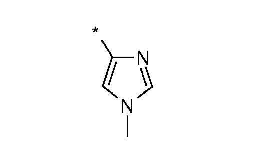 | 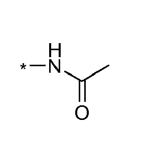 | > 300 |
| 168. | 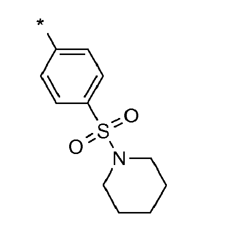 | H | 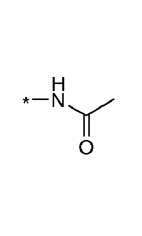 | 248-251 |
| 169. | 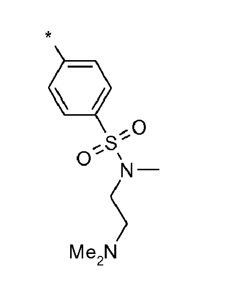 | H | 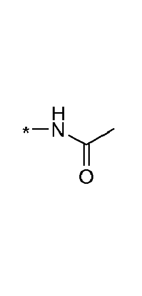 | 242-245 |
| 170. | 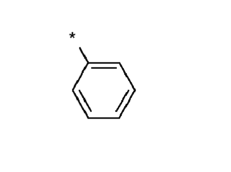 | 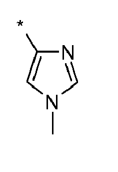 | 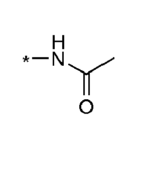 | >300 |
| 171. | 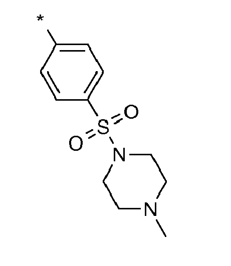 | H | 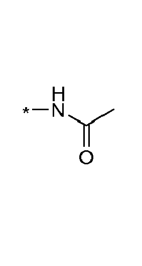 | 298->300 |
| 172. | 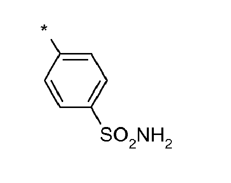 | H | 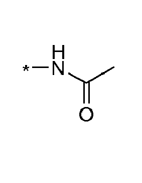 | >300 |
| 173. | 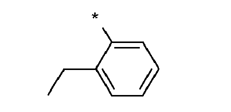 | H | 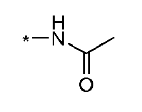 | 209-211 |
| 174. | 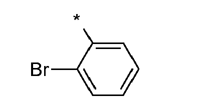 | 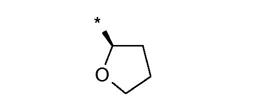 | 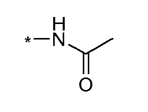 | |
| 175. | 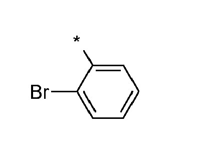 | 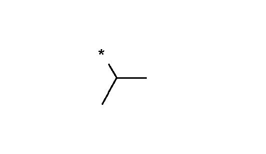 | 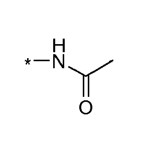 | |
| 176. | 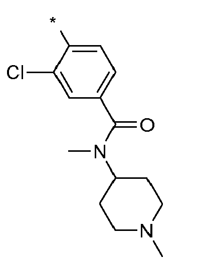 | 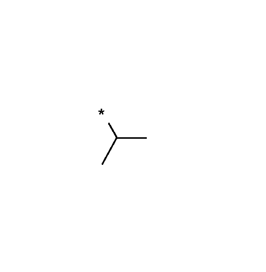 | 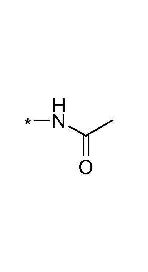 | |
| 177. | 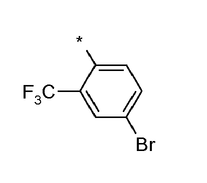 | 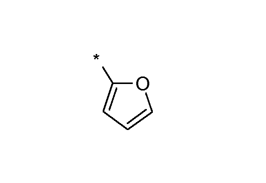 | 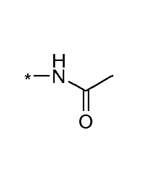 | |
| 178. | 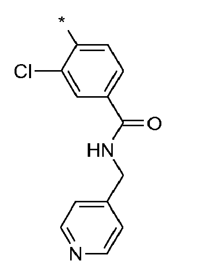 | 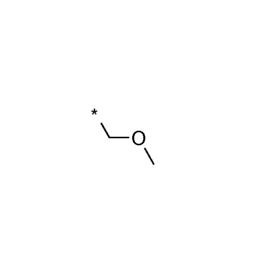 | 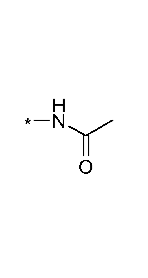 | |
| 179. | 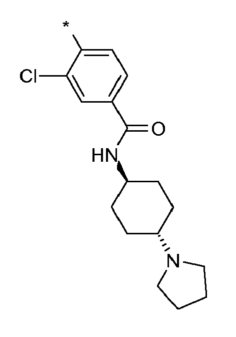 | 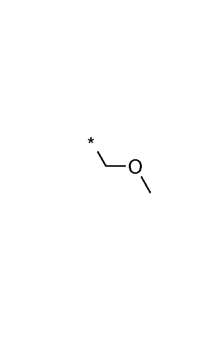 | 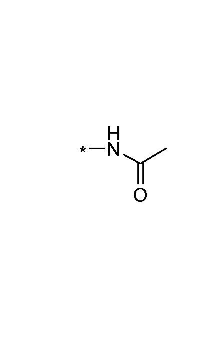 | |
| 180. | 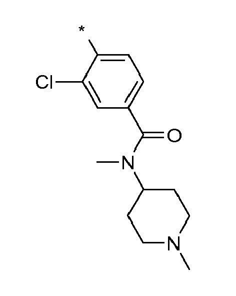 | 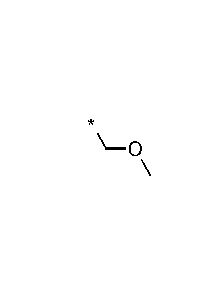 | 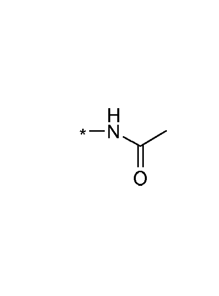 | |
| 181. | 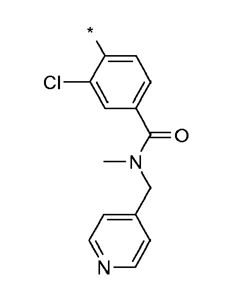 | 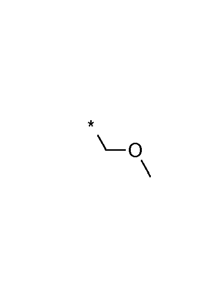 | 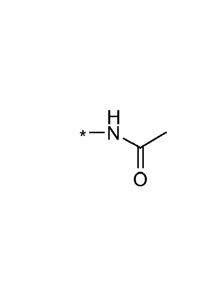 | |
| 182. | 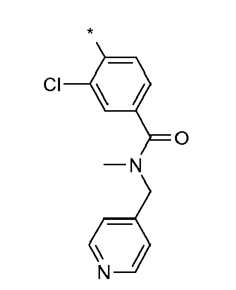 | 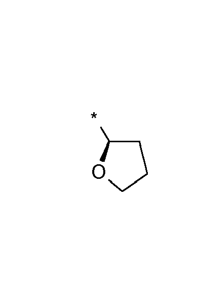 | 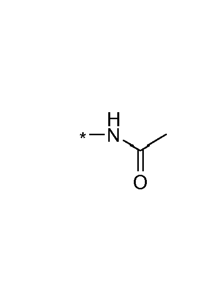 | |
| 183. | 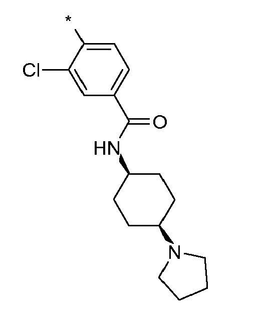 | 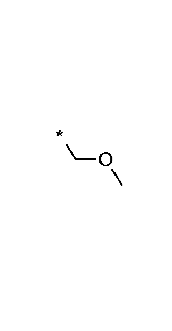 | 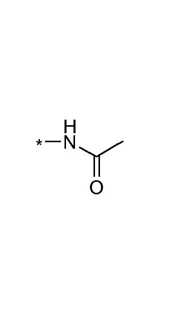 | |
| 184. | 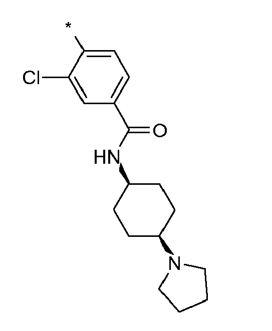 | 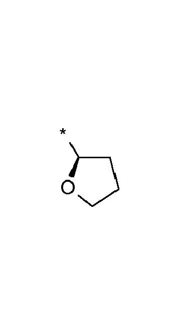 | 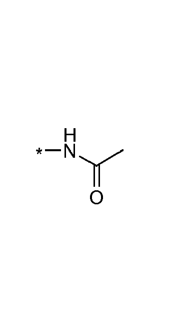 | |
| 185. | 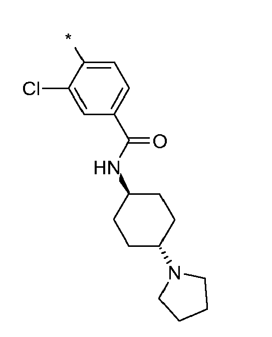 | 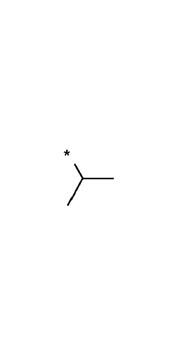 | 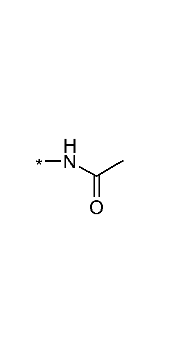 | |
| 186. | 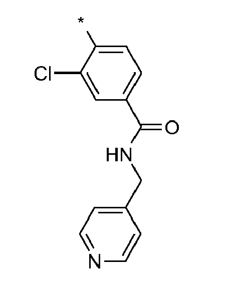 | 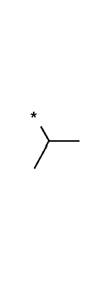 | 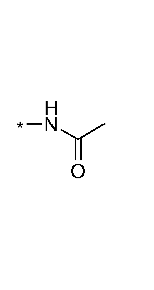 | |
| 187. | 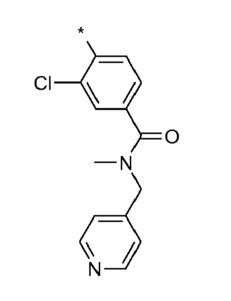 | 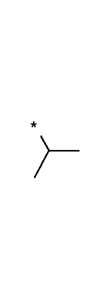 | 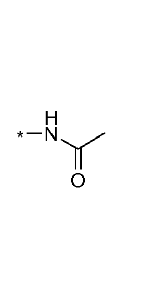 | |
| 188. | 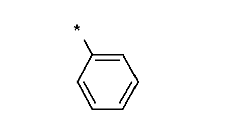 | 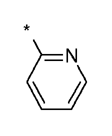 | 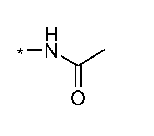 | |
| 189. | 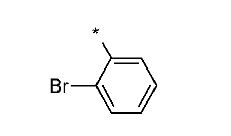 | 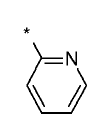 | 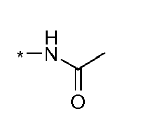 | |
| 190. | 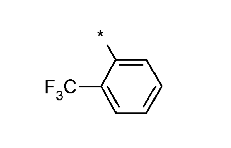 | 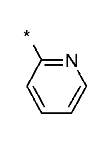 | 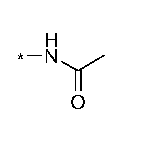 | |
| 191. | 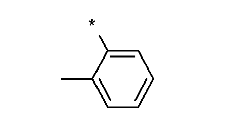 | 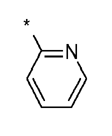 | 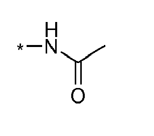 | |
| 192. | 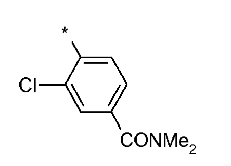 | 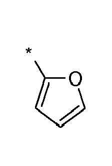 | 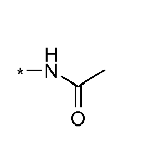 | |
| 193. | 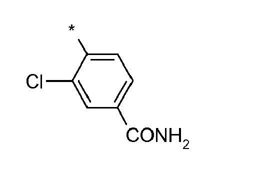 | 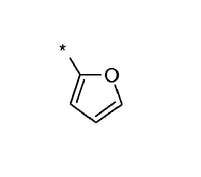 | 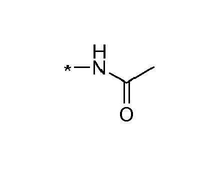 | |
| 194. | 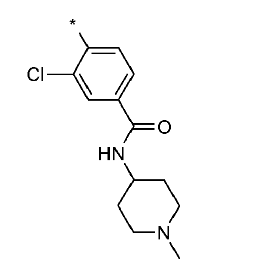 | 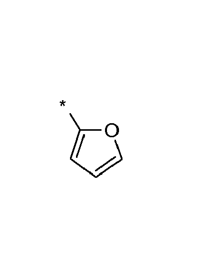 | 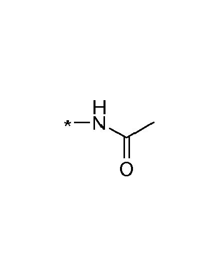 | |
| 195. | 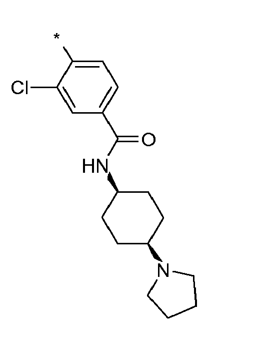 | 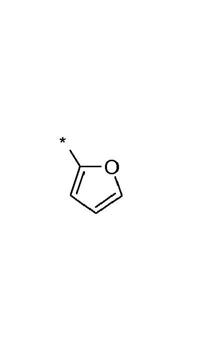 | 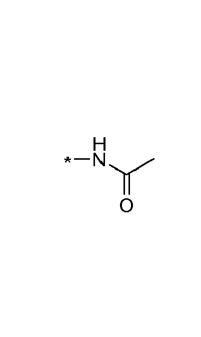 | |
| 196. | 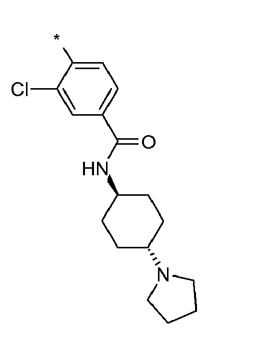 | 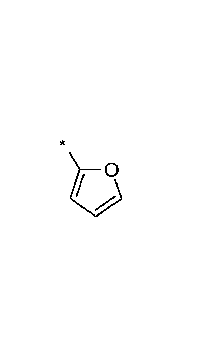 | 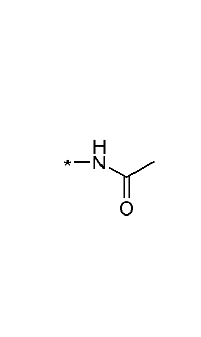 | |
| 197. | 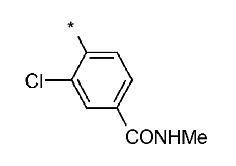 | 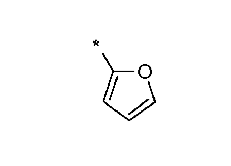 | 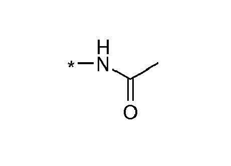 | |
| 198. | 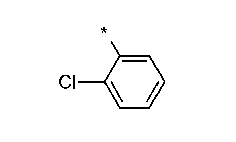 | 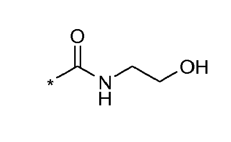 | 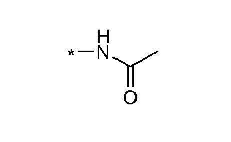 | |
| 199. | 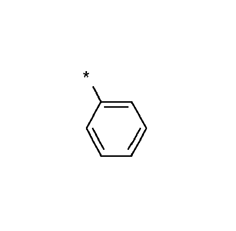 | H | 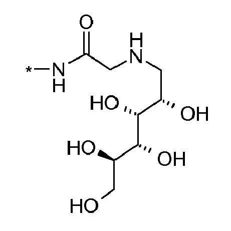 | |
| 200. | 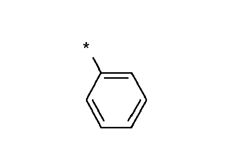 | H | 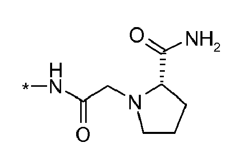 | |
| 201. | 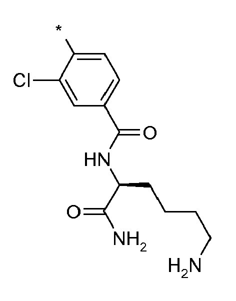 | H | 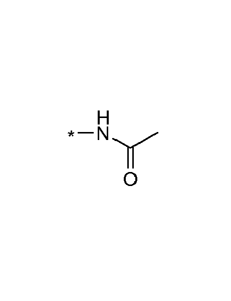 | |
| 202. | 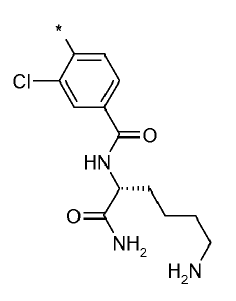 | H | 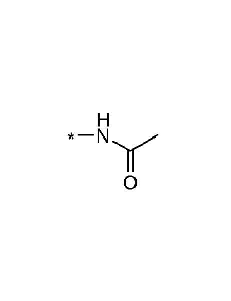 | |
| 203. | 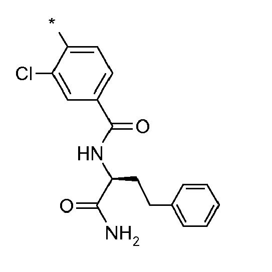 | H | 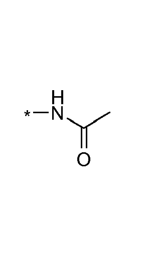 | |
| 204. | 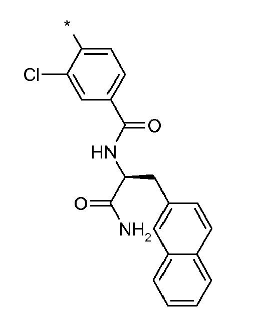 | H | 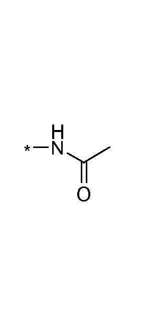 | |
| 205. | 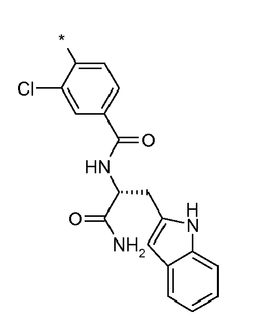 | H | 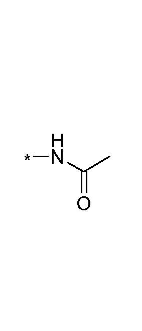 | |
| 206. | 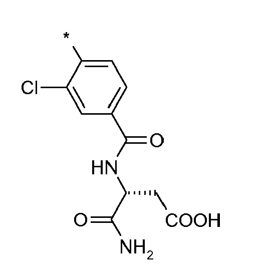 | H | 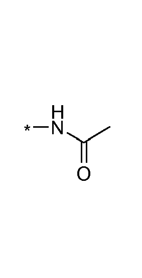 | |
| 207. | 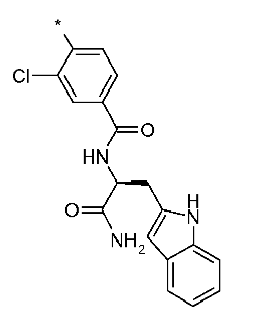 | H | 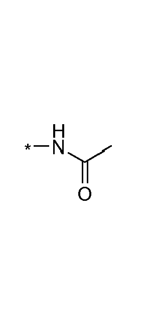 | |
| 208. | 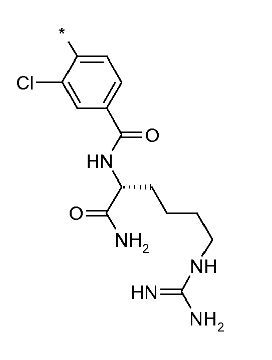 | H | 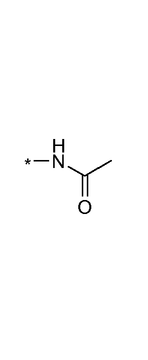 | |
| 209. | 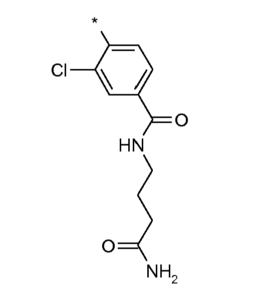 | H | 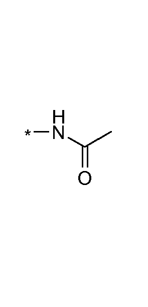 | |
| 210. | 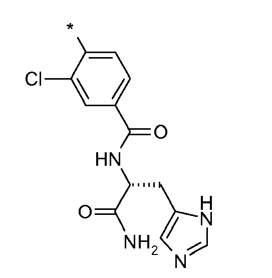 | H | 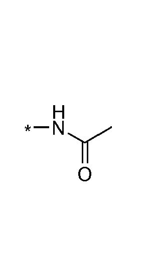 | |
| 211. | 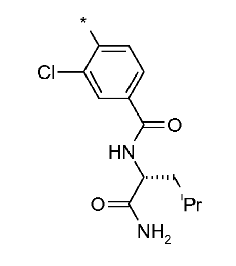 | H | 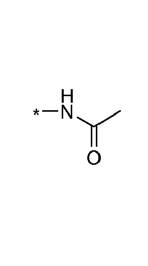 | |
| 212. | 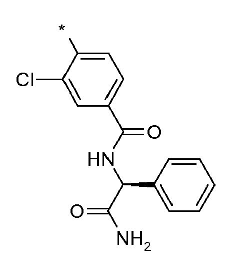 | H | 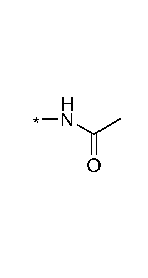 | |
| 213. | 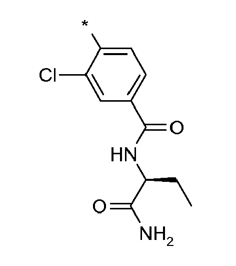 | H | 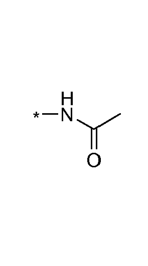 | |
| 214. | 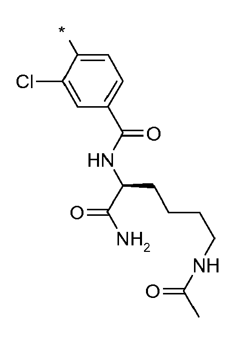 | H | 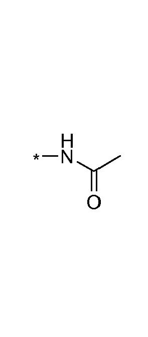 | |
| 215. | 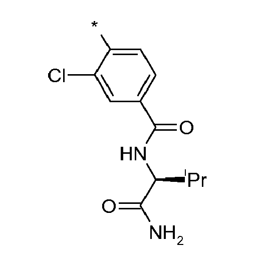 | H | 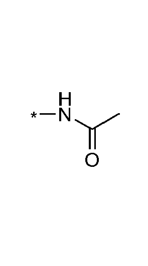 | |
| 216. | 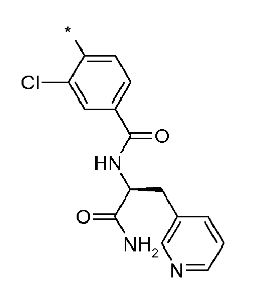 | H | 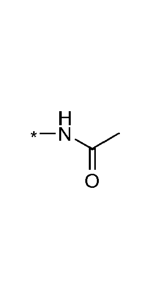 | |
| 217. | 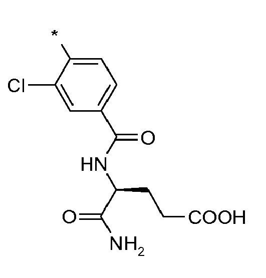 | H | 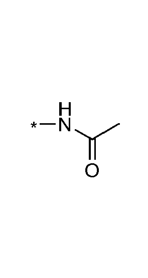 | |
| 218. | 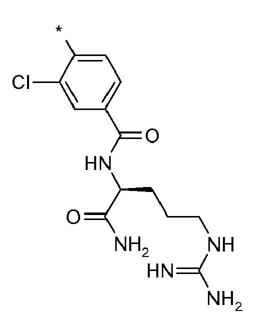 | H | 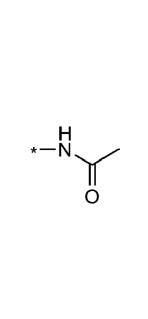 | |
| 219. | 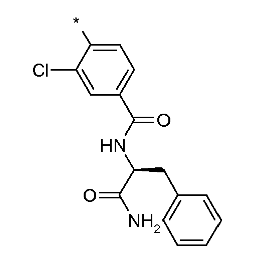 | H | 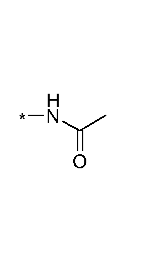 | |
| 220. | 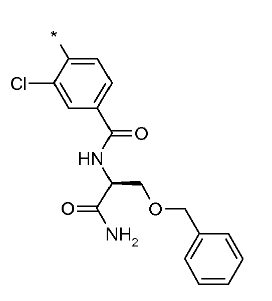 | H | 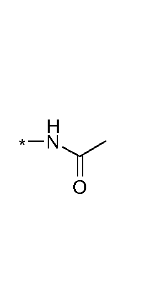 | |
| 221. | 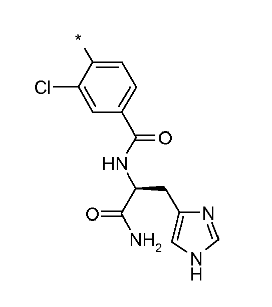 | H | 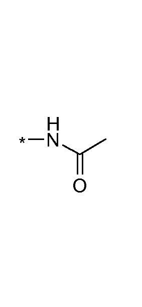 | |
| 222. | 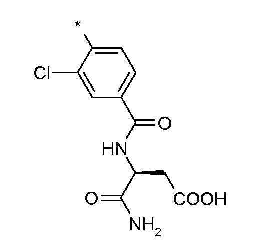 | H | 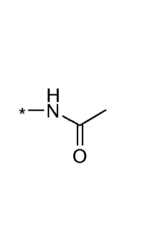 | |
| 223. | 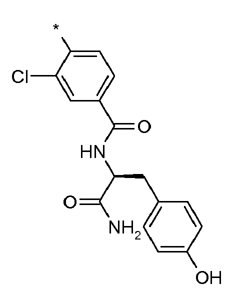 | H | 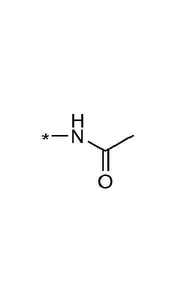 | |
| 224. | 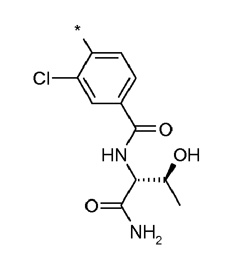 | H | 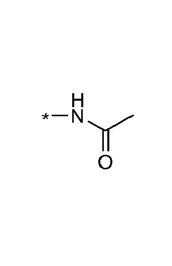 | |
| 225. | 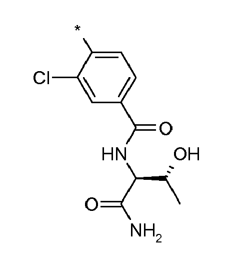 | H | 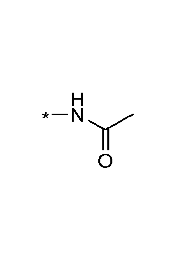 | |
| 226. | 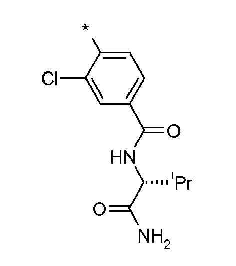 | H | 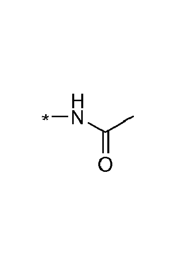 | |
| 227. | 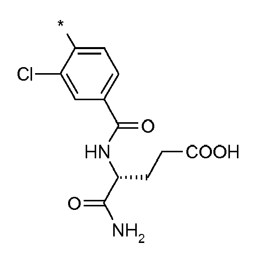 | H | 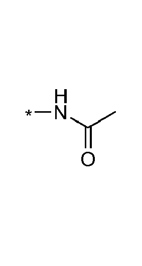 | |
| 228. | 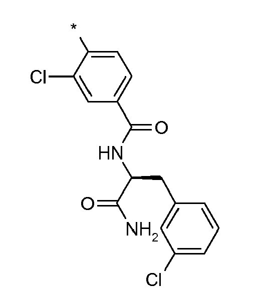 | H | 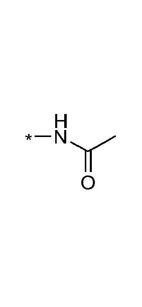 | |
| 229. | 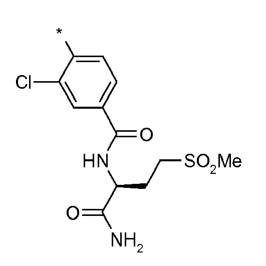 | H | 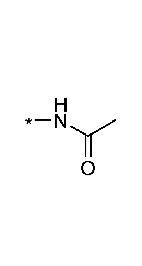 | |
| 230. | 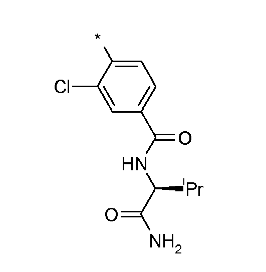 | H | 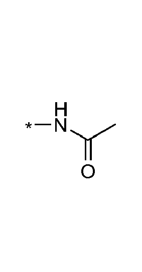 | |
| 231. | 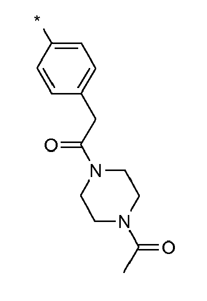 | H | 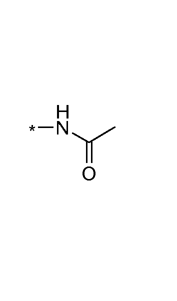 | |
| 232. | 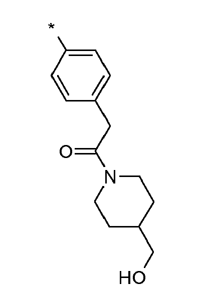 | H | 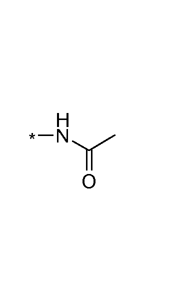 | |
| 233. | 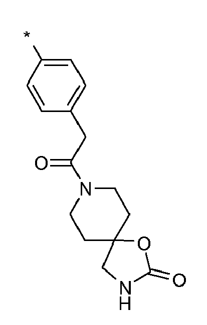 | H | 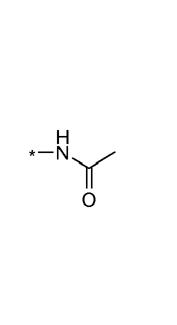 | |
| 234. | 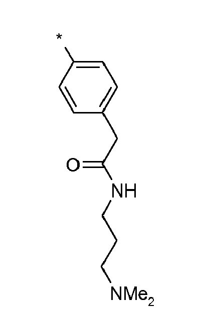 | H | 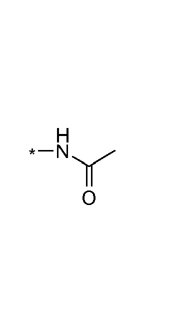 | |
| 235. | 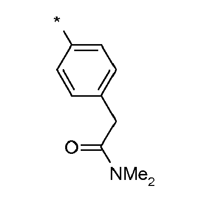 | H | 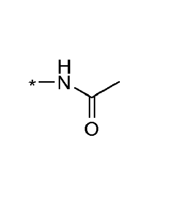 | |
| 236. | 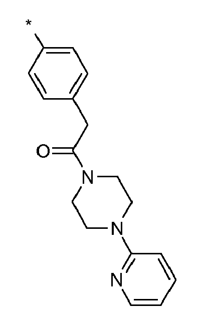 | H | 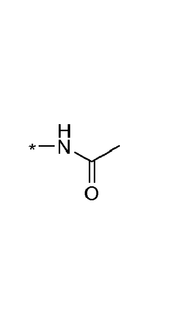 | |
| 237. | 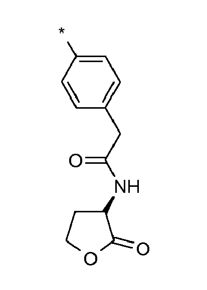 | H | 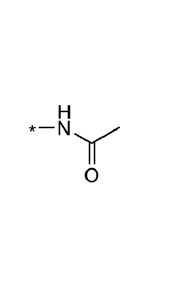 | |
| 238. | 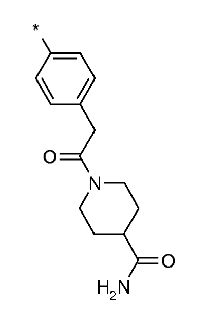 | H | 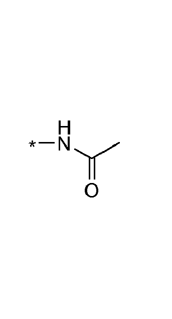 | |
| 239. | 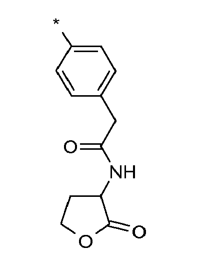 | H | 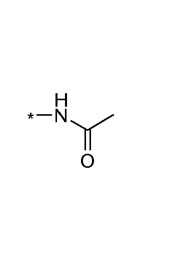 | |
| 240. | 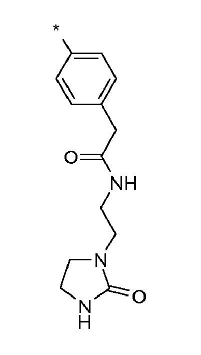 | H | 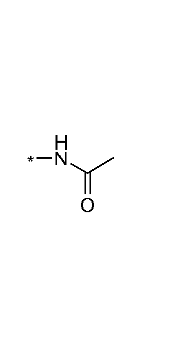 | |
| 241. | 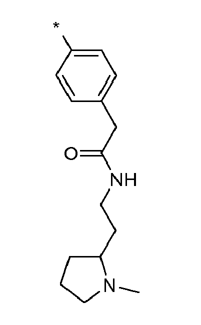 | H | 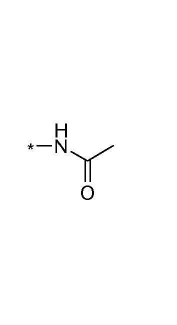 | |
| 242. | 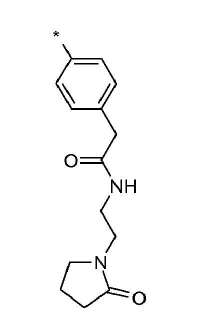 | H | 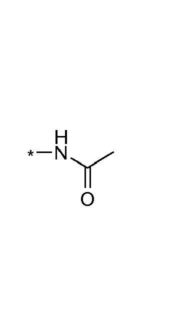 | |
| 243. | 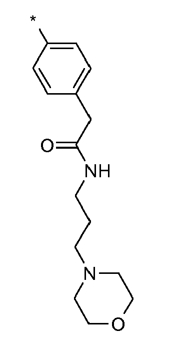 | H | 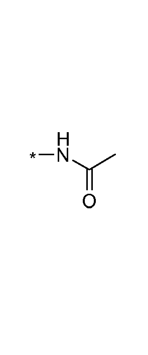 | |
| 244. | 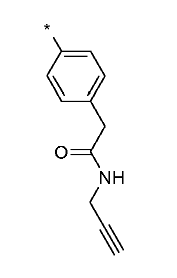 | H | 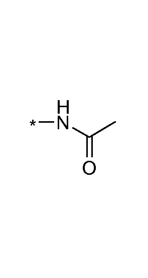 | |
| 245. | 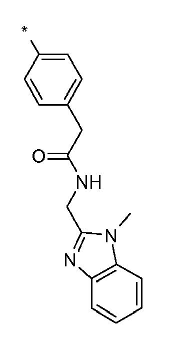 | H | 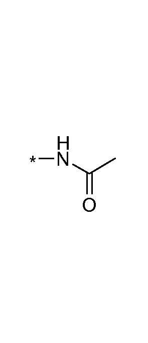 | |
| 246. | 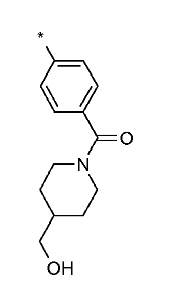 | H | 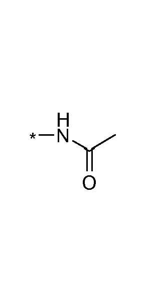 | |
| 247. | 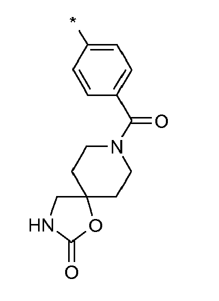 | H | 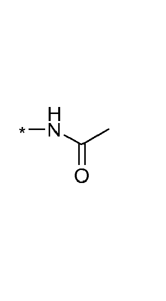 | |
| 248. | 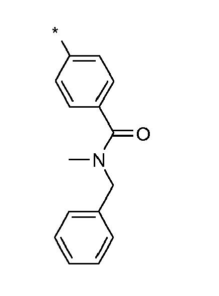 | H | 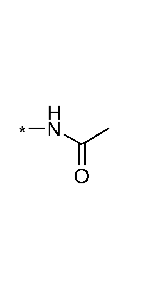 | |
| 249. | 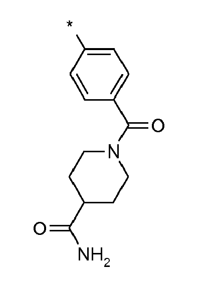 | H | 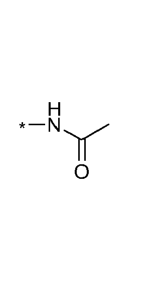 | |
| 250. | 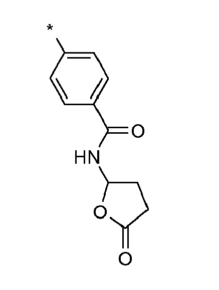 | H | 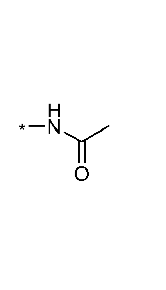 | |
| 251. | 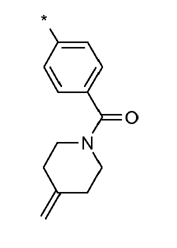 | H | 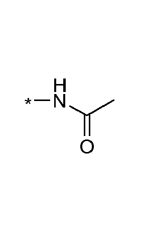 | |
| 252. | 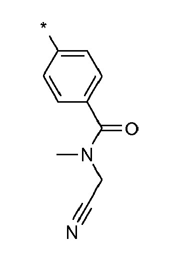 | H | 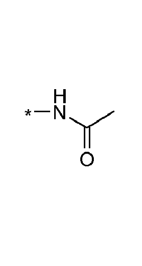 | |
| 253. | 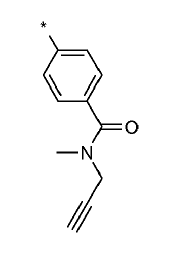 | H | 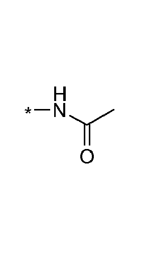 | |
| 254. | 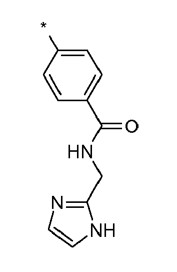 | H | 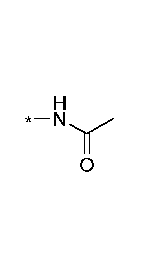 | |
| 255. | 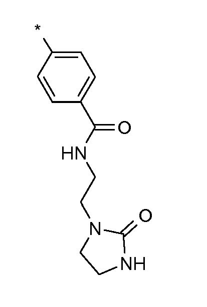 | H | 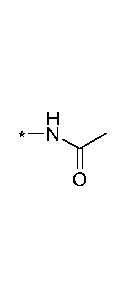 | |
| 256. | 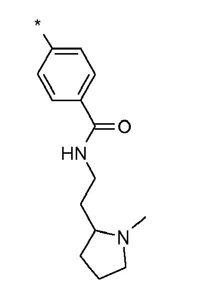 | H | 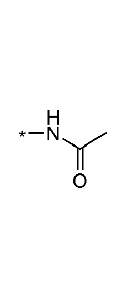 | |
| 257. | 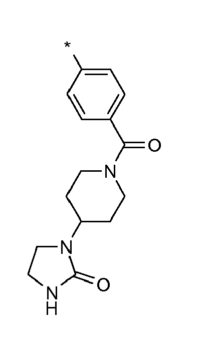 | H | 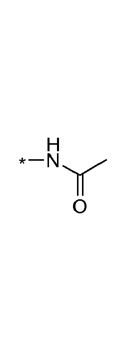 | |
| 258. | 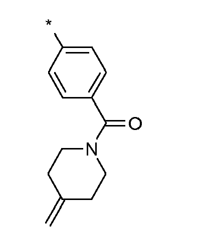 | H | 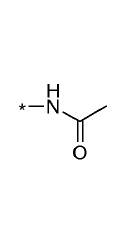 | |
| 259. | 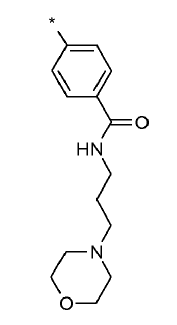 | H | 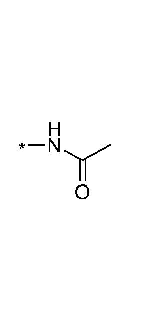 | |
| 260. | 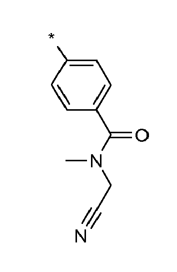 | H | 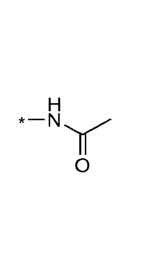 | |
| 261. | 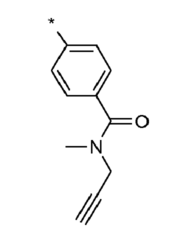 | H | 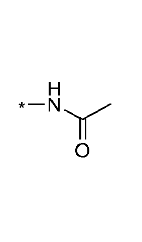 | |
| 262. | 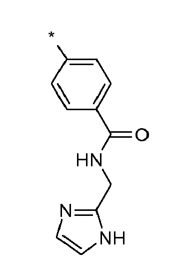 | H | 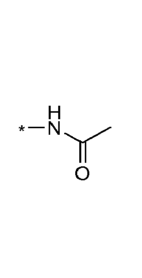 | |
| 263. | 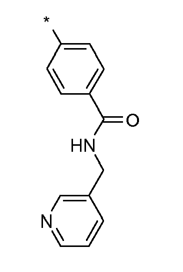 | H | 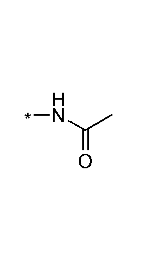 | |
| 264. | 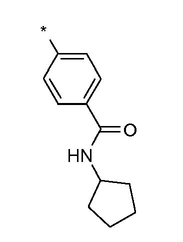 | H | 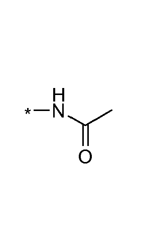 | |
| 265. | 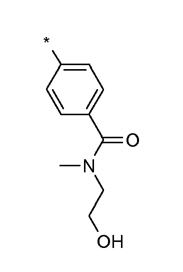 | H | 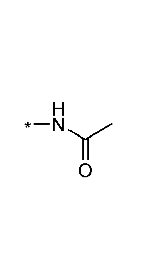 | |
| 266. | 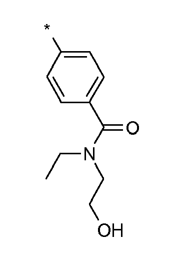 | H | 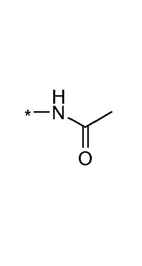 | |
| 267. | 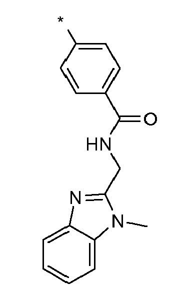 | H | 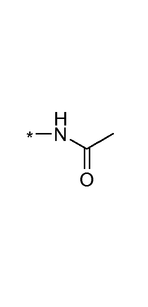 | |
| 268. | 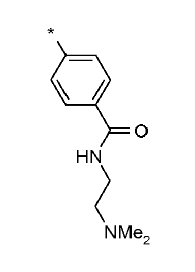 | H | 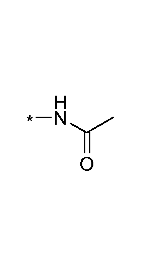 | |
| 269. | 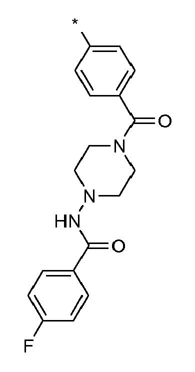 | H | 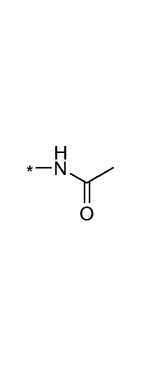 | |
| 270. | 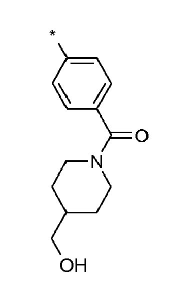 | H | 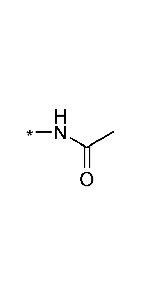 | |
| 271. | 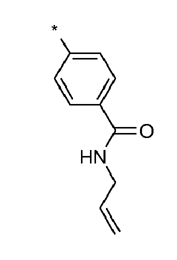 | H | 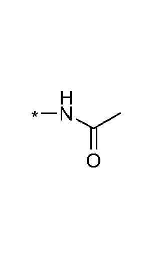 | |
| 272. | 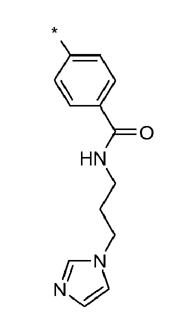 | H | 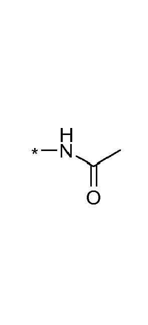 | |
| 273. | 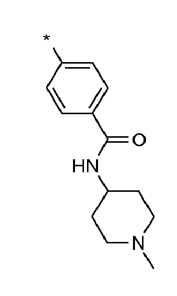 | H | 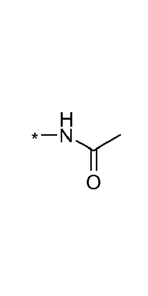 | |
| 274. | 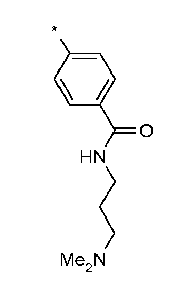 | H | 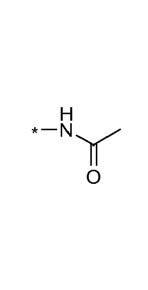 | |
| 275. | 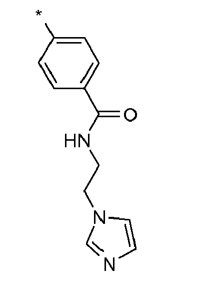 | H | 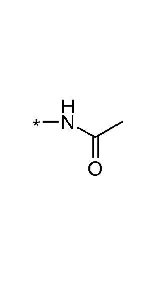 | |
| 276. | 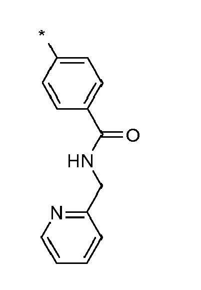 | H | 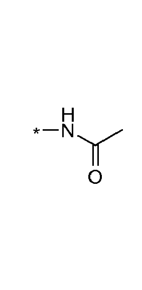 | |
| 277. | 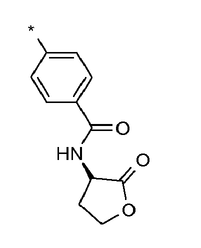 | H | 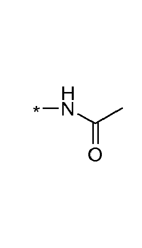 | |
| 278. | 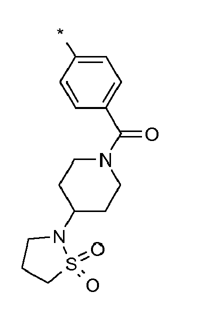 | H | 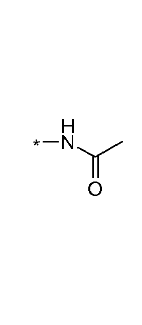 | |
| 279. | 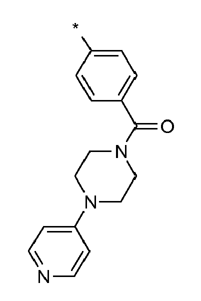 | H | 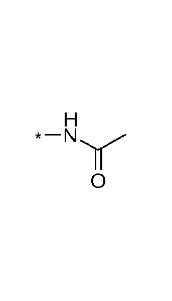 | |
| 280. | 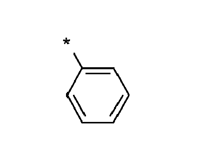 | 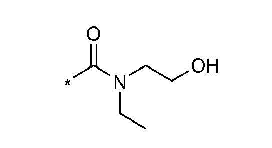 | 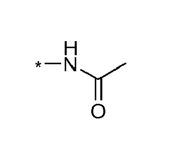 | |
| 281. | 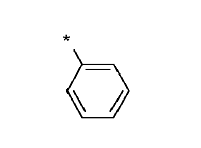 | 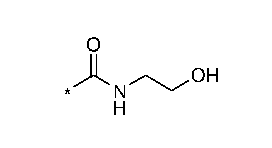 | 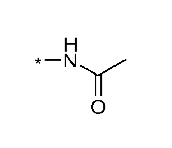 | |
| 282. | 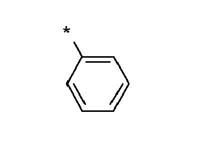 | 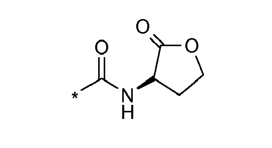 | 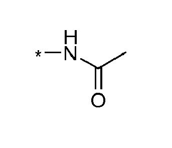 | |
| 283. | 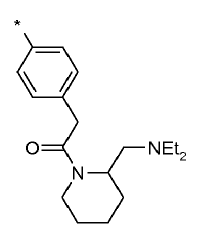 | H | 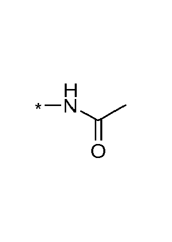 | |
| 284. | 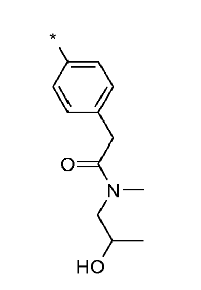 | H | 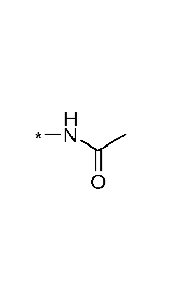 | |
| 285. | 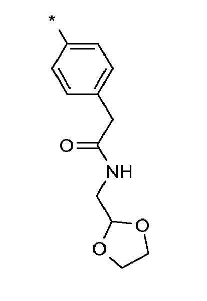 | H | 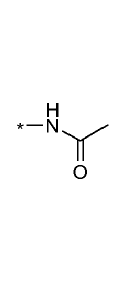 | |
| 286. | 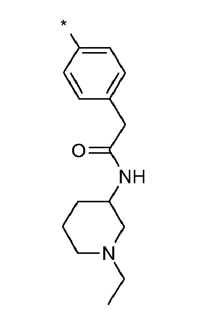 | H | 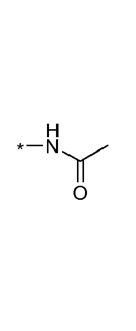 | |
| 287. | 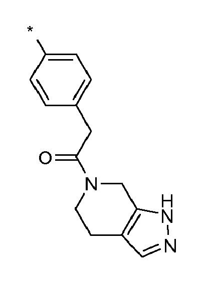 | H | 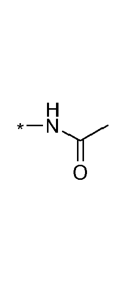 | |
| 288. | 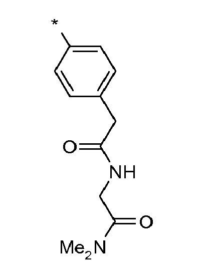 | H | 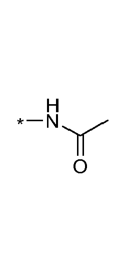 | |
| 289. | 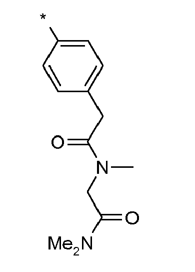 | H | 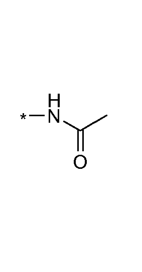 | |
| 290. | 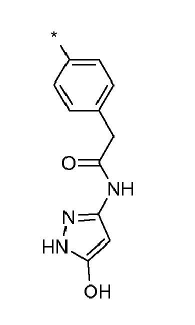 | H | 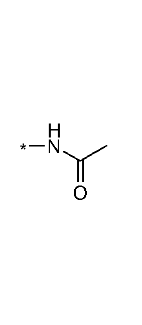 | |
| 291. | 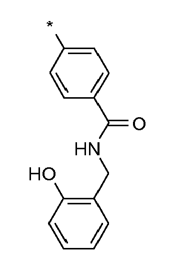 | H | 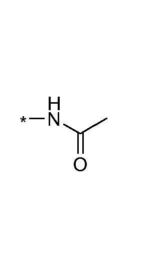 | |
| 292. | 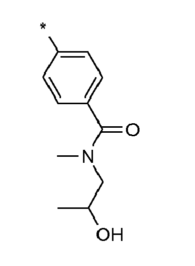 | H | 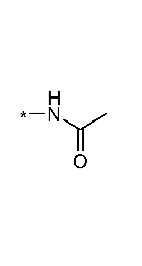 | |
| 293. | 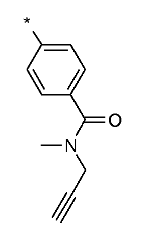 | H | 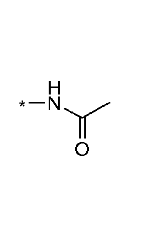 | |
| 294. | 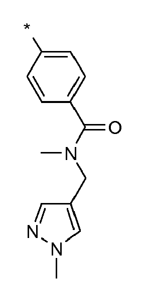 | H | 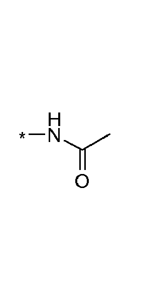 | |
| 295. | 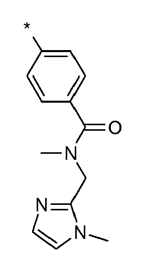 | H | 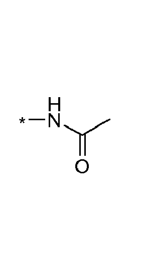 | |
| 296. | 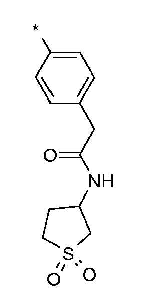 | H | 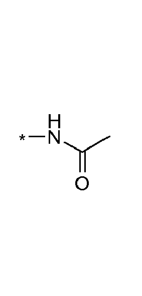 | |
| 297. | 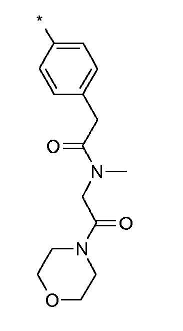 | H | 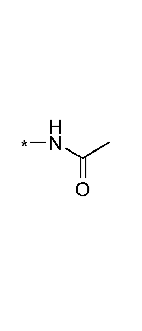 | |
| 298. | 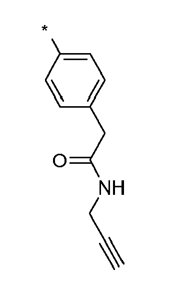 | H | 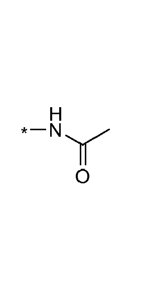 | |
| 299. | 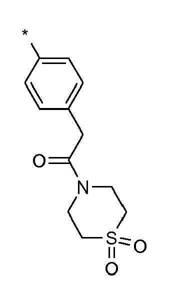 | H | 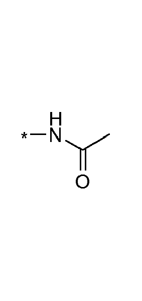 | |
| 300. | 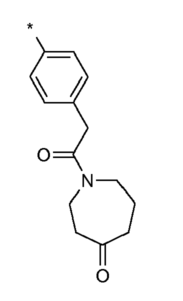 | H | 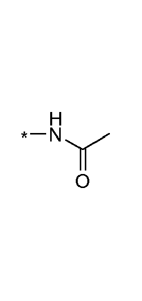 | |
| 301. | 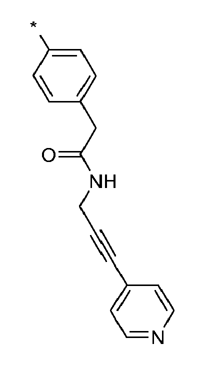 | H | 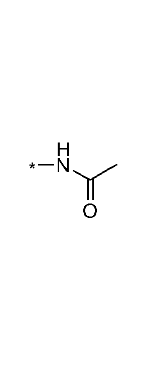 | |
| 302. | 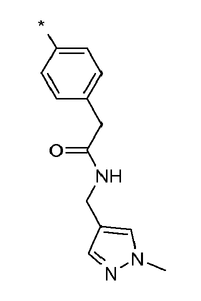 | H | 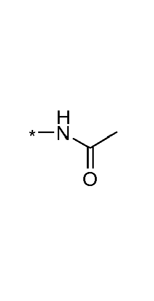 | |
| 303. | 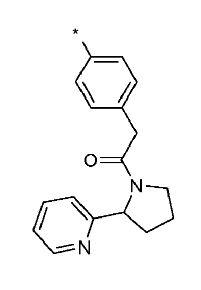 | H | 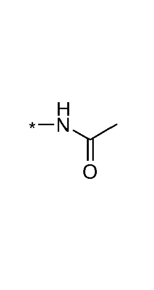 | |
| 304. | 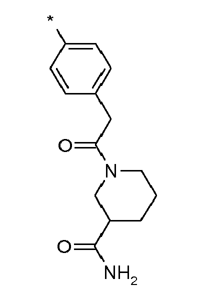 | H | 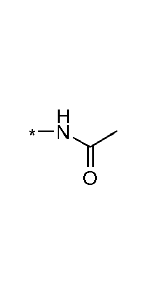 | |
| 305. | 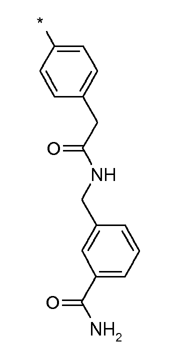 | H | 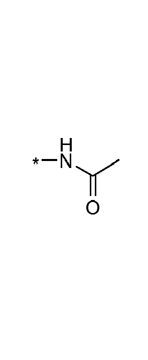 | |
| 306. | 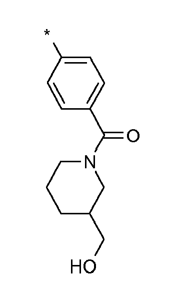 | H | 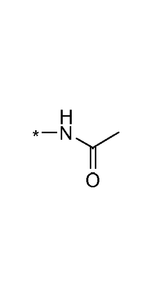 | |
| 307. | 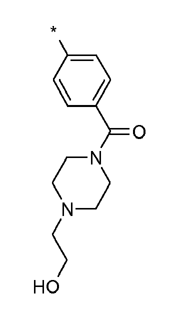 | H | 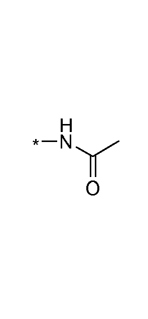 | |
| 308. | 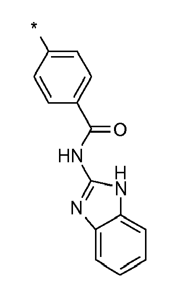 | H | 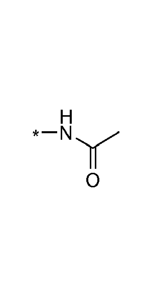 | |
| 309. | 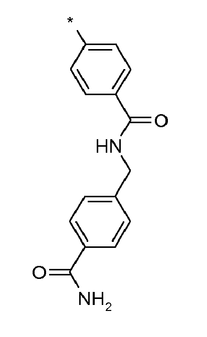 | H | 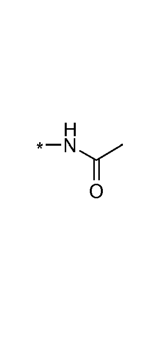 | |
| 310. | 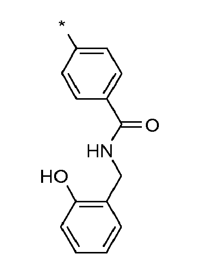 | H | 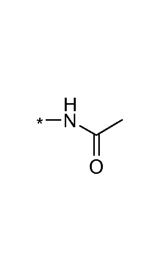 | |
| 311. | 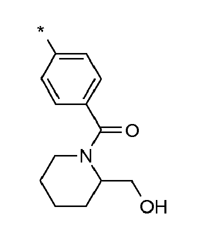 | H | 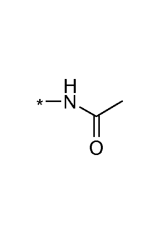 | |
| 312. | 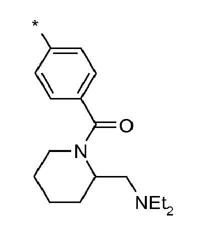 | H | 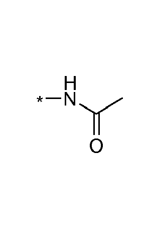 | |
| 313. | 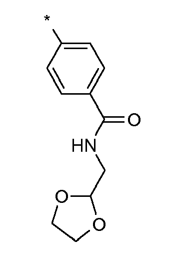 | H | 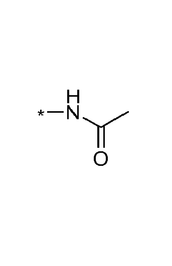 | |
| 314. | 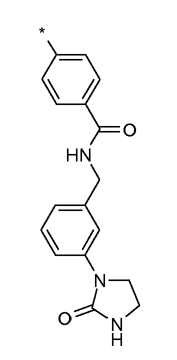 | H | 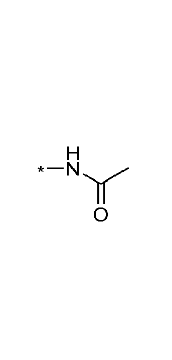 | |
| 315. | 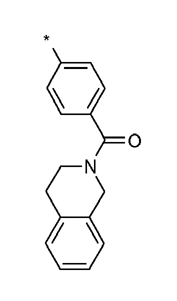 | H | 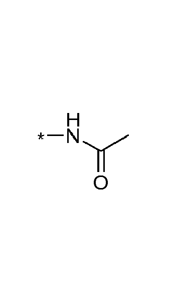 | |
| 316. | 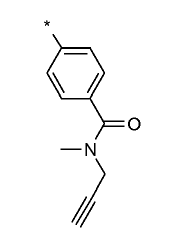 | H | 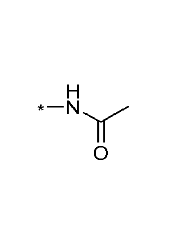 | |
| 317. | 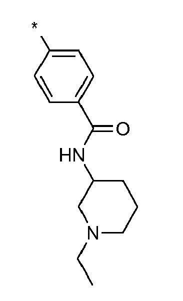 | H | 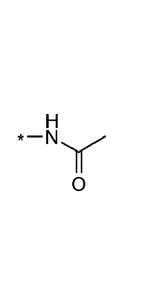 | |
| 318. | 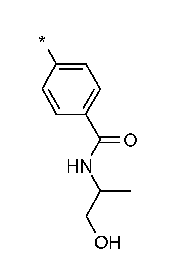 | H | 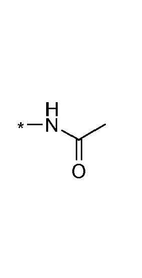 | |
| 319. | 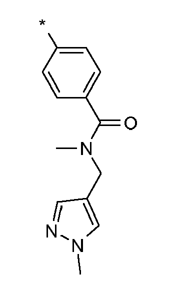 | H | 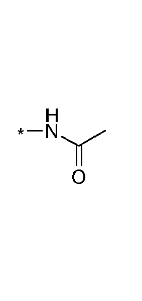 | |
| 320. | 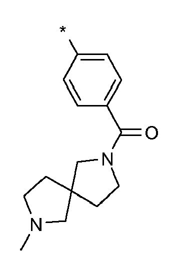 | H | 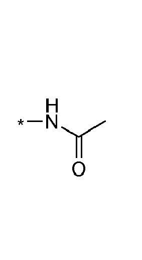 | |
| 321. | 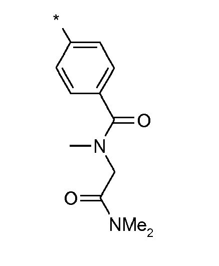 | H | 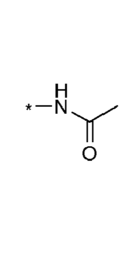 | |
| 322. | 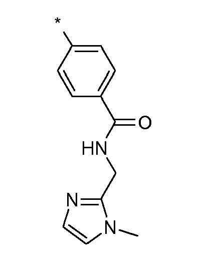 | H | 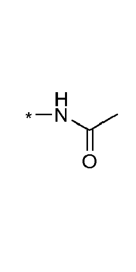 | |
| 323. | 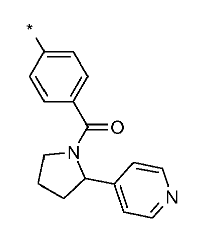 | H | 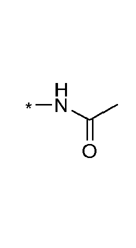 | |
| 324. | 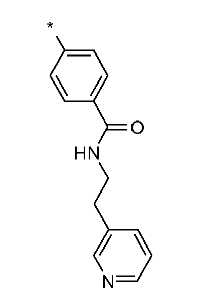 | H | 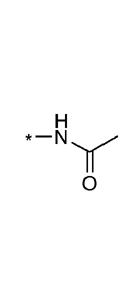 | |
| 325. | 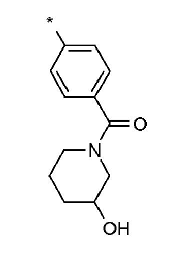 | H | 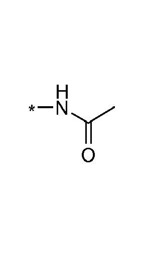 | |
| 326. | 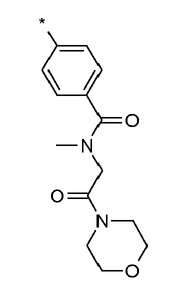 | H | 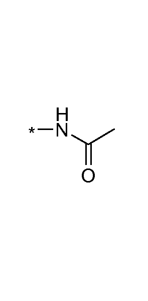 | |
| 327. | 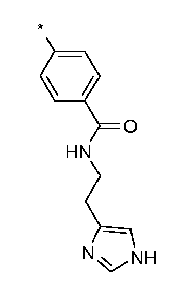 | H | 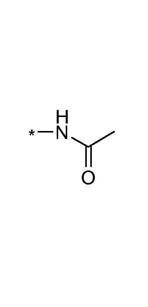 | |
| 328. | 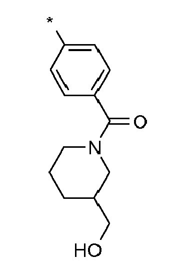 | H | 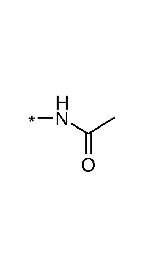 | |
| 329. | 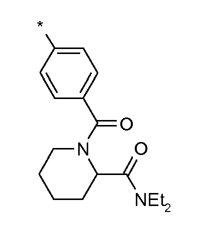 | H | 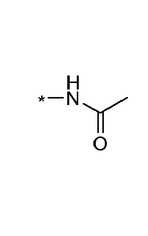 | |
| 330. | 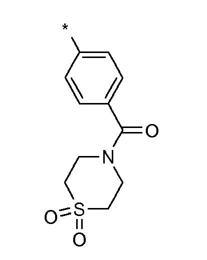 | H | 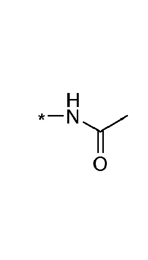 | |
| 331. | 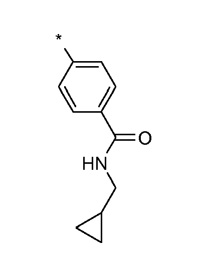 | H | 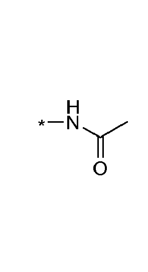 | |
| 332. | 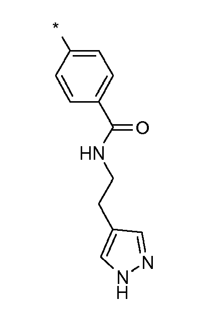 | H | 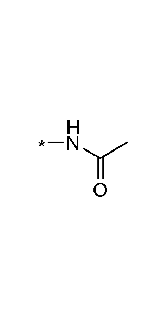 | |
| 333. | 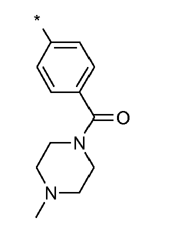 | H | 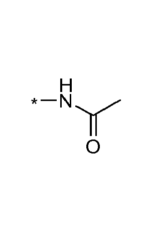 | |
| 334. | 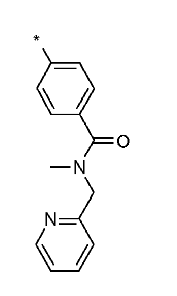 | H | 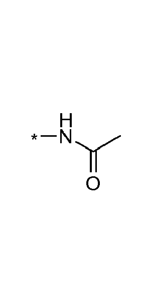 | |
| 335. | 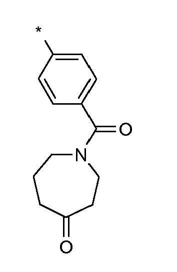 | H | 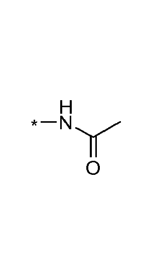 | |
| 336. | 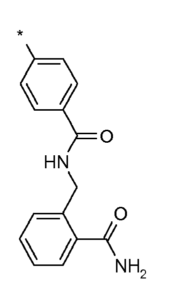 | H | 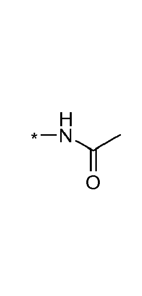 | |
| 337. | 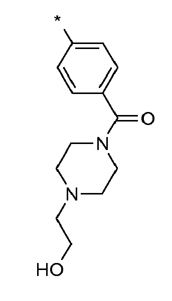 | H | 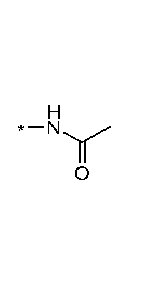 | |
| 338. | 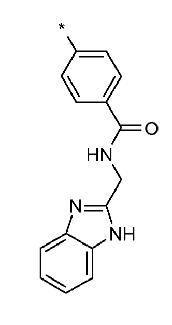 | H | 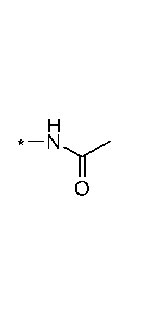 | |
| 339. | 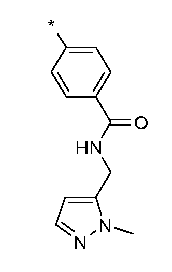 | H | 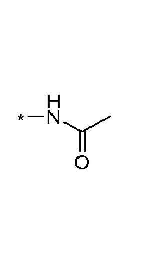 | |
| 340. | 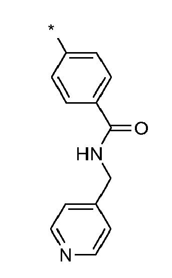 | H | 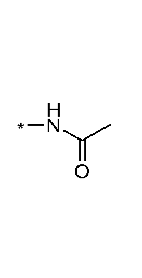 | |
| 341. | 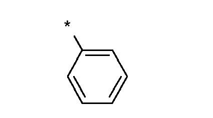 | 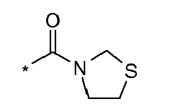 | 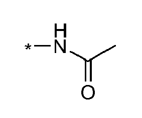 | |
| 342. | 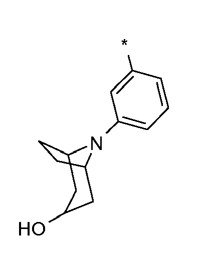 | H | 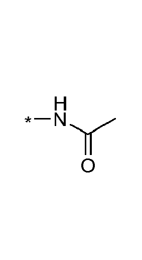 | |
| 343. | 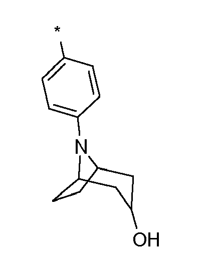 | H | 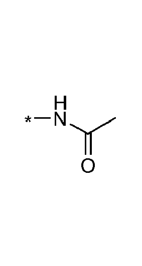 | |
| | | | | |

### BEISPIELE B

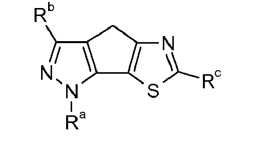

| | **R^{a}** | **R^{b}** | **R^{c}** | **mₚ** |
|---|---|---|---|---|
| 344. | 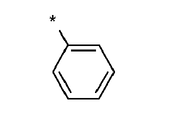 | H | 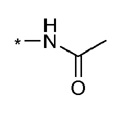 | >300 |
| 345. | 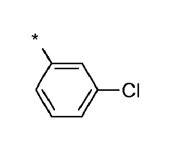 | H | 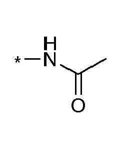 | 298-305 |
| 346. | 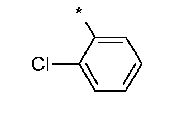 | H | 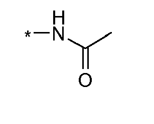 | 255-260 |
| 347. | 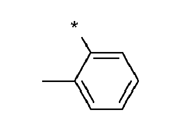 | H | 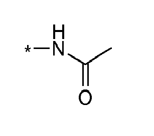 | 245-252 |
| 348. | 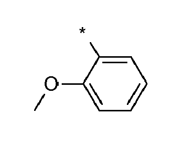 | H | 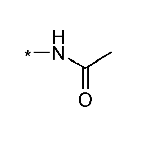 | 288-295 |
| 349. | 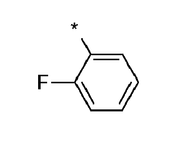 | H | 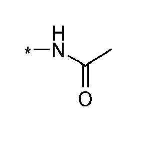 | |
| 350. | 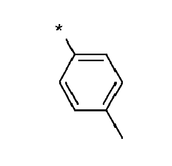 | H | 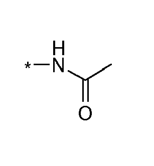 | >300 |
| 351. | 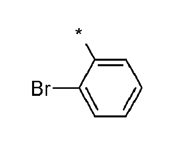 | H | 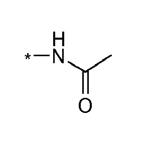 | 296-300 |
| 352. | 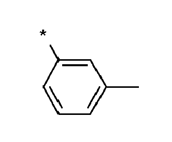 | H | 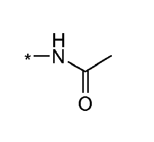 | 292-300 |
| 353. | 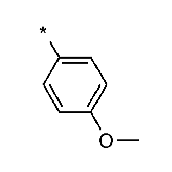 | H | 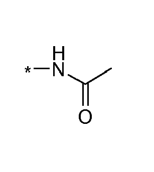 | >280 |
| 354. | 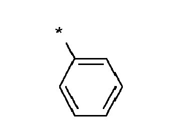 | H | 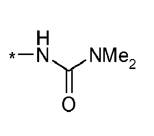 | 238-243 |
| 355. | 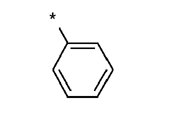 | H | 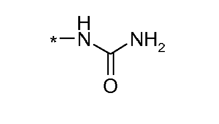 | >300 |
| 356. | 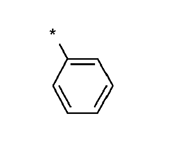 | H | 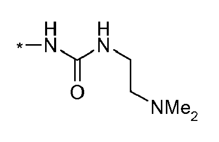 | 205-210 |
| 357. | 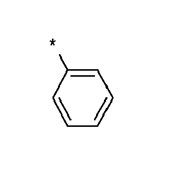 | H | 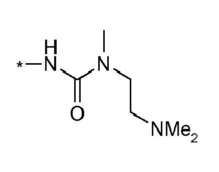 | 200-204 |
| 358. | 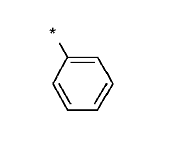 | H | 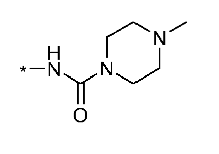 | 108-115 |
| 359. | 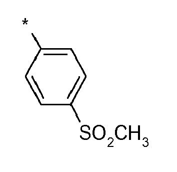 | H | 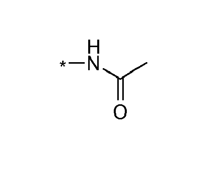 | >300 |
| 360. | 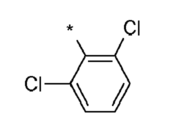 | H | 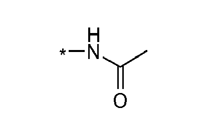 | 175-180 |
| 361. | 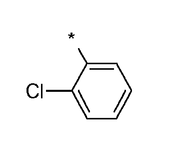 | H | 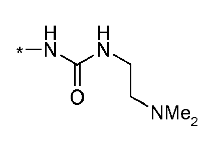 | 172-175 |
| 362. | 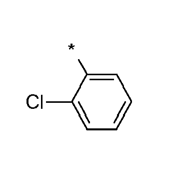 | H | 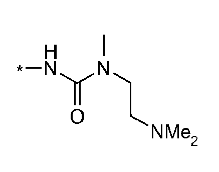 | 220-222 |
| 363. | 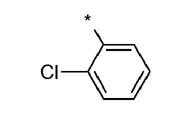 | H | 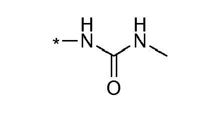 | >250 |
| 364. | 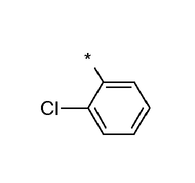 | H | 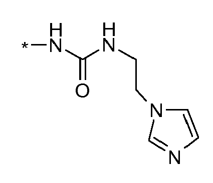 | 239-242 |
| 365. | 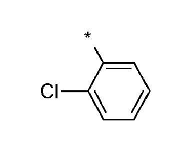 | H | 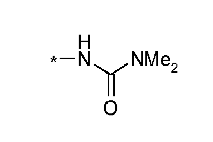 | 216-219 |
| 366. | 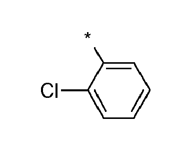 | H | 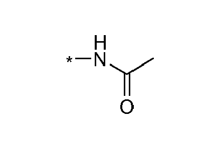 | >300 |
| 367. | 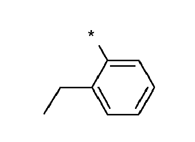 | H | 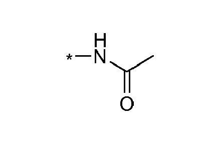 | 199-201 |
| 368. | 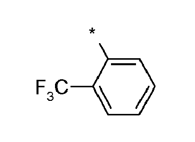 | H | 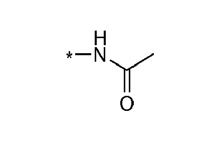 | >300 |
| 369. | 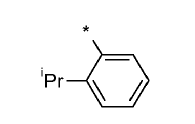 | H | 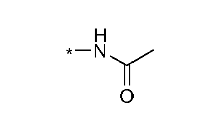 | 246-247 |
| | | | | |

### BEISPIELE C

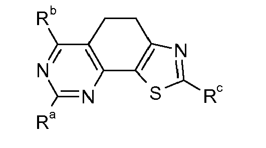

| | **R^{a}** | **R^{b}** | **R^{c}** | **mₚ** |
|---|---|---|---|---|
| 370. | 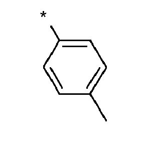 | H | 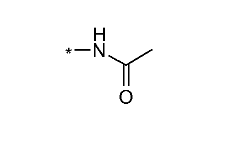 | > 275 |
| 371. | 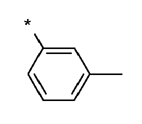 | H | 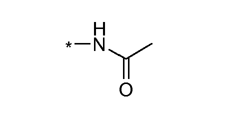 | 267-268 |
| 372. | 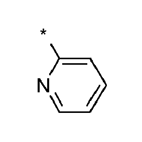 | H | 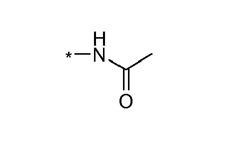 | |
| 373. | 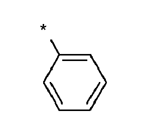 | H | 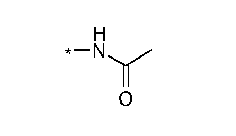 | > 295 |
| 374. | 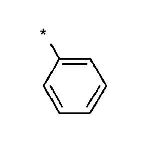 | H | 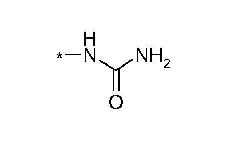 | >300 |
| 375. | 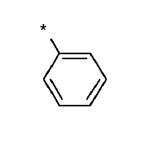 | H | 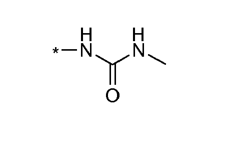 | > 300 |
| 376. | 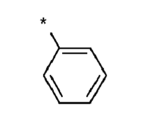 | H | 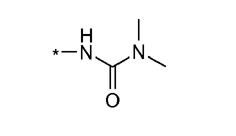 | 208-209 |
| 377. | 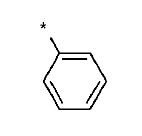 | H | 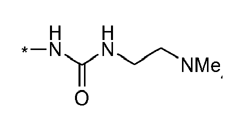 | 132-133 |
| 378. | 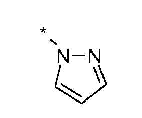 | H | 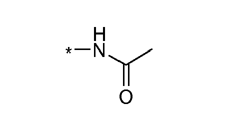 | > 300 |
| 379. | 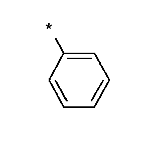 | H | 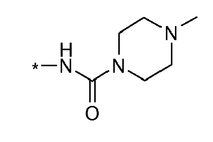 | 147.4-150 |
| 380. | 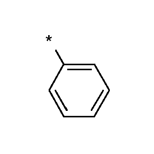 | H | 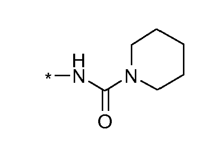 | 147-149.3 |
| 381. | 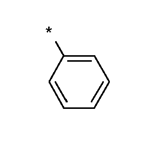 | H | 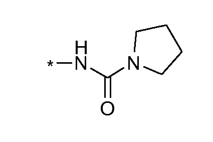 | 263.4-264.5 |
| 382. | 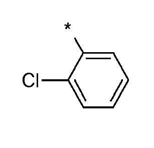 | H | 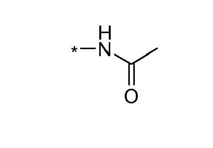 | 237.9-239.4 |
| 383. | 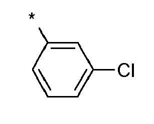 | H | 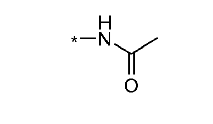 | 272-273 |
| 384. | 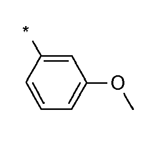 | H | 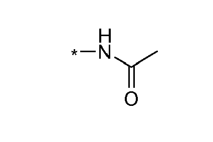 | 242-243 |
| 385. | 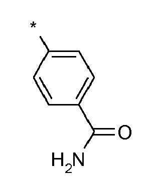 | H | 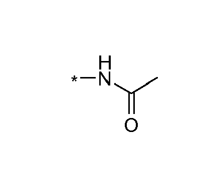 | >300 |
| 386. | 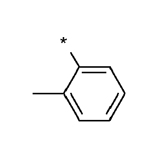 | H | 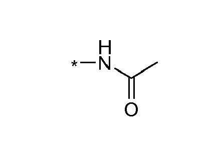 | 196-199 |
| 387. | 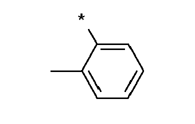 | H | 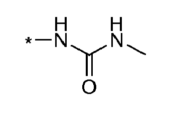 | 221-222 |
| 388. | 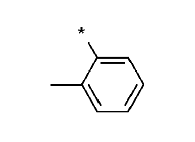 | H | 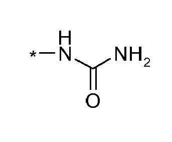 | > 300 |
| 389. | 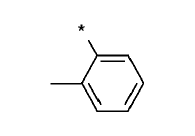 | H | 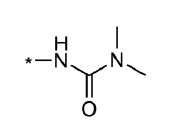 | 231-232 |
| 390. | 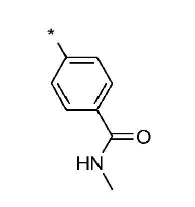 | H | 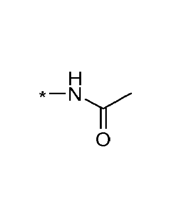 | >300 |
| 391. | 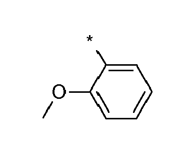 | H | 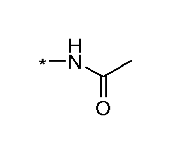 | 234-234.5 |
| 392. | 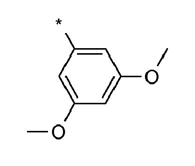 | H | 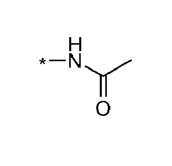 | 273-274 |
| 393. | 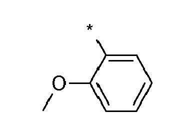 | H | 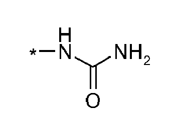 | 194-196 |
| 394. | 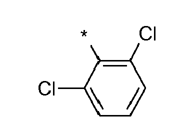 | H | 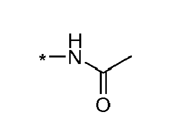 | 287.7-289 |
| 395. | 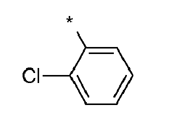 | OH | 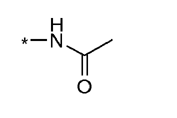 | |
| | | | | |

### BEISPIELE D

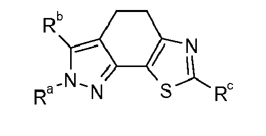

| | **R^{a}** | **R^{b}** | **R^{c}** | **mₚ** |
|---|---|---|---|---|
| 396. | 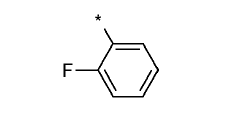 | 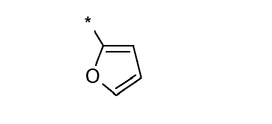 | 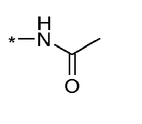 | 289-290 |
| 397. | 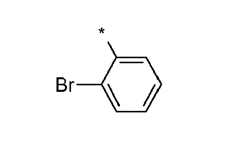 | 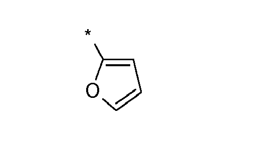 | 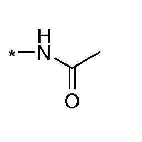 | >300 |
| 398. | 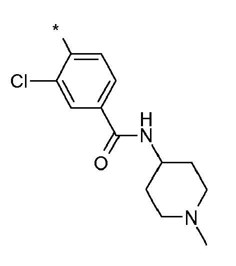 | 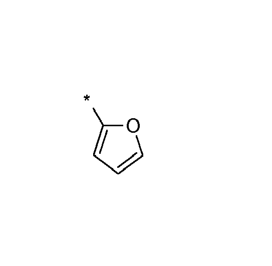 | 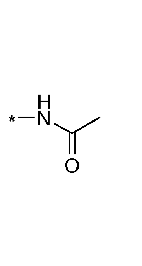 | |
| 399. | 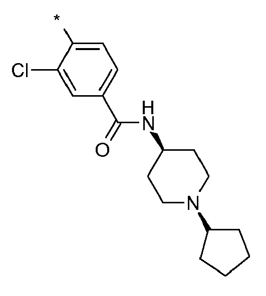 | 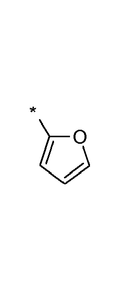 | 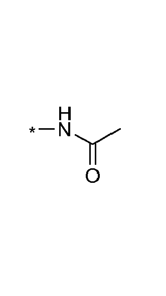 | |
| 400. | 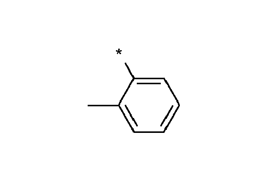 | 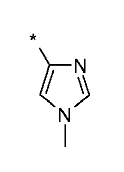 | 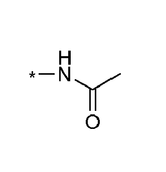 | >300 |
| 401. | 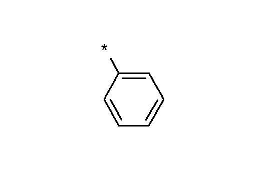 | 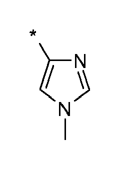 | 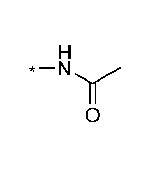 | > 300 |
| 402. | 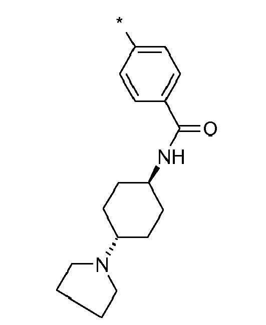 | 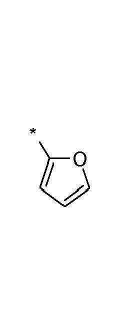 | 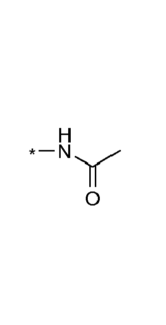 | |
| | | | | |

### BEISPIELE E

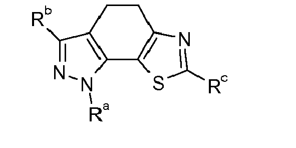

| | **R^{a}** | **R^{b}** | **R^{c}** | **mₚ** |
|---|---|---|---|---|
| 403. | -CH₃ | H | 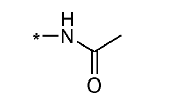 | |
| 404. | 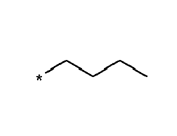 | H | 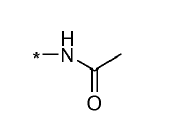 | |
| 405. | 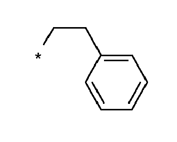 | H | 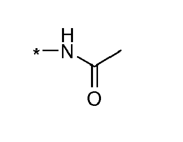 | |
| 406. | 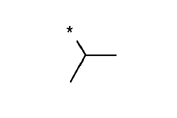 | H | 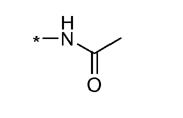 | |
| 407. | 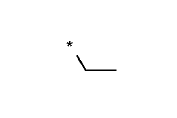 | H | 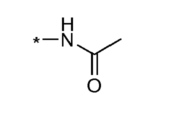 | |
| 408. | 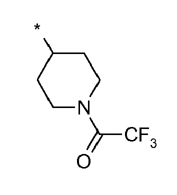 | H | 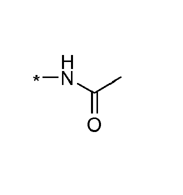 | |
| 409. | 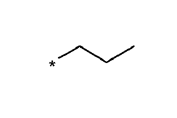 | H | 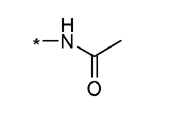 | 246-247 |
| 410. | 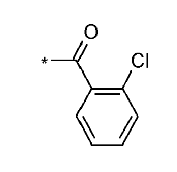 | H | 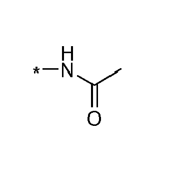 | |
| 411. | 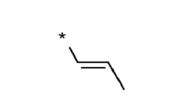 | H | 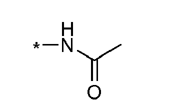 | |
| 412. | 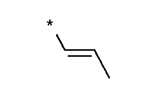 | H | 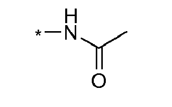 | |
| 413. | 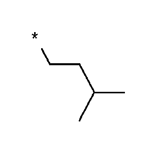 | H | 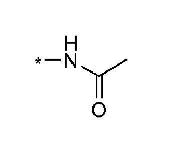 | 191 |
| 414. | 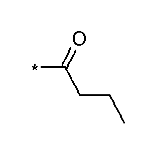 | H | 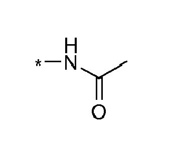 | |
| 415. | 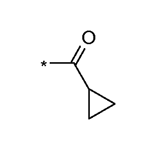 | H | 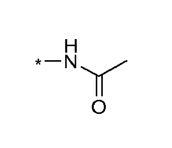 | |
| 416. | 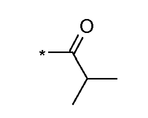 | H | 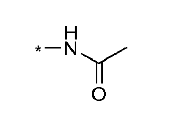 | |
| | | | | |

### BEISPIELE F

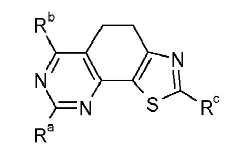

| | **R^{a}** | **R^{b}** | **R^{c}** | **mₚ** |
|---|---|---|---|---|
| 417. | 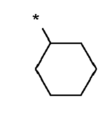 | H | 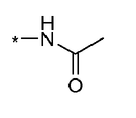 | |
| 418. | 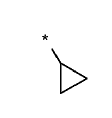 | H | 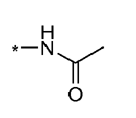 | |
| 419. | 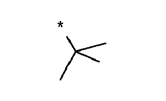 | H | 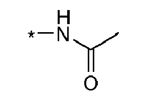 | |
| 420. | 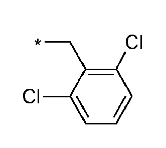 | H | 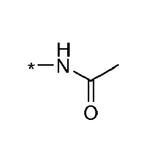 | |
| 421. | 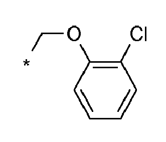 | H | 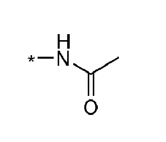 | |
| 422. | 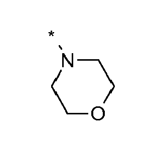 | H | 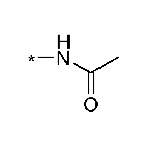 | |
| 423. | 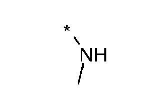 | H | 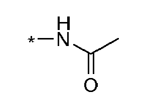 | 235-238 |
| 424. | 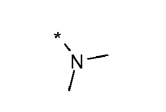 | H | 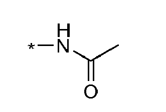 | 281-282 |
| 425. | -CF₃ | H | 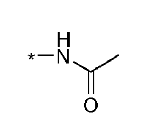 | 366-370 |
| 426. | 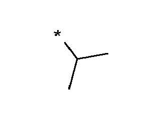 | H | 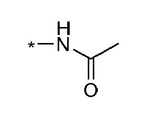 | 248.4-250.6 |
| 427. | 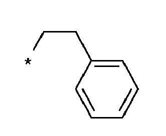 | H | 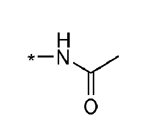 | 198-199 |
| 428. | -CH₃ | H | 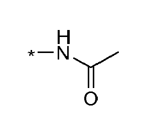 | 224-225 |
| 429. | 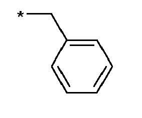 | H | 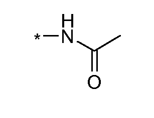 | 94-103 |
| 430. | 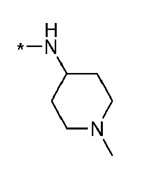 | H | 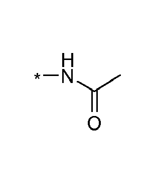 | 220.5-221.5 |
| 431. | 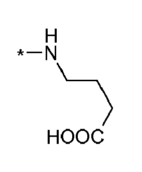 | H | 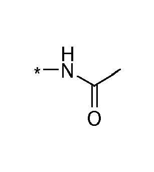 | |
| 432. | 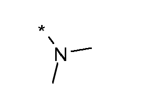 | H | 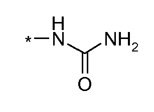 | 367.9-368.5 |
| 433. | 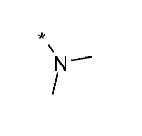 | H | 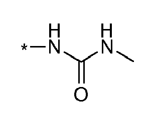 | 369.4-370.4 |
| 434. | 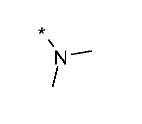 | H | 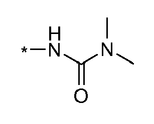 | 294.2-296.7 |
| 435. | 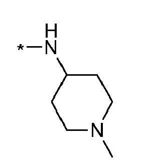 | H | 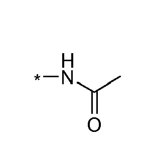 | 274-276 |
| 436. | 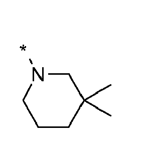 | H | 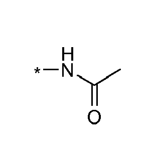 | 299-301 |
| 437. | 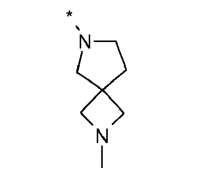 | H | 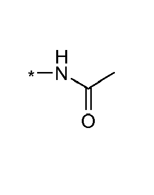 | 267-269 |
| 438. | 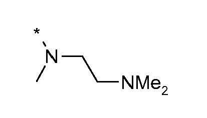 | H | 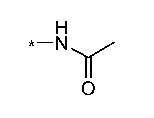 | |
| 439. | 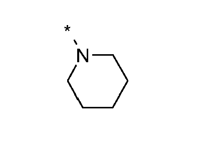 | H | 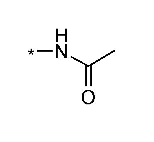 | |
| 440. | 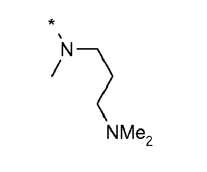 | H | 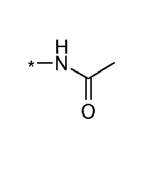 | |
| 441. | 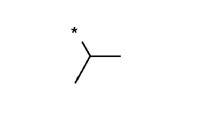 | OH | 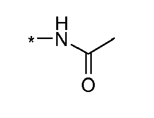 | |
| 442. | 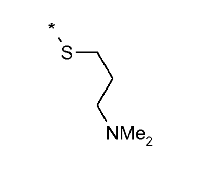 | H | 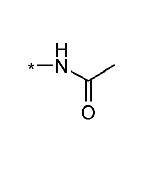 | |
| 443. | 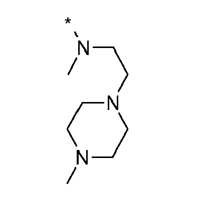 | H | 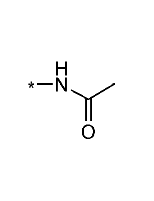 | |
| 444. | 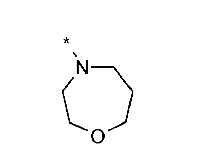 | H | 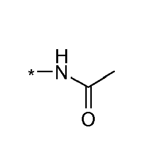 | |
| | | | | |

### BEISPIELE G

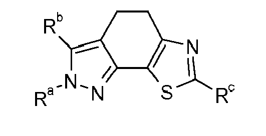

| | **R^{a}** | **R^{b}** | **R^{c}** | **mₚ** |
|---|---|---|---|---|
| 445. | 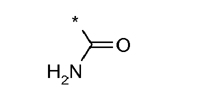 | H | 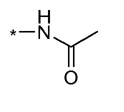 | |
| 446. | 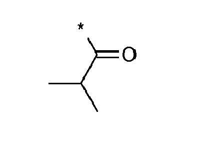 | H | 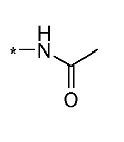 | |
| 447. | 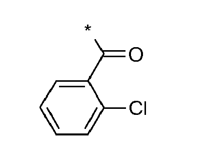 | H | 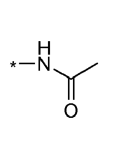 | |
| 448. | 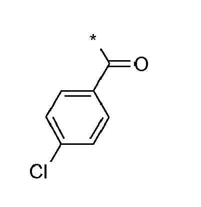 | H | 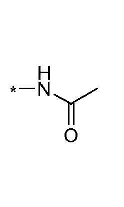 | |
| 449. | 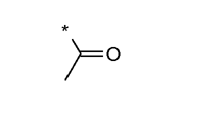 | H | 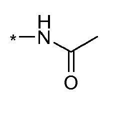 | |
| 450. | 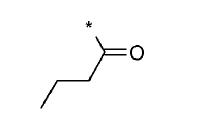 | H | 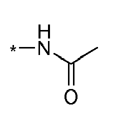 | |
| 451. | 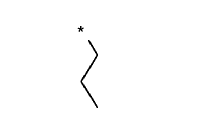 | H | 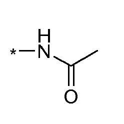 | 208-209 |
| 452. | 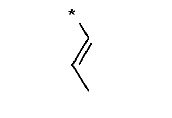 | H | 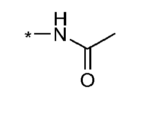 | |
| 453. | 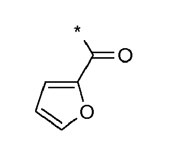 | H | 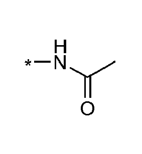 | |
| 454. | 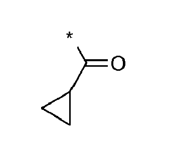 | H | 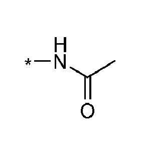 | |
| | | | | |

### BEISPIELE H

| | | **mₚ** |
|---|---|---|
| 455. | 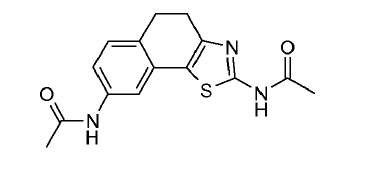 | 181-183 Zersetz. |
| 456. | 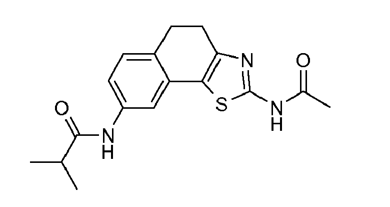 | 272-274 Zersetz. |
| 457. | 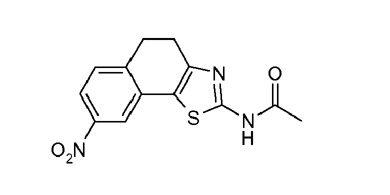 | > 315 |
| 458. | 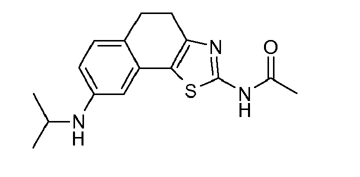 | 183-184 |
| | | |

### INDIKATIONSGEBIETE

Wie gefunden wurde, zeichnen sich die Verbindungen der Formel **1** durch vielfältige Anwendungsmöglichkeiten auf therapeutischem Gebiet aus. Hervorzuheben sind solche Anwendungsmöglichkeiten, für welche die erfindungsgemäßen Verbindungen der Formel **1** aufgrund ihrer pharmazeutischen Wirksamkeit als PI3-Kinase Modulator bevorzugt zur Anwendung gelangen können.

Allgemein ausgedrückt sind dies Erkrankungen, bei deren Pathologie eine Aktivität von PI3-Kinasen beteiligt ist, insbesondere entzündliche und allergische Erkrankungen. Insbesondere seien entzündliche und allergische Atemwegserkrankungen, entzündliche Erkrankungen des Magen-Darm Traktes, entzündliche Erkrankungen des Bewegungsapparates, entzündliche und allergische Hauterkrankungen, entzündliche Augenerkrankungen, Erkrankungen der Nasenschleimhaut, entzündliche oder allergische Krankheitszustände, bei denen Autoimmun-Reaktionen beteiligt sind oder Nierenentzündungen genannt. Die Behandlung kann dabei symptomatisch, adaptiv, kurativ oder präventiv erfolgen.

Bevorzugt genannte Atemwegserkrankungen wären hierbei chronische und/oder obstruktive Atemwegserkrankungen. Die erfindungsgemäßen Verbindungen der Formel **1** können dabei, auf Grund ihrer pharmakologischen Eigenschaften eine Reduzierung der
- Gewebezerstörung
- der Entzündung der Atemwege
- der bronchialen Hyperreaktivität
- des Umbauprozesses der Lunge infolge der Entzündung
- der Verschlechterung der Krankheit (Progression)
bewirken. Besonders bevorzugt sind die erfindungsgemäßen Verbindungen zur Herstellung eines Medikaments zur Behandlung von Chronischer Bronchitis, akuter Bronchitis, Bronchitis aufgrund bakterieller oder viraler Infektion oder Pilzen oder Helminthen, allergischer Bronchitis, toxischer Bronchitis, chronisch obstruktiver Bronchitis (COPD), Asthma (intrinsisch oder allergisch), pediatrischem Asthma, Bronchiektasien, allergischer Alveolitis, allergischer oder nicht-allergischer Rhinitis, chronischer Sinusitis, zystischer Fibrose oder Mukoviszidose, alpha-1-Antitrypsin-Mangel, Husten, Lungenemphysem, interstitieller Lungenerkrankungen wie z.B. Lungenfibrose, Asbestose und Silikose und Alveolitis; hyperreaktiver Atemwege, Nasenpolypen, Lungenödemen wie z.B. toxischem Lungenödem und ARDS / IRDS, Pneumonitis aufgrund unterschiedlicher Genese wie Strahlen-induziert oder durch Aspiration oder infektiöse, Kollagenosen wie Lupus eryth, systemische Sklerodermie, Sarkoidose oder M. Boeck.

Ebenfalls geeignet sind die Verbindungen der Formel **1** zur Behandlung von Erkrankungen der Haut, wie z.B. Psoriasis, Kontakt-Dermatitis, atopische Dermatitis, Alopecia areata (kreisrunder Haarausfall), Erythema exsudativum multiforme (Stevens-Johnson-Syndrom), Dermatitis herpetiformis, Sklerodermie, Vitiligo, Nesselsucht (Urticaria), Lupus erythematodes, follikuläre und flächenhafte Pyodermien, endogene und exogene Akne, Akne rosacea sowie andere entzündliche oder allergische oder proliferative Hauterkrankungen.

Weiterhin sind die Verbindungen der Formel **1** zum therapeutischen Einsatz geeignet bei entzündlichen oder allergischen Krankheitszuständen, bei denen Autoimmun-Reaktionen beteiligt sind, wie z.B. entzündliche Darmerkrankungen, z.B. Morbus Crohn oder Colitis ulzerosa; Krankheiten aus dem Formenkreis der Arthritis, wie z.B. rheumatoide oder psoriatrische Arthritis, Osteoarthritis, rheumatoide Spondylitis und andere arthritische Zustände oder Multiple Sklerose.

Ferner seien folgende allgemeine entzündliche oder allergische Erkrankungen genannt, die sich durch Medikamente beinhaltend Verbindungen der Formel **1** behandeln lassen:
- Entzündungen am Auge, wie z.B. Bindehautentzündung (Konjunktivitis) verschiedener Arten, wie z.B. durch Infektionen mit Pilzen oder Bakterien, allergische Konjunktivitis, Reizkonjunktivitis, durch Medikamenten induzierten Konjunktivitis, Keratitis, Uveitis
- Krankheiten der Naseschleimhaut, wie z.B. allergische Rhinitis/Sinusitis oder Nasenpolypen
- Entzündliche oder allergische Krankheitszustände, wie z.B. systemische Lupus erythematodes, chronische Hepatitis, Nierenentzündungen, wie Glomerulonephritis, interstitielle Nephritis oder idiopathisches nephrotisches Syndrom.

Als weitere Krankheiten, die auf Grund der pharmakologischen Wirksamkeit der Verbindungen der Formel **1** mit einem Medikament selbige beinhaltend behandelt werden können, seien toxische oder septische Schocksyndrome, Atherosklerose, Mittelohrentzündung, (Otitis media), Hypertrophie des Herzens, Herzinsuffizienz, Schlaganfall, Ischämie-Reperfusionsschäden oder neurodegenerative Erkrankungen wie Parkinson'sche Krankheit oder Alzheimer genannt.

### KOMBINATIONEN

Die Verbindungen der Formel **1** können allein oder in Kombination mit anderen erfindungsgemäßen Wirkstoffen der Formel **1** zur Anwendung gelangen. Gegebenenfalls können die Verbindungen der Formel **1** auch in Kombination mit weiteren pharmakologisch aktiven Wirkstoffen eingesetzt werden. Bevorzugt gelangen hierbei solche Wirkstoffe zur Anwendung, die beispielsweise ausgewählt sind aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren oder zwei- oder dreifach Kombinationen davon, wie beispielsweise Kombinationen von
- Betamimetika mit Corticosteroiden, PDE4-Inhibitoren, EGFR-Hemmern oder LTD4-Antagonisten,
- Anticholinergika mit Betamimetika, Corticosteroiden, PDE4-Inhibitoren, EGFR-Hemmern oder LTD4-Antagonisten,
- Corticosteroiden mit PDE4-Inhibitoren, EGFR-Hemmern oder LTD4-Antagonisten
- PDE4-Inhibitoren mit EGFR-Hemmern oder LTD4-Antagonisten
- EGFR-Hemmern mit LTD4-Antagonisten.
Auch die Kombinationen dreier Wirkstoffe je einer der o.g. Verbindungsklassen ist Bestandteil der Erfindung.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HOKU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluoro-4-hydroxyphenyl)-2-[4-(1-benzimidazolyl)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chloro-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethylethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodid und Methansulfonat besonders bevorzugt. Weiterhin genannte Verbindungen sind:
- 2,2-Diphenylpropionsäuretropenolester-methobromid
- 2,2-Diphenylpropionsäurescopinester-methobromid
- 2-Fluor-2,2-diphenylessigsäurescopinester-methobromid
- 2-Fluor-2,2-Diphenylessigsäuretropenolester-methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester -Methobromid
- 9-Fluor-fluoren-9-carbonsäuretropenolester -Methobromid
- 9-Hydroxy-fluoren-9-carbonsäurescopinester -Methobromid
- 9-Fluor-fluoren-9-carbonsäurescopinester Methobromid
- 9-Methyl-fluoren-9-carbonsäuretropenolesterMethobromid
- 9-Methyl-fluoren-9-carbonsäurescopinesterMethobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinesterMethobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester -Methobromid
- 4,4'-Difluorbenzilsäuremethylestercyclopropyltropinester-Methobromid
- 9-Hydroxy-xanthen-9-carbonsäuretropenolester -Methobromid
- 9-Hydroxy-xanthen-9-carbonsäurescopinester Methobromid
- 9-Methyl-xanthen-9-carbonsäuretropenolester -Methobromid
- 9-Methyl-xanthen-9-carbonsäurescopinesterMethobromid
- 9-Ethyl-xanthen-9-carbonsäuretropenolester Methobromid
- 9-Difluormethyl-xanthen-9-carbonsäuretropenolester -Methobromid
- 9-Hydroxymethyl-xanthen-9-carbonsäurescopinester -Methobromid

Als Corticosteroide gelangen hierbei vorzugsweise Verbindungen zur Anwendung, die ausgewählt sind aus der Gruppe bestehend aus Prednisolon, Prednison, Butixocortpropionat, Flunisolid, Beclomethason, Triamcinolon, Budesonid, Fluticason, Mometason, Ciclesonid, Rofleponid, Dexamethason, Betamethason, Deflazacort, RPR-106541, NS-126, ST-26 und
- 6,9-Difluoro-17-[(2-furanylcarbonyl)oxy]-11-hydroxy-16-methyl-3-oxo-androsta-1,4-dien-17-carbothionsäure (S)-fluoromethylester
- 6,9-Difluoro-11-hydroxy-16-methyl-3-oxo-17-propionyloxy-androsta-1,4-dien-17-carbothionsäure (S)-(2-oxo-tetrahydro-furan-3S-yl)ester,
- Etiprednol-dichloroacetat
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, CI-1018, CDC-801, CDC-3052, D-22888, YM-58997, Z-15370 und
- N-(3,5-Dichloro-1-oxo-pyridin-4-yl)-4-difluoromethoxy-3-cyclopropylmethoxybenzamid
- (-)p-[(4*a*R*,10*b*S*)-9-Ethoxy-1,2,3,4,4a,10b-hexahydro-8-methoxy-2-methylbenzo[s][1,6]naphthyridin-6-yl]-N,N-dsiiopropylbenzamid
- (R)-(+)-1-(4-Bromobenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methylisothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxyphenyl)cyclohexan-1-on
- cis[4-Cyano-4-(3-cyclopropylmethoxy-4-difluoromethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9H-pyrazolo[3,4-c]-1,2,4-triazolo[4,3-a]pyridin
- 9-Cyclopentyl-5,6-dihydro-7-ethyl-3-(tert-butyl)-9H-pyrazolo[3,4-c]-1,2,4-triazolo[4,3-a]pyridin
gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluoro-2-quinolinyl)ethenyl)phenyl)-3-(2-(2- hydroxy-2-propyl)phenyl)thio)methylcyclopropan-essigsäure,
- 1-(((1 (R)-3(3-(2-(2,3-Dichlorothieno[3,2-b]pyridin-5-yl)-(E)-ethenyl)phenyl)-3-(2-(1-hydroxy-1-methylethyl)phenyl)propyl)thio)methyl)cyclopropanessigsäure
- [2-[[2-(4-tert-Butyl-2-thiazolyl)-5-benzofuranyl]oxymethyl]phenyl]essigsäure
gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-[N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-[N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonylethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxychinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylaminoethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methylamino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methylamino}-cyclohexan-1-yloxy)-7-methoxy- chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxychinazolin
- 4-[(3-Chlor-4-fluor-phenyl)amino]-6-(1-methansulfonyl-piperidin-4-yloxy)-7-(2-methoxyethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxychinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methylamino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxychinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxychinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4-{N-[(morpholin-4-yl)carbonyl]-N-methylamino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxychinazolin
- 4-[(3-Chlor-4-fluor-phenyl)amino]-6-(1-cyano-piperidin-4-yloxy)-7-methoxy-chinazolin gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als PAF-Antagonisten gelangen hierbei vorzugsweise Verbindungen zur Anwendung, die ausgewählt sind aus der Gruppe bestehend aus
- 4-(2-Chlorphenyl)-9-methyl-2-[3(4-morpholinyl)-3-propanon-1-yl]-6H-thieno-[3,2-f]-[1,2,4]triazolo[4,3-a][1,4]diazepin
- 6-(2-Chlorphenyl)-8,9-dihydro-1-methyl-8-[(4-morpholinyl)carbonyl]-4H,7H-cyclopenta-[4,5]thieno-[3,2-f][1,2,4]triazolo[4,3-a][1,4]diazepin
gegebenenfalls in Form ihrer Racemate, Enantiomere, Diasteromere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

### FORMULIERUNGEN

Die erfindungsgemäßen Verbindungen können oral, transdermal, inhalativ, parenteral oder sublingual verabreicht werden. Die erfindungsgemäßen Verbindungen liegen hierbei als aktive Bestandteile in üblichen Darreichungsformen vor, beispielsweise in Zusammensetzungen, die im wesentlichen aus einem inerten pharmazeutischen Träger und einer effektiven Dosis des Wirkstoffs bestehen, wie beispielsweise Tabletten, Dragees, Kapseln, Oblaten, Pulver, Lösungen, Suspensionen, Emulsionen, Sirupe, Suppositorien, transdermale Systeme etc.. Eine wirksame Dosis der erfindungsgemäßen Verbindungen liegt bei einer oralen Anwendung zwischen 0.1 und 5000, vorzugsweise zwischen 1 und 500, besonders bevorzugt zwischen 5-300 mg/Dosis, bei intravenöser, subcutaner oder intramuskulärer Anwendung zwischen 0,001 und 50, vorzugsweise zwischen 0,1 und 10 mg/Dosis. Für die Inhalation sind erfindungsgemäß Lösungen geeignet, die 0,01 bis 1,0, vorzugsweise 0,1 bis 0,5 % Wirkstoff enthalten. Für die inhalative Applikation ist die Verwendung von Pulvern, ethanolischen oder wässrigen Lösungen bevorzugt. Gleichfalls ist es möglich, die erfindungsgemäßen Verbindungen als Infusionslösung, vorzugsweise in einer physiologischen Kochsalzlösung oder Nährsalzlösung einzusetzen.

Die erfindungsgemäßen Verbindungen können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, Säfte, Emulsionen oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure hergestellt und in Injektionsflaschen oder Ampullen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Eine therapeutisch wirksame Tagesdosis beträgt zwischen 1 und 2000 mg, bevorzugt 10-500 mg pro Erwachsener

Die nachfolgenden Beispiele illustrieren die vorliegende Erfindung ohne sie jedoch in ihrem Umfang zu beschränken:

### Pharmazeutische Formulierungsbeispiele

| A) | Tabletten | pro Tablette | |
|---|---|---|---|
| | Wirkstoff | 100 | mg |
| | Milchzucker | 140 | mg |
| | Maisstärke | 240 | mg |
| | Polyvinylpyrrolidon | 15 | mg |
| | Magnesiumstearat | 5 | mg |
| | | 500 | mg |

Der feingemahlene Wirkstoff, Milchzucker und ein Teil der Maisstärke werden miteinander vermischt. Die Mischung wird gesiebt, worauf man sie mit einer Lösung von Polyvinylpyrrolidon in Wasser befeuchtet, knetet, feuchtgranuliert und trocknet. Das Granulat, der Rest der Maisstärke und das Magnesiumstearat werden gesiebt und miteinander vermischt. Das Gemisch wird zu Tabletten geeigneter Form und Größe verpresst.

| B) | Tabletten | pro Tablette | |
|---|---|---|---|
| | Wirkstoff | 80 | mg |
| | Maisstärke | 190 | mg |
| | Milchzucker | 55 | mg |
| | Mikrokristalline Cellulose | 35 | mg |
| | Polyvinylpyrrolidon | 15 | mg |
| | Natrium-carboxymethylstärke | 23 | mg |
| | Magnesiumstearat | 2 | mg |
| | | 400 | mg |

Der feingemahlene Wirkstoff, ein Teil der Maisstärke, Milchzucker, mikrokristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natrium-carboxymethylstärke und das Magnesiumstearat, vermischt und verpreßt das Gemisch zu Tabletten geeigneter Größe.

| C) | Dragées | pro Dragée | |
|---|---|---|---|
| | Wirkstoff | 5 | mg |
| | Maisstärke | 41,5 | mg |
| | Milchzucker | 30 | mg |
| | Polyvinylpyrrolidon | 3 | mg |
| | Magnesiumstearat | 0.5 | mg |
| | | 80 | mg |

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepresst. Die so hergestellten Dragéekerne werden auf bekannte Weise mit einer Schicht überzogen, die im wesentlichsten aus Zucker und Talkum besteht. Die fertigen Dragees werden mit Wachs poliert.

| D) | Kapseln | pro Kapsel | |
|---|---|---|---|
| | Wirkstoff | 50 | mg |
| | Maisstärke | 268,5 | mg |
| | Magnesiumstearat | 1,5 | mg |
| | | 320 | mg |

Substanz und Maisstärke werden gemischt und mit Wasser befeuchtet. Die feuchte Masse wird gesiebt und getrocknet. Das trockene Granulat wird gesiebt und mit Magensiumstearat gemischt. Die Endmischung wird in Hartgelatinekapseln Größe 1 abgefüllt.

| E) | Ampullenlösung | | |
|---|---|---|---|
| | Wirkstoff | 50 | mg |
| | Natriumchlorid | 50 | mg |
| | Aqua pro inj. | 5 | ml |

Der Wirkstoff wird bei Eigen-pH oder gegebenenfalls bei pH 5,5 bis 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

| F) | Suppositorien | | |
|---|---|---|---|
| | Wirkstoff | 50 | mg |
| | Adeps solidus | 1650 | mg |
| | | 1700 | mg |

Das Hartfett wird geschmolzen. Bei 40°C wird die gemahlene Wirksubstanz homogen dispergiert. Es wird auf 38°C abgekühlt und in schwach vorgekühlte Suppositorienformen ausgegossen

| G) | orale Suspension | | |
|---|---|---|---|
| | Wirkstoff | 50 | mg |
| | Hydroxyethylcellulose | 50 | mg |
| | Sorbinsäure | 5 | mg |
| | Sorbit (70%ig) | 600 | mg |
| | Glycerin | 200 | mg |
| | Aroma | 15 | mg |
| | Wasser ad | 5 | ml |

Destilliertes Wasser wird auf 70°C erhitzt. Hierin wird unter Rühren Hydroxyethyl-cellulose gelöst. Nach Zugabe von Sorbitlösung und Glycerin wird auf Raumtemperatur abgekühlt. Bei Raumtemperatur werden Sorbinsäure, Aroma und Substanz zugegeben. Zur Entlüftung der Suspension wird unter Rühren evakuiert,
und 50 mg Wirkstoff.

## Patentansprüche

1. Verbindungen der Formel **1**; 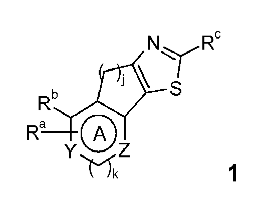 mit der Maßgabe, dass die mit A gekennzeichnete, kreisförmige Linie ein aromatisches System bedeutet; worin
**Y** Kohlenstoff, Stickstoffatom, Schwefel;
**Z** Kohlenstoff, Stickstoffatom, Schwefel;
**j** 1, 2 oder 3;
**k** 0 oder 1;
**R^{a}** H, COR⁸, NR⁹R¹⁰, NO₂, OR⁸, SR¹¹, SOR¹¹, SO₂R¹¹, NHCO-C₁₋₆-Alkyl-NH₂, oder ein Rest ausgewählt aus der Gruppe bestehend aus C₁₋₆-Alkyl, C₂₋₆-Alkenyl, C₃₋₈-Cycloalkyl, C₃₋₈-Cycloalkenyl, C₁₋₆-Haloalkyl, Aryl, C₇₋₁₁-Aralkyl, Spiro, Het, Hetaryl und CH₂-O-Aryl, der gegebenenfalls substituiert sein kann;
R⁸ C₁₋₆-Alkyl, C₃₋₆-Cycloalkyl, NH₂, Hetaryl oder Aryl, gegebenenfalls substituiert mit einem oder mehreren Halogen oder C₁₋₄-Alkyl;
R⁹ H, COOR¹², CONR¹² oder C₁₋₆-Alkyl, gegebenenfalls substituiert mit einem oder mehreren COOH, N(C₁₋₆-Alkyl)₂ oder Het, gegebenenfalls substituiert mit einem oder mehreren C₁₋₆-Alkyl; oder R⁹ bedeutet Het, gegebenenfalls substituiert mit einem oder mehreren C₁₋₄-Alkyl;
R¹⁰ H, C₁₋₆-Alkyl, CO-C₁₋₆-Alkyl oder C₂₋₆-Alkinyl;
R¹¹ C₁₋₆-Alkyl, gegebenenfalls substituiert mit einem oder mehreren N(C₁₋₄-Alkyl)₂;
**R^{b}** R⁴, OR⁴, -CH₂OR⁴, COR⁴, COOR⁴, CONR⁴R⁵, NR⁴R⁵, NR⁵COR⁴, NR⁵COOR⁴, NR⁵CONR⁴R⁵, NR⁵SOR⁴ oder NR⁵SO₂R⁴;
R⁴, R⁵ H, C₁₋₆-Alkyl, C₁₋₆-Haloalkyl, C₁₋₆-Alkylen-OH, C₂₋₆-Alkenyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl;
oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
**R^{c}** NHR⁶ oder ein Rest ausgewählt aus der Gruppe bestehend aus 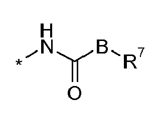 worin
B Bindung, C₁₋₆-Alkyl, C₂₋₆-Alkenyl oder C₂₋₆-Alkinyl;
R⁶ H oder ein Rest ausgewählt aus der Gruppe C₁₋₄-Alkyl, C₂₋₄-Alkenyl, C₂₋₄-Alkinyl, C₃₋₆-Cycloalkyl, C₃₋₆-Cycloalkenyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
R⁷ H, C₁₋₄-Alkyl, C₂₋₄-Alkenyl, C₂₋₄-Alkinyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₆-Alkyl, (CH₂)₂₋₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2}, Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}** = H und **R^{c}=** NHCONH-Et bedeutet.

2. Verbindungen der Formel **1** nach Anspruch 1; worin
**R^{a}** ein Rest ausgewählt aus der Gruppe bestehend aus Aryl, C₇₋₁₁-Aralkyl und Hetaryl, der gegebenenfalls substituiert durch einen oder mehrere Reste ausgewählt aus der Gruppe bestehend aus R¹, R² und R³ sein kann;
R¹ und R² unabhängig voneinander C₁₋₆-Alkyl, C₂₋₆-Alkenyl C₂₋₆-Alkinyl, C₃₋₆-Cycloalkyl, C₃₋₆-Cycloalkenyl, C₁₋₆-Haloalkyl, C₁₋₆-Alkylen-COOH, C₁₋₆-Alkoxy, Halogen, OH, CN, COR^{1.1}, O-C₁₋₄-Haloalkyl, NO₂ oder SR^{1.1}, SOR^{1.1}, SO₂^{R1.1}, Het oder Hetaryl,
R^{1.1} OH, C₁₋₆-Alkyl, C₂₋₆-Alkenyl C₂₋₆-Alkinyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl;
oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
R³ ein Rest ausgewählt aus der Gruppe bestehend aus 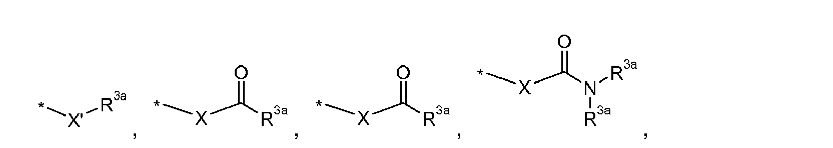 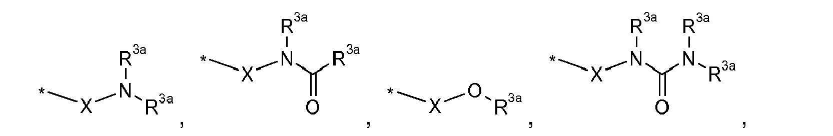 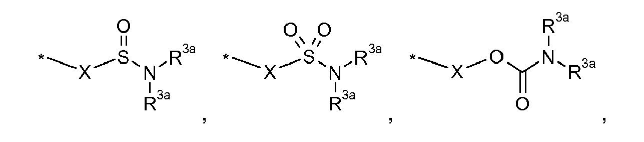 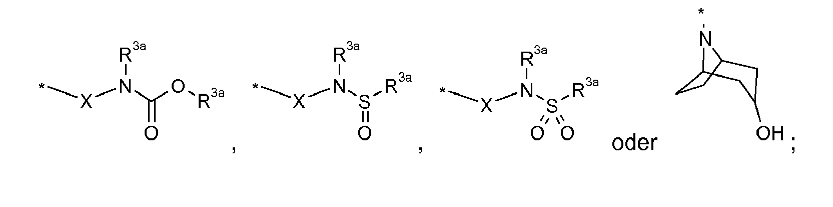 worin
X Bindung oder C₁₋₄-Alkylen;
X' C₁₋₄-Alkylen, C₂₋₄-Alkenylen oder C₁₋₄-Alkinylen
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} C₁₋₆-Alkyl, C₂₋₆-Alkenyl, OH, C₁₋₄-Alkylen-OH, C₁₋₄-Alkylen-NR^{3.1.1.1}R^{3.1.1.2,}, COR^{3.1.1.1}, COOR^{3.1.1.1}, CONR^{3.1.1.1}R^{3.1.1.2}, NR^{3.1.1.1}R^{3.1.1.2}, Het, Hetaryl, NHCOR^{3.1.1.1}
R^{3.1.1.1} ein Rest ausgewählt aus der Gruppe bestehend aus H, C₁₋₄-Alkyl, Aryl und C₇₋₁₁-Aralkyl; gegebenenfalls substituiert mit einem Rest ausgewählt aus der Gruppe Halogen, OH und CN;
R^{3.1.1.2} H, C₁₋₄-Alkyl;
R^{3.2} ein Rest ausgewählt aus der Gruppe bestehend aus C₃₋₆-Cycloalkyl, Het, Hetaryl und Spiro der gegebenenfalls substituiert ist mit einem oder mehreren R^{3.2.1}
R^{3.2.1} C₁₋₆-Alkyl, C₃₋₆-Cycloalkyl, OH, NR^{3.2.1.1}R^{3.2.1.2}, NHCOR^{3.2.1.3} oder Het, gegebenenfalls substituiert mit einem oder mehreren Resten ausgewählt aus der Gruppe bestehend aus C₁₋₄-Alkyl, SO₂R^{3.2.1.1}, CH₂-C₃₋₆-Cycloalkyl und Aryl;
R^{3.2.1.1} H, C₁₋₄-Alkyl oder C₇₋₁₁- Aralkyl;
R^{3.2.1.2} H, C₁₋₄-Alkyl oder C₇₋₁₁- Aralkyl;
R^{3.2.1.2} Aryl, C₇₋₁₁Aralkyl; oder
C₁₋₆-alkyl, das gegebenenfalls substituiert ist mit einem oder zwei R^{3.2.2};
R^{3.2.2} C₁₋₆-Alkyl, C₂₋₆-Alkenyl, C₂₋₆-Alkinyl, COOR^{3.2.2.1}, CONR^{3.2.2.1}R^{3.2.2.2}, NR^{3.2.2.1}R^{3.2.2.2}, NHCOR^{3.2.2.1}, C₁₋₆-Haloalkyl, CN, OR^{3.2.2.1}, SO₂R^{3.2.2.1}, C₃₋₆-Cycloalkyl, CO-Het, C₂₋₄-Alkinyl-Hetaryl, Guanidin oder ein Rest ausgewählt aus der Gruppe bestehend aus Het, Hetaryl und Aryl, der gegebenenfalls substituiert ist mit einem oder mehreren Resten ausgewählt aus der Gruppe Halogen, C₁₋₆-Alkyl, CONR^{3.2.2.1}R^{3.2.2.2}, OH, Imidazolidinon;
R^{3.2.2.1} H oder C₁₋₆-Alkyl, Aryl, C₇₋₁₁-Aralkyl
R^{3.2.2.2.} H oder C₁₋₆-Alkyl; oder
Aryl, das gegebenenfalls substituiert ist mit einem oder zwei R^{3.2.3}
R^{3.2.3} ein Rest ausgewählt aus der Gruppe bestehend aus NH-C₁₋₆-alkyl-N(C₁₋₆-alkyl)₂ oder Het, wobei Het gegebenenfalls substituiert mit einem Rest C₁₋₆-Alkyl sein kann;
R^{3.3} H oder ein Rest ausgewählt aus der Gruppe bestehend aus C₁₋₆-Alkyl, C₂₋₆-Alkenyl, C₂₋₆-Alkinyl, C₁₋₆-Haloalkyl und Aryl, der gegebenenfalls substituiert sein kann mit einem oder mehreren Resten R^{3.3.1};
R^{3.3.1} C₃₋₆-Cycloalkyl, C₃₋₆-Cycloalkenyl, OR^{3.3.1.1}, NR^{3.3.1.1}R^{3.3.1.2}, CONR^{3.3.1}R^{3.3.1.2}, COOR^{3.3.1.1}, NR^{3.3.1.1}COR^{3.3.1.2},SOR^{3.3.1.1} SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het, gegebenenfalls substituiert mit einem oder mehreren Resten ausgewählt aus der Gruppe bestehend aus C₁₋₄-Alkyl, SO₂R^{3.2.1.1}, SO₂C₁₋₄-Alkyl, SO₂C₇₋₁₁- Aralkyl, CH₂-C₃₋₆-Cyclloalkyl und Aryl;
R^{3.3.1.1}, R^{3.3.1.2} und R^{3.3.1.3} ein Rest ausgewählt aus der Gruppe bestehend aus C₁₋₆-Alkyl, C₂₋₆-Alkenyl, C₂₋₆-Alkinyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, COC₁₋₄-Alkyl-Hetaryl, COC₂₋₄-Alkenyl-Hetaryl, COC₂₋₄-Alkinyl-Hetaryl; oder
zwei der Gruppen R^{3.3.1.1}, ^{R3.3.1.2} und R^{3.3.1.3} bilden gemeinsam einen Ring, bestehend aus Kohlenstoffatomen und gegebenenfalls einem Heteroatom ausgewählt aus der Gruppe bestehend aus Sauerstoff, Stickstoff und Schwefel;
**R^{a}** H, C₁₋₆-Alkyl, C₂₋₆-Alkenyl, C₃₋₈-Cycloalkyl, C₃₋₈-Cycloalkenyl, C₁₋₆-Haloalkyl, COR⁸, NR⁹R¹⁰, NO₂, OR⁸, SR¹¹, SOR¹¹, SO₂R¹¹, NHCO-C₁₋₆-Alkyl-NH₂, Spiro oder ein Rest ausgewählt aus der Gruppe bestehend aus C₇₋₁₁- Aralkyl, CH₂-O-Aryl und Het der gegebenenfalls substituiert sein kann mit einem oder mehreren Halogen, C₁₋₆-Alkyl, CO-C₁₋₄-Haloalkyl, C₁₋₄-Alkyl-NH₂ oder CH₂NHCOOR¹²;
R⁸ C₁₋₆-Alkyl, C₃₋₆-Cycloalkyl, NH₂, Hetaryl oder Aryl, gegebenenfalls substituiert mit einem oder mehreren Halogen oder C₁₋₄-Alkyl;
R⁹ H, COOR¹², CONR¹² oder C₁₋₆-Alkyl, gegebenenfalls substituiert mit einem oder mehreren COOH, N(C₁₋₆-Alkyl)₂ oder Het, gegebenenfalls substituiert mit einem oder mehreren C₁₋₆-Alkyl; oder R⁹ bedeutet Het, gegebenenfalls substituiert mit einem oder mehreren C₁₋₄-Alkyl;
R¹⁰ H, C₁₋₆-Alkyl, CO-C₁₋₆-Alkyl oder C₂₋₆-Alkinyl;
R¹¹ C₁₋₆-Alkyl, gegebenenfalls substituiert mit einem oder mehreren N(C₁₋₄-Alkyl)₂;
R¹² H, C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}** = H und **R^{c}** = NHCONH-Et bedeutet.

3. Verbindungen der Formel **1** nach einem der Ansprüche 1 oder 2; worin
**R^{a}** ein Rest ausgewählt aus der Gruppe bestehend aus Aryl, C₇₋₁₁-Aralkyl und Hetaryl, der gegebenenfalls substituiert durch einen oder mehrere Reste ausgewählt aus der Gruppe bestehend aus R¹, R² und R³ sein kann;
R¹ und R² unabhängig voneinander C₁₋₆-Alkyl, C₂₋₆-Alkenyl C₂₋₆-Alkinyl, C₃₋₆-Cycloalkyl, C₃₋₆-Cycloalkenyl, C₁₋6-Haloalkyl, C₁₋₆-Alkylen-COOH, C₁₋₆-Alkoxy, Halogen, OH, CN, COR^{1.1}, O-C₁₋₄-Haloalkyl, NO₂ oder SR^{1.1}, SOR^{1.1}, SO₂R^{1.1}, Het oder Hetaryl,
R^{1.1} OH, C₁₋₆-Alkyl, C₂₋₆-Alkenyl C₂₋₆-Alkinyl, NR^{1.1.1}R^{1.1.2}
R^{1.1.1} H, C₁₋₆-Alkyl, gegebenenfalls substituiert mit einem Rest ausgewählt aus der Gruppe bestehend aus NH₂, NHMe, NMe₂;
R^{1.1.2} H, C₁₋₆-Alkyl;
oder R^{1.1.1} und R^{1.1.2} bilden zusammen einen fünf- oder sechsgliedrigen heterocyclischen Ring, der gegebenenfalls substituiert sein kann mit einem Rest ausgewählt aus der Gruppe bestehend aus Methyl, Ethyl, Propyl;
R³ ein Rest ausgewählt aus der Gruppe bestehend aus 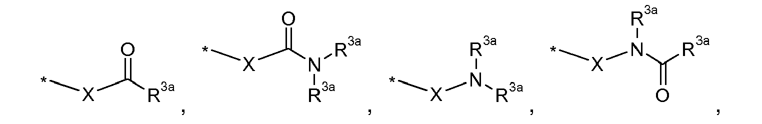 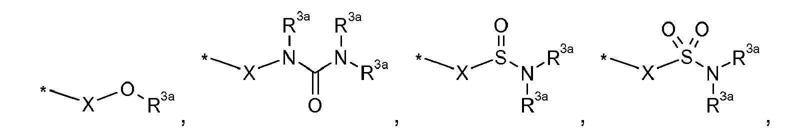 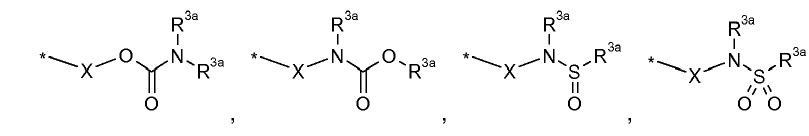 oder 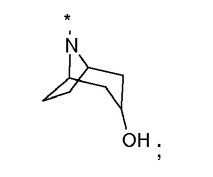 worin
X Bindung oder C₁₋₄-alkylen;
R^{3a} ein Rest, gleich oder verschieden, ausgewählt aus der Gruppe bestehend aus R^{3.1}, R^{3.2} und R^{3.3};
R^{3.1} Spiro oder Het, wobei Het gegebenenfalls substituiert mit einem oder mehreren R^{3.1.1} sein kann;
R^{3.1.1} C₁₋₆-Alkyl, C₂₋₆-Alkenyl, OH, C₁₋₄-Alkylen-OH, C₁₋₄-Alkylen-NR^{3.1.1}R^{3.1.1.2,}, COR^{3.1.1.1}, COOR^{3.1.1.1}, CONR^{3.1.1.1}R^{3.1.1.2}, NR^{3.1.1.1}R^{3.1.1.2}, Het, Hetaryl, NHCOR^{3.1.1.1}
R^{3.1.1.1} ein Rest ausgewählt aus der Gruppe bestehend aus H, C₁₋₄-Alkyl, Aryl und C₇₋₁₁-Aralkyl; gegebenenfalls substituiert mit einem Rest ausgewählt aus der Gruppe Halogen, OH und CN;
R^{3.1.1.2} H, C₁₋₄-Alkyl;
R^{3.2} ein Rest ausgewählt aus der Gruppe bestehend aus C₃₋₆-Cycloalkyl, Het, Hetaryl und Spiro der gegebenenfalls substituiert ist mit einem oder mehreren R^{3.2.1}
R^{3.2.1} C₁₋₆-Alkyl, C₃₋₆-Cycloalkyl, OH, -NR^{3.2.1.1}R^{3.2.1.2}, NHCOR^{3.2.1.3} oder Het, gegebenenfalls substituiert mit einem oder mehreren Resten ausgewählt aus der Gruppe bestehend aus C₁₋₄-Alkyl, SO₂R^{3.2.1.1}, CH₂-C₃₋₆-Cycloalkyl und Aryl;
R^{3.2.1.1} H, C₁₋₄-Alkyl oder C₇₋₁₁- Aralkyl;
R^{3.2.1.2} H, C₁₋₄-Alkyl oder C₇₋₁₁- Aralkyl;
R^{3.2.1.2} Aryl, C₇₋₁₁-Aralkyl; oder
-C₁₋₆-alkyl, das gegebenenfalls substituiert ist mit einem oder zwei R^{3.2.2};
R^{3.2.2} C₁₋₆-Alkyl, C₂₋₆-Alkenyl, C₂₋₆-Alkinyl, COOR^{3.2.2.1}, CONR^{3.2.2.1}R^{3.2.2.2}, NR^{3.2.2.1}R^{3.2.2.2}, NHCOR^{3.2.2.1}, C₁₋₆-Haloalkyl, CN, OR^{3.2.2.1}, SO₂R^{3.2.2.1}, C₃₋₆-Cycloalkyl, CO-Het, C₂₋₄-Alkinyl-Hetaryl, Guanidin oder ein Rest ausgewählt aus der Gruppe bestehend aus Het, Hetaryl und Aryl, der gegebenenfalls substituiert ist mit einem oder mehreren Resten ausgewählt aus der Gruppe Halogen, C₁₋₆-Alkyl, CONR^{3.2.2.1}R^{3.2.2.2}, OH, Imidazolidinon;
R^{3.2.2.1} H oder C₁₋₆-Alkyl, Aryl, C₇₋₁₁-Aralkyl
R^{3.2.2.2.} H oder C₁₋₆-Alkyl; oder
Aryl, das gegebenenfalls substituiert ist mit einem oder zwei R^{3.2.3}
R^{3.2.3} ein Rest ausgewählt aus der Gruppe bestehend aus NH-C₁₋₆-alkyl-N(C₁₋₆-alkyl)₂ oder Het, wobei Het gegebenenfalls substituiert mit einem Rest C₁₋₆-Alkyl sein kann;
R^{3.3} H oder ein Rest ausgewählt aus der Gruppe bestehend aus C₁₋₆-Alkyl, C₂₋₄-Alkenyl, C₂₋₄-Alkinyl, C₁₋₄-Haloalkyl und Aryl, der gegebenenfalls substituiert sein kann mit einem oder mehreren Resten R^{3.3.1};
R^{3.3.1} C₅₋₆-Cycloalkyl, C₅₋₆-Cycloalkenyl, OR^{3.3.1.1}, NR^{3.3.1.1}R^{3.3.1.2}, CONR ^{3.3.1.1}R^{3.3.1.2}, COOR^{3.3.1.1}, NR^{3.3.1.1}COR^{3.3.1.2}, SOR^{3.3.1.1}, SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het, gegebenenfalls substituiert mit einem oder mehreren Resten ausgewählt aus der Gruppe bestehend aus C₁₋₄-Alkyl, SO₂R^{3.2.1.1}, SO₂C₁₋₄-Alkyl, SO₂C₇₋₁₁- Aralkyl, CH₂-C₃₋₆-Cycloalkyl und Aryl;
R^{3.3.1.1}, R^{3.3.1.2} und R^{3.3.1.3} ein Rest ausgewählt aus der Gruppe bestehend aus C₁₋₄-Alkyl, C₂₋₄-Alkenyl, C₂₋₄-Alkinyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, COC₁₋₄-Alkyl-Hetaryl, COC₂₋₄-Alkenyl-Hetaryl, COC₂₋₄-Alkinyl-Hetaryl; oder
zwei der Gruppen R^{3.3.1.1}, R^{3.3.1.2} und R^{3.3.1.3} bilden gemeinsam einen fünf-, sechs oder siebengliedrigen Ring, bestehend aus Kohlenstoffatomen und gegebenenfalls einem Heteroatom ausgewählt aus der Gruppe bestehend aus Sauerstoff, Stickstoff und Schwefel;
**R^{a}** H, C₁₋₆-Alkyl, C₂₋₆-Alkenyl, C₃₋₈-Cycloalkyl, C₃₋₈-Cycloalkenyl, C₁₋₆-Haloalkyl, COR⁸, NR⁹R¹⁰, NO₂, OR⁸, SR¹¹, SOR¹¹, SO₂R¹¹, NHCO-C₁₋₆-Alkyl-NH₂, Spiro oder ein Rest ausgewählt aus der Gruppe bestehend aus C₇₋₁₁- Aralkyl, CH₂-O-Aryl und Het der gegebenenfalls substituiert sein kann mit einem oder mehreren Halogen, C₁₋₆-Alkyl, CO-C₁₋₄-Haloalkyl, C₁₋₄-Alkyl-NH₂ oder CH₂NHCOOR¹²;
R⁸ C₁₋₆-Alkyl, C₃₋₆-Cycloalkyl, NH₂, Hetaryl oder Aryl, gegebenenfalls substituiert mit einem oder mehreren Halogen oder C₁₋₄-Alkyl;
R⁹ H, COOR¹² oder C₁₋₄-Alkyl, gegebenenfalls substituiert mit einem oder mehreren COOH, N(C₁₋₄-Alkyl)₂ oder Het, gegebenenfalls substituiert mit einem oder mehreren C₁₋₄-Alkyl; oder R⁹ bedeutet Het, gegebenenfalls substituiert mit einem oder mehreren C₁₋₄-Alkyl;
R¹⁰ H, C₁₋₆-Alkyl, CO-C₁₋₄-Alkyl oder C₂₋₆-Alkinyl;
R¹¹ C₁₋₆-Alkyl, gegebenenfalls substituiert mit einem oder mehreren N(C₁₋₄-Alkyl)₂;
R¹² C₁₋₆-Alkyl;
**R^{b}** R⁴, OR⁴, -CH₂OR⁴, COR⁴, COOR⁴, CONR⁴R⁵, NR⁴R⁵, NR⁵COR⁴, NR⁵COOR⁴, NR⁵CONR⁴R⁵, NR⁵SOR⁴ oder NR⁵SO₂R⁴;
R⁴ H, C₁₋₆-Alkyl, C₁₋₆-Haloalkyl, C₁₋₆-Alkylen-OH, C₂₋₆-Alkenyl, C₇₋₁₁-Aralkyl, C₂₋₄-Alkenyl-Aryl, C₂₋₄-Alkinyl-Aryl, C₁₋₄-Alkyl-Hetaryl, C₂₋₄-Alkenyl-Hetaryl, C₂₋₄-Alkinyl-Hetaryl, C₂₋₆-Alkinyl, gegebenenfalls substituiert mit Si(C₁₋₄-Alkyl)₃, oder R⁴ bedeutet ein Rest ausgewählt aus der Gruppe bestehend aus Aryl, Het, Hetaryl und gegebenenfalls substituiert mit C₁₋₄-Alkyl; R⁵ H oder C₁₋₆-Alkyl;
oder R⁴ und R⁵ bilden gemeinsam einen fünf-, sechs- oder siebengliedrigen Ring bestehend aus Kohlenstoffatomen und gegebenenfalls einem Heteroatom ausgewählt aus der Gruppe bestehend aus Sauerstoff, Stickstoff und Schwefel;
**R^{c}** NHR⁶ oder ein Rest ausgewählt aus der Gruppe bestehend aus 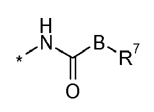 worin
B Bindung, C₁₋₄-Alkyl oder C₂₋₄-Alkinyl;
R⁶ H oder ein Rest ausgewählt aus der Gruppe C₁₋₄-Alkyl, C₂₋₄-Alkenyl, C₂₋₄-Alkinyl, C₃₋₆-Cycloalkyl, C₃₋₆-Cycloalkenyl, Het, Aryl, Hetaryl gegebenenfalls substituiert mit einem oder mehreren Resten R^{6.1};
R^{6.1} Halogen, CF₃, OH, CN, OMe, SO₂(C₁₋₄-Alkyl);
R⁷ H, C₁₋₄-Alkyl, C₂₋₄-Alkenyl, C₂₋₄-Alkinyl, C₃₋₆-Cycloalkyl, NR^{7.1}R^{7.2}, OR^{7.2}, SR^{7.2}, Hetaryl, Het, gegebenenfalls substituiert mit C₁₋₄-Alkyl oder CONH₂;
R^{7.1} H, C₁₋₄-Alkyl, (CH₂)₂₋₄R^{7.1.1} oder COOButyl;
R^{7.2} H, C₁₋₆-Alkyl, gegebenenfalls substituiert mit einem oder mehreren OH;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R**^{a} nicht H oder Me sein kann, wenn **Y** = Stickstoff; **Z** = Stickstoff; **j** = 2; **k** = 0; **R**^{b}= H und **R**^{c}= NHCONH-Et bedeutet.

4. Verbindungen der Formel **1** nach einem der Ansprüche 1 bis 3; worin
**R**^{a} ein Rest ausgewählt aus der Gruppe bestehend aus Aryl und C₇₋₁₁-Aralkyl, der gegebenenfalls substituiert durch einen oder mehrere Reste ausgewählt aus der Gruppe bestehend aus R¹, R² und R³ sein kann; oder Hetaryl gegebenenfalls substituiert durch ein oder mehrere C₁-₄-Alkyl;
R¹ C₁₋₄-Alkyl, C₁₋₄-Haloalkyl, C₁₋₄-Alkylen-COOH, C₁₋₄-Alkoxy, Halogen, OH, CN, COR^{1.1}, O-C₁₋₄-Haloalkyl, NO₂ oder SO₂R^{1.1};
R^{1.1} OH, Methyl, NH₂, NHMe, NMe₂, 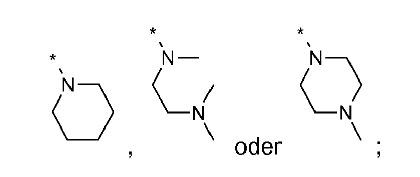
R² C₁₋₄-Alkyl, C₁₋₄-Alkoxy oder Halogen;
R³ ein Rest ausgewählt aus der Gruppe bestehend aus 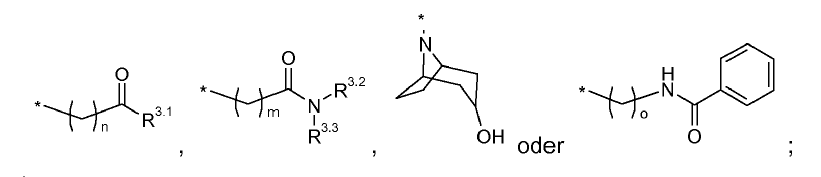 worin
n 0 oder 1;
m 0 oder 1;
o 2;
R^{3.1} Spiro oder Het, wobei Het gegebenenfalls substituiert mit einem oder mehreren R^{3.1.1} sein kann;
R^{3.1.1} C₁₋₄-Alkyl, C₂₋₄-Alkenyl, OH, C₁₋₄-Alkylen-OH, CH₂NEt₂, COMe, COOH, CONH₂, NH₂, Het, Hetaryl, 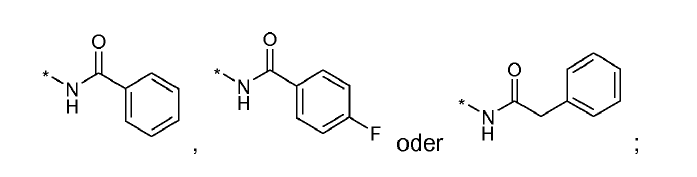
R^{3.2} ein Rest ausgewählt aus der Gruppe bestehend aus C₃₋₆-Cycloalkyl, Het, Hetaryl und Spiro der gegebenenfalls substituiert ist mit einem oder zwei R^{3.2.1}
R^{3.2.1} C₁₋₄-Alkyl, Cyclopentyl, OH, -NR^{3.2.1.1}R^{3.2.1.2} oder 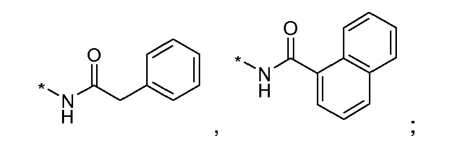 oder Het, gegebenenfalls substituiert ist mit einem oder mehreren Resten ausgewählt aus der Gruppe bestehend aus Methyl, SO₂R^{3.2.1.1}, 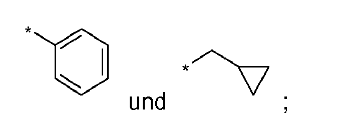
R^{3.2.1.1} H, Methyl oder Benzyl;
R^{3.2.1.2} H, Methyl oder Benzyl; oder
-C₁₋₆-alkyl, das gegebenenfalls substituiert ist mit einem oder zwei R^{3.2.2};
R^{3.2.2} C₂₋₄-Alkenyl, C₂₋₄-Alkinyl, COOR^{3.2.2.2.1}, CONR^{3.2.2.1}R^{3.2.2.2}, NR^{3.2.2.1}R^{3.2.2.2}, NHCOR^{3.2.2.1}, C₁₋₄-Haloalkyl, CN, OH, SO₂R^{3.2.2.1}, C₃₋₆-Cycloalkyl oder ein Rest ausgewählt aus der Gruppe bestehend aus 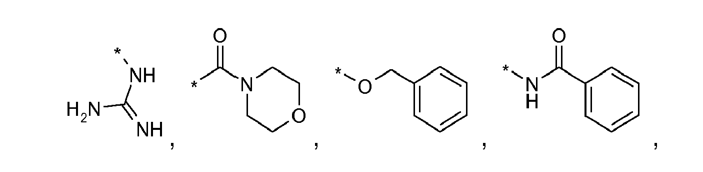 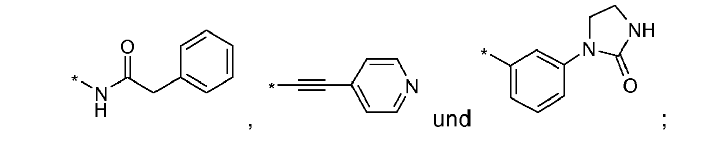 oder ein Rest ausgewählt aus der Gruppe bestehend aus Het, Hetaryl und Aryl, der gegebenenfalls substituiert ist mit einem oder mehreren Resten ausgewählt aus der Gruppe Cl, Methyl, CONR^{3.2.2.1}R^{3.2.2.2}, OH;
R^{3.2.2.1} H oder Methyl;
R^{3.2.2.2.} H oder Methyl; oder
Aryl, das gegebenenfalls substituiert ist mit einem oder zwei R^{3.2.3}
R^{3.2.3} ein Rest ausgewählt aus der Gruppe bestehend aus 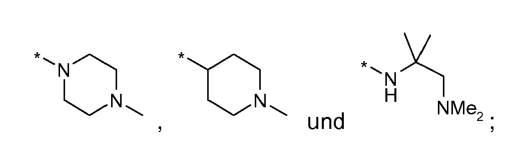
R^{3.3} H oder ein Rest ausgewählt aus der Gruppe bestehend aus C₁₋₆-Alkyl und Aryl, der gegebenenfalls substituiert sein kann mit einem oder mehreren Resten R^{3.3.1};
R^{3.3.1} C₅₋₆-Cycloalkyl, C₅₋₆-Cycloalkenyl, OR^{3.3.1.1}, NR^{3.3.1.1}R^{3.3.1.2}, CONR^{3.3.1.1}R^{3.3.1.2},COOR^{3.3.1.1}, NR^{3.3.1.1}COR^{3.3.1.2},SOR^{3.3.1.1}, SO₂R^{3.3.1.1}, C(NR^{3.3.1.1}R^{3.3.1.2})NR^{3.3.1.3}, NR^{3.3.1.1}CONR^{3.3.1.2}R^{3.3.1.3}, OH, CN, Halogen oder Het, gegebenenfalls substituiert mit einem oder mehreren Resten ausgewählt aus der Gruppe bestehend aus C₁₋₄-Alkyl, SO₂R^{3.2.1.1}, SO₂C₁₋₄-Alkyl, SO₂C₇₋₁₁- Aralkyl, 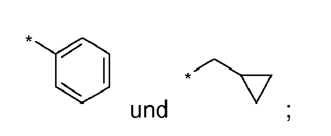
R^{3.3.1.1} R^{3.3.1.2} und R^{3.3.1.3} ein Rest ausgewählt aus der Gruppe bestehend aus C₁₋₄-Alkyl, C₇₋₁₁-Aralkyl, C₁₋₄-Alkyl-Hetaryl, COC₁₋₄-Alkyl-Hetaryl;
**R^{a}** H, C₁₋₆-Alklyl, C₂₋₆-Alkenyl, C₃₋₆-Cycloalkyl, CF₃, COR⁸, NR⁹R¹⁰, NO₂, S(O)ₙR¹¹, oder ein Rest ausgewählt aus der Gruppe bestehend aus 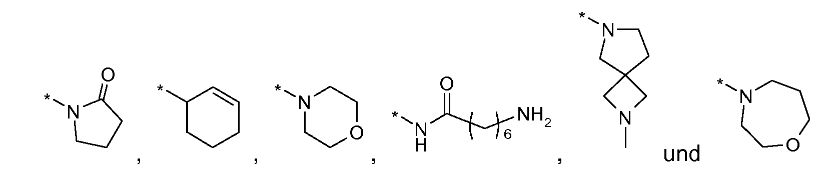 oder ein Rest ausgewählt aus der Gruppe bestehend aus 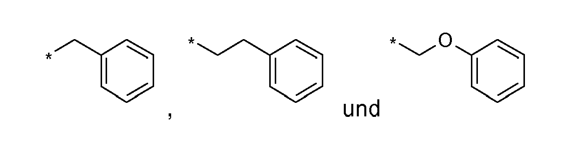 der gegebenenfalls substituiert sein kann mit einem oder mehreren Cl;
oder ein Rest ausgewählt aus der Gruppe bestehend aus 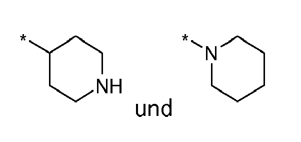 der gegebenenfalls substituiert sein kann mit einem oder mehreren CH₃, COCF₃, CH₂NH₂ oder CH₂NHCOOR¹²;
R⁸ C₁₋₄-Alkyl, C₃₋₆-Cycloalkyl, NH₂, Furanyl oder Phenyl, gegebenenfalls substituiert mit einem oder mehreren Chlor;
R⁹ H, COOR¹² oder Piperidino, gegebenenfalls substituiert mit einem oder mehreren CH₃, oder ein Rest ausgewählt aus der Gruppe bestehend aus C₁₋₄-Alkyl, der gegebenenfalls substituiert sein kann mit einem oder mehreren COOH, NMe₂ oder 4-Methylpiperazin;
R¹⁰ H, C₁₋₄-Alkyl, C₂₋₄-Alkinyl oder COCH₃;
R¹¹ C₁₋₄-Alkyl, gegebenenfalls substituiert mit einem oder mehreren NMe₂,
R¹² C1-₄-Alkyl;
**R^{b}** R⁴, CH₂OR⁴, COR⁴, COOR⁴, CONR⁴R⁵, NH₂, NHCOOR⁴, NHCONR⁴R⁵ oder OH;
R⁴ H, C₁₋₄-Alkyl, C₁₋₄-Alkylen-OH, C₂₋₄-Alkinyl, C₁₋₆-Haloalkyl, Aryl, Het, Hetaryl, 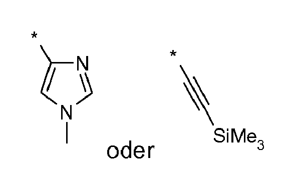
R⁵ H oder C₁₋₄-Alkyl;
**R^{c}** NHR⁶ oder ein Rest ausgewählt aus der Gruppe bestehend aus 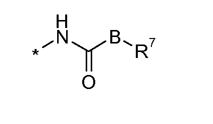 worin
B Bindung, C₁₋₄-Alkyl oder C₂₋₄-Alkinyl;
R⁶ H, C₁₋₄-Alkyl, C₃₋₆-Cycloalkyl, C₇-₁₁₋ Aralkyl, Aryl, gegebenenfalls substituiert mit SO₂CH₃;
R⁷ H, NR^{7.1}R^{7.2}, OR^{7.2}, SR^{7.2}, Hetaryl, Het, gegebenenfalls substituiert mit C₁₋₄-Alkyl oder CONH₂, oder ein Rest ausgewählt aus der Gruppe bestehend aus 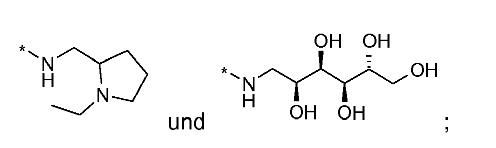
R^{7.1} H, C₁₋₄-Alkyl, (CH₂)₂R^{7.1.1} oder COOButyl;
R^{7.2} H, C₁₋₄-Alkyl;
R^{7.1.1} NR^{7.1.1.1}R^{7.1.1.2} Het oder 1-Imidazolyl, 2-(N-Ethylpyrollidin);
R^{7.1.1.1} oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

5. Verbindungen der Formel **1** nach einem der Ansprüche 1 bis 4; worin
**R^{a}** Phenyl oder Benzyl, jeweils gegebenenfalls substituiert durch einen oder mehrere Reste ausgewählt aus der Gruppe bestehend aus R¹, R² und R³; oder 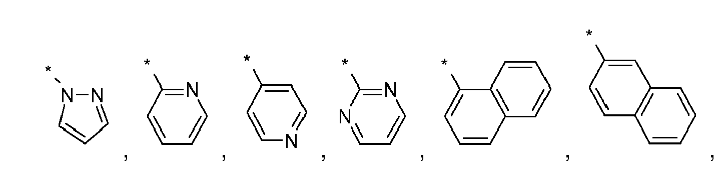 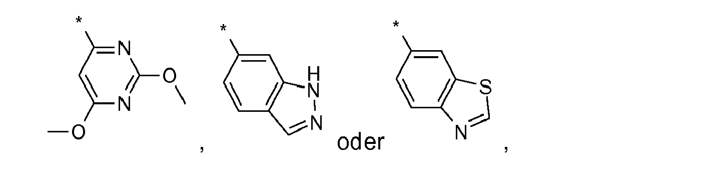
R¹ Methyl, Ethyl, Propyl, Butyl, CF₃, CH₂COOH, Methoxy, F, Cl, Br, OH, CN, COR^{1.1}, OCF₃, NO₂ oder SO₂R^{1.1};
R^{1.1} OH, Methyl, NH₂, NHMe, NMe₂, 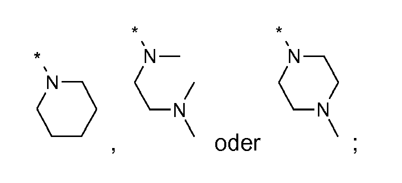
R² Methyl, Methoxy, F, Cl oder Br;
R³ ein Rest ausgewählt aus der Gruppe bestehend aus 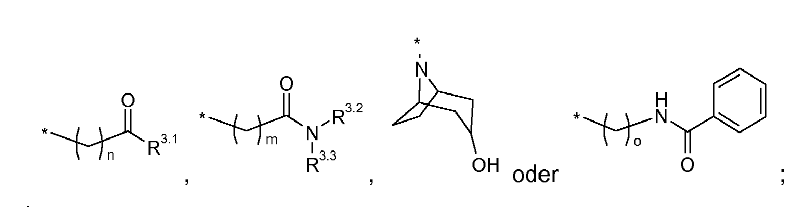 worin
n 0 oder 1;
m 0 oder 1;
o 2;
R^{3.1} ein Rest ausgewählt aus der Gruppe bestehend aus 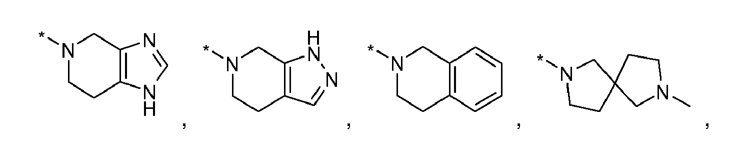 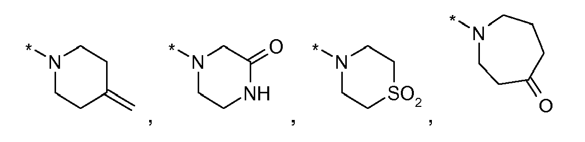 und 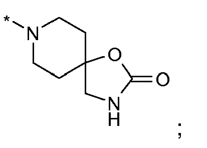 oder ein Rest ausgewählt aus der Gruppe bestehend aus 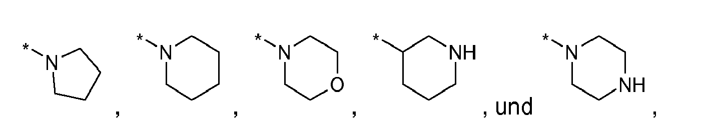 der gegebenenfalls substituiert sein kann mit einem oder mehreren R^{3.1.1};
R^{3.1.1} Methyl, Ethyl, OH, CH₂OH, CH₂CH₂OH, CH₂NEt₂, COMe, COOH, CONH₂, NH₂, 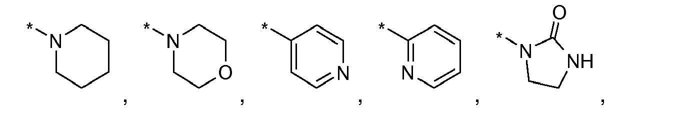 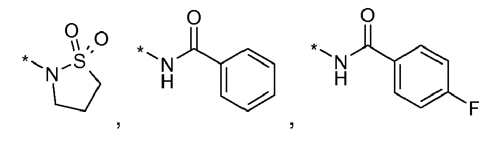 oder 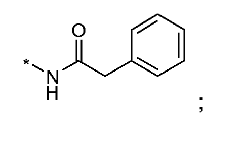
R^{3.2} ein Rest ausgewählt aus der Gruppe bestehend aus 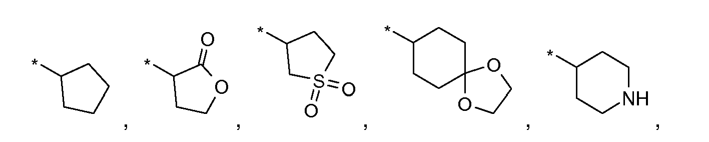 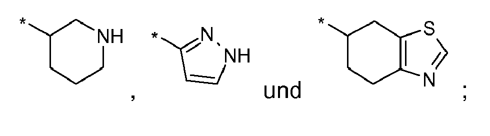 der gegebenenfalls substituiert ist mit einem oder mehreren Resten ausgewählt aus der Gruppe bestehend aus Methyl, Ethyl, Cyclopentyl, OH, NH₂; oder
Cyclohexyl, das gegebenenfalls substituiert ist mit einem oder zwei R^{3.2.1}
R^{3.2.1} -NR^{3.2.1.1}R^{3.2.1.2} oder ein Rest ausgewählt aus der Gruppe bestehend aus 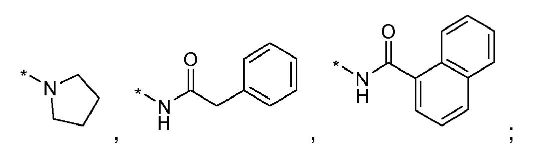 oder ein Rest ausgewählt aus der Gruppe bestehend aus 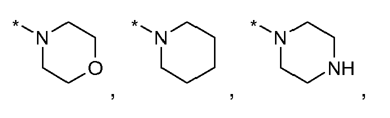 der gegebenenfalls substituiert ist mit einem oder mehreren Resten ausgewählt aus der Gruppe bestehend aus Methyl, SO₂R^{3.2.1.1}, 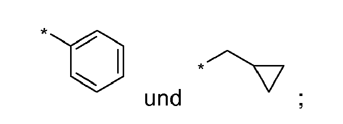
R^{3.2.1.1} H, Methyl oder Benzyl;
R^{3.2.1.2} H, Methyl oder Benzyl; oder
-C₁₋₆-alkyl, gerade oder verzweigt, das gegebenenfalls substituiert ist mit einem oder zwei R^{3.2.2}_{;}
R^{3.2.2} C=CH₂, C≡CH, COOR^{3.2.2.1}, CONR^{3.2.2.1}R^{3.2.2.2}, NR^{3.2.2.1}R^{3.2.2.2}, NHCOR^{3.2.2.1}, CF₃, CN, OH, SO₂R^{3.2.2.1} oder ein Rest ausgewählt aus der Gruppe bestehend aus 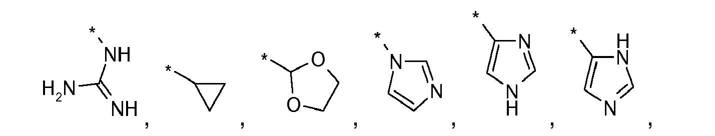 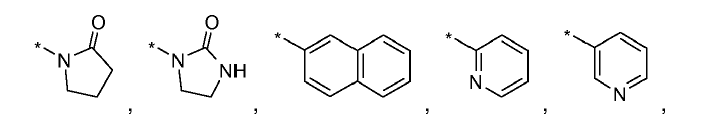 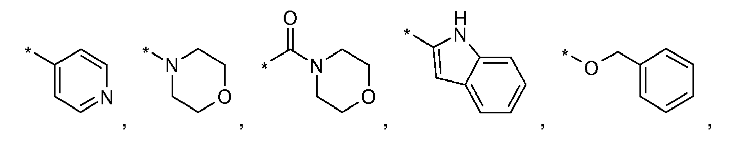 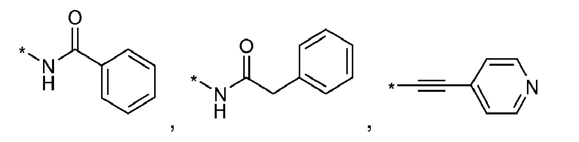 und 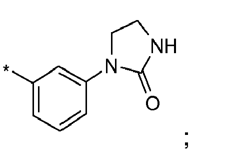 oder ein Rest ausgewählt aus der Gruppe bestehend aus 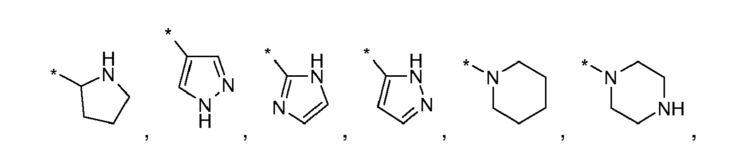 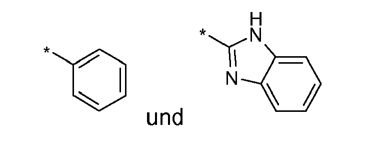 der gegebenenfalls substituiert ist mit einem oder mehreren Resten ausgewählt aus der Gruppe Cl, Methyl, CONR^{3.2.2.1}R^{3.2.2.2}, OH;
R^{3.2.2.1} H oder Methyl;
R^{3.2.2.2.} H oder Methyl; oder
Phenyl, das gegebenenfalls substituiert ist mit einem oder zwei R^{3.2.3}
R^{3.2.3} ein Rest ausgewählt aus der Gruppe bestehend aus 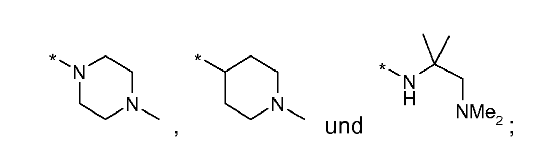
R^{3.3} H, C₁₋₆-Alkyl, gegebenenfalls substituiert mit einem oder mehreren R^{3.3.1},
R^{3.3.1} C₅₋₆-Cycloalkyl, C₅₋₆-Cycloalkenyl, OR^{3.3.1.1} NR^{3.3.1.1}R^{3.3.1.2}, CONR^{3.3.1.1}R^{3.3.1.2}, COOR^{3.3.1.1}, NR^{3.3.1.1}, COR^{3.3.1.2},SOR^{3.3.1.1}, SO₂R^{3.3.1.1}, C(NR^{3.3.1.1}R^{3.3.1.2})NR^{3.3.1.3}, NR^{3.3.1}CONR^{3.3.1.2}R^{3.3.1.3}, OH, CN, Halogen oder Het der gegebenenfalls substituiert ist mit einem oder mehreren Resten ausgewählt aus der Gruppe bestehend aus Methyl, SO₂H, SO₂CH₃, SO₂CH₂Phenyl, 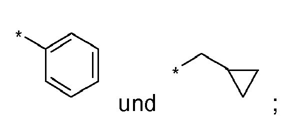
R^{3.3.1.1}, R^{3.3.1.2} und R^{3.3.1.3} ein Rest ausgewählt aus der Gruppe bestehend aus C₁₋₄-Alkyl, C₇₋₁₁-Aralkyl, C₁₋₄-Alkyl-Hetaryl, COC₁₋₄-Alkyl-Hetaryl;
**R^{a}** H, Methyl, Ethyl, Propyl, Butyl, 3-Methyl-butyl, Propenyl, Cyclopropyl, Cyclohexyl, CF₃, COR⁸, NR⁹R¹⁰, NO₂, OR⁸, SR¹¹, SOR¹¹, SO₂R¹¹ oder ein Rest ausgewählt aus der Gruppe bestehend aus 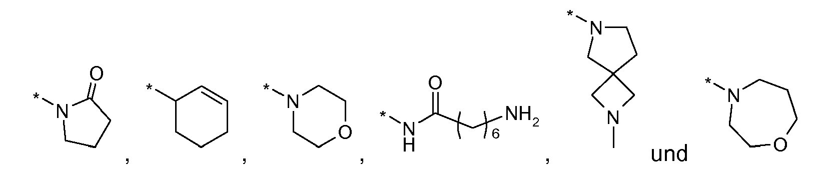 oder ein Rest ausgewählt aus der Gruppe bestehend aus 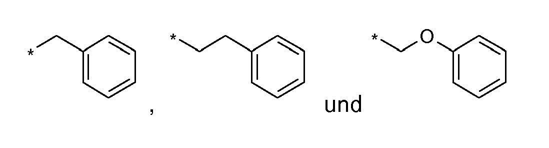 der gegebenenfalls substituiert sein kann mit einem oder mehreren Cl,
oder ein Rest ausgewählt aus der Gruppe bestehend aus 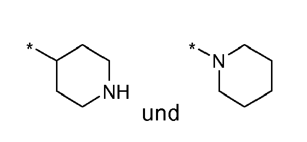 der gegebenenfalls substituiert sein kann mit einem oder mehreren CH₃, COCF₃, CH₂NH₂ oder CH₂NHCOOR¹²;
R⁸ Methyl, Propyl, Cyclopropyl, NH₂, Furanyl oder Phenyl, gegebenenfalls substituiert mit einem oder mehreren Chlor;
R⁹ H, COOR¹² oder Piperidino, gegebenenfalls substituiert mit einem oder mehreren CH₃, oder ein Rest ausgewählt aus der Gruppe bestehend aus Methyl, Ethyl und Propyl, der gegebenenfalls substituiert sein kann mit einem oder mehreren COOH, NMe₂ oder 4-Methylpiperazin;
R¹⁰ H, Methyl, COCH₃, C≡CH oder CH₂C≡CH;
R¹¹ Ethyl oder Propyl, gegebenenfalls substituiert mit einem oder mehreren NMe₂,
R¹² Butyl
**R^{b}** R⁴, CH₂O_{R}⁴, COR⁴, COOR⁴, CONR⁴R⁵, NH₂, NHCOOR⁴, NHCONR⁴R⁵ oder OH; R⁴ H, Methyl, Ethyl, 2-Hydroxyethyl, Propyl, C≡CH, CF₃, Phenyl, 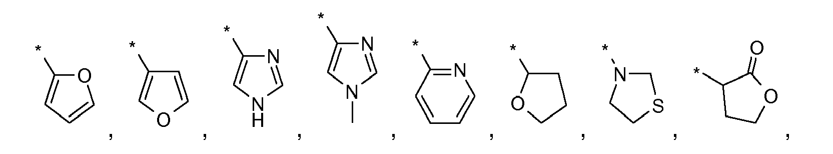 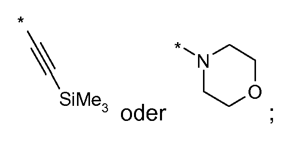
R⁵ H, Methyl oder Ethyl;
**R^{c}** NHR⁶ oder ein Rest ausgewählt aus der Gruppe bestehend aus 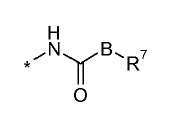 worin
B Bindung, Methylen, Ethylen, Propylen oder Butinylen;
R⁶ H, C₁₋₄-Alkyl, Aryl, gegebenenfalls substituiert mit SO₂CH₃;
R⁷ H, NR^{7.1}R^{7.2}, OR^{7.2}, SR^{7.2} oder ein Rest ausgewählt aus der Gruppe bestehend aus 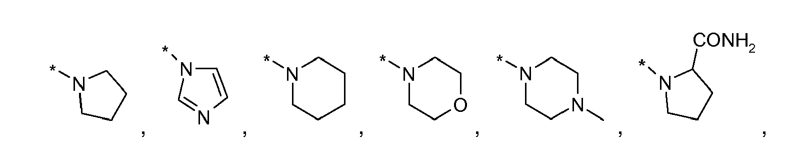 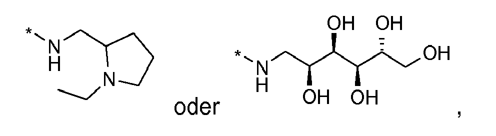
R^{7.1} H, Methyl, Ethyl, (CH₂)₂R^{7.1.1} oder COOButyl;
R^{7.2} H, Methyl oder Ethyl;
R^{7.1.1} NR^{7.1.1.1}R^{7.1.1.2} Het oder 1-Imidazolyl, 2-(N-Ethylpyrollidin);
R^{7.1.1.1} H oder C₁₋₆-Alkyl;
R^{7.1.1.2} H oder C₁₋₆-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}**= H und **R^{c}**= NHCONH-Et bedeutet.

6. Verbindungen der Formel **1** nach einem der Ansprüche 1 bis 5; worin
**R^{a}** Phenyl oder Benzyl, jeweils gegebenenfalls substituiert durch einen oder mehrere Reste ausgewählt aus der Gruppe bestehend aus R¹, R² und R³; oder 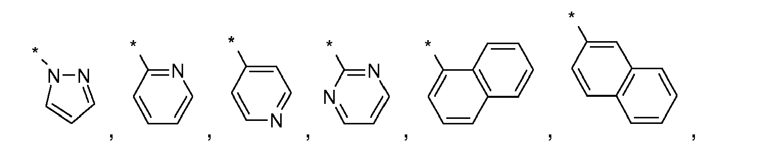 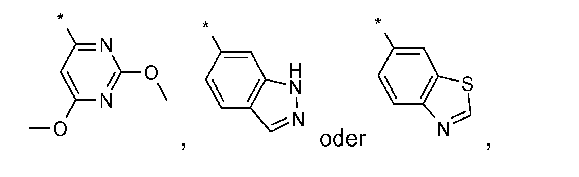
R¹ Methyl, Ethyl, Propyl, Butyl, CF₃, CH₂COOH, Methoxy, F, Cl, Br, OH, CN, COR^{1.1}, OCF₃, NO₂ oder SO₂R^{1.1};
R^{1.1} OH, Methyl, NH₂, NHMe, NMe₂, 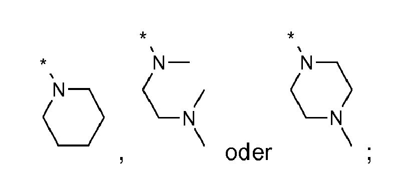
R² Methyl, Methoxy, F, Cl oder Br;
R³ ein Rest ausgewählt aus der Gruppe bestehend aus 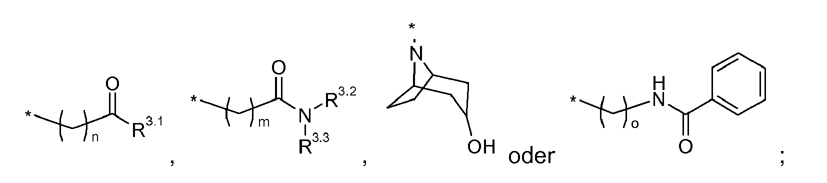 worin
n 0 oder 1;
m 0 oder 1;
o 2;
R^{3.1} ein Rest ausgewählt aus der Gruppe bestehend aus 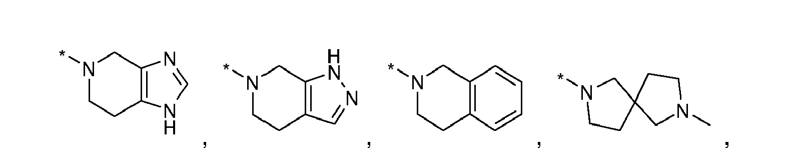 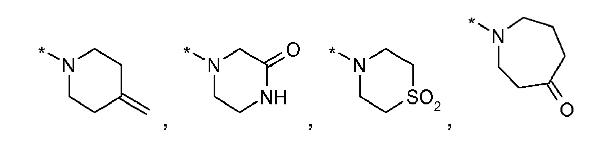 und 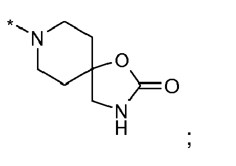 oder ein Rest ausgewählt aus der Gruppe bestehend aus 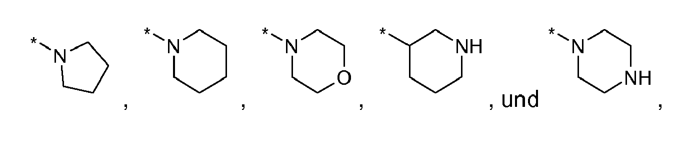 der gegebenenfalls substituiert sein kann mit einem oder mehreren R^{3.1.1};
R^{3.1.1} Methyl, Ethyl, OH, CH₂OH, CH₂CH₂OH, CH₂NEt₂, COMe, COOH, CONH₂, NH₂, 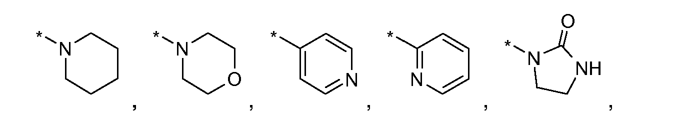 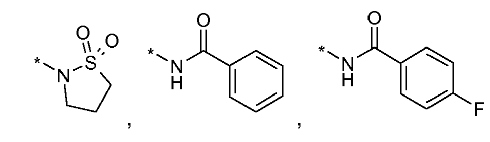 oder 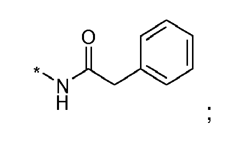
R^{3.2} ein Rest ausgewählt aus der Gruppe bestehend aus 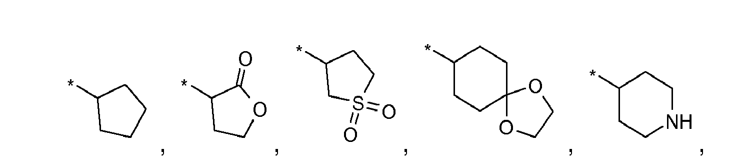 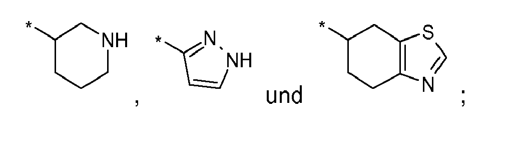 der gegebenenfalls substituiert ist mit einem oder mehreren Resten ausgewählt aus der Gruppe bestehend aus Methyl, Ethyl, Cyclopentyl, OH, NH₂; oder
Cyclohexyl, das gegebenenfalls substituiert ist mit einem oder zwei R^{3.2.1}
R^{3.2.1} -NR^{3.2.1.1}R^{3.2.1.2} oder ein Rest ausgewählt aus der Gruppe bestehend aus 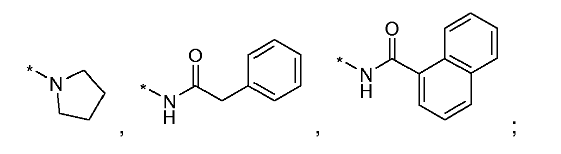 oder ein Rest ausgewählt aus der Gruppe bestehend aus 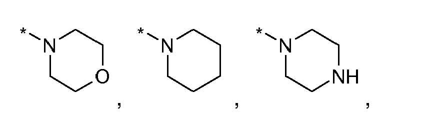 der gegebenenfalls substituiert ist mit einem oder mehreren Resten ausgewählt aus der Gruppe bestehend aus Methyl, SO₂R^{3.2.1.1}, 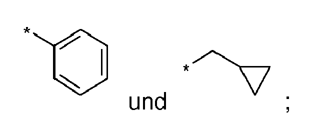
R^{3.2.1.1} H, Methyl oder Benzyl;
R^{3.2.1.2} H, Methyl oder Benzyl; oder
-C₁₋₆-alkyl, gerade oder verzweigt, das gegebenenfalls substituiert ist mit einem oder zwei R^{3.2.2}_{;}
R^{3.2.2} C=CH₂, C=CH, COOR^{3.2.2.1}, CONR^{3.2.2.1}R^{3.2.2.2}, NR^{3.2.2.1}R^{3.2.2.2}, NHCOR^{3.2.2.1}, CF₃, CN, OH, SO₂R^{3.2.2.1} oder ein Rest ausgewählt aus der Gruppe bestehend aus 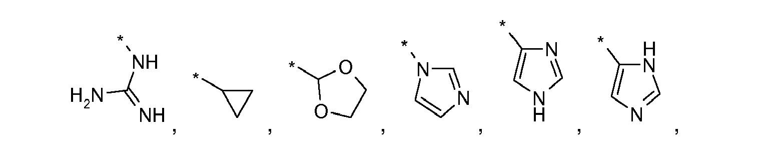 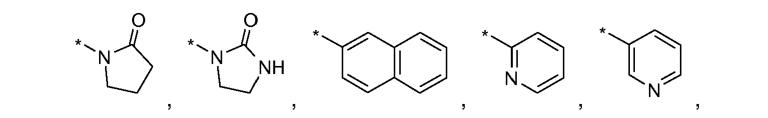 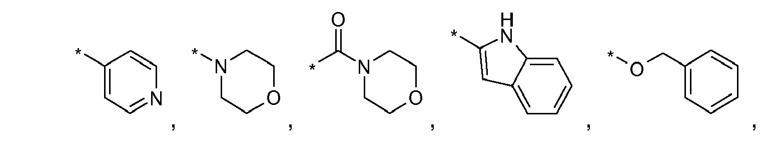 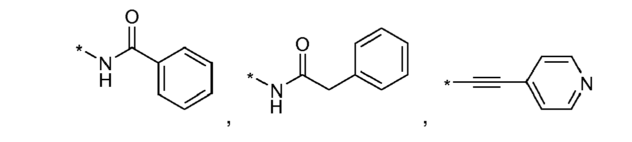 und 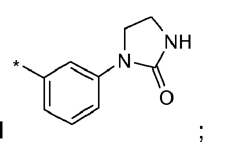 oder ein Rest ausgewählt aus der Gruppe bestehend aus 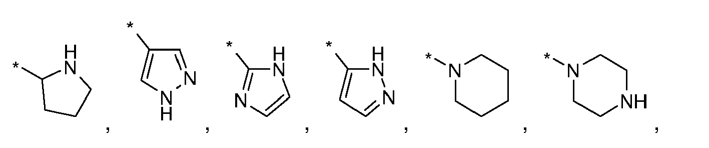 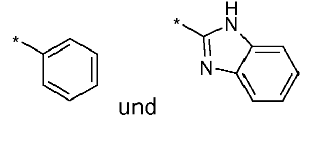 der gegebenenfalls substituiert ist mit einem oder mehreren Resten ausgewählt aus der Gruppe Cl, Methyl, CONR^{3.2.2.1}R^{3.2.2.2}, OH;
R^{3.2.2.1} H oder Methyl;
R^{3.2.2.2.} H oder Methyl; oder
Phenyl, das gegebenenfalls substituiert ist mit einem oder zwei R^{3.2.3}
R^{3.2.3} ein Rest ausgewählt aus der Gruppe bestehend aus 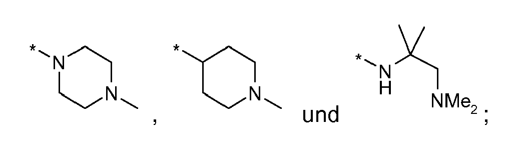
R^{3.3} H, Methyl oder Ethyl;
**R^{b}** R⁴, CH₂O_{R}⁴, COR⁴, COOR⁴, CONR⁴R⁵, NH₂, NHCOOR⁴, NHCONR⁴R⁵ oder OH;
R⁴ H, Methyl, Ethyl, 2-Hydroxyethyl, Propyl, C≡CH, CF₃, Phenyl, 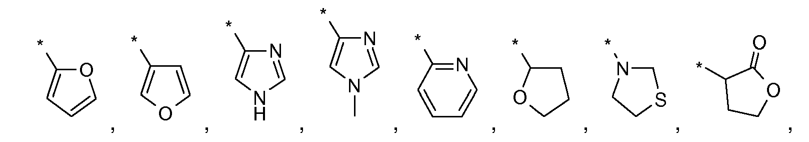 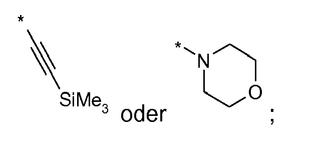
R⁵ H, Methyl oder Ethyl;
**R^{c}** NH₂ oder ein Rest ausgewählt aus der Gruppe bestehend aus 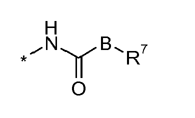 worin
B Bindung, Methylen, Ethylen, Propylen oder Butinylen;
R⁶ H, Phenyl, gegebenenfalls substituiert mit SO₂CH₃;
R⁷ H, NR^{7.1}R^{7.2}, OR^{7.2}, SR^{7.2} oder ein Rest ausgewählt aus der Gruppe bestehend aus 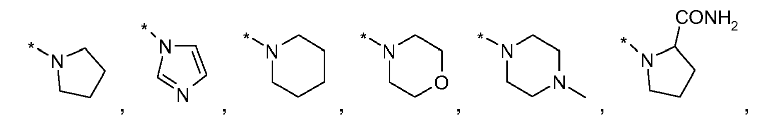 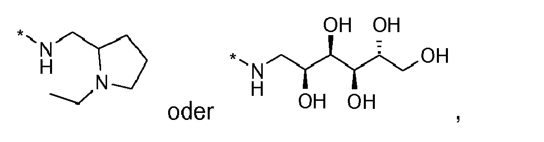
R^{7.1} H, Methyl, Ethyl, (CH₂)₂R^{7.1.1} oder COOButyl;
R^{7.2} H, Methyl oder Ethyl;
R^{7.1.1} NMe₂ oder 1-Imidazolyl
bedeuten, sowie pharmakologisch verträgliche Salz, Diastereomere, Enantiomere, Racemate, Hydrate oder Solvate davon.

7. Verbindungen der Formel 1 nach einem der Ansprüche 1 bis 6; worin
**R^{c}** ein Rest 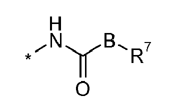 worin
B Methylen, Propylen;
R⁷ H, NR^{7.1}R^{7.2} oder 1-Imidazolyl;
R^{7.1} H oder Methyl;
R^{7.2} H oder Methyl;
bedeuten, sowie pharmakologisch verträgliche Salz, Diastereomere, Enantiomere, Racemate, Hydrate oder Solvate davon.

8. Verbindungen der Formel **1** nach einem der Ansprüche 1 bis 7; worin
**R^{a}** Phenyl, gegebenenfalls substituiert durch einen oder mehrere Reste ausgewählt aus der Gruppe bestehend aus R¹, R² und R³;
bedeuten, sowie pharmakologisch verträgliche Salz, Diastereomere, Enantiomere, Racemate, Hydrate oder Solvate davon.

9. Verbindungen der Formel **1** nach einem der Ansprüche 1 bis 8; worin
**R^{a}** Phenyl, gegebenenfalls substituiert durch einen oder mehrere Reste ausgewählt aus der Gruppe bestehend aus R¹ und R³;
R¹ Methyl, Ethyl, Propyl, CF₃, Methoxy, F, Cl oder Br;
R³ ein Rest 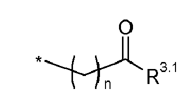
bedeuten, sowie pharmakologisch verträgliche Salz, Diastereomere, Enantiomere, Racemate, Hydrate oder Solvate davon.

10. Verbindungen der Formel **1** nach einem der Ansprüche 1 bis 9; worin
**R^{a}** Phenyl, gegebenenfalls substituiert durch einen oder mehrere Reste ausgewählt aus der Gruppe bestehend aus R¹ und R³;
R¹ Methyl, Ethyl, Propyl, CF₃, Methoxy, F, Cl oder Br;
R³ ein Rest 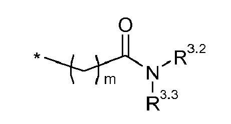
bedeuten, sowie pharmakologisch verträgliche Salz, Diastereomere, Enantiomere, Racemate, Hydrate oder Solvate davon.

11. Verbindungen der Formel **1.1** nach einem der Ansprüche 1 bis 10; 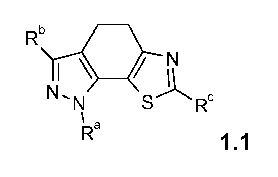 worin **R^{a}**, **R^{b}** und **R^{c}** die oben genannte Bedeutung haben, sowie pharmakologisch verträgliche Salz, Diastereomere, Enantiomere, Racemate, Hydrate oder Solvate davon.

12. Verbindungen der Formel **1.1** nach einem der Ansprüche 1 bis 11;
**R^{a}** Phenyl, gegebenenfalls substituiert durch einen oder mehrere Reste ausgewählt aus der Gruppe bestehend aus R¹, R² und R³; oder 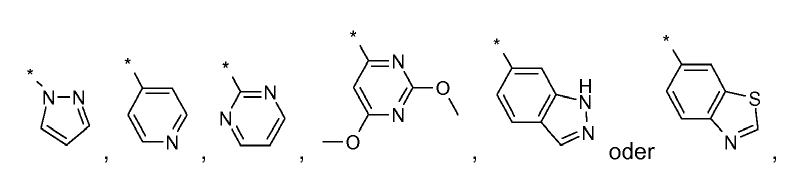
R¹ Methyl, Ethyl, Propyl, CF₃, CH₂COOH, Methoxy, F, Cl, Br, CN, COR^{1.1} oder SO₂R^{1.1}_{;}
R^{1.1} OH, Methyl, NH₂, NHMe, NMe₂ oder 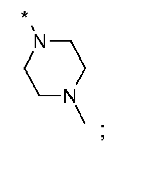
R² Methyl, F, Cl oder Br;
R³ ein Rest ausgewählt aus der Gruppe bestehend aus 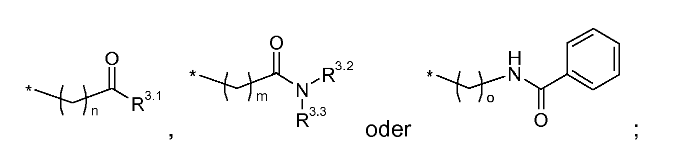 worin
n 0 oder 1;
m 0 oder 1;
o 2;
R^{3.1} ein Rest ausgewählt aus der Gruppe bestehend aus 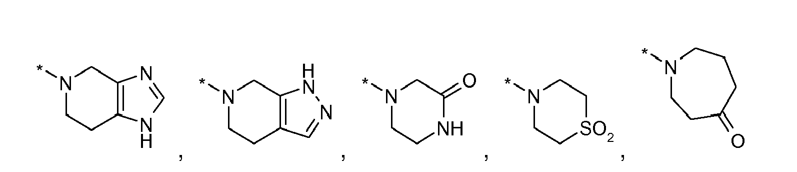 und 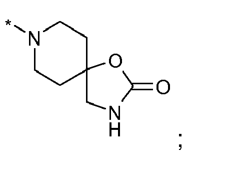 oder ein Rest ausgewählt aus der Gruppe bestehend aus 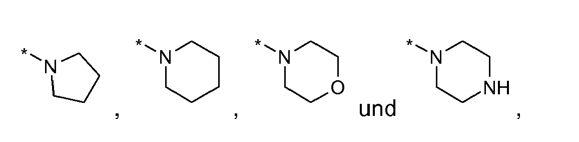 der gegebenenfalls substituiert sein kann mit einem oder mehreren R^{3.1.1};
R^{3.1.1} Methyl, OH, CH₂OH, CH₂CH₂OH, CH₂NEt₂, COMe, COOH, CONH₂, 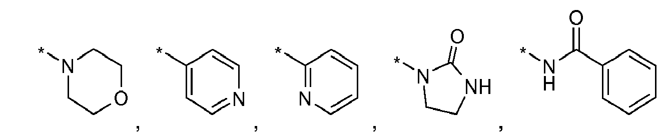 oder 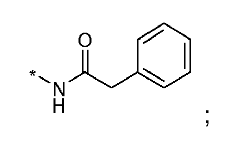
R^{3.2} ein Rest ausgewählt aus der Gruppe bestehend aus 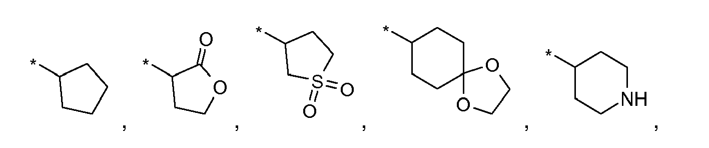 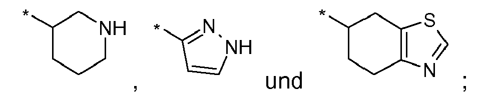 der gegebenenfalls substituiert ist mit einem oder mehreren Resten ausgewählt aus der Gruppe bestehend aus Methyl, Ethyl, Cyclopentyl, OH, NH_{2;} oder
Cyclohexyl, das gegebenenfalls substituiert ist mit einem oder zwei R^{3.2.1}
R^{3.2.1} -NR^{3.2.1.1}R^{3.2.1.2} oder ein Rest ausgewählt aus der Gruppe bestehend aus 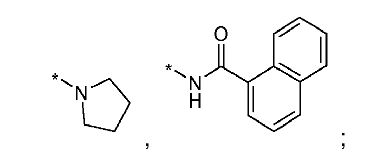 oder ein Rest ausgewählt aus der Gruppe bestehend aus 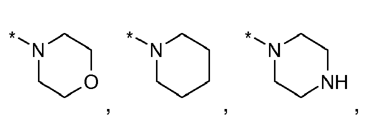 der gegebenenfalls substituiert ist mit einer oder mehreren Methylgruppen
R^{3.2.1.1} H oder Methyl;
R^{3.2.1.2} H oder Methyl; oder
-C₁₋₆-alkyl, gerade oder verzweigt, das gegebenenfalls substituiert ist mit einem oder zwei R^{3.2.2};
R^{3.2.2} C=CH₂, C-CH, COOR^{3.2.2.1}, CONR^{3.2.2.1}R^{3.2.2.2}, NR^{3.2.2.1}R^{3.2.2.2}, CN, OH oder ein Rest ausgewählt aus der Gruppe bestehend aus 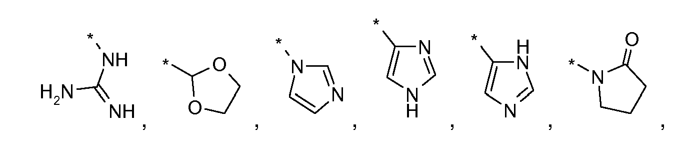 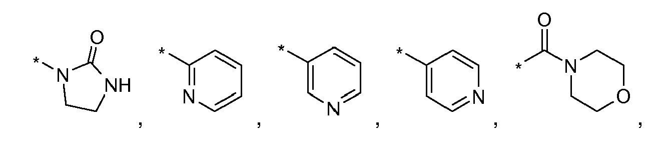 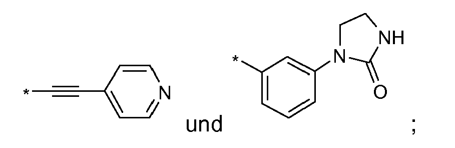 oder ein Rest ausgewählt aus der Gruppe bestehend aus 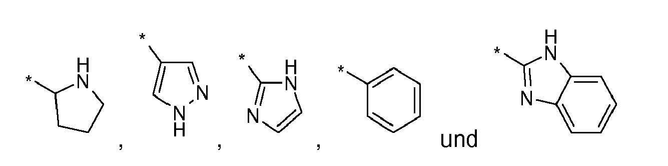 der gegebenenfalls substituiert ist mit einem oder mehreren Resten ausgewählt aus der Gruppe Methyl, CONR^{3.2.2.1}R^{3.2.2.2}, OH;
R^{3.2.2.1} H oder Methyl;
R^{3.2.2.2.} H oder Methyl; oder
Phenyl, das gegebenenfalls substituiert ist mit einem oder zwei R^{3.2.3}
R^{3.2.3} ein Rest ausgewählt aus der Gruppe bestehend aus 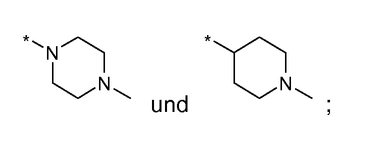
R^{3.3} H, Methyl oder Ethyl;
**R^{b}** R⁴, CH₂O_{C}H₃, COR⁴, COOH, COOCH₃ CONR⁴R⁵, NH₂, NHCOOR⁴ oder OH;
R⁴ H, Methyl, Propyl, C≡CH, Phenyl, 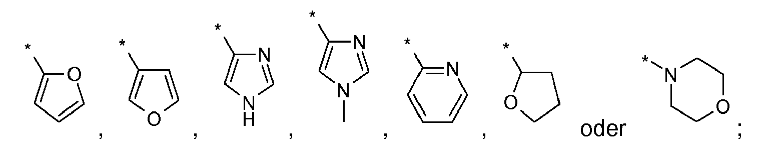
R⁵ H oder Methyl;
**R^{c}** ein Rest 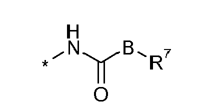 worin
B Methylen, Propylen;
R⁷ H, NR^{7.1}R^{7.2} oder 1-Imidazolyl;
R^{7.1} H oder Methyl;
R^{7.2} H oder Methyl;
bedeuten, sowie pharmakologisch verträgliche Salz, Diastereomere, Enantiomere, Racemate, Hydrate oder Solvate davon.

13. Verbindungen der Formel **1.2** nach einem der Ansprüche 1 bis 10; 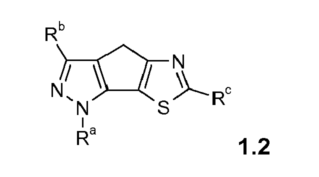 worin **R^{a}, R^{b}** und **R^{c}** die oben genannte Bedeutung haben, sowie pharmakologisch verträgliche Salz, Diastereomere, Enantiomere, Racemate, Hydrate oder Solvate davon.

14. Verbindungen der Formel **1.2** nach einem der Ansprüche 1 bis 10 und 13;
**R^{a}** Phenyl, gegebenenfalls substituiert durch einen oder mehrere Reste ausgewählt aus der Gruppe bestehend aus R¹ und R²;
R¹ Methyl, Ethyl, Propyl, CF₃, F, Cl, COR^{1.1} oder SO₂R^{1.1};
R^{1.1} Methyl;
R² Methyl, F oder Cl;
**R^{b}** R⁴;
R⁴ H;
**R^{c}** NHR⁶ oder ein Rest 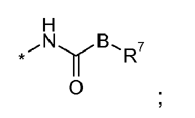 worin
B Bindung, Methylen, Ethylen oder Propylen;
R⁶ H;
R⁷ H oder NR^{7.1}R^{7.2}, 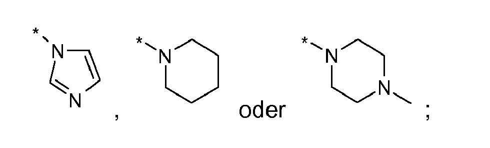
R^{7.1} H, Methyl, Ethyl, (CH₂)₂R^{7.1.1} oder COOButyl;
R^{7.2} H, Methyl oder Ethyl;
R^{7.1.1} NMe₂ oder 1-Imidazolyl
bedeuten, sowie pharmakologisch verträgliche Salz, Diastereomere, Enantiomere, Racemate, Hydrate oder Solvate davon.

15. Verbindungen der Formel 1.3 nach einem der Ansprüche 1 bis 10; 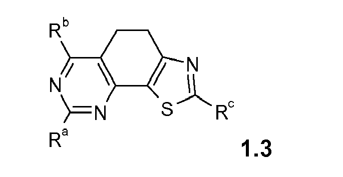 worin **R^{a}, R^{b}** und **R^{c}** die oben genannte Bedeutung haben, sowie pharmakologisch verträgliche Salz, Diastereomere, Enantiomere, Racemate, Hydrate oder Solvate davon.

16. Verbindungen der Formel **1.3** nach einem der Ansprüche 1 bis 10 und 15; worin
**R^{a}** Phenyl, gegebenenfalls substituiert durch einen oder mehrere Reste ausgewählt aus der Gruppe bestehend aus R¹ und R²; oder 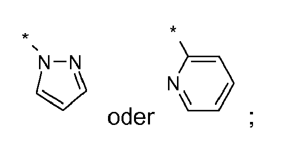
R¹ Methyl, Methoxy, Cl, OH oder COR^{1.1};
R^{1.1} NH₂, NHMe oder NMe₂;
R² Methoxy oder Cl;
**R^{b}** R⁴ oder OH;
R⁴ H;
**R^{c}** NHR⁶ oder ein Rest 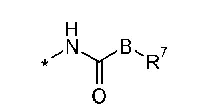 worin
B Bindung, Methylen, Ethylen oder Propylen;
R⁶ H;
R⁷ H, NR^{7.1}R^{7.2} oder ein Rest ausgewählt aus der Gruppe bestehend aus 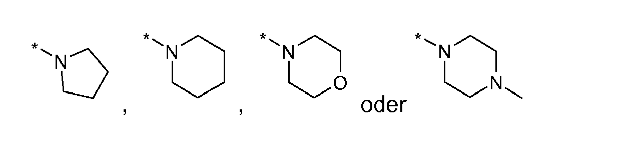
R^{7.1} H, Methyl oder (CH₂)₂R^{7.1.1};
R^{7.2} H, Methyl oder Ethyl;
R^{7.1.1} NMe₂;
bedeuten, sowie pharmakologisch verträgliche Salz, Diastereomere, Enantiomere, Racemate, Hydrate oder Solvate davon.

17. Verbindungen der Formel **1.4** nach einem der Ansprüche 1 bis 10; worin 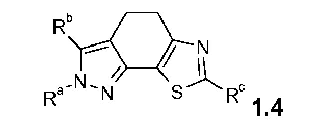 worin **R^{a}, R^{b} und R^{c}** die oben genannte Bedeutung haben, sowie pharmakologisch verträgliche Salz, Diastereomere, Enantiomere, Racemate, Hydrate oder Solvate davon.

18. Verbindungen der Formel **1.4** nach einem der Ansprüche 1 bis 10, 17; worin
**R^{a}** Phenyl, gegebenenfalls substituiert durch einen oder mehrere Reste ausgewählt aus der Gruppe bestehend aus R¹ und R³; oder
R¹ Methyl, F, Cl oder Br;
R³ ein Rest ausgewählt aus der Gruppe bestehend aus 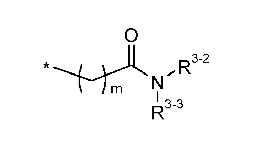 worin
m 0;
R^{3.2} ein Rest 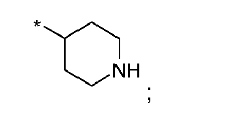 der gegebenenfalls substituiert ist mit einem Rest ausgewählt aus der Gruppe bestehend aus Methyl und Cyclopentyl;
Cyclohexyl, das gegebenenfalls substituiert ist mit einem R^{3.2.1}
R^{3.2.1} ein Rest 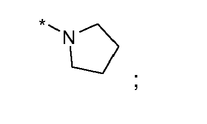
R^{3.3} H;
**R^{b}** R⁴.
R⁴ ein Rest ausgewählt aus der Gruppe bestehend aus 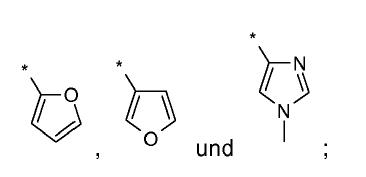
**R^{c}** ein Rest 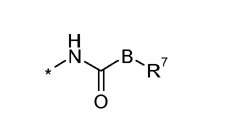 worin
B Methylen;
R⁷ H;
bedeuten, sowie pharmakologisch verträgliche Salz, Diastereomere, Enantiomere, Racemate, Hydrate oder Solvate davon.

19. Verbindungen der Formel **1** nach einem der Ansprüche 1 bis 5; worin
**R^{a}** H, C₁₋₆-Alklyl, C₂₋₆-Alkenyl, C₃₋₆-Cycloalkyl, C₃₋₆-Cycloalkenyl, CF₃, COR⁸, NR⁹R¹⁰, NO₂, S(O)ₙR¹¹, Spiro, NHCO-C₁₋₆-Alkyl-NH₂ oder ein Rest ausgewählt aus der Gruppe bestehend aus C₇₋₁₁-Aralkyl und CH₂O-Aryl, der gegebenenfalls substituiert sein kann mit einem oder mehreren Cl; oder ein Rest ausgewählt aus der Gruppe bestehend aus Het, der gegebenenfalls substituiert sein kann mit einem oder mehreren C₁₋₄-Alkyl, COCF₃, CH₂NH₂ oder CH₂NHCOOR¹²;
R⁸ C₁₋₄-Alkyl, C₃₋₆-Cycloalkyl, NH₂, Hetaryl, Aryl, gegebenenfalls substituiert mit einem oder mehreren Chlor;
R⁹ H, COOR¹² oder Het, gegebenenfalls substituiert mit einem oder mehreren C₁₋₄-Alkyl, oder ein Rest ausgewählt aus der Gruppe bestehend aus C₁₋₄-Alkyl, der gegebenenfalls substituiert sein kann mit einem oder mehreren COOH, N(C₁₋₄-Alkyl)₂ oder 4-Methylpiperazin;
R¹⁰ H, C₁₋₄-Alkyl, C₂₋₄-Alkinyl oder COCH₃;
R¹¹ C₁₋₄-Alkyl, gegebenenfalls substituiert mit einem oder mehreren N(C₁₋₄-Alkyl)₂,
R¹² C₁₋₄-Alkyl;
n 0 oder 2;
**R^{b}** H, OH oder COOEt;
**R^{c}** NH₂ oder NHCOR¹³;
R¹³ C₁₋₄-Alkyl, oder NR^{13.1}R^{13.2},
R^{13.1} H oder C₁₋₄-Alkyl;
R^{13.2} H oder C₁₋₄-Alkyl;
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; **j** = 2; **k** = 0; **R^{b}=** H und **R^{c}=** NHCONH-Et bedeutet.

20. Verbindungen der Formel **1** nach einem der Ansprüche 1 bis 5 und 19; worin
**R^{a}** H, Methyl, Ethyl, Propyl, Butyl, 3-Methyl-butyl, Propenyl, Cyclopropyl, Cyclohexyl, CF₃, COR⁸, NR⁹R¹⁰, NO₂, S(O)ₙR¹¹, oder ein Rest ausgewählt aus der Gruppe bestehend aus 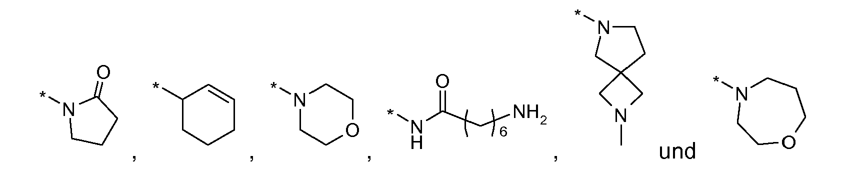 oder ein Rest ausgewählt aus der Gruppe bestehend aus 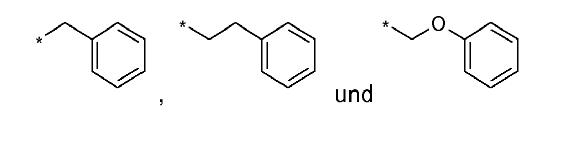 der gegebenenfalls substituiert sein kann mit einem oder mehreren Cl;
oder ein Rest ausgewählt aus der Gruppe bestehend aus 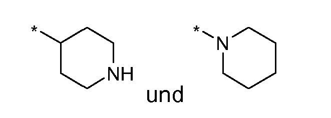 der gegebenenfalls substituiert sein kann mit einem oder mehreren CH₃, COCF₃, CH₂NH₂ oder CH₂NHCOOR¹²;
R⁸ Methyl, Propyl, Cyclopropyl, NH₂, Furanyl oder Phenyl, gegebenenfalls substituiert mit einem oder mehreren Chlor;
R⁹ H, COOR¹² oder Piperidino, gegebenenfalls substituiert mit einem oder mehreren CH₃, oder ein Rest ausgewählt aus der Gruppe bestehend aus Methyl, Ethyl und Propyl, der gegebenenfalls substituiert sein kann mit einem oder mehreren COOH, NMe₂ oder 4-Methylpiperazin;
R¹⁰ H, Methyl, COCH₃, C≡CH oder CH₂C≡CH;
R¹¹ Ethyl oder Propyl, gegebenenfalls substituiert mit einem oder mehreren NMe₂,
R¹² Butyl
**R^{b}** H, OH oder COOEt;
**R^{c}** NH₂ oder NHCOR¹³;
R¹³ Methyl oder NR^{13.1}R^{13.2},
R^{13.1} H oder Methyl;
R^{13.2} H oder Methyl.
bedeuten, sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** = Stickstoff; **Z** = Stickstoff; j = 2; **k** = 0; **R^{b}=** H und **R^{c}=** NHCONH-Et bedeutet.

21. Verbindungen der Formel **1** nach einem der Ansprüche 1 bis 20 als Arzneimittel.

22. Verbindungen nach einem der Ansprüche 1 bis 19; sowie pharmakologisch verträgliche Salz, Diastereomere, Enantiomere, Racemate, Hydrate oder Solvate davon, mit der Maßgabe, dass **R^{a}** nicht H oder Me sein kann, wenn **Y** und **Z** = Stickstoff; j = 2; k = 0; **R^{b}=** H und **R^{c}=** NHCONH-Et bedeutet und dass **R^{c}** nicht NH₂ sein kann, wenn **Y** und **Z** = Stickstoff; **j** = 2; **k** = 1; **R^{b}=** H und **R^{a}=** 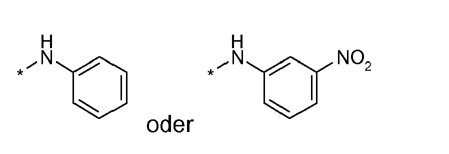 bedeutet.
